# EUROPEAN PATENT SPECIFICATION

(11) **EP 3 953 360 B1**
(45) Date of publication and mention of the grant of the patent: **28.02.2024**
(21) Application number: 20719132.1
(22) Date of filing: 06.04.2020
(51) Int. Cl.: C07D 498/04, C07D 519/00, A61P 25/00, A61P 25/08, A61P 25/16, A61P 25/28, A61P 29/00, A61P 35/00, A61K 9/00, A61K 31/496, A61K 31/4995

(54) **HETEROCYCLIC COMPOUNDS AS INHIBITORS OF MONOACYLGLYCEROL LIPASE (MAGL)**
HETEROCYCLISCHE VERBINDUNGEN ALS INHIBITOREN DER MONOACYLGLYCEROL-LIPASE (MAGL)
COMPOSÉS HÉTÉROCYCLIQUES EN TANT QU' INHIBITEURS DE LA LIPASE MONOACYLGLYCEROL (MAGL)

(30) Priority: 09.04.2019 EP 19168245
(43) Date of publication of application: 16.02.2022
(73) Proprietor: F. Hoffmann-La Roche AG, 4070 Basel (CH)
(72) Inventor: BENZ, Joerg, 4070 Basel (CH); DÉCORET, Guillaume, 4070 Basel (CH); GRETHER, Uwe, 4070 Basel (CH); GROEBKE ZBINDEN, Katrin, 4070 Basel (CH); HORNSPERGER, Benoit, 4070 Basel (CH); KUHN, Bernd, 4070 Basel (CH); RICHTER, Hans, 4070 Basel (CH); ROMBACH, Didier, 40470 Basel (CH); O'HARA, Fionn, 4070 Basel (CH); KROLL, Carsten, 4070 Basel (CH)
(74) Representative: Neuhaus, Christian Michel
(86) International application number: PCT/EP2020/059709
(87) International publication number: WO 2020/207941

(56) References cited:
- EP-A2- 3 312 177
- WO-A1-2006/051410
- WO-A2-2015/179559
- WO-A2-2016/014975
- WO-A2-2020/198526
- GRANCHI, C. ET AL.: "A patent review of Monoacylglycerol Lipase (MAGL) inhibitors (2013-2017)", EXPERT OPINION ON THERAPEUTIC PATENTS, vol. 27, no. 12, 2017, pages 1341-1351, XP055675734, ISSN: 1354-3776, DOI: 10.1080/13543776.2018.1389899

## Description

### Field of the Invention

The present invention relates to organic compounds useful for therapy or prophylaxis in a mammal, and in particular to monoacylglycerol lipase (MAGL) inhibitors for the treatment or prophylaxis of neuroinflammation, neurodegenerative diseases, pain, cancer, mental disorders, multiple sclerosis, Alzheimer's disease, Parkinson's disease, amyotrophic lateral sclerosis, traumatic brain injury, neurotoxicity, stroke, epilepsy, anxiety, migraine and/or depression in a mammal.

### Background of the Invention

Endocannabinoids (ECs) are signaling lipids that exert their biological actions by interacting with cannabinoid receptors (CBRs), CB1 and CB2. They modulate multiple physiological processes including neuroinflammation, neurodegeneration and tissue regeneration (Iannotti, F.A., et al., Progress in lipid research 2016, 62, 107-28.). In the brain, the main endocannabinoid, 2-arachidonoylglycerol (2-AG), is produced by diacyglycerol lipases (DAGL) and hydrolyzed by the monoacylglycerol lipase, MAGL. MAGL hydrolyses 85% of 2-AG; the remaining 15% being hydrolysed by ABHD6 and ABDH12 (Nomura, D.K., et al., Science 2011, 334, 809.). MAGL is expressed throughout the brain and in most brain cell types, including neurons, astrocytes, oligodendrocytes and microglia cells (Chanda, P.K., et al., Molecular pharmacology 2010, 78, 996; Viader, A., et al., Cell reports 2015, 12, 798.). 2-AG hydrolysis results in the formation of arachidonic acid (AA), the precursor of prostaglandins (PGs) and leukotrienes (LTs). Oxidative metabolism of AA is increased in inflamed tissues. There are two principal enzyme pathways of arachidonic acid oxygenation involved in inflammatory processes, the cyclooxygenase which produces PGs and the 5-lipoxygenase which produces LTs. Of the various cyclooxygenase products formed during inflammation, PGE2 is one of the most important. These products have been detected at sites of inflammation, e.g. in the cerebrospinal fluid of patients suffering from neurodegenerative disorders and are believed to contribute to inflammatory response and disease progression. Mice lacking MAGL (Mgll-/-) exhibit dramatically reduced 2-AG hydrolase activity and elevated 2-AG levels in the nervous system while other arachidonoyl-containing phospho- and neutral lipid species including anandamide (AEA), as well as other free fatty acids, are unaltered. Conversely, levels of AA and AA-derived prostaglandins and other eicosanoids, including prostaglandin E2 (PGE2), D2 (PGD2), F2 (PGF2), and thromboxane B2 (TXB2), are strongly decreased. Phospholipase A₂ (PLA₂) enzymes have been viewed as the principal source of AA, but cPLA₂-deficient mice have unaltered AA levels in their brain, reinforcing the key role of MAGL in the brain for AA production and regulation of the brain inflammatory process.

Neuroinflammation is a common pathological change characteristic of diseases of the brain including, but not restricted to, neurodegenerative diseases (e.g. multiple sclerosis, Alzheimer's disease, Parkinson disease, amyotrophic lateral sclerosis, traumatic brain injury, neurotoxicity, stroke, epilepsy and mental disorders such as anxiety and migraine). In the brain, production of eicosanoids and prostaglandins controls the neuroinflammation process. The pro-inflammatory agent lipopolysaccharide (LPS) produces a robust, timedependent increase in brain eicosanoids that is markedly blunted in Mgll-/- mice. LPS treatment also induces a widespread elevation in pro-inflammatory cytokines including interleukin-1-a (IL-1-a), IL-1b, IL-6, and tumor necrosis factor-a (TNF-a) that is prevented in Mgll-/- mice.

Neuroinflammation is characterized by the activation of the innate immune cells of the central nervous system, the microglia and the astrocytes. It has been reported that antiinflammatory drugs can suppress in preclinical models the activation of glia cells and the progression of disease including Alzheimer's disease and mutiple sclerosis (Lleo A., Cell Mol Life Sci. 2007, 64, 1403.). Importantly, genetic and/or pharmacological disruption of MAGL activity also blocks LPS-induced activation of microglial cells in the brain (Nomura, D.K., et al., Science 2011, 334, 809.).

In addition, genetic and/or pharmacological disruption of MAGL activity was shown to be protective in several animal models of neurodegeneration including, but not restricted to, Alzheimer's disease, Parkinson's disease and multiple sclerosis. For example, an irreversible MAGL inhibitor has been widely used in preclinical models of neuroinflammation and neurodegeneration (Long, J.Z., et al., Nature chemical biology 2009, 5, 37.). Systemic injection of such inhibitor recapitulates the Mgll-/- mice phenotype in the brain, including an increase in 2-AG levels, a reduction in AA levels and related eicosanoids production, as well as the prevention of cytokines production and microglia activation following LPS-induced neuroinflammation (Nomura, D.K., et al., Science 2011, 334, 809.), altogether confirming that MAGL is a druggable target.

Consecutive to the genetic and/or pharmacological disruption of MAGL activity, the endogenous levels of the MAGL natural substrate in the brain, 2-AG, are increased. 2-AG has been reported to show beneficial effects on pain with, for example, anti-nociceptive effects in mice (Ignatowska-Jankowska B. et al., J. Pharmacol. Exp. Ther. 2015, 353, 424.) and on mental disorders, such as depression in chronic stress models (Zhong P. et al., Neuropsychopharmacology 2014, 39, 1763.).

Furthermore, oligodendrocytes (OLs), the myelinating cells of the central nervous system, and their precursors (OPCs) express the cannabinoid receptor 2 (CB2) on their membrane. 2-AG is the endogenous ligand of CB1 and CB2 receptors. It has been reported that both cannabinoids and pharmacological inhibition of MAGL attenuate OLs's and OPCs's vulnerability to excitotoxic insults and therefore may be neuroprotective (Bernal-Chico, A., et al., Glia 2015, 63, 163.). Additionally, pharmacological inhibition of MAGL increases the number of myelinating OLs in the brain of mice, suggesting that MAGL inhibition may promote differentiation of OPCs in myelinating OLs in vivo (Alpar, A., et al., Nature communications 2014, 5, 4421.). Inhibition of MAGL was also shown to promote remyelination and functional recovery in a mouse model of progressive multiple sclerosis (Feliu A. et al., Journal of Neuroscience 2017, 37 (35), 8385.).

Finally, in recent years, metabolism is talked highly important in cancer research, especially the lipid metabolism. Researchers believe that the de novo fatty acid synthesis plays an important role in tumor development. Many studies illustrated that endocannabinoids have anti-tumorigenic actions, including anti-proliferation, apoptosis induction and anti-metastatic effects. MAGL as an important decomposing enzyme for both lipid metabolism and the endocannabinoids system, additionally as a part of a gene expression signature, contributes to different aspects of tumourigenesis (Qin, H., et al., Cell Biochem. Biophys. 2014, 70, 33; Nomura DK et al., Cell 2009, 140(1), 49-61; Nomura DK et al., Chem. Biol. 2011, 18(7), 846-856).

In conclusion, suppressing the action and/or the activation of MAGL is a promising new therapeutic strategy for the treatment or prevention of neuroinflammation, neurodegenerative diseases, pain, cancer and mental disorders. Furthermore, suppressing the action and/or the activation of MAGL is a promising new therapeutic strategy for providing neuroprotection and myelin regeneration. Accordingly, there is a high unmet medical need for new MAGL inhibitors.

Granchi et al., Expert Opinion on Therapeutic Patents 2017, 27, 1341 provides a patent review of MAGL inhibitors for the years 2013-2017.

WO2016/014975 discloses compounds that may be used to inhibit the action of fatty acid amide hydrolase (FAAH), monoacylglycerol lipase (MAGL) or dual FAAH/MAGL.

WO2015/179559 discloses pyrazole compounds that are useful as modulators of one or more of MAGL, ABHD6, and FAAH.

WO2006/051410 discloses a7 nicotinic receptor agonists.

EP3312177 discloses tricyclic derivative compounds that are active against PARP-1, tankyrase-1 or tankyrase-2.

### Summary of the Invention

In a first aspect, the present invention provides compounds of Formula (I), or pharmaceutically acceptable salts thereof, 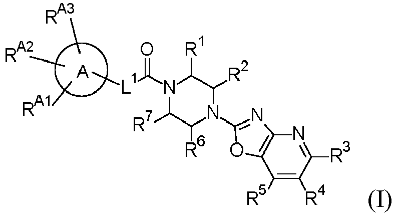 wherein R¹ to R⁷, A, L¹, and R^{A1} to R^{A3} are as defined herein.

In a further aspect, the present invention provides a process of manufacturing the compounds of formula (I) as described herein, comprising:
reacting an acid of formula **1**, wherein L¹, A, and R^{A1} to R^{A3} are as defined herein, 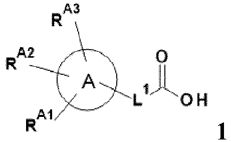
with an amine of formula **2**, wherein R¹ to R⁷ are as defined herein 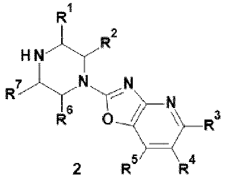
in the presence of a base and a coupling reagent,
to form said compound of formula (I).

In a further aspect, the present invention provides a compound of formula (I) as described herein, for use as therapeutically active substance.

In a further aspect, the present invention provides a pharmaceutical composition comprising a compound of formula (I) as described herein and a therapeutically inert carrier.

In a further aspect, the present invention provides a compound of formula (I) as described herein for use in a method of inhibiting monoacylglycerol lipase in a mammal.

In a further aspect, the present invention provides a compound of formula (I) as described herein for use in the treatment or prophylaxis of neuroinflammation, neurodegenerative diseases, pain, cancer and/or mental disorders in a mammal.

In a further aspect, the present invention provides a compound of formula (I) as described herein, for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

### Detailed Description of the Invention

### Definitions

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The term "C₁-C₆-alkyl" refers to a mono- or multivalent, e.g., a mono- or bivalent, linear or branched saturated hydrocarbon group of 1 to 6 carbon atoms, e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl.

The term "alkoxy" refers to an alkyl group, as previously defined, attached to the parent molecular moiety via an oxygen atom. In some preferred embodiments, the alkoxy group contains 1 to 6 carbon atoms, e.g. 1, 2, 3, 4, 5, or 6 carbon atoms. In other embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy, 1,1-dimethylpropoxy, 2,2-dimethylpropoxy, and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "C₃₋₁₂-cycloalkyl" as used herein refers to a saturated or partly unsaturated monocyclic or bicyclic hydrocarbon group of 3 to 12 ring carbon atoms. In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. In one embodiment, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, and 1-bicyclo [1.1.1]pentanyl.

The term "heterocyclyl" as used herein refers to a saturated or partly unsaturated mono- or bicyclic, preferably monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of monocyclic heterocyclyl groups include azetidin-3-yl, azetidin-2-yl, 2-azaspiro[3.3]heptan-2-yl, 2,6-diazaspiro[3.3]heptan-2-yl, 2-azaspiro[3.4]octan-2-yl, 5-oxa-2-azaspiro[3.4]octan-2-yl, pyrrolidinyl (e.g. pyrrolidin-1-yl), thiomorpholino, oxetan-3-yl, oxetan-2-yl, tetrahydrofuranyl (e.g. tetrahydrofuran-2-yl), tetrahydropyranyl (e.g. tetrahydropyran-2-yl), 1-piperidinyl, 2-piperidinyl, 3-piperidinyl, 4-piperidinyl, piperazinyl (e.g. piperazin-1-yl), morpholino, morpholin-2-yl and morpholin-3-yl.

The term " C₆₋₁₄-aryl" refers to a monocyclic, bicyclic, or tricyclic carbocyclic ring system having a total of 6 to 14 ring members, preferably, 6 to 12 ring members, and more preferably 6 to 10 ring members, and wherein at least one ring in the system is aromatic. A preferred, yet non-limiting example of aryl includes phenyl.

The term " C₁₋₁₃-heteroaryl" refers to a mono- or multivalent, monocyclic, bicyclic or tricyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, pyrimidin-2-yl, pyrimidin-4-yl, pyrimidin-5-yl, pyrimidin-6-yl, 1,3-benzoxazol-2-yl, 1,3-benzoxazol-4-yl, 1,3-benzoxazol-5-yl, 1,3-benzoxazol-6-yl, 1,3-benzoxazol-7-yl, 1H-indazol-3-yl, 1H-indazol-4-yl, oxadiazolyl (e.g. 1,2,4-oxadiazol-3-yl, 1,3,4-oxadiazol-2-yl), pyrazolyl (e.g. 1H-pyrazol-4-yl), triazolyl (e.g. 1H-1,2,4-triazol-3-yl, 1H-triazol-4-yl, 2H-triazol-4-yl), and tetrazolyl (e.g. 2H-tetrazol-5-yl).

The term "hydroxy" refers to an -OH group.

The term "cyano" refers to a -CN (nitrile) group.

The term "oxo" refers to a double bonded oxygen (=O).

The term "acetyl" refers to a group CH₃C(O)-.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are fluoromethyl, 3,3,3-trifluoropropyl, 2,2-difluoropropyl, 1-fluoro-1-methyl-ethyl, 2-fluoro-1,1-dimethyl-ethyl, 2-fluoro-2-methylpropyl, trifluoromethyl and 2,2,2-trifluoroethyl.

The term "cyanoalkyl" refers to an alkyl group, wherein at least one of the hydrogen atoms of the alkyl group has been replaced by a cyano group. Preferably, "cyanoalkyl" refers to an alkyl group wherein 1, 2 or 3 hydrogen atoms of the alkyl group have been replaced by a cyano group. Particularly preferred, yet non-limiting examples of cyanoalkyl are cyanomethyl, and 2-cyano-2,2-dimethyl-ethyl.

The term "alkanoyl" refers to alkyl-C(O)-.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. Prticularly preferred, yet non-limiting examples of haloalkoxy are trifluoromethoxy, 3,3,3-trifluoropropoxy, 2,2,2-trifluoro-1-methyl-ethoxy, and 3-fluoro-2-fluoro-propoxy.

The term "alkenyl" denotes a mono- or bivalent linear or branched hydrocarbon group of 2 to 6 carbon atoms with at least one double bond ("C₂-C₆-alkenyl"), e.g. 1 or 2 double bonds. In particular embodiments, alkenyl has 2 to 4 carbon atoms with at least one double bond, e.g. 1 or 2 double bonds. Examples of alkenyl include ethenyl, propenyl, prop-2-enyl, isopropenyl, n-butenyl (e.g. (Z)-but-1-enyl), iso-butenyl, allyl, 2-methylallyl, 2-methylprop-1-enyl, and propa-1,2-dienyl.

The term "alkynyl" denotes a mono- or bivalent linear or branched hydrocarbon group of 2 to 6 carbon atoms with at least one triple bond ("C₂-C₆-alkynyl"). In particular embodiments, alkynyl has 2 to 4 carbon atoms with at least one triple bond. Examples of alkynyl include ethynyl, ethyndiyl, propynyl, n-butynyl or isobutynyl.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are 2-hydroxy-1,1-dimethylethyl, 2-hydroxy-2-methyl-propyl, hydroxymethyl and hydroxyethyl (e.g. 2-hydroxyethyl).

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably 1 hydrogen atom of the alkyl group have been replaced by an alkoxy group. Some particularly preferred, yet non-limiting examples of alkoxyalkyl are 2-methoxy-2,2-dimethyl-ethyl, methoxymethyl, and 2-methoxyethyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochloride salts.

The term "protective group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wuts, 5th Ed., 2014, John Wiley & Sons, N.Y.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The abbreviation "MAGL" refers to the enzyme monoacylglycerol lipase. The terms "MAGL" and "monoacylglycerol lipase" are used herein interchangeably.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "neuroinflammation" as used herein relates to acute and chronic inflammation of the nervous tissue, which is the main tissue component of the two parts of the nervous system; the brain and spinal cord of the central nervous system (CNS), and the branching peripheral nerves of the peripheral nervous system (PNS). Chronic neuroinflammation is associated with neurodegenerative diseases such as Alzheimer's disease, Parkinson's disease and multiple sclerosis. Acute neuroinflammation usually follows injury to the central nervous system immediately, e.g., as a result of traumatic brain injury (TBI).

The term "traumatic brain injury" ("TBI", also known as "intracranial injury"), relates to damage to the brain resulting from external mechanical force, such as rapid acceleration or deceleration, impact, blast waves, or penetration by a projectile.

The term "neurodegenerative diseases" relates to diseases that are related to the progressive loss of structure or function of neurons, including death of neurons. Examples of neurodegenerative diseases include, but are not limited to, multiple sclerosis, Alzheimer's disease, Parkinson's disease and amyotrophic lateral sclerosis.

The term "mental disorders" (also called mental illnesses or psychiatric disorders) relates to behavioral or mental patterns that may cause suffering or a poor ability to function in life. Such features may be persistent, relapsing and remitting, or occur as a single episode. Examples of mental disorders include, but are not limited to, anxiety and depression.

The term "pain" relates to an unpleasant sensory and emotional experience associated with actual or potential tissue damage. Examples of pain include, but are not limited to, nociceptive pain, chronic pain (including idiopathic pain), neuropathic pain including chemotherapy induced neuropathy, phantom pain and phsychogenic pain. A particular example of pain is neuropathic pain, which is caused by damage or disease affecting any part of the nervous system involved in bodily feelings (i.e., the somatosensory system). In one embodiment, "pain" is neuropathic pain resulting from amputation or thoracotomy. In one embodiment, "pain" is chemotherapy induced neuropathy.

The term "neurotoxicity" relates to toxicity in the nervous system. It occurs when exposure to natural or artificial toxic substances (neurotoxins) alter the normal activity of the nervous system in such a way as to cause damage to nervous tissue. Examples of neurotoxicity include, but are not limited to, neurotoxicity resulting from exposure to substances used in chemotherapy, radiation treatment, drug therapies, drug abuse, and organ transplants, as well as exposure to heavy metals, certain foods and food additives, pesticides, industrial and/or cleaning solvents, cosmetics, and some naturally occurring substances.

The term "cancer" refers to a disease characterized by the presence of a neoplasm or tumor resulting from abnormal uncontrolled growth of cells (such cells being "cancer cells"). As used herein, the term cancer explicitly includes, but is not limited to, hepatocellular carcinoma, colon carcinogenesis and ovarian cancer.

The term "mammal" as used herein includes both humans and non-humans and includes but is not limited to humans, non-human primates, canines, felines, murines, bovines, equines, and porcines. In a particularly preferred embodiment, the term "mammal" refers to humans.

### Compounds of the Invention

In a first aspect, the present invention provides compounds of Formula (I) 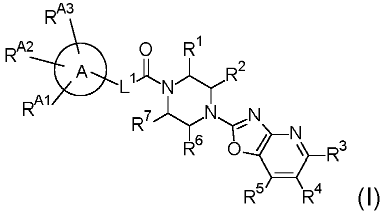 or pharmaceutically acceptable salts thereof, wherein:
(i) R¹, R², R⁶, and R⁷ are each independently hydrogen, C₁-C₆-alkyl or hydroxy-C₁-C₆-alkyl; or
(ii) R¹ and R⁶ together form a bridge of formula -(CH₂)ₘ-, wherein m is 1, 2 or 3; and R² and R⁷ are independently hydrogen, C₁-C₆-alkyl or hydroxy-C₁-C₆-alkyl; or
(iii) R¹ and R⁷ together form a bridge of formula -(CH₂)ₘ-, wherein m is 1, 2 or 3; and R² and R⁶ are independently hydrogen, C₁-C₆-alkyl or hydroxy-C₁-C₆-alkyl; or
(iv) R² and R⁶ together form a bridge of formula -(CH₂)ₘ-, wherein m is 1, 2 or 3; and R¹ and R⁷ are independently hydrogen, C₁-C₆-alkyl or hydroxy-C₁-C₆-alkyl; or
(v) R² and R⁷ together form a bridge of formula -(CH₂)ₘ-, wherein m is 1, 2 or 3; and R¹ and R⁶ are independently hydrogen, C₁-C₆-alkyl or hydroxy-C₁-C₆-alkyl;
   - R³, R⁴, and R⁵: are each independently hydrogen, halogen, cyano, C₁-C₆-alkyl, halo-C₁-C₆-alkyl, hydroxy-C₁-C₆-alkyl, C₃₋₁₂-cycloalkyl, C₁-C₆-alkoxy or halo-C₁-C₆-alkoxy;
   - R^{A1}: is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 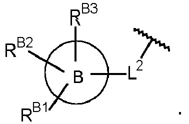
   - R^{A2}: is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 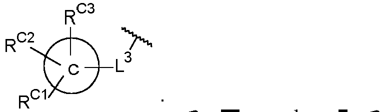
   - R^{A3}: is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy or halo-C₁-C₆-alkyl;
   - R^{B1}: is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 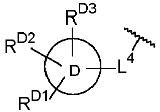
   - R^{B2}, R^{B3}, R^{C1}, R^{C2}, R^{C3}, R^{D1}, R^{D2}, and R^{D3}: are each independently hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy or halo-C₁-C₆-alkyl;
   - L¹: is a covalent bond, C₂-C₆-alkynyl, carbonyl, -C(O)NH-, -NHC(O)-, -CH₂O-, -OCH₂- or SO₂;
   - L², L³, and L⁴: are each independently a covalent bond, C₂-C₆-alkynyl, -CH₂-, -O-, carbonyl, -C(O)NH-, -NHC(O)-, -CH₂O-, -OCH₂- or SO₂; and
   - A, B, C, and D: are each independently C₆₋₁₄-aryl, C₁₋₁₃-heteroaryl, C₃₋₁₂-cycloalkyl
   or C₂₋₉-heterocyclyl.

In one embodiment, the present invention provides a compound of formula (I) as described herein, or a pharmaceutically acceptable salt thereof, wherein:
(i) R¹ and R² are independently hydrogen, C₁-C₆-alkyl or hydroxy-C₁-C₆-alkyl; and R⁶ and R⁷ are hydrogen; or
(ii) R¹ and R⁷ together form a bridge of formula -(CH₂)₂-; and R² and R⁶ are both hydrogen; or
(iii) R² and R⁶ together form a bridge of formula -(CH₂)₂-; and R¹ and R⁷ are both hydrogen.

In a preferred embodiment, the present invention provides a compound of formula (I) as described herein, or a pharmaceutically acceptable salt thereof, wherein:
R¹, R⁶, and R⁷ are all hydrogen; and
R² is hydrogen or C₁-C₆-alkyl.

In a particularly preferred embodiment, the present invention provides a compound of formula (I) as described herein, or a pharmaceutically acceptable salt thereof, wherein:
R¹, R⁶, and R⁷ are all hydrogen; and
R² is hydrogen or methyl.

In one embodiment, the present invention provides a compound of formula (I) as described herein, or a pharmaceutically acceptable salt thereof, wherein R³ is hydrogen, halogen, cyano, C₁-C₆-alkyl, halo-C₁-C₆-alkyl, C₃₋₁₂-cycloalkyl or C₁-C₆-alkoxy.

In a preferred embodiment, the present invention provides a compound of formula (I) as described herein, or a pharmaceutically acceptable salt thereof, wherein R³ is C₁-C₆-alkyl or halogen.

In a particularly preferred embodiment, the present invention provides a compound of formula (I) as described herein, or a pharmaceutically acceptable salt thereof, wherein R³ is methyl or chloro.

In one embodiment, the present invention provides a compound of formula (I) as described herein, or a pharmaceutically acceptable salt thereof, wherein R⁴ is hydrogen.

In one embodiment, the present invention provides a compound of formula (I) as described herein, or a pharmaceutically acceptable salt thereof, wherein R⁵ is hydrogen or halogen.

In a preferred embodiment, the present invention provides a compound of formula (I) as described herein, or a pharmaceutically acceptable salt thereof, wherein R⁵ is hydrogen.

In one embodiment, the present invention provides a compound of formula (I) as described herein, or a pharmaceutically acceptable salt thereof, wherein:
- L¹: is a covalent bond or C₂-C₆-alkynyl;
- A: is C₆₋₁₄-aryl, C₁₋₁₃-heteroaryl or C₂₋₉-heterocyclyl;
- R^{A1}: is hydrogen, halogen, C₁-C₆-alkyl, C₂-C₆-alkenyl, C₂-C₆-alkynyl, C₁-C₆-alkoxy, halo-C₁-C₆-alkoxy, halo-C₁-C₆-alkyl or a group 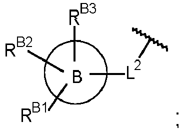
- R^{A2}: is hydrogen, halogen, cyano, hydroxy, C₁-C₆-alkyl, C₁-C₆-alkoxy, halo-C₁-C₆-alkoxy, halo-C₁-C₆-alkyl or a group 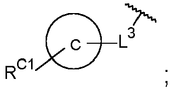
- R^{A3}: is hydrogen or halogen;
- L²: is a covalent bond, -C(O)NH-, -NHC(O)-, -CH₂O-, -OCH₂- or SO₂;
- B: is C₆₋₁₄-aryl, C₁₋₁₃-heteroaryl, C₃₋₁₂-cycloalkyl or C₂₋₉-heterocyclyl;
- R^{B1}: is hydrogen, halogen, hydroxy, acetyl, cyano, cyano-C₁-C₆-alkyl, C₁-C₆-alkyl, halo-C₁-C₆-alkyl, hydroxy-C₁-C₆-alkyl, C₂-C₆-alkenyl, C₁-C₆-alkoxy, halo-C₁-C₆-alkoxy, C₁-C₆-alkoxy-C₁-C₆-alkyl, C₁-C₆-alkanoyl, oxo or a group 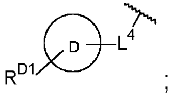
- R^{B2}: is hydrogen, halogen, oxo or C₁-C₆-alkyl;
- R^{B3}: is hydrogen or halogen;
- L³: is a covalent bond or -CH₂O-;
- C: is C₆₋₁₄-aryl, C₁₋₁₃-heteroaryl, C₃₋₁₂-cycloalkyl or C₂₋₉-heterocyclyl;
- R^{C1}: is hydrogen, halogen, C₁-C₆-alkyl or halo-C₁-C₆-alkyl;
- L⁴: is a covalent bond, -CH₂- or -O-;
- D: is C₆₋₁₄-aryl, C₃₋₁₂-cycloalkyl or C₂₋₉-heterocyclyl; and
- R^{D1}: is C₁-C₆-alkyl, halo-C₁-C₆-alkyl or halogen.

In a preferred embodiment, the present invention provides a compound of formula (I) as described herein, or a pharmaceutically acceptable salt thereof, wherein:
- L¹: is a covalent bond;
- A: is C₆₋₁₄-aryl or C₁₋₁₃-heteroaryl;
- R^{A1}: is a group 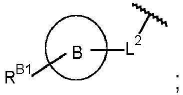
- R^{A2}: is hydrogen, halogen or C₁-C₆-alkyl;
- R^{A3}: is hydrogen or halogen;
- L²: is a covalent bond or -CH₂O-;
- B: is C₁₋₁₃-heteroaryl or C₃₋₁₂-cycloalkyl; and
- R^{B1}: is C₁-C₆-alkyl or halo-C₁-C₆-alkyl.

In a particularly preferred embodiment, the present invention provides a compound of formula (I) as described herein, or a pharmaceutically acceptable salt thereof, wherein:
- **L**¹: is a covalent bond;
- **A**: is phenyl or pyridyl;
- **R**^{A1}: is a group 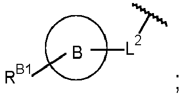
- **R**^{A2}: is hydrogen, fluoro, chloro or methyl;
- **R**^{A3}: is hydrogen or fluoro;
- **L**²: is a covalent bond or -CH₂O-;
- **B**: is oxadiazolyl (e.g. 1,2,4-oxadiazol-3-yl, 1,3,4-oxadiazol-2-yl), pyrazolyl (e.g. 1H-pyrazol-4-yl), triazolyl (e.g. 1H-1,2,4-triazol-3-yl, 1H-triazol-4-yl, 2H-triazol-4-yl), tetrazolyl (e.g. 2H-tetrazol-5-yl) or cyclopropyl; and
- **R**^{B1}: is *tert*-butyl, 2,2-dimethylpropyl, trifluoromethyl or 2-fluoro-1,1-dimethyl-ethyl.

In one embodiment, the present invention provides a compound of formula (I) as described herein, or a pharmaceutically acceptable salt thereof, wherein:
(i) R¹ and R² are independently hydrogen, C₁-C₆-alkyl or hydroxy-C₁-C₆-alkyl; and R⁶ and R⁷ are hydrogen; or
(ii) R¹ and R⁷ together form a bridge of formula -(CH₂)₂-; and R² and R⁶ are both hydrogen; or
(iii) R² and R⁶ together form a bridge of formula -(CH₂)₂-; and R¹ and R⁷ are both hydrogen;

- **R**³: is hydrogen, halogen, cyano, C₁-C₆-alkyl, halo-C₁-C₆-alkyl, C₃₋₁₂-cycloalkyl or C₁-C₆-alkoxy;
- **R**⁴: is hydrogen;
- **R**⁵: is hydrogen or halogen;
- **L**¹: is a covalent bond or C₂-C₆-alkynyl;
- **A**: is C₆₋₁₄-aryl, C₁₋₁₃-heteroaryl or C₂₋₉-heterocyclyl;
- **R**^{A1}: is hydrogen, halogen, C₁-C₆-alkyl, C₂-C₆-alkenyl, C₂-C₆-alkynyl, C₁-C₆-alkoxy, halo-C₁-C₆-alkoxy, halo-C₁-C₆-alkyl or a group 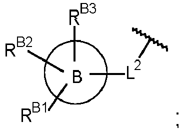
- **R**^{A2}: is hydrogen, halogen, cyano, hydroxy, C₁-C₆-alkyl, C₁-C₆-alkoxy, halo-C₁-C₆-alkoxy, halo-C₁-C₆-alkyl or a group 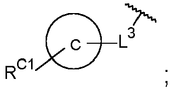
- **R**^{A3}: is hydrogen or halogen;
- **L**²: is a covalent bond, -C(O)NH-, -NHC(O)-, -CH₂O-, -OCH₂- or SO₂;
- **B**: is C₆₋₁₄-aryl, C₁₋₁₃-heteroaryl, C₃₋₁₂-cycloalkyl or C₂₋₉-heterocyclyl;
- **R**^{B1}: is hydrogen, halogen, hydroxy, acetyl, cyano, cyano-C₁-C₆-alkyl, C₁-C₆-alkyl, halo-C₁-C₆-alkyl, hydroxy-C₁-C₆-alkyl, C₂-C₆-alkenyl, C₁-C₆-alkoxy, halo-C₁-C₆-alkoxy, C₁-C₆-alkoxy-C₁-C₆-alkyl, C₁-C₆-alkanoyl, oxo or a group 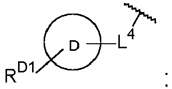
- **R**^{B2}: is hydrogen, halogen, oxo or C₁-C₆-alkyl;
- **R**^{B3}: is hydrogen or halogen;
- **L**³: is a covalent bond or -CH₂O-;
- **C**: is C₆₋₁₄-aryl, C₁₋₁₃-heteroaryl, C₃₋₁₂-cycloalkyl or C₂₋₉-heterocyclyl;
- **R**^{C1}: is hydrogen, halogen, C₁-C₆-alkyl or halo-C₁-C₆-alkyl;
- **L**⁴: is a covalent bond, -CH₂- or -O-;
- **D**: is C₆₋₁₄-aryl, C₃₋₁₂-cycloalkyl or C₂₋₉-heterocyclyl; and
- **R**^{D1}: is C₁-C₆-alkyl, halo-C₁-C₆-alkyl or halogen.

In a preferred embodiment, the present invention provides a compound of formula (I) as described herein, or a pharmaceutically acceptable salt thereof, wherein:
- **R**¹, R⁴, R⁵, R⁶, and R⁷: are all hydrogen;
- **R**²: is hydrogen or C₁-C₆-alkyl;
- **R**³: is C₁-C₆-alkyl or halogen;
- **L**¹: is a covalent bond;
- **A**: is C₆₋₁₄-aryl or C₁₋₁₃-heteroaryl;
- **R**^{A1}: is a group 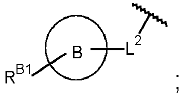
- **R**^{A2}: is hydrogen, halogen or C₁-C₆-alkyl;
- **R**^{A3}: is hydrogen or halogen;
- **L**²: is a covalent bond or -CH₂O-;
- **B**: is C₁₋₁₃-heteroaryl or C₃₋₁₂-cycloalkyl; and
- **R**^{B1}: is C₁-C₆-alkyl or halo-C₁-C₆-alkyl.

In a particularly preferred embodiment, the present invention provides a compound of formula (I) as described herein, or a pharmaceutically acceptable salt thereof, wherein:
- **R**¹, R⁴, R⁵, R⁶, and R⁷: are all hydrogen;
- **R**²: is hydrogen or methyl;
- **R**³: is methyl or chloro;
- **L**¹: is a covalent bond;
- **A**: is phenyl or pyridyl;
- **R**^{A1}: is a group 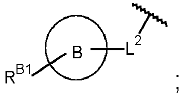
- **R**^{A2}: is hydrogen, fluoro, chloro or methyl;
- **R**^{A3}: is hydrogen or fluoro;
- **L**²: is a covalent bond or -CH₂O-;
- **B**: is oxadiazolyl, pyrazolyl, triazolyl, tetrazolyl or cyclopropyl; and
- **R**^{B1}: is *tert*-butyl, 2,2-dimethylpropyl, trifluoromethyl or 2-fluoro-1,1-dimethyl-ethyl.

In one embodiment, the present invention provides a compound of formula (I) as described herein, or a pharmaceutically acceptable salt thereof, wherein L¹ is a covalent bond or C₂-C₆-alkynyl.

In a preferred embodiment, the present invention provides a compound of formula (I) as described herein, or a pharmaceutically acceptable salt thereof, wherein L¹ is a covalent bond.

In one embodiment, the present invention provides a compound of formula (I) as described herein, or a pharmaceutically acceptable salt thereof, wherein A is C₆₋₁₄-aryl, C₁₋₁₃-heteroaryl or C₂₋₉-heterocyclyl.

In a preferred embodiment, the present invention provides a compound of formula (I) as described herein, or a pharmaceutically acceptable salt thereof, wherein A is C₆₋₁₄-aryl or C₁₋₁₃-heteroaryl.

In a particularly preferred embodiment, the present invention provides a compound of formula (I) as described herein, or a pharmaceutically acceptable salt thereof, wherein A is phenyl or pyridyl.

In one embodiment, the present invention provides a compound of formula (I) as described herein, or a pharmaceutically acceptable salt thereof, wherein R^{A1} is hydrogen, halogen, C₁-C₆-alkyl, C₂-C₆-alkenyl, C₂-C₆-alkynyl, C₁-C₆-alkoxy, halo-C₁-C₆-alkoxy, halo-C₁-C₆-alkyl or a group

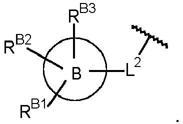

In a preferred embodiment, the present invention provides a compound of formula (I) as described herein, or a pharmaceutically acceptable salt thereof, wherein R^{A1} is a group

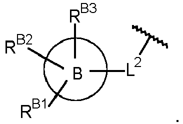

In one embodiment, the present invention provides a compound of formula (I) as described herein, or a pharmaceutically acceptable salt thereof, wherein R^{A2} is hydrogen, halogen, cyano, hydroxy, C₁-C₆-alkyl, C₁-C₆-alkoxy, halo-C₁-C₆-alkoxy, halo-C₁-C₆-alkyl or a group

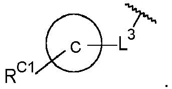

In a preferred embodiment, the present invention provides a compound of formula (I) as described herein, or a pharmaceutically acceptable salt thereof, wherein R^{A2} is hydrogen, halogen or C₁-C₆-alkyl.

In a particularly preferred embodiment, the present invention provides a compound of formula (I) as described herein, or a pharmaceutically acceptable salt thereof, wherein R^{A2} is hydrogen, fluoro, chloro or methyl.

In one embodiment, the present invention provides a compound of formula (I) as described herein, or a pharmaceutically acceptable salt thereof, wherein R A3 is hydrogen or halogen. In a preferred embodiment, the present invention provides a compound of formula (I) as described herein, or a pharmaceutically acceptable salt thereof, wherein R A3 is hydrogen or fluoro.

In one embodiment, the present invention provides a compound of formula (I) as described herein, or a pharmaceutically acceptable salt thereof, wherein L² is a covalent bond, - C(O)NH-, -NHC(O)-, -CH₂O-, -OCH₂- or SO₂.

In a preferred embodiment, the present invention provides a compound of formula (I) as described herein, or a pharmaceutically acceptable salt thereof, wherein L² is a covalent bond or -CH₂O-.

In one embodiment, the present invention provides a compound of formula (I) as described herein, or a pharmaceutically acceptable salt thereof, wherein B is C₆₋₁₄-aryl, C₁₋₁₃-heteroaryl, C₃₋₁₂-cycloalkyl or C₂₋₉-heterocyclyl.

In a preferred embodiment, the present invention provides a compound of formula (I) as described herein, or a pharmaceutically acceptable salt thereof, wherein B is C₁₋₁₃-heteroaryl or C₃₋₁₂-cycloalkyl.

In a particularly preferred embodiment, the present invention provides a compound of formula (I) as described herein, or a pharmaceutically acceptable salt thereof, wherein B is oxadiazolyl, pyrazolyl, triazolyl, tetrazolyl or cyclopropyl.

In one embodiment, the present invention provides a compound of formula (I) as described herein, or a pharmaceutically acceptable salt thereof, wherein R^{B1} is hydrogen, halogen, hydroxy, acetyl, cyano, cyano-C₁-C₆-alkyl, C₁-C₆-alkyl, halo-C₁-C₆-alkyl, hydroxy-C₁-C₆-alkyl, C₂-C₆-alkenyl, C₁-C₆-alkoxy, halo-C₁-C₆-alkoxy, C₁-C₆-alkoxy-C₁-C₆-alkyl, C₁-C₆-alkanoyl, oxo or a group

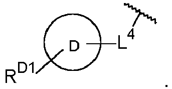

In a preferred embodiment, the present invention provides a compound of formula (I) as described herein, or a pharmaceutically acceptable salt thereof, wherein R^{B1} is C₁-C₆-alkyl or halo-C₁-C₆-alkyl.

In a particularly preferred embodiment, the present invention provides a compound of formula (I) as described herein, or a pharmaceutically acceptable salt thereof, wherein R^{B1} is *tert*-butyl, 2,2-dimethylpropyl, trifluoromethyl or 2-fluoro-1,1-dimethyl-ethyl.

In one embodiment, the present invention provides a compound of formula (I) as described herein, or a pharmaceutically acceptable salt thereof, wherein R^{B2} is hydrogen, halogen, oxo or C₁-C₆-alkyl.

In one embodiment, the present invention provides a compound of formula (I) as described herein, or a pharmaceutically acceptable salt thereof, wherein R^{B3} is hydrogen or halogen.

In one embodiment, the present invention provides a compound of formula (I) as described herein, or a pharmaceutically acceptable salt thereof, wherein L³ is a covalent bond or - CH₂O-.

In one embodiment, the present invention provides a compound of formula (I) as described herein, or a pharmaceutically acceptable salt thereof, wherein C is C₆₋₁₄-aryl, C₁₋₁₃-heteroaryl, C₃₋₁₂-cycloalkyl or C₂₋₉-heterocyclyl.

In one embodiment, the present invention provides a compound of formula (I) as described herein, or a pharmaceutically acceptable salt thereof, wherein R^{C1} is hydrogen, halogen, C₁-C₆-alkyl or halo-C₁-C₆-alkyl.

In one embodiment, the present invention provides a compound of formula (I) as described herein, or a pharmaceutically acceptable salt thereof, wherein L⁴ is a covalent bond, -CH₂- or -O-.

In one embodiment, the present invention provides a compound of formula (I) as described herein, or a pharmaceutically acceptable salt thereof, wherein D is C₆₋₁₄-aryl, C₃₋₁₂-cycloalkyl or C₂₋₉-heterocyclyl.

In one embodiment, the present invention provides a compound of formula (I) as described herein, or a pharmaceutically acceptable salt thereof, wherein R^{D1} is C₁-C₆-alkyl, halo-C₁-C₆-alkyl or halogen.

In one embodiment, the present invention provides a compound of formula (I) as described herein, or a pharmaceutically acceptable salt thereof, wherein the compound of formula (I) is:
[4-(5-methyloxazolo[4,5-b]pyridin-2-yl)piperazin-1-yl]-(2-phenyl-1,3-benzoxazol-6-yl)methanone;
(4-oxazolo[4,5-b]pyridin-2-ylpiperazin-1-yl)-(2-phenyl-1,3-benzoxazol-6-yl)methanone;
[4-(5-methyloxazolo[4,5-b]pyridin-2-yl)piperazin-1-yl]-(2-pyrrolidin-1-yl-1,3-benzoxazol-6-yl)methanone;
[4-(5-tert-butyl-1,2,4-oxadiazol-3-yl)phenyl]-[4-(5-methyloxazolo[4,5-b]pyridin-2-yl)piperazin-1-yl]methanone;
[4-[5-(2,2-dimethylpropyl)-1,2,4-oxadiazol-3-yl]phenyl]-[4-(5-methyloxazolo[4,5-b]pyridin-2-yl)piperazin-1-yl]methanone;
[2-(2-azaspiro [3.4] octan-2-yl)-1,3 -benzoxazol-6-yl]- [4-(5 -methyloxazolo [4,5 -b]pyridin-2-yl)piperazin-1-yl]methanone;
[4-[5-(2-hydroxy-1,1-dimethyl-ethyl)-1,2,4-oxadiazol-3-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(7-chlorooxazolo[4,5-b]pyridin-2-yl)piperazin-1-yl]methanone;
[4-(7-chlorooxazolo[4,5-b]pyridin-2-yl)piperazin-1-yl]-[4-[5-(2,2-dimethylpropyl)-1,2,4-oxadiazol-3-yl]phenyl]methanone;
[4-(5-chlorooxazolo[4,5-b]pyridin-2-yl)piperazin-1-yl]-[4-[5-(2,2-dimethylpropyl)-1,2,4-oxadiazol-3-yl]phenyl]methanone;
[4-[5-(2-fluoro-1,1-dimethyl-ethyl)-1,2,4-oxadiazol-3-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(5-chlorooxazolo[4,5-b]pyridin-2-yl)piperazin-1-yl]methanone;
[4-[5-(2,2-dimethylpropyl)-1,2,4-oxadiazol-3-yl]phenyl]-(4-oxazolo[4,5-b]pyridin-2-ylpiperazin-1-yl)methanone;
[4-(5-tert-butyl-1,2,4-oxadiazol-3-yl)phenyl]-(4-oxazolo [4, 5-b]pyridin-2-ylpiperazin-1-yl)methanone;
2-[4-[4-[5-(2,2-dimethylpropyl)-1,2,4-oxadiazol-3-yl]benzoyl]piperazin-1-yl]oxazolo[4,5-b]pyridine-5-carbonitrile;
[4-[5-(2,2-dimethylpropyl)-1,2,4-oxadiazol-3-yl]phenyl]-[4-[5-(trifluoromethyl)oxazolo [4, 5-b]pyridin-2-yl]piperazin-1-yl] methanone;
[2-(1-ethylpropyl)-1,3-benzoxazol-6-yl]-[4-(5-methyloxazolo[4,5-b]pyridin-2-yl)piperazin-1-yl]methanone;
[4-(5-chlorooxazolo [4, 5-b]pyridin-2-yl)piperazin-1-yl]-[4-[5-(2,2-dimethylpropyl)-1, 3,4-oxadiazol-2-yl]phenyl]methanone;
(2-cyclohexyl-1,3-benzoxazol-6-yl)-[4-(5-methyloxazolo[4,5-b]pyridin-2-yl)piperazin- 1 - yl]methanone;
[4-(5-methyloxazolo [4, 5-b]pyridin-2-yl)piperazin-1-yl]-[2-(3,3, 3-trifluoropropyl)-1,3-benzoxazol-6-yl]methanone;
[4-(5-chlorooxazolo[4,5-b]pyridin-2-yl)piperazin-1-yl]-[4-[1-(2,2-dimethylpropyl)pyrazol-4-yl]phenyl]methanone;
[3-chloro-5-[4-(hydroxymethyl)phenyl]phenyl]-[4-(5-methyloxazolo[4,5-b]pyridin-2-yl)piperazin-1-yl]methanone;
[3-chloro-5-(1,1-dioxo-1,4-thiazinan-4-yl)phenyl]-[4-(5-methyloxazolo[4,5-b]pyridin-2-yl)piperazin-1-yl]methanone;
1-[4-[3-chloro-5-[4-(5-methyloxazolo[4,5-b]pyridin-2-yl)piperazine-1-carbonyl]phenyl]piperazin-1-yl]ethanone;
[3-chloro-5-(4,4-difluoro-1-piperidyl)phenyl]-[4-(5-methyloxazolo[4,5-b]pyridin-2-yl)piperazin-1-yl]methanone;
[3-chloro-5-[4-(hydroxymethyl)-1-piperidyl]phenyl]-[4-(5-methyloxazolo[4,5-b]pyridin-2-yl)piperazin-1-yl]methanone;
[3-chloro-5-(6-hydroxy-2-azaspiro[3.3]heptan-2-yl)phenyl]-[4-(5-methyloxazolo[4,5-b]pyridin-2-yl)piperazin-1-yl]methanone;
[2-(3-chlorophenyl)sulfonyl-2,6-diazaspiro[3.3]heptan-6-yl]-[4-(5-methyloxazolo[4,5-b]pyridin-2-yl)piperazin-1-yl]methanone;
[3-[4-(hydroxymethyl)phenyl]-5-(3,3,3-trifluoropropoxy)phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2-fluoro-1,1-dimethylethyl)-1,2,4-oxadiazol-3-yl]phenyl]methanone;
[2-[(E)-but-1-enyl]-1,3-benzoxazol-6-yl]-[4-(5-methyloxazolo[4,5-b]pyridin-2-yl)piperazin-1-yl]methanone;
(2-cyclopropyl-1,3-benzoxazol-6-yl)-[4-(5-methyloxazolo [4, 5-b]pyridin-2-yl)piperazin-1-yl]methanone;
1-[3-[4-[4-(5-chlorooxazolo[4,5-b]pyridin-2-yl)piperazine-1-carbonyl]phenyl]-1,2,4-oxadiazol-5-yl]propan-2-one;
[4-(5-chlorooxazolo[4,5-b]pyridin-2-yl)piperazin-1-yl]-[4-[5-(2,2-difluoropropyl)-1,2,4-oxadiazol-3-yl]phenyl]methanone;
[4-(5-chlorooxazolo[4,5-b]pyridin-2-yl)piperazin-1-yl]-[4-[5-(1-fluoro-1-methyl-ethyl)-1,2,4-oxadiazol-3-yl]phenyl]methanone;
[4-(5-chlorooxazolo[4,5-b]pyridin-2-yl)piperazin-1-yl]-[4-[3-(2,2-dimethylpropyl)-1,2,4-oxadiazol-5-yl]phenyl]methanone;
[3-[4-(hydroxymethyl)phenyl]-4-[[1-(trifluoromethyl)cyclopropyl]methoxy]phenyl]-[4-(5-methyloxazolo[4,5-b]pyridin-2-yl)piperazin-1-yl]methanone;
[3-[4-(hydroxymethyl)phenyl]-4-(3,3,3-trifluoropropoxy)phenyl]-[4-(5-methyloxazolo[4,5-b]pyridin-2-yl)piperazin-1-yl]methanone;
[3-[4-(hydroxymethyl)phenyl]-5-[[1-(trifluoromethyl)cyclopropyl]methoxy]phenyl]-[4-(5-methyloxazolo[4,5-b]pyridin-2-yl)piperazin-1-yl]methanone;
(2-butyl-1,3-benzoxazol-6-yl)-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(1-fluorocyclopropyl)-1,2,4-oxadiazol-3-yl]phenyl]methanone;
[4-(5-chlorooxazolo[4,5-b]pyridin-2-yl)piperazin-1-yl]-[4-[1-(2,2-dimethylpropyl)-1,2,4-triazol-3-yl]phenyl] methanone;
[4-[1-(2,2-dimethylpropyl)-1,2,4-triazol-3-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)imidazol-4-yl]phenyl]methanone;
[4-(5-tert-butyl-1,3,4-oxadiazol-2-yl)phenyl]-[4-(5-chlorooxazolo[4,5-b]pyridin-2-yl)piperazin-1-yl]methanone;
[4-(5-oxa-2-azaspiro[3.4]octan-2-yl)phenyl]-(4-oxazolo[4,5-b]pyridin-2-ylpiperazin-1-yl)methanone;
(4-oxazolo[4,5-b]pyridin-2-ylpiperazin-1-yl)-[4-[3-(2,2,2-trifluoro-1-methyl-ethoxy)azetidin-1-yl]phenyl]methanone;
[4-(5-tert-butyl-1,3,4-oxadiazol-2-yl)phenyl]-[4-(5-methyloxazolo[4,5-b]pyridin-2-yl)piperazin-1-yl]methanone;
[4-(5-methyloxazolo [4,5-b]pyridin-2-yl)piperazin-1-yl]-[4-(5-oxa-2-azaspiro[3.4]octan-2-yl)phenyl] methanone;
[4-(5-methyloxazolo[4,5-b]pyridin-2-yl)piperazin-1-yl]-[4-[3-(2,2,2-trifluoro-1-methyl-ethoxy)azetidin-1-yl]phenyl]methanone;
[4-(5-chlorooxazolo[4,5-b]pyridin-2-yl)piperazin-1-yl]-[4-[5-(2-fluoro-2-methyl-propyl)-1,2,4-oxadiazol-3-yl]phenyl]methanone;
[4-(5-chlorooxazolo[4,5-b]pyridin-2-yl)piperazin-1-yl]-[4-[5-(2-methylallyl)-1,2,4-oxadiazol-3-yl]phenyl]methanone;
[4-(5-chlorooxazolo[4,5-b]pyridin-2-yl)piperazin-1-yl]-[4-[5-(2-methylprop-1-enyl)-1,2,4-oxadiazol-3-yl]phenyl]methanone;
[4-(5-chlorooxazolo[4,5-b]pyridin-2-yl)piperazin-1-yl]-[4-[5-(2-hydroxy-2-methylpropyl)-1,2,4-oxadiazol-3-yl]phenyl]methanone;
(4-oxazolo[4,5-b]pyridin-2-ylpiperazin-1-yl)-[4-(3-phenoxyazetidin-1-yl)phenyl] methanone;
[4-[5-(2-fluoro-1,1-dimethyl-ethyl)-1,3,4-oxadiazol-2-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(5-methoxyoxazolo[4,5-b]pyridin-2-yl)piperazin-1-yl]methanone;
[4-(5-chlorooxazolo[4,5-b]pyridin-2-yl)piperazin-1-yl]-[4-[5-(2-fluoro-1,1-dimethylethyl)- 1,3,4-oxadiazol-2-yl]phenyl]methanone;
[4-[5-(1-fluorocyclopropyl)-1,2,4-oxadiazol-3-yl]phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[4-(3-phenoxyazetidin-1-yl)phenyl] methanone;
[4-[5-(2,2-dimethylpropyl)-1,2,4-oxadiazol-3-yl]-3-[4-(hydroxymethyl)phenyl]phenyl]-[4-(5-methyloxazolo[4,5-b]pyridin-2-yl)piperazin-1-yl]methanone;
[3-[4-(hydroxymethyl)phenyl]-4-[(1-methylcyclopropyl)methoxy]phenyl]-[4-(5-methyloxazolo[4,5-b]pyridin-2-yl)piperazin-1-yl]methanone;
[3-[4-(hydroxymethyl)phenyl]-5-[(1-methylcyclopropyl)methoxy]phenyl]-[4-(5-methyloxazolo[4,5-b]pyridin-2-yl)piperazin-1-yl]methanone;
[4-[(3-chlorophenyl)methoxy]-3-(1H-imidazol-2-yl)phenyl]-[4-(5-methyloxazolo[4,5-b]pyridin-2-yl)piperazin-1-yl]methanone;
[4-(2-azaspiro[3.4]octan-2-yl)-3-(1H-imidazol-2-yl)phenyl]-[4-(5-methyloxazolo[4,5-b]pyridin-2-yl)piperazin-1-yl]methanone;
[4-[5-[1-(fluoromethyl)cyclopropyl]-1,2,4-oxadiazol-3-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-[1-(fluoromethyl)cyclopropyl]-1,2,4-oxadiazol-3-yl]phenyl]methanone;
[4-(3-tert-butyl-1,2,4-oxadiazol-5-yl)phenyl]-[4-(5-methyloxazolo[4,5-b]pyridin-2-yl)piperazin-1-yl]methanone;
[4-(3-tert-butyl-1,2,4-oxadiazol-5-yl)phenyl]-[4-(5-chlorooxazolo[4,5-b]pyridin-2-yl)piperazin-1-yl]methanone;
[4-(5-tert-butyl-1,2,4-oxadiazol-3-yl)phenyl]-[4-(5-cyclopropyloxazolo[4,5-b]pyridin-2-yl)piperazin-1-yl]methanone;
[4-(5-chlorooxazolo[4,5-b]pyridin-2-yl)piperazin-1-yl]-[4-[5-[(1-methylcyclopropyl)methyl]-1,2,4-oxadiazol-3-yl]phenyl]methanone;
[4-(5-methyloxazolo[4,5-b]pyridin-2-yl)piperazin-1-yl]-[4-[3-[(1S)-2,2,2-trifluoro-1-methyl-ethoxy]azetidin-1-yl]phenyl]methanone;
[4-(5-methyloxazolo[4,5-b]pyridin-2-yl)piperazin-1-yl]-[4-[3-[(1R)-2,2,2-trifluoro-1-methyl-ethoxy]azetidin-1-yl]phenyl]methanone;
[4-(5-chlorooxazolo[4,5-b]pyridin-2-yl)piperazin-1-yl]-[4-(2,2-difluoro-5-azaspiro[2.4]heptan-5-yl)phenyl]methanone;
[4-(5-chlorooxazolo[4,5-b]pyridin-2-yl)piperazin-1-yl]-[4-(7,7-difluoro-2-azaspiro[3.3]heptan-2-yl)phenyl]methanone;
[4-(3-tert-butoxyazetidin-1-yl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[3-(1,1-dimethylpropoxy)azetidin-1-yl]phenyl]methanone;
[4-(1-tert-butyl-1,2,4-triazol-3-yl)phenyl]-[4-(5-chlorooxazolo[4,5-b]pyridin-2-yl)piperazin-1-yl]methanone;
[4-(1-tert-butyl-1,2,4-triazol-3-yl)phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]phenyl]methanone;
[4-(5-chlorooxazolo [4, 5-b]pyridin-2-yl)piperazin-1-yl]-[4-[2-(2,2-dimethylpropyl)triazol-4-yl]phenyl]methanone;
[4-(5-tert-butyl-1,2,4-oxadiazol-3-yl)phenyl]-[4-(5-chlorooxazolo[4,5-b]pyridin-2-yl)-2-methyl-piperazin-1-yl]methanone;
[4-(5-tert-butyl-1,2,4-oxadiazol-3-yl)phenyl]-[4-(5-chlorooxazolo[4,5-b]pyridin-2-yl)-3-methyl-piperazin-1-yl]methanone;
[4-(1-tert-butylpyrazol-4-yl)phenyl]-[4-(5-methyloxazolo[4,5-b]pyridin-2-yl)piperazin-1-yl]methanone;
[4-(1-tert-butylpyrazol-4-yl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[1-[(1-methylcyclopropyl)methyl]-1,2,4-triazol-3-yl]phenyl]methanone;
[4-(5-chlorooxazolo[4,5-b]pyridin-2-yl)piperazin-1-yl]-[4-[2-[(1-methylcyclopropyl)methyl]-1,2,4-triazol-3-yl]phenyl]methanone;
[4-[5-(1-methylcyclopropyl)-1,2,4-oxadiazol-3-yl]phenyl]-[4-(5-methyloxazolo [4,5-b]pyridin-2-yl)piperazin-1-yl]methanone;
[4-(4-tert-butylpyrazol-1-yl)phenyl]-[4-(5-chlorooxazolo[4,5-b]pyridin-2-yl)piperazin-1-yl]methanone;
[4-(4-tert-butylpyrazol-1-yl)phenyl]-[4-(5-methyloxazolo[4,5-b]pyridin-2-yl)piperazin- 1 - yl]methanone;
[4-[1-(2,2-dimethylpropyl)pyrazol-4-yl]phenyl]-[4-(5-methyloxazolo[4,5-b]pyridin-2-yl)piperazin-1-yl]methanone;
[4-(7,7-difluoro-2-azaspiro[3.3]heptan-2-yl)phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[6-[3-(2,2,2-trifluoro-1-methyl-ethoxy)azetidin-1-yl]-3-pyridyl]methanone;
[4-(4-tert-butylimidazol-1-yl)phenyl]-[4-(5-methyloxazolo [4, 5-b]pyridin-2-yl)piperazin-1-yl]methanone;
[4-(5-tert-butyl-1,2,4-oxadiazol-3-yl)-3-methyl-phenyl]-[4-(5-methyloxazolo[4,5-b]pyridin-2-yl)piperazin-1-yl]methanone;
[4-(5-tert-butyl-1,2,4-oxadiazol-3-yl)-3-methyl-phenyl]-[4-(5-chlorooxazolo[4,5-b]pyridin-2-yl)piperazin-1-yl]methanone;
[4-(5-tert-butyl-1,2,4-oxadiazol-3-yl)-3-fluoro-phenyl]-[4-(5-methyloxazolo[4,5-b]pyridin-2-yl)piperazin-1-yl]methanone;
[4-(5-tert-butyl-1,2,4-oxadiazol-3-yl)-3-fluoro-phenyl]-[4-(5-chlorooxazolo[4,5-b]pyridin-2-yl)piperazin-1-yl]methanone;
[3-chloro-4-[(1-methylcyclopropyl)methoxy]phenyl]-[4-(5-chlorooxazolo[4,5-b]pyridin-2-yl)piperazin-1-yl]methanone;
[6-[3-(1,1-dimethylpropoxy)azetidin-1-yl]-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[5-methyl-6-[3-(2,2,2-trifluoro-1-methyl-ethoxy)azetidin-1-yl]-3-pyridyl]methanone;
[6-(5-tert-butyl-1,2,4-oxadiazol-3-yl)-3-pyridyl]-[4-(5-chlorooxazolo[4,5-b]pyridin-2-yl)piperazin-1-yl]methanone;
[6-(5-tert-butyl-1,2,4-oxadiazol-3-yl)-3-pyridyl]-[4-(5-methyloxazolo[4,5-b]pyridin-2-yl)piperazin-1-yl]methanone;
[4-(4-tert-butylimidazol-1-yl)phenyl]-[4-(5-chlorooxazolo [4, 5-b]pyridin-2-yl)piperazin-1-yl]methanone;
[2-fluoro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[4-(5-chlorooxazolo [4, 5-b]pyridin-2-yl)piperazin-1-yl]-[4-(2, 2-dimethylpropoxy)-3-methyl-phenyl] methanone;
[4-(2,2-dimethylpropoxy)-3-methyl-phenyl]-[4-(5-methyloxazolo[4,5-b]pyridin-2-yl)piperazin-1-yl]methanone;
[4-(5-chlorooxazolo [4, 5-b]pyridin-2-yl)piperazin-1-yl]-[4-[3-(2,2,2-trifluoro-1-methyl-ethoxy)azetidin-1-yl]phenyl]methanone;
[4-(5-chlorooxazolo [4, 5-b]pyridin-2-yl)piperazin-1-yl]-[4-(3-ethoxy-3-methyl-azetidin-1-yl)phenyl] methanone;
[3-chloro-4-[(1-methylcyclopropyl)methoxy]phenyl]-[4-(5-methyloxazolo[4,5-b]pyridin-2-yl)piperazin-1-yl]methanone;
[3-fluoro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[4-(5-methyloxazolo [4,5-b]pyridin-2-yl)piperazin-1-yl]-[4-(3-methyloxetan-3-yl)phenyl]methanone;
[4-(1-fluorocyclopropyl)phenyl]-[4-(5-methyloxazolo[4,5-b]pyridin-2-yl)piperazin-1-yl]methanone;
[4-chloro-5-fluoro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[6-[3-(2,2,2-trifluoro-1-methyl-ethoxy)azetidin-1-yl]pyridazin-3-yl]methanone;
[5-methyl-6-[(1-methylcyclopropyl)methoxy]-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[2-[3-(2,2,2-trifluoro-1-methyl-ethoxy)azetidin-1-yl]pyrimidin-5-yl] methanone;
[4-(5-methyloxazolo[4,5-b]pyridin-2-yl)piperazin-1-yl]-[4-methyl-6-[3-(2,2,2-trifluoro-1-methyl-ethoxy)azetidin-1-yl]-3-pyridyl]methanone;
[6-(7,7-difluoro-2-azaspiro[3.3]heptan-2-yl)-4-methyl-3-pyridyl]-[4-(5-methyloxazolo[4,5-b]pyridin-2-yl)piperazin-1-yl]methanone;
[2-(2,2-difluoro-5-azaspiro[2.4]heptan-5-yl)pyrimidin-5-yl]-[4-(5-methyloxazolo[4,5-b]pyridin-2-yl)piperazin-1-yl]methanone;
[6-(2,2-difluoro-5-azaspiro[2.4]heptan-5-yl)-4-methyl-3-pyridyl]-[4-(5-methyloxazolo[4,5-b]pyridin-2-yl)piperazin-1-yl]methanone;
[5-fluoro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[6-(2,2-difluoro-5-azaspiro[2.4]heptan-5-yl)-5-methyl-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[2-methyl-4-[3-(2,2,2-trifluoro-1-methyl-ethoxy)azetidin-1-yl]phenyl]methanone;
[2,6-difluoro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[2-chloro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[5-methyl-6-[[1-(trifluoromethyl)cyclopropyl]methoxy]-3-pyridyl]methanone;
[4-(5-chlorooxazolo[4,5-b]pyridin-2-yl)piperazin-1-yl]-[3-fluoro-4-[(1-methylcyclopropyl)methoxy]phenyl]methanone;
[4-(3-tert-butyl-1,2,4-triazol-1-yl)phenyl]-[4-(5-methyloxazolo[4,5-b]pyridin-2-yl)piperazin-1-yl]methanone;
[3-fluoro-4-[(1-methylcyclopropyl)methoxy]phenyl]-[4-(5-methyloxazolo[4,5-b]pyridin-2-yl)piperazin-1-yl]methanone;
[4-(3-tert-butyl-1,2,4-triazol-1-yl)phenyl]-[4-(5-chlorooxazolo[4,5-b]pyridin-2-yl)piperazin-1-yl]methanone;
[5-chloro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2,2-dimethylpropyl)-4-methyl-1,2,4-triazol-3-yl]phenyl]methanone;
[5-chloro-6-(2,2-difluoro-5-azaspiro[2.4]heptan-5-yl)-3-pyridyl]-[4-(5-methyloxazolo[4,5-b]pyridin-2-yl)piperazin-1-yl]methanone;
[4-[5-(2,2-dimethylpropyl)-4-methyl-1,2,4-triazol-3-yl]phenyl]-[4-(5-methyloxazolo[4,5-b]pyridin-2-yl)piperazin-1-yl]methanone;
[6-(2,2-difluoro-5-azaspiro[2.4]heptan-5-yl)-5-fluoro-3-pyridyl]-[4-(5-methyloxazolo[4,5-b]pyridin-2-yl)piperazin-1-yl]methanone;
[4-[1-(2,2-dimethylpropyl)triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-fluoro-6-[3-(2,2,2-trifluoro-1-methyl-ethoxy)azetidin-1-yl]-3-pyridyl]methanone;
[4-(5-chlorooxazolo[4,5-b]pyridin-2-yl)piperazin-1-yl]-[6-(2,2-difluoro-5-azaspiro[2.4]heptan-5-yl)-5-fluoro-3-pyridyl]methanone;
[4-(5-chlorooxazolo[4,5-b]pyridin-2-yl)piperazin-1-yl]-[5-fluoro-6-[[1-(trifluoromethyl)cyclopropyl]methoxy]-3-pyridyl]methanone;
[4-(5-chlorooxazolo[4,5-b]pyridin-2-yl)piperazin-1-yl]-[5-fluoro-6-[(1-methylcyclopropyl)methoxy]-3-pyridyl]methanone;
[4-(5-tert-butyl-4-methyl-1,2,4-triazol-3-yl)phenyl]-[4-(5-chlorooxazolo[4,5-b]pyridin-2-yl)piperazin-1-yl]methanone;
[4-(5-tert-butyl-4-methyl-1,2,4-triazol-3-yl)phenyl]-[4-(5-methyloxazolo[4,5-b]pyridin-2-yl)piperazin-1-yl]methanone;
[5-chloro-6-[(1-methylcyclopropyl)methoxy]-3-pyridyl]-[4-(5-methyloxazolo[4,5-b]pyridin-2-yl)piperazin-1-yl]methanone;
[5-chloro-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-tert-butyl-1,2,4-oxadiazol-3-yl)-3-chloro-phenyl]-[4-(5-chlorooxazolo[4,5-b]pyridin-2-yl)piperazin-1-yl]methanone;
[4-(5-tert-butyl-1,2,4-oxadiazol-3-yl)-3-chloro-phenyl]-[4-(5-methyloxazolo[4,5-b]pyridin-2-yl)piperazin-1-yl]methanone;
[4-(5-tert-butyl-1,2,4-oxadiazol-3-yl)-3,5-difluoro-phenyl]-[4-(5-chlorooxazolo[4,5-b]pyridin-2-yl)piperazin-1-yl]methanone;
[4-(5-tert-butyl-1,2,4-oxadiazol-3-yl)-3-(trifluoromethyl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3-methyl-phenyl]methanone;
[4-(5-chlorooxazolo[4,5-b]pyridin-2-yl)piperazin-1-yl]-[4-[2-(2,2-dimethylpropyl)triazol-4-yl]-3-methyl-phenyl]methanone;
[5-fluoro-6-[(1-methylcyclopropyl)methoxy]-3-pyridyl]-[4-(5-methyloxazolo[4,5-b]pyridin-2-yl)piperazin-1-yl]methanone;
[5-chloro-6-[(1-methylcyclopropyl)methoxy]-3-pyridyl]-[4-(5-chlorooxazolo[4,5-b]pyridin-2-yl)piperazin-1-yl]methanone;
[5-fluoro-6-[[1-(trifluoromethyl)cyclopropyl]methoxy]-3-pyridyl]-[4-(5-methyloxazolo[4,5-b]pyridin-2-yl)piperazin-1-yl]methanone;
[5,6-bis[[1-(trifluoromethyl)cyclopropyl]methoxy]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-chloro-6-[[1-(trifluoromethyl)cyclopropyl]methoxy]-3-pyridyl]methanone;
[4-(5-chlorooxazolo[4,5-b]pyridin-2-yl)piperazin-1-yl]-[5-methyl-6-[[1-(trifluoromethyl)cyclopropyl]methoxy]-3-pyridyl]methanone;
[4-(5-chlorooxazolo[4,5-b]pyridin-2-yl)piperazin-1-yl]-[5-(trifluoromethyl)-6-[[1-(trifluoromethyl)cyclopropyl]methoxy]-3-pyridyl]methanone;
[4-(5-tert-butyl-1,3,4-oxadiazol-2-yl)-3-methyl-phenyl]-[4-(5-methyloxazolo[4,5-b]pyridin-2-yl)piperazin-1-yl]methanone;
[4-(5-tert-butyl-1,3,4-oxadiazol-2-yl)-3-methyl-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)triazol-4-yl]-3-fluoro-phenyl]methanone;
[4-[2-(2,2-dimethylpropyl)triazol-4-yl]-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3-fluoro-phenyl]methanone;
[4-(1-tert-butyltriazol-4-yl)phenyl]-[4-(5-methyloxazolo[4,5-b]pyridin-2-yl)piperazin-1-yl]methanone;
[4-(1-tert-butyltriazol-4-yl)phenyl]-[4-(5-chlorooxazolo[4,5-b]pyridin-2-yl)piperazin-1-yl]methanone;
[4-(1-tert-butyl-1,2,4-triazol-3-yl)-3-chloro-phenyl]-[4-(5-methyloxazolo[4,5-b]pyridin-2-yl)piperazin-1-yl]methanone;
[4-(1-tert-butyl-1,2,4-triazol-3-yl)-3-chloro-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)triazol-4-yl] -3,5 -difluoro-phenyl] methanone;
[4-[2-(2,2-dimethylpropyl)triazol-4-yl]-3,5-difluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3,5-difluoro-phenyl]methanone;
[4-(5-chlorooxazolo[4,5-b]pyridin-2-yl)piperazin-1-yl]-[6-(cyclopropylmethoxy)-5-methyl-3-pyridyl] methanone;
[4-(5-chlorooxazolo[4,5-b]pyridin-2-yl)piperazin-1-yl]-[5-methyl-6-[(1-methylcyclopropyl)methoxy]-3-pyridyl]methanone;
[4-(5-chlorooxazolo[4,5-b]pyridin-2-yl)piperazin-1-yl]-[5-methyl-6-[(3-methyloxetan-3-yl)methoxy]-3-pyridyl]methanone;
[4-(5-chlorooxazolo[4,5-b]pyridin-2-yl)piperazin-1-yl]-[6-[(2,2-difluoro-1-methylcyclopropyl)methoxy]-5-methyl-3-pyridyl] methanone;
[4-(5-chlorooxazolo[4,5-b]pyridin-2-yl)piperazin-1-yl]-[6-[(3-ethyloxetan-3-yl)methoxy]-5-methyl-3-pyridyl]methanone;
[4-(5-chlorooxazolo[4,5-b]pyridin-2-yl)piperazin-1-yl]-[6-[[1-(methoxymethyl)cyclopropyl]methoxy]-5-methyl-3-pyridyl]methanone; [4-(5-chlorooxazolo[4,5-b]pyridin-2-yl)piperazin-1-yl]-[6-[(3-fluorooxetan-3-yl)methoxy]-5-methyl-3-pyridyl]methanone;
2-[1-[[5-[4-(5-chlorooxazolo[4,5-b]pyridin-2-yl)piperazine-1-carbonyl]-3-methyl-2-pyridyl] oxymethyl] cyclopropyl] acetonitrile;
1-[[5-[4-(5-chlorooxazolo[4,5-b]pyridin-2-yl)piperazine-1-carbonyl]-3-methyl-2-pyridyl]oxymethyl]cyclopropanecarbonitrile;
3-[[5-[4-(5-chlorooxazolo[4,5-b]pyridin-2-yl)piperazine-1-carbonyl]-3-methyl-2-pyridyl]oxymethyl]oxetane-3-carbonitrile;
[4-[1-(1-bicyclo[1.1.1]pentanyl)triazol-4-yl]phenyl]-[4-(5-methyloxazolo[4,5-b]pyridin-2-yl)piperazin-1-yl]methanone;
[4-[1-(1-bicyclo[1.1.1]pentanyl)triazol-4-yl]phenyl]-[4-(5-chlorooxazolo[4,5-b]pyridin-2-yl)piperazin-1-yl]methanone;
[4-(1-tert-butyl-1,2,4-triazol-3-yl)-3-methyl-phenyl]-[4-(5-methyloxazolo[4,5-b]pyridin-2-yl)piperazin-1-yl]methanone;
[4-(1-tert-butyl-1,2,4-triazol-3-yl)-3-methyl-phenyl]-[4-(5-chlorooxazolo[4,5-b]pyridin-2-yl)piperazin-1-yl]methanone;
3-[[5-[4-(5-chlorooxazolo[4,5-b]pyridin-2-yl)piperazine-1-carbonyl]-3-methyl-2-pyridyl]oxymethyl]-5-fluoro-benzonitrile;
[4-(5-chlorooxazolo[4,5-b]pyridin-2-yl)piperazin-1-yl]-[4-[1-(2,2,2-trifluoroethyl)triazol-4-yl]phenyl]methanone;
[4-(5-methyloxazolo[4,5-b]pyridin-2-yl)piperazin-1-yl]-[4-[1-(2,2,2-trifluoroethyl)triazol-4-yl]phenyl]methanone;
[4-(5-methyloxazolo[4,5-b]pyridin-2-yl)piperazin-1-y1]-[4-[1-[(3-methyloxetan-3-yl)methyl]triazol-4-yl]phenyl]methanone;
[4-(5-chlorooxazolo[4,5-b]pyridin-2-yl)piperazin-1-yl]-[4-[1-[(3-methyloxetan-3-yl)methyl]triazol-4-yl]phenyl]methanone;
1-[4-(5-chlorooxazolo[4,5-b]pyridin-2-yl)piperazin-1-yl]-3-phenyl-prop-2-yn-1-one;
1-[4-(5-chlorooxazolo[4,5-b]pyridin-2-yl)piperazin-1-yl]-3-(4-chlorophenyl)prop-2-yn-1-one;
[4-(5-chlorooxazolo[4,5-b]pyridin-2-yl)piperazin-1-yl]-[3-[2-(2,6-dichlorophenyl)ethynyl]azetidin-1-yl]methanone;
[3-[2-(2-chloro-4-fluoro-phenyl)ethynyl] azetidin-1-yl]-[4-(5-chlorooxazolo [4, 5-b]pyridin-2-yl)piperazin-1-yl]methanone;
[4-(5-chlorooxazolo[4,5-b]pyridin-2-yl)piperazin-1-yl]-(4-ethynyl-3-methoxyphenyl)methanone;
[4-[5-(1-bicyclo[1.1.1]pentanyl)-1,2,4-oxadiazol-3-yl]phenyl]-[4-(5-chlorooxazolo[4,5-b]pyridin-2-yl)piperazin-1-yl]methanone;
[4-(1-tert-butyl-1,2,4-triazol-3-yl)-3-fluoro-phenyl]-[4-(5-methyloxazolo[4,5-b]pyridin-2-yl)piperazin-1-yl]methanone;
[4-(1-tert-butyl-1,2,4-triazol-3-yl)-3-fluoro-phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[4-[1-[1-(trifluoromethyl)cyclopropyl]triazol-4-yl]phenyl]methanone;
[4-(5-methyloxazolo[4,5-b]pyridin-2-yl)piperazin-1-yl]-[4-[1-(3-methyloxetan-3-yl)triazol-4-yl]phenyl]methanone;
[4-(5-chlorooxazolo[4,5-b]pyridin-2-yl)piperazin-1-yl]-[4-[1-(3-methyloxetan-3-yl)triazol-4-yl]phenyl]methanone;
[4-(1-tert-butyl-1,2,4-triazol-3-yl)-3-(trifluoromethyl)phenyl]-[4-(5-methyloxazolo[4,5-b]pyridin-2-yl)piperazin-1-yl]methanone;
[4-(1-tert-butyl-1,2,4-triazol-3-yl)-3-(trifluoromethyl)phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[4-[1-(oxetan-3-ylmethyl)triazol-4-yl]phenyl]methanone;
[4-(5-chlorooxazolo[4,5-b]pyridin-2-yl)piperazin-1-yl]-[4-[1-(oxetan-3-ylmethyl)triazol-4-yl]phenyl]methanone;
[4-(5-tert-butyl-1,3,4-oxadiazol-2-yl)-3-chloro-phenyl]-[4-(5-chlorooxazolo[4,5-b]pyridin-2-yl)piperazin-1-yl]methanone;
[4-(5-tert-butyl-1,3,4-oxadiazol-2-yl)-3-fluoro-5-hydroxy-phenyl]-[4-(5-chlorooxazolo[4,5-b]pyridin-2-yl)piperazin-1-yl]methanone;
[4-(5-tert-butyl-1,3,4-oxadiazol-2-yl)-3-fluoro-5-hydroxy-phenyl]-[4-(5-methyloxazolo[4,5-b]pyridin-2-yl)piperazin-1-yl]methanone;
[4-(5-tert-butyl-2-methyl-1,2,4-triazol-3-yl)phenyl]-[4-(5-chlorooxazolo[4,5-b]pyridin-2-yl)piperazin-1-yl]methanone;
5-[4-(5-chlorooxazolo[4,5-b]pyridin-2-yl)piperazine-1-carbonyl]-2-[1-(2,2-dimethylpropyl)triazol-4-yl]benzonitrile;
5-[4-(5-chlorooxazolo[4,5-b]pyridin-2-yl)piperazine-1-carbonyl]-2-[2-(2,2-dimethylpropyl)triazol-4-yl]benzonitrile;
[6-(1-tert-butylpyrazol-4-yl)-5-methyl-3-pyridyl]-[4-(5-chlorooxazolo[4,5-b]pyridin-2-yl)piperazin-1-yl]methanone;
[6-(4-tert-butylpyrazol-1-yl)-5-methyl-3-pyridyl]-[4-(5-chlorooxazolo[4,5-b]pyridin-2-yl)piperazin-1-yl]methanone;
[6-(1-tert-butylpyrazol-4-yl)-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-methyl-6-(tetrahydrofuran-2-ylmethoxy)-3-pyridyl]methanone;
[4-(5-tert-butyl-1,3,4-oxadiazol-2-yl)-3-(trifluoromethyl)phenyl]-[4-(5-methyloxazolo[4,5-b]pyridin-2-yl)piperazin-1-yl]methanone;
[4-(5-tert-butyl-1,3,4-oxadiazol-2-yl)-3-(trifluoromethyl)phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[6-methyl-5-[[1-(trifluoromethyl)cyclopropyl]methoxy]pyrazin-2-yl]methanone;
[4-(5-chlorooxazolo[4,5-b]pyridin-2-yl)piperazin-1-yl]-[5-methyl-6-(oxetan-2-ylmethoxy)-3-pyridyl]methanone;
2-[1-(2,2-dimethylpropyl)triazol-4-yl]-5-[4-(5-methyloxazolo[4,5-b]pyridin-2-yl)piperazine-1-carbonyl]benzonitrile;
2-[2-(2,2-dimethylpropyl)triazol-4-yl]-5-[4-(5-methyloxazolo[4,5-b]pyridin-2-yl)piperazine-1-carbonyl]benzonitrile;
[4-(5-chlorooxazolo[4,5-b]pyridin-2-yl)piperazin-1-yl]-[4-[1-(2,2-dimethylpropyl)triazol-4-yl]-3-methoxy-phenyl]methanone;
[4-(5-chlorooxazolo[4,5-b]pyridin-2-yl)piperazin-1-yl]-[4-[2-(2,2-dimethylpropyl)triazol-4-yl]-3-methoxy-phenyl]methanone;
[4-[1-(2,2-dimethylpropyl)triazol-4-yl]-3-methoxy-phenyl]-[4-(5-methyloxazolo[4,5-b]pyridin-2-yl)piperazin-1-yl]methanone;
[4-[2-(2,2-dimethylpropyl)triazol-4-yl]-3-methoxy-phenyl]-[4-(5-methyloxazolo[4,5-b]pyridin-2-yl)piperazin-1-yl]methanone;
[4-(1-tert-butyl-1,2,4-triazol-3-yl)-3-methoxy-phenyl]-[4-(5-methyloxazolo[4,5-b]pyridin-2-yl)piperazin-1-yl]methanone;
[4-(1-tert-butyl-1,2,4-triazol-3-yl)-3-methoxy-phenyl]-[4-(5-chlorooxazolo[4,5-b]pyridin-2-yl)piperazin-1-yl]methanone;
[5-methoxy-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-[(1-fluorocyclopropyl)methyl]triazol-4-yl]phenyl]methanone;
[4-(5-chlorooxazolo[4,5-b]pyridin-2-yl)piperazin-1-yl]-[5-methyl-6-(tetrahydropyran-2-ylmethoxy)-3-pyridyl]methanone;
[4-(5-chlorooxazolo[4,5-b]pyridin-2-yl)piperazin-1-yl]-[6-[(1-methoxycyclopropyl)methoxy]-5-methyl-3-pyridyl]methanone;
[4-[1-[(1-fluorocyclopropyl)methyl]triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[6-[1-(2,2-dimethylpropyl)triazol-4-yl]-5-fluoro-3-pyridyl]methanone;
[4-(5-chlorooxazolo[4,5-b]pyridin-2-yl)piperazin-1-yl]-[6-[3-(2,2-dimethylpropyl)triazol-4-yl]-5-fluoro-3-pyridyl]methanone;
[4-(5-chlorooxazolo[4,5-b]pyridin-2-yl)piperazin-1-yl]-[6-[2-(2,2-dimethylpropyl)triazol-4-yl]-5-fluoro-3-pyridyl]methanone;
[3-[(2-chloro-4-fluoro-phenoxy)methyl]azetidin-1-yl]-[4-(5-chlorooxazolo[4,5-b]pyridin-2-yl)piperazin-1-yl]methanone;
[3-[(2-chloro-4-fluoro-phenoxy)methyl]azetidin-1-yl]-[4-(5-methyloxazolo[4,5-b]pyridin-2-yl)piperazin-1-yl]methanone;
2-chloro-N-[1-[4-(5-chlorooxazolo[4,5-b]pyridin-2-yl)piperazine-1-carbonyl]azetidin-3-yl]-4-fluoro-benzamide;
2-chloro-4-fluoro-N-[1-[4-(5-methyloxazolo[4,5-b]pyridin-2-yl)piperazine-1-carbonyl]azetidin-3-yl]benzamide;
[4-(5-chlorooxazolo[4,5-b]pyridin-2-yl)piperazin-1-yl]-[6-[(2S)-2,3-difluoropropoxy]-5-methyl-3-pyridyl]methanone;
N-(2-chloro-4-fluoro-phenyl)-1-[4-(5-chlorooxazolo[4,5-b]pyridin-2-yl)piperazine-1-carbonyl]azetidine-3-carboxamide;
N-(2-chloro-4-fluoro-phenyl)-1-[4-(5-methyloxazolo[4,5-b]pyridin-2-yl)piperazine-1-carbonyl]azetidine-3-carboxamide;
[4-(5-chlorooxazolo[4,5-b]pyridin-2-yl)piperazin-1-yl]-[5-methyl-6-(3,3,3-trifluoropropoxy)-3 -pyridyl] methanone;
[4-(1-tert-butyltriazol-4-yl)-3-methyl-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[6-[(2R)-2,3-difluoropropoxy]-5-methyl-3 -pyridyl] methanone;
[4-(5-tert-butyl-2-methyl-1,2,4-triazol-3-yl)phenyl]-[4-(5-methyloxazolo[4,5-b]pyridin-2-yl)piperazin-1-yl]methanone;
[6-[1-(2,2-dimethylpropyl)triazol-4-yl]-5-fluoro-3-pyridyl]-[4-(5-methyloxazolo[4,5-b]pyridin-2-yl)piperazin-1-yl]methanone;
[6-[2-(2,2-dimethylpropyl)triazol-4-yl]-5-fluoro-3-pyridyl]-[4-(5-methyloxazolo[4,5-b]pyridin-2-yl)piperazin-1-yl]methanone;
[6-[3-(2,2-dimethylpropyl)triazol-4-yl]-5-fluoro-3-pyridyl]-[4-(5-methyloxazolo[4,5-b]pyridin-2-yl)piperazin-1-yl]methanone;
[4-(5-tert-butyl-1,3,4-oxadiazol-2-yl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-(1H-triazol-5-yl)phenyl] methanone;
[4-(5-tert-butyl-1,3,4-oxadiazol-2-yl)-3-chloro-phenyl]-[4-(5-methyloxazolo[4,5-b]pyridin-2-yl)piperazin-1-yl]methanone;
[6-(3-tert-butyl-1,2,4-triazol-1-yl)-5-methyl-3-pyridyl]-[4-(5-methyloxazolo[4,5-b]pyridin-2-yl)piperazin-1-yl]methanone;
[6-(3-tert-butyl-1,2,4-triazol-1-yl)-5-methyl-3-pyridyl]-[4-(5-chlorooxazolo[4,5-b]pyridin-2-yl)piperazin-1-yl]methanone;
[6-(4-tert-butylpyrazol-1-yl)-5-methyl-3-pyridyl]-[4-(5-methyloxazolo [4, 5-b]pyridin-2-yl)piperazin-1-yl]methanone;
3-[4-[4-[4-(5-chlorooxazolo[4,5-b]pyridin-2-yl)piperazine-1-carbonyl]phenyl]triazol-1-yl]-2,2-dimethyl-propanenitrile;
\[4-(5-chlorooxazolo[4,5-b]pyridin-2-yl)piperazin-1-yl]-[4-[1-(2-fluoro-2-methylpropyl)triazol-4-yl]phenyl]methanone;
[4-[1-(2-fluoro-2-methyl-propyl)triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2-fluoro-1,1-dimethylethyl)triazol-4-yl]phenyl]methanone;
[4-[1-(2-fluoro-1,1-dimethyl-ethyl)triazol-4-yl]phenyl]-[4-(5-methyloxazolo[4,5-b]pyridin-2-yl)piperazin-1-yl]methanone;
3-[4-[4-[4-(5-chlorooxazolo[4,5-b]pyridin-2-yl)piperazine-1-carbonyl]phenyl]triazol-2-yl]-2,2-dimethyl-propanenitrile;
[4-(5-chlorooxazolo[4,5-b]pyridin-2-yl)piperazin-1-yl]-[6-methyl-5-[[1-(trifluoromethyl)cyclopropyl]methoxy]pyrazin-2-yl]methanone;
[4-(5-chlorooxazolo[4,5-b]pyridin-2-yl)piperazin-1-yl]-[6-[1-(2,2-dimethylpropyl)pyrazol-4-yl]-5-methyl-3-pyridyl]methanone;
[6-[1-(2,2-dimethylpropyl)pyrazol-4-yl]-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-methoxy-6-[[1-(trifluoromethyl)cyclopropyl]methoxy]-3-pyridyl]methanone;
[4-(5-chlorooxazolo[4,5-b]pyridin-2-yl)piperazin-1-yl]-[4-[1-(2-methoxy-2-methylpropyl)triazol-4-yl]phenyl]methanone;
[4-(5-chlorooxazolo[4,5-b]pyridin-2-yl)piperazin-1-yl]-[4-(5-isobutyl-1,3,4-oxadiazol-2-yl)phenyl] methanone;
[4-(5-methyloxazolo[4,5-b]pyridin-2-yl)piperazin-1-yl]-[6-methyl-5-[[1-(trifluoromethyl)cyclopropyl]methoxy]-2-pyridyl]methanone;
[4-(5-chlorooxazolo[4,5-b]pyridin-2-yl)piperazin-1-yl]-[6-methyl-5-[[1-(trifluoromethyl)cyclopropyl]methoxy]-2-pyridyl]methanone;
[4-(5-chlorooxazolo[4,5-b]pyridin-2-yl)piperazin-1-yl]-[4-[1-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[4-[2-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[4-[3-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[6-[1-(2,2-dimethylpropyl)triazol-4-yl]-5-methyl-3-pyridyl]methanone;
[4-(5-chlorooxazolo[4,5-b]pyridin-2-yl)piperazin-1-yl]-[6-[2-(2,2-dimethylpropyl)triazol-4-yl]-5-methyl-3-pyridyl]methanone;
[(3R)-4-(5-chlorooxazolo[4,5-b]pyridin-2-yl)-3-methyl-piperazin-1-yl]-[4-[1-(2,2-dimethylpropyl)triazol-4-yl]phenyl]methanone;
[(3S)-4-(5-chlorooxazolo[4,5-b]pyridin-2-yl)-3-methyl-piperazin-1-yl]-[4-[1-(2,2-dimethylpropyl)triazol-4-yl]phenyl]methanone;
[4-(5-chlorooxazolo[4,5-b]pyridin-2-yl)piperazin-1-yl]-[4-(5-isobutyl-1,3,4-oxadiazol-2-yl)-3-methyl-phenyl]methanone;
[4-(5-chlorooxazolo[4,5-b]pyridin-2-yl)piperazin-1-yl]-[2-chloro-6-[[1-(trifluoromethyl)cyclopropyl]methoxy]-3-pyridyl]methanone;
[4-[2-(2,2-dimethylpropyl)tetrazol-5-yl]phenyl]-[4-(5-methyloxazolo[4,5-b]pyridin-2-yl)piperazin-1-yl]methanone;
[6-(5-tert-butyl-1,3,4-oxadiazol-2-yl)-5-fluoro-3-pyridyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[6-[1-(2,2-dimethylpropyl)pyrazol-4-yl]-5-(trifluoromethyl)-3-pyridyl]methanone;
[4-(5-chlorooxazolo[4,5-b]pyridin-2-yl)piperazin-1-yl]-[3-fluoro-4-[5-(2-fluoro-1,1-dimethyl-ethyl)-1,3,4-oxadiazol-2-yl]phenyl]methanone;
[4-(5-tert-butyl-1,3,4-oxadiazol-2-yl)-3-fluoro-phenyl]-[4-(5-chlorooxazolo[4,5-b]pyridin-2-yl)piperazin-1-yl]methanone;
[6-[1-(2,2-dimethylpropyl)pyrazol-4-yl]-5-(trifluoromethyl)-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]phenyl]methanone;
[4-(5-tert-butyl-1,2,4-oxadiazol-3-yl)phenyl]-[8-(5-chlorooxazolo[4,5-b]pyridin-2-yl)-3,8-diazabicyclo[3.2.1]octan-3-yl]methanone;
[4-(5-chlorooxazolo[4,5-b]pyridin-2-yl)-3-(hydroxymethyl)piperazin-1-yl]-[4-[1-(2,2-dimethylpropyl)triazol-4-yl]phenyl]methanone;
[4-[1-(2,2-dimethylpropyl)triazol-4-yl]phenyl]-[4-(5-fluorooxazolo[4,5-b]pyridin-2-yl)piperazin-1-yl]methanone;
[4-(3-tert-butyl-1,2,4-triazol-1-yl)-3-methyl-phenyl]-[4-(5-chlorooxazolo[4,5-b]pyridin-2-yl)piperazin-1-yl]methanone;
[4-(3-tert-butyl-1,2,4-triazol-1-yl)-3-methyl-phenyl]-[4-(5-methyloxazolo [4, 5-b]pyridin-2-yl)piperazin-1-yl]methanone;
[(2S)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone;
[(2R)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone;
[4-(3-tert-butyl-1,2,4-triazol-1-yl)-3-chloro-phenyl]-[4-(5-methyloxazolo[4,5-b]pyridin-2-yl)piperazin-1-yl]methanone;
[4-(3-tert-butyl-1,2,4-triazol-1-yl)-3-chloro-phenyl]-[4-(5-chlorooxazolo [4, 5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)-1,2,4-triazol-3-yl]-3-fluoro-phenyl]methanone;
[4-[1-(2,2-dimethylpropyl)-1,2,4-triazol-3-yl]-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)-2-methyl-piperazin-1-yl]-[4-[1-(2,2-dimethylpropyl)triazol-4-yl]phenyl]methanone;
[2-chloro-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-tert-butyl-1,2,4-oxadiazol-3-yl)phenyl]-[3-(5-chlorooxazolo[4,5-b]pyridin-2-yl)-3,8-diazabicyclo[3.2.1]octan-8-yl]methanone;
[4-(5-chlorooxazolo[4,5-b]pyridin-2-yl)piperazin-1-yl]-[3-fluoro-4-[5-(2-fluoro-2-methylpropyl)-1,3,4-oxadiazol-2-yl]phenyl]methanone;
[4-[2-(2,2-dimethylpropyl)tetrazol-5-yl]-3,5-difluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5 -yl] -3, 5 -difluoro-phenyl] methanone;
[4-[2-(2,2-dimethylpropyl)tetrazol-5-yl]-3-methyl-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]-3-methyl-phenyl]methanone;
[4-[2-(2,2-dimethylpropyl)tetrazol-5-yl]-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]-3-fluoro-phenyl]methanone;
[4-(5-chlorooxazolo[4,5-b]pyridin-2-yl)piperazin-1-yl]-[6-[2-(2,2-dimethylpropyl)tetrazol-5-yl]-5-methoxy-3-pyridyl]methanone;
[6-[2-(2,2-dimethylpropyl)tetrazol-5-yl]-5-methoxy-3-pyridyl]-[4-(5-methyloxazolo[4,5-b]pyridin-2-yl)piperazin-1-yl]methanone;
[6-[2-(2,2-dimethylpropyl)tetrazol-5-yl]-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[6-[2-(2,2-dimethylpropyl)tetrazol-5 -yl] -5 -methyl-3 -pyridyl] methanone;
[6-[2-(2,2-dimethylpropyl)tetrazol-5-yl]-5-fluoro-3-pyridyl]-[4-(5-methyloxazolo[4,5-b]pyridin-2-yl)piperazin-1-yl]methanone; or
[4-(5-chlorooxazolo[4,5-b]pyridin-2-yl)piperazin-1-yl]-[6-[2-(2,2-dimethylpropyl)tetrazol-5 -yl] -5 -fluoro-3 -pyridyl] methanone.

In a preferred embodiment, the present invention provides a compound of formula (I) as described herein, or a pharmaceutically acceptable salt thereof, wherein the compound of formula (I) is:
[4-(5-chlorooxazolo[4,5-b]pyridin-2-yl)piperazin-1-yl]-[4-[1-(2,2-dimethylpropyl)triazol-4-yl]-3-fluoro-phenyl]methanone;
[4-(5-chlorooxazolo[4,5-b]pyridin-2-yl)piperazin-1-yl]-[4-[1-(2,2-dimethylpropyl)pyrazol-4-yl]phenyl]methanone;
[4-(5-chlorooxazolo[4,5-b]pyridin-2-yl)piperazin-1-yl]-[4-[2-(2,2-dimethylpropyl)tetrazol-5-yl]phenyl]methanone;
[4-(5-chlorooxazolo[4,5-b]pyridin-2-yl)piperazin-1-yl]-[4-[2-(2,2-dimethylpropyl)triazol-4-yl]phenyl]methanone;
[4-(5-tert-butyl-1,2,4-oxadiazol-3-yl)-3-methyl-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[5-(2,2-dimethylpropyl)-1,2,4-oxadiazol-3-yl]phenyl]methanone;
[4-[1-(2,2-dimethylpropyl)pyrazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-methyl-6-[[1-(trifluoromethyl)cyclopropyl]methoxy]-3-pyridyl]methanone;
[4-(5-chlorooxazolo[4,5-b]pyridin-2-yl)piperazin-1-yl]-[4-[1-(2,2-dimethylpropyl)triazol-4-yl]-3,5-difluoro-phenyl]methanone;
[4-(5-chlorooxazolo[4,5-b]pyridin-2-yl)piperazin-1-yl]-[4-[1-(2,2-dimethylpropyl)triazol-4-yl]phenyl]methanone;
[4-(5-chlorooxazolo[4,5-b]pyridin-2-yl)piperazin-1-yl]-[4-[1-(2,2-dimethylpropyl)-1,2,4-triazol-3-yl]phenyl]methanone;
[4-(5-tert-butyl-1,2,4-oxadiazol-3-yl)phenyl]-[4-(5-chlorooxazolo[4,5-b]pyridin-2-yl)piperazin-1-yl]methanone;
[4-(5-tert-butyl-1,2,4-oxadiazol-3-yl)-3-fluoro-phenyl]-[4-(5-chlorooxazolo[4,5-b]pyridin-2-yl)piperazin-1-yl]methanone;
[4-(5-tert-butyl-1,3,4-oxadiazol-2-yl)-3-chloro-phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[5-methyl-6-[[1-(trifluoromethyl)cyclopropyl]methoxy]-3-pyridyl]methanone;
[4-(5-tert-butyl-1,3,4-oxadiazol-2-yl)-3-methyl-phenyl]-[4-(5-chlorooxazolo[4,5-b]pyridin-2-yl)piperazin-1-yl]methanone;
[4-[1-(2,2-dimethylpropyl)-1,2,4-triazol-3-yl]phenyl]-[4-(5-methyloxazolo[4,5-b]pyridin-2-yl)piperazin-1-yl]methanone;
[4-(1-tert-butyl-1,2,4-triazol-3-yl)-3-chloro-phenyl]-[4-(5-chlorooxazolo[4,5-b]pyridin-2-yl)piperazin-1-yl]methanone;
[4-(1-tert-butyl-1,2,4-triazol-3-yl)phenyl]-[4-(5-chlorooxazolo[4,5-b]pyridin-2-yl)piperazin-1-yl]methanone;
[4-(1-tert-butylpyrazol-4-yl)phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(5-chlorooxazolo[4,5-b]pyridin-2-yl)-3-methyl-piperazin-1-yl]methanone;
[4-(5-tert-butyl-1,2,4-oxadiazol-3-yl)-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2,2-dimethylpropyl)-1,3,4-oxadiazol-2-yl]phenyl]methanone;
[4-(5-chlorooxazolo[4,5-b]pyridin-2-yl)piperazin-1-yl]-[5-fluoro-6-[[1-(trifluoromethyl)cyclopropyl]methoxy]-3-pyridyl]methanone;
[4-(5-tert-butyl-1,2,4-oxadiazol-3-yl)phenyl]-[4-(5-methyloxazolo[4,5-b]pyridin-2-yl)piperazin-1-yl]methanone;
[4-(5-tert-butyl-1,3,4-oxadiazol-2-yl)-3-methyl-phenyl]-[4-(5-methyloxazolo[4,5-b]pyridin-2-yl)piperazin-1-yl]methanone;
[4-(3-tert-butyl-1,2,4-triazol-1-yl)phenyl]-[4-(5-methyloxazolo[4,5-b]pyridin-2-yl)piperazin-1-yl]methanone;
[4-(5-tert-butyl-1,3,4-oxadiazol-2-yl)phenyl]-[4-(5-chlorooxazolo[4,5-b]pyridin-2-yl)piperazin-1-yl]methanone;
[4-[5-(2-fluoro-1,1-dimethyl-ethyl)-1,2,4-oxadiazol-3-yl]phenyl]-[4-(5-methyloxazolo[4,5-b]pyridin-2-yl)piperazin-1-yl]methanone;
[4-(1-tert-butyl-1,2,4-triazol-3-yl)phenyl]-[4-(5-methyloxazolo[4,5-b]pyridin-2-yl)piperazin-1-yl]methanone; or
[4-[2-(2,2-dimethylpropyl)tetrazol-5-yl]-3-methyl-phenyl]-[4-(5-methyloxazolo[4,5-b]pyridin-2-yl)piperazin-1-yl]methanone.

In a particular embodiment, the present invention provides pharmaceutically acceptable salts of the compounds of formula (I) as described herein, especially hydrochloride salts. In a further particular embodiment, the present invention provides compounds according to formula (I) as described herein.

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H , ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

The preparation of compounds of formula (I) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (I) contain one or more functional groups which are not stable or are reactive under the reaction conditions of one or more reaction steps, appropriate protective groups (as described e.g., in "Protective Groups in Organic Chemistry" by T. W. Greene and P. G. M. Wuts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

The present compounds of formula I can be prepared by reacting an acid of formula 1 with a compound of formula 2 using a coupling reagent, such as HATU or T3P, a suitable base such as DIPEA or TEA in a solvent such as DMF, THF or acetonitrile (Scheme 1).

Intermediates which also fall under formula I (for example 3-10), can also be prepared in this manner.

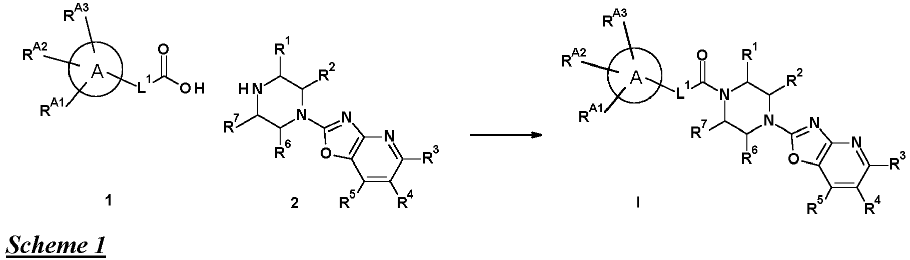

Alternatively, compounds of formula I with A = (substituted) pyridyl, (substituted) pyrimidinyl or (substituted) pyridazinyl were prepared by reacting compounds of formula **3** (X = F, Cl) with either the corresponding alcohol and a suitable base such as NaH or KOtBu as base in a solvent such as DMA, NMP or DMF at elevated temperature if required. Alternatively, if X = OH, compounds of formula I can be generated from the corresponding alcohol by a Mitsunobu reaction (for example, using cyanomethyl tributylphosphorane) (Scheme 2).

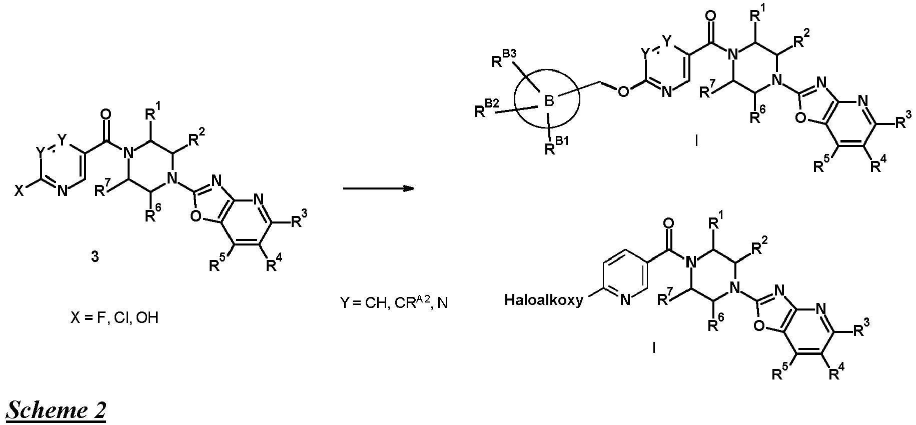

Compounds of formula I with L1 = bond, A = (substituted)aryl or (substituted)heteroaryl, B = N-linked heterocyclyl (such as piperazinyl, (spiro)piperidinyl) can be prepared from a building block **4** (X = Br, I, OTf) and an amine building block via a metal-catalyzed cross-coupling reaction, for example Buchwald conditions (using Pd(OAc)₂, a ligand such as BINAP, a base such as Cs₂CO₃ and a solvent such as toluene at elevated temperatures for several hours under an Argon atmosphere) or Ullmann conditions (using a copper source such as CuI, a ligand such as N,N-dimethyl glycine in a solvent such as DMSO at elevated temperature overnight under an Argon atmosphere). Alternatively, where X= F, Cl, and at least one adjacent Y = N, compounds of formula I can be prepared via a nucleophilic aromatic substitution reaction (for example, with DIPEA, NMP solvent, at 100 °C) (Scheme 3).

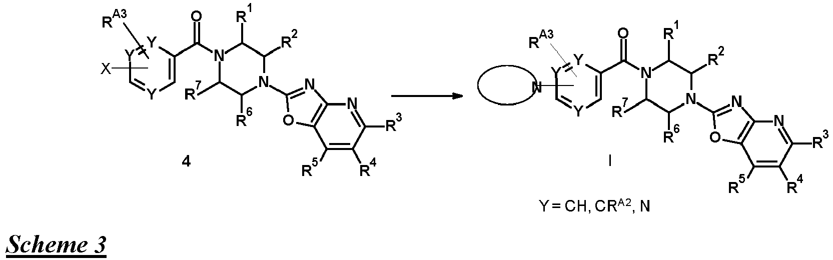

Compounds of formula I with A = (substituted)aryl or (substituted)heteroaryl, B = (hetero)aryl were prepared using a metal-catalysed cross-coupling reaction, for example a Suzuki reaction by reacting a compound of formula 5, where X is a halogen, such as I or Br, with a boronic acid derivative, a catalyst such as Pd(dppf)Cl₂·CH₂Cl₂, a base such as potassium phosphate or Cs₂CO₃ and a solvent such as 1,4-dioxane (Scheme 4).

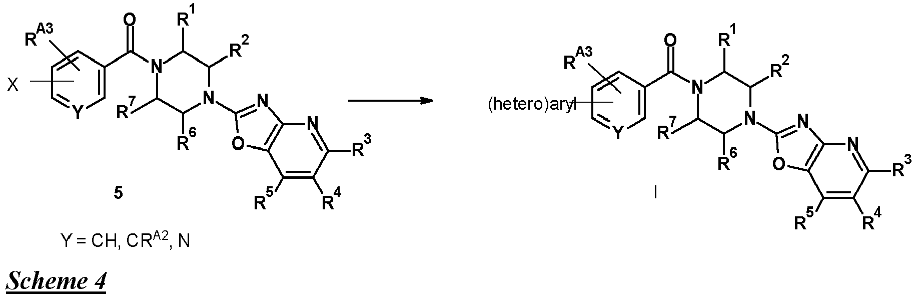

Alternatively, compounds of formula I could be prepared from compounds of formula 6, where X is a halogen, such as I or Br, using a metal-catalyzed cross-coupling reaction (for example reaction with dicyanozinc under Pd-catalysis to give R³ = CN) or a reagent such as (1,10-Phenanthroline)(trifluoromethyl)copper(I) to give R³=CF₃ (Scheme 5).

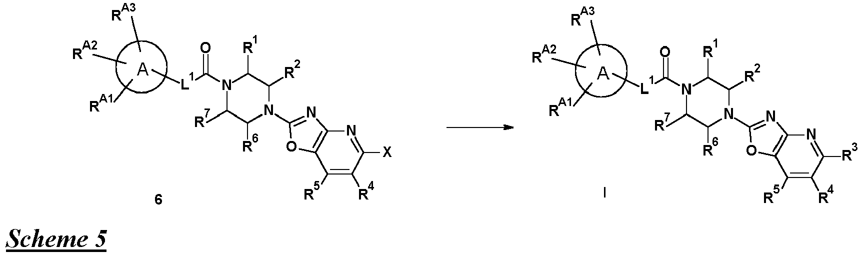

Compounds of formula I with A = (substituted) phenyl or heteroaryl, B = heteroaryl such as triazole or pyrazole could be prepared from an N-H heteroaryl of formula 7 and a suitable alkylating agent R^{B2}-X under basic conditions (for example using an alkyl iodide and K₂CO₃) (Scheme 6).

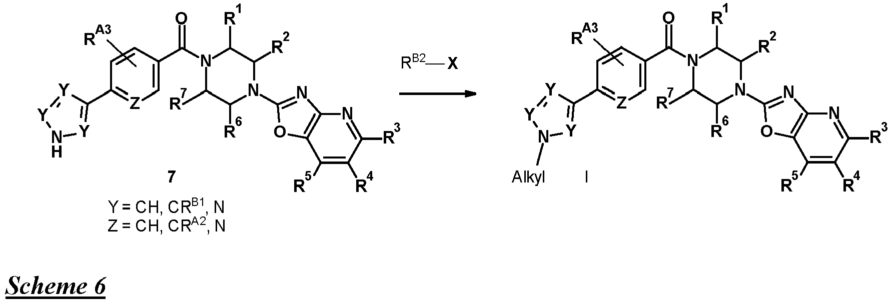

Compounds of formula I with A = azetidinyl or spiroazetidinyl, L2 = alkynyl, -O-CH₂-, - CO-NH- -NH-CO-, -SO₂-, B = (substituted)aryl could be prepared by reaction of a nitrophenyl compound of formula **8** with a suitable amine, a base such as TEA or DIPEA, a catalyst such as DMAP in a solvent such as acetonitrile at elevated temperature and prolonged reaction times. Alternatively, a similar reaction can be carried out using other isolated activated derivatives of compounds of formula **2**, such as a carbonyl chloride (Scheme 7).

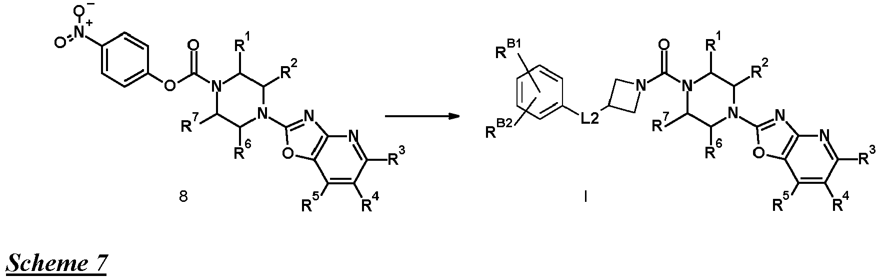

Compounds of formula I with A = (substituted)aryl or (substituted)heteroaryl, and B = triazole could be prepared from an alkyne of formula **9** and a suitable amine R^{B1}-NH₂ via *in situ* formation of the azide intermediate (for example using 2-azido-1,3-dimethyl-4,5-dihydro-1H-imidazol-3-ium hexafluorophosphate(V)) followed by a copper -catalyzed cycloaddition (Scheme 8).

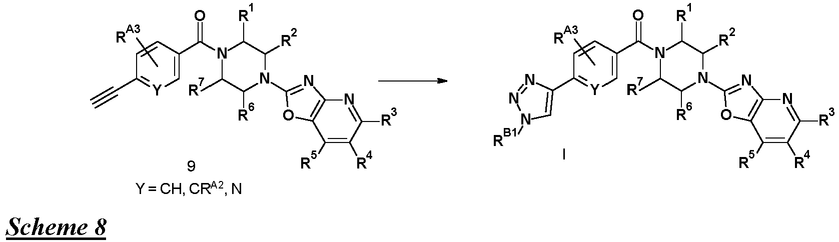

Compounds of formula I with L1 = bond, A = (substituted)phenyl, R^{A1} = alkoxy or haloalkoxy could be prepared from the corresponding phenol **10** using standard techniques, for example reaction with a suitable alkylating agent R-X (X = Cl, Br, I, mesylate) under basic conditions (e.g. NaH) (Scheme 9), or alternatively using Mitsunobu conditions with a suitable alcohol R-OH.

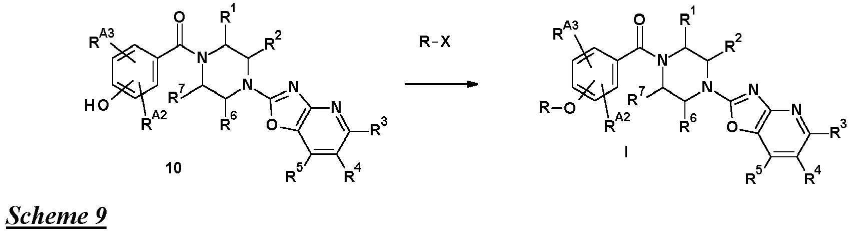

Fluoroalkyl compounds of formula I or the fluorinated ester building blocks of formula **11** could be prepared from suitable alcohol (to give fluoro) or ketone (to give difluoro) intermediates using a fluorinating agent such as (Diethylamino)sulfur trifluoride or XtalFluor-E.

Compounds of Formula I could also be prepared from other compounds of formula I by minor modifications such as reduction of an alkene (e.g. using Pd/C and H₂ gas) or removal of a protecting group under standard techniques. Additional compounds of Formula I or building blocks of formula **11** were sometimes also formed as side products of planned syntheses.

Acid building blocks of formula 1 can be prepared by hydrolysis of the corresponding esters **11** using standard techniques, for example using an aqueous base such as NaOH or LiOH for R= Me, Et or TFA for R = tBu (Scheme 10).

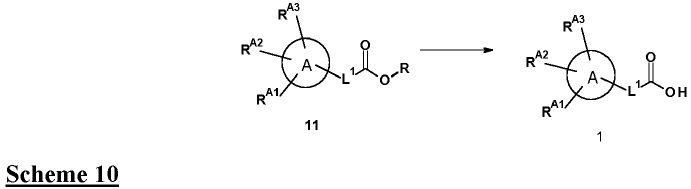

Building blocks of formula **11** with A = (substituted)aryl or (substituted)heteroaryl, B = heteroaryl can be prepared using standard heterocyclic synthesis techniques.

For example, where B = oxadiazole, building blocks **15** can be prepared from condensation of a suitable acid derivative **14** with an alkyl (Z)-4-(N'-hydroxycarbamimidoyl)benzoate **13**, for example using a coupling agent such as EDC/HOBt or CDI. Non-commercial alkyl (Z)-4-(N'-hydroxycarbamimidoyl)benzoates can be generated from the 4-formylbenzoate derivatives **12** using standard techniques (e.g. condensation to the aldoxime, oxidation with NCS, and treatment with ammonium hydroxide). Alternatively, building blocks of formula **15** can be prepared from the 4-bromo-N-hydroxy-benzamidines **17** by condensation with a suitable acid or acid chloride **18** to produce **19**, followed by conversion to the esters **15** using standard techniques, such as a palladium-catalyzed carbonylative cross coupling with MeOH and CO. Non-commercial 4-bromo-N-hydroxy-benzamidines **17** can be generated from 4-cyanoarylbromides **16** using standard techniques (e.g. hydroxylamine hydrochloride/NaOH in EtOH/water) (Scheme 11).

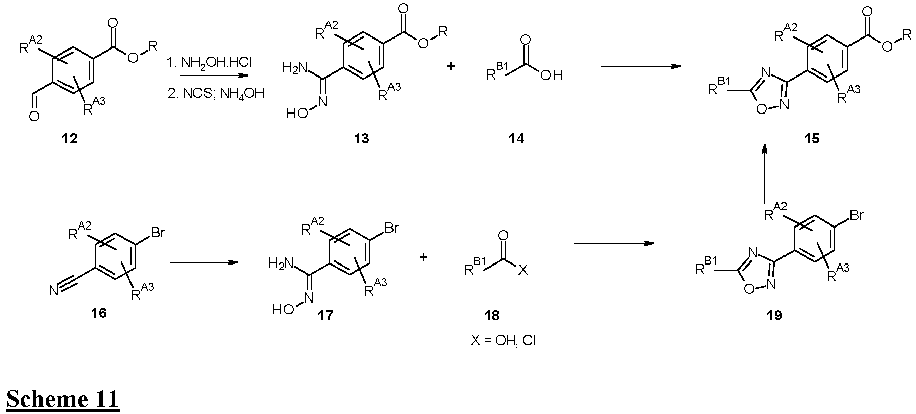

Building blocks of formula **21** with A = (substituted)(hetero)aryl, B = oxadiazole can be prepared by reacting a 4-bromo(hetero)arylhydrazide with an acid chloride to produce an intermediate **23**, which can be further reacted with POCl₃ to form **24**, followed by conversion to the ester **21** using standard techniques, such as a palladium-catalyzed carbonylative cross coupling with MeOH and CO. If required, the acid chlorides R^{B1}-COCl can be prepared from the corresponding carboxylic acids R^{B1}-COOH using standard techniques. Alternatively, the 4-bromo(hetero)arylhydrazide **22** can be condensed with a suitable aldehyde R^{B1}-CHO (e.g. trimethylacetaldehyde) to give imine **20** which could be converted to oxadiazole building block **24** by heating with iodine and K₂CO₃ in DMSO. If required, suitable 4-bromo(hetero)arylhydrazides can be prepared from a suitable 4-bromobenzoic acid, for example by reaction with tert-butyl carbazate to form the Boc-protected 4-bromo(hetero)arylhydrazide followed by removal of the Boc group with HCl/dioxane (Scheme 12).

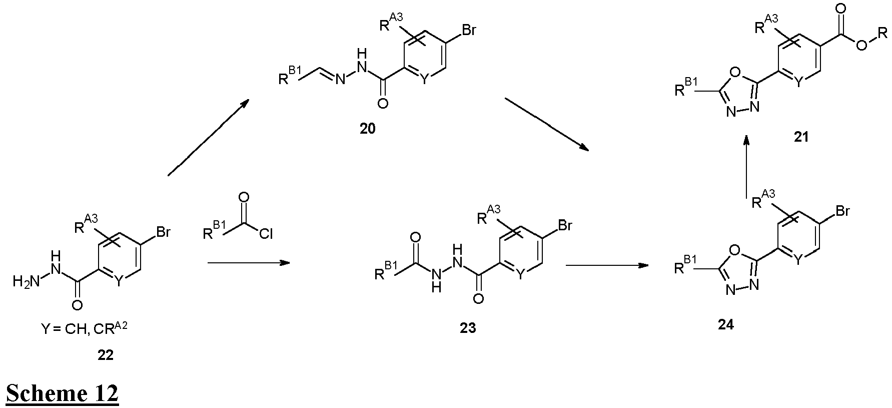

Building blocks of formula **25** with A = (substituted) aryl, B = 4-alkyl-1,2,4-triazole can be prepared by reacting a suitable amide R^{B1}-CONHR^{B2} with oxalyl chloride and an alkyl 4-(hydrazinecarbonyl)benzoate (Scheme 13).

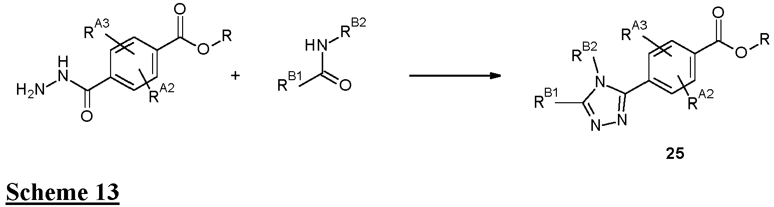

Building blocks of formula **27** with A = substituted (hetero)aryl, B = heteroaryl could be prepared from building blocks **26** and **28** by alkylation with a suitable alkylating agent under basic conditions (e.g. alkyl halide, K₂CO₃), by N,N-dimethylformamide di-tert-butyl acetal in DMF to install the t-Bu group (NH to R^{B1} = tert-butyl), or using Mitsunobu conditions (e.g. an alcohol R^{B1}-OH, PPh₃ and diisopropyl azodicarboxylate). The bromides **29** could be converted to the esters **27** using standard techniques, such as a palladium-catalyzed carbonylative cross coupling with MeOH and CO. (Scheme 14).

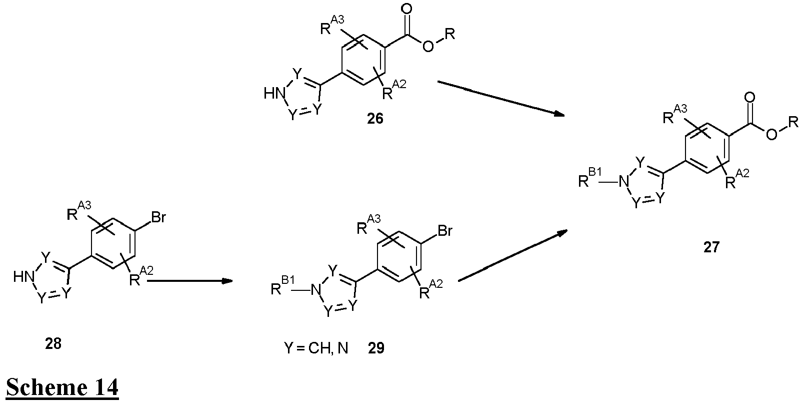

Building blocks of formula **33** with A = (substituted)aryl, B = triazole (one Y being a CH, the other one an N) could be prepared from 4-halobenzoates **30** (X=Br, I) by conversion to the alkyne **31** using ethynyltrimethylsilane under Stille conditions followed by cleavage of the silane (for example by hydrolysis, or using TBAF). The alkynes **31** could be converted to the N-H triazoles **32** using standard conditions (for example reaction with azidotrimethylsilane under copper catalysis with aqueous workup). The triazoles **32** could be converted to **33** (both 1-alkyltriazole and 2-alkyltriazole regioisomers) by alkylation, for example using a base such as K₂CO₃ or NaH and a suitable alkylation agent R^{B1}-X (X = Cl, Br, I, mesylate, triflate) which if not commercial could be generated from the alcohols using standard techniques. Alternatively, the alkyne **31** could be converted directly to **33** (1-alkyltriazole regioisomer) using a suitable amine R^{B1}-NH₂ via in situ formation of the R^{B1}-azide intermediate (for example using 2-azido-1,3-dimethyl-4,5-dihydro-1H-imidazol-3-ium hexafluorophosphate(V)) followed by a copper -catalyzed cycloaddition (Scheme 15).

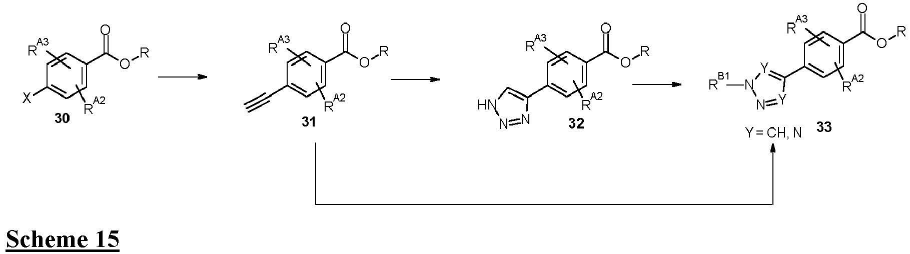

Acetylenes of formula **35** could be prepared from 4-halo(hetero)aryl esters **34** (X=Br, I) by using ethynyltrimethylsilane under Stille conditions followed by hydrolysis (for example with NaOH then HCl, or with TBAF) (Scheme 16).

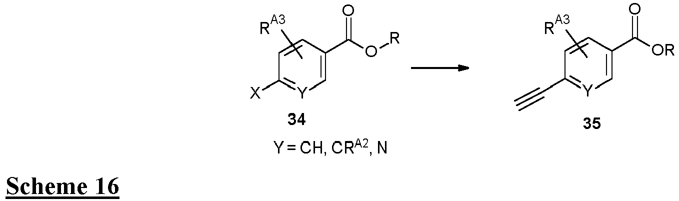

Ethers of formula **37** could be prepared from 4-halo(hetero)aryl alcohols **36** by alkylation using a suitable alcohol R^{A1}-OH in a Mitsunobu reaction (e.g. with diisopropyl azodicarboxylate and triphenylphosphine) or using a suitable alkyl halide, triflate or mesylate R^{A1}-X under basic conditions (e.g. alkyl iodide, K₂CO₃), followed by hydrolysis of the ester to the acid (Scheme 17).

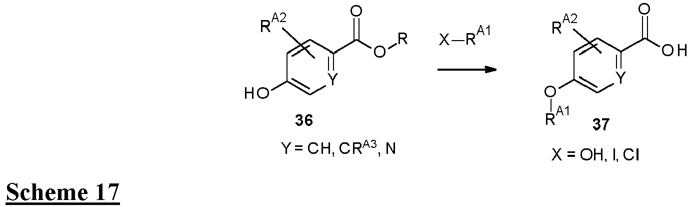

Building blocks of formula **40** with A = substituted (hetero)aryl, B = heteroaryl could be prepared from heteroaryl halides (X = Br, Cl, I) and a suitable coupling partner **39** in a transition metal catalysed cross coupling reaction such as a Suzuki reaction (M= boronic acid derivative) or a Stille reaction (M= stannane) (Scheme 18). The heteroarenes 10d-a were generated by alkylation of N-H heteroarenes using, for example NaH and a suitable alkylation agent R^{B1}-X (X = Cl, Br, I, mesylate) which if not commercial could be generated from the alcohols using standard techniques. Alternatively, certain building blocks **38** could be obtained by alkylation of an amino-heteroaryl precursor (X = NH₂), for example with 2-methylpropan-2-ol in perchloric acid where R^{B1} = tert-Bu, followed by Sandmeyer conditions to obtain the heteroaryl halide.

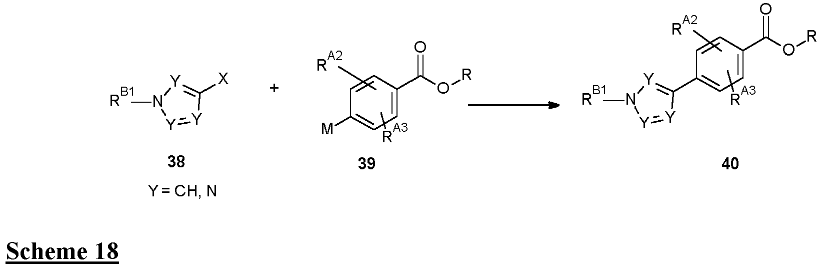

Building blocks of formula **43** with A = substituted (hetero)aryl, B = N-linked heteroaryl could be prepared by reacting a N-H heteroarene **41** with a 4-halo(hetero)aryl carboxylic acid (R = H) or ester (R ≠ H) **42**, either by nucleophilic substitution with a suitable base such as Cs₂CO₃ (X = F) or using a metal-catalyzed cross-coupling, for example copper-catalysis (Scheme 19).

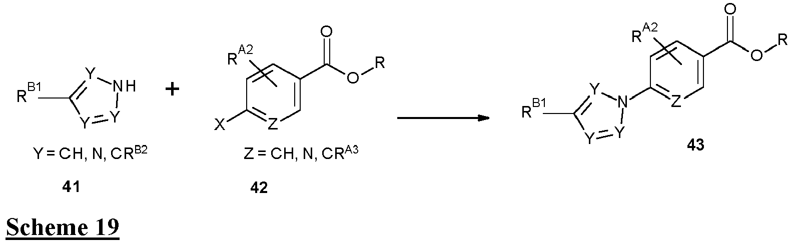

Building blocks of formula **46** with A = substituted (hetero)aryl, B = tetrazole could be generated by reacting a benzoate derivative of formula **45** (X = halogen, triflate) with a 2-alkyl-5-(tributylstannyl)-2H-tetrazole of formula **44** using a metal catalyzed cross coupling such as a Stille reaction (Scheme 20). The 2-alkyl-5-(tributylstannyl)-2H-tetrazoles **44** were generated from 1H-tetrazol-5-amine via alkylation (for example with an alkylating agent such as 1-iodo-2,2-dimethylpropane and a base such as NaH), conversion to the iodide under Sandmeyer conditions, and finally conversion to the stannane (for example by Li-halogen exchange by reaction with nBuLi and attack on tributylchlorostannane).

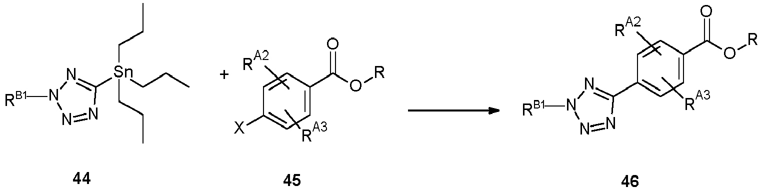

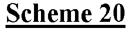

Alternatively, building blocks of formula **46** with A = (substituted)aryl, B = tetrazole could be generated by reacting a 4-cyano-benzoate with sodium azide, followed by alkylation with a suitable alkylating agent R^{B1}-X (for example using an alkyl iodide and NaH) (Scheme 21).

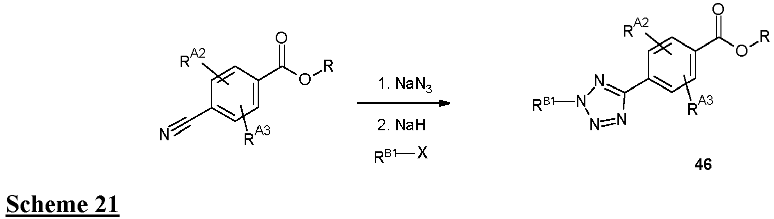

Building blocks of formula **47** can be prepared from tert-butyl 3-ethynylazetidine-1-carboxylate and a suitable (substituted)aryl halide (X = Br, I, OTf) using a metal-catalyzed cross coupling reaction e.g. applying Sonogashira conditions, followed by removal of the protecting group (for example using TFA) (Scheme 22).

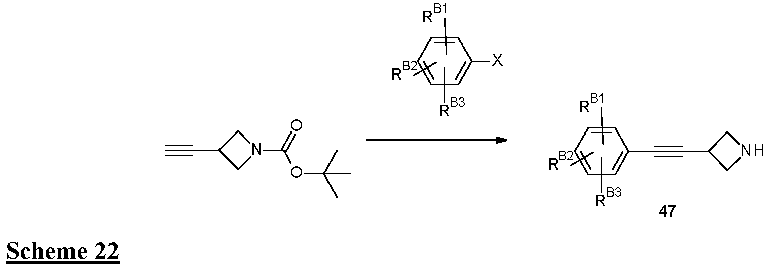

Building blocks of formula **48** can be prepared from a suitable (substituted)heteroaryl halide (X = Cl, Br; Y = N, CH, CR^{A1}) and a suitable alcohol Rⁿ-OH under basic conditions (e.g. NaH) (Scheme 23).

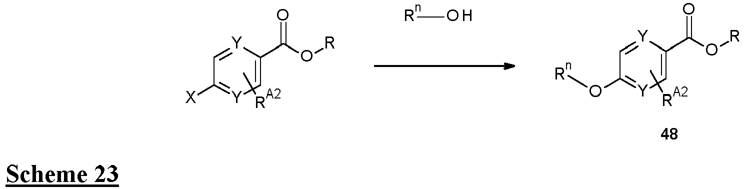

Building blocks of formula 49 can be prepared from a suitable protected heterocyclyl building block and a suitable sulfonyl chloride under basic conditions, followed by deprotection (for example TFA where PG = Boc) (Scheme 24).

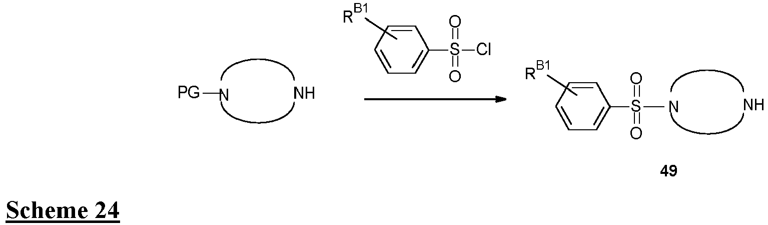

Building blocks of formula 50 can be prepared from a suitable 3-formylbenzoate under standard heterocyclic synthesis techniques, for example condensation with triethylammonium acetate, ammonium acetate and oxaldehyde (Scheme 25).

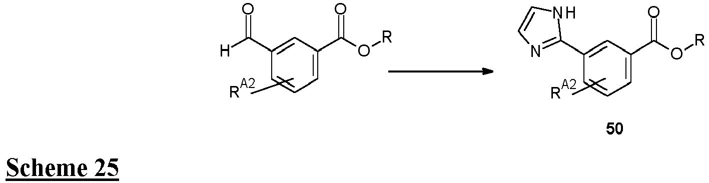

Building blocks of formula **53** can be prepared from a suitable 4-amino-3-hydroxybenzoate derivative **51** and a suitable acid R^{A1}-COOH in a condensation reaction (e.g. using EDCI and HOBt). Alternatively when R^{A1} is a nucleophilic moiety, for example an amine, building blocks of formula **53** can be generated from a suitable 2-(methylthio)benzo[d]oxazole-6-carboxylate derivative **52** under basic conditions (Scheme 26).

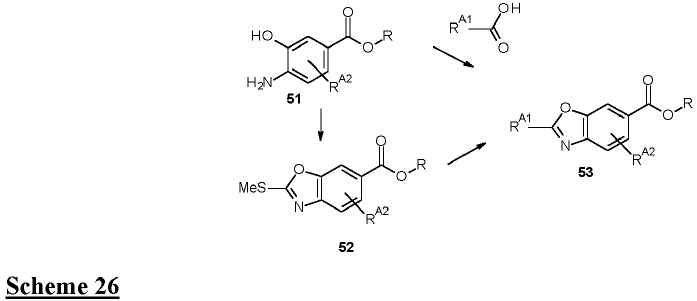

Building blocks of formula **2** can be generated by reacting a piperazine derivative with a 2-substitued oxazolo[4,5-b]pyridine derivative **54**, where X is a leaving group such as Cl or SMe (Scheme 27). Non-commercial 2-substitued oxazolo[4,5-b]pyridine derivatives **54** may be constructed from 2-nitropyridin-3-ol building blocks via reduction to the amine, condensation with a reagent such as 1,1'-thiocarbonyldiimidazole to give a oxazolo[4,5-b]pyridine-2-thione, and conversion to **54** (for example by methylation with MeI or by reaction with oxalyl chloride). Protecting groups, if used, can be removed using standard techniques (e.g. TFA for Boc groups, piperidine for Fmoc).

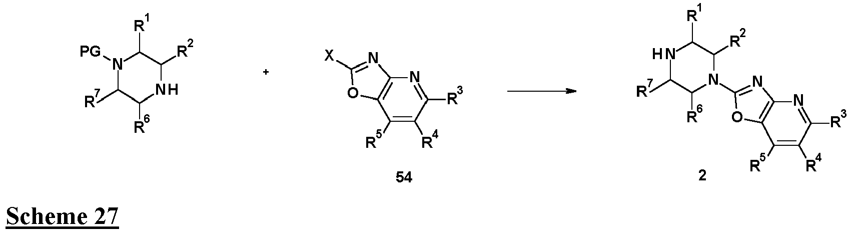

Alternatively, building blocks of formula **2**, can be generated through modification of an lesser-substituted oxazolo[4,5-b]pyridine core **55**, wherein Rⁿ an optional substituent, via formation of the N-oxide (for example using mCPBA), reaction with a reagent such as iodine or oxalyl chloride, and deprotection of the Boc group (for example using TFA) (Scheme 28).

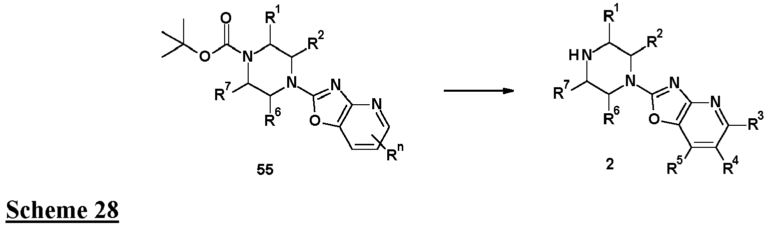

In one aspect, the present invention provides a process of manufacturing the compounds of formula (I) as described in any one of schemes 1 to 28, or a combination thereof.

In a further aspect, the present invention provides a process of manufacturing the compounds of formula (I) as described herein, comprising:
reacting an acid of formula **1**, wherein L¹, A, and R^{A1} to R^{A3} are as defined herein, 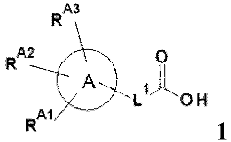
with an amine of formula **2**, wherein R¹ to R⁷ are as defined herein 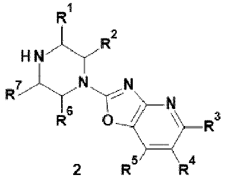 in the presence of a base and a coupling reagent,
to form said compound of formula (I).

In one embodiment, there is provided a process according to the invention, wherein said base is diisopropylethylamine or triethylamine.

In one embodiment, there is provided a process according to the invention, wherein said coupling reagent is HATU or T3P.

In one embodiment, there is provided a process according to the invention, wherein the process is conducted in a solvent, preferably in DMF or THF.

### MAGL Inhibitory Activity

Compounds of the present invention are MAGL inhibitors. Thus, in one aspect, the present invention provides the use of compounds of formula (I) as described herein for inhibiting MAGL in a mammal.

In a further aspect, the present invention provides compounds of formula (I) as described herein for use in a method of inhibiting MAGL in a mammal.

In a further aspect, the present invention provides the use of compounds of formula (I) as described herein for the preparation of a medicament for inhibiting MAGL in a mammal. In a further aspect, the present invention provides a method for inhibiting MAGL in a mammal, which method comprises administering an effective amount of a compound of formula (I) as described herein to the mammal.

Compounds were profiled for MAGL inhibitory activity by measuring the enzymatic activity of MAGL by following the hydrolysis of 4-nitrophenylacetate resulting in 4-nitrophenol, which absorbs at 405-412 nm (G.G. Muccioli, G. Labar, D.M. Lambert, Chem. Bio. Chem. 2008, 9, 2704-2710). This assay is hereinafter abbreviated "4-NPA assay".

The assay was carried out in 384 well assay plates (black with clear bottom, non-binding surface treated, Corning Ref. 3655) in a total volume of 40 µL. Compound dilutions were made in 100% DMSO (VWR Chemicals 23500.297) in a polypropylene plate in 3-fold dilution steps to give a final concentration range in the assay from 25 µM to 1.7 nM. 1 µL compound dilutions (100% DMSO) were added to 19 µL MAGL (recombinant wild-type) in assay buffer (50 mM TRIS (GIBCO, 15567-027), 1 mM EDTA (Fluka, 03690-100mL)). The plate was shaked for 1 min at 2000 rpm (Variomag Teleshake) and then incubated for 15 min at RT. To start the reaction, 20 µL 4-Nitrophenlyacetate (Sigma N-8130) in assay buffer with 6% EtOH was added. The final concentrations in the assay were 1 nM MAGL and 300 µM 4-Nitrophenylacetate. After shaking (1 min, 2000 rpm) and 5 min incubation at RT, the absorbance at 405 nm was measured for a fist time (Molecular Devices, SpectraMax Paradigm). A second measurement was then done after incubation for 80 min at RT. From the two measurements, the slope was calculated by substracting the first from the second measurement.

Alternatively, compounds were profiled for MAGL inhibitory activity by determining the enzymatic activity by following the hydrolysis of the natural substrate 2-arachidonoylglycerol (2-AG) resulting in arachidonic acid, which can be followed by mass spectrometry. This assay is hereinafter abbreviated "2-AG assay".

The 2-AG assay was carried out in 384 well assay plates (PP, Greiner Cat# 784201) in a total volume of 20 µL. Compound dilutions were made in 100% DMSO (VWR Chemicals 23500.297) in a polypropylene plate in 3-fold dilution steps to give a final concentration range in the assay from 12.5 µM to 0.8 pM. 0.25µL compound dilutions (100% DMSO) were added to 9 µL MAGL in assay buffer (50 mM TRIS (GIBCO, 15567-027), 1 mM EDTA (Fluka, 03690-100 mL), 0.01% (v/v) Tween. After shaking, the plate was incubated for 15 min at RT. To start the reaction, 10 µL 2-arachidonoylglycerol in assay buffer was added. The final concentrations in the assay was 50 pM MAGL and 8 µM 2-arachidonoylglyerol. After shaking and 30 min incubation at RT, the reaction was quenched by the addition of 40µL of ACN containing 4µM of d8-arachidonic acid. The amount of arachidonic acid was traced by an online SPE system (Agilent Rapidfire) coupled to a triple quadrupole mass spectrometer (Agilent 6460). A C18 SPE cartridge (G9205A) was used in an ACN/water liquid setup. The mass spectrometer was operated in negative electrospray mode following the mass transitions 303.1 → 259.1 for arachidonic acid and 311.1 → 267.0 for d8-arachidonic acid. The activity of the compounds was calculated based on the ratio of intensities [arachidonic acid / d8-arachidonic acid].

**Table 1**

| **Example** | **Systematic Name** | **IC50 MAGL [µM]^{[a]}** |
|---|---|---|
| 1 | (4-(5-methyloxazolo[4,5-b]pyridin-2-yl)piperazin-1-yl)(2-phenylbenzo[d]oxazol-6-yl)methanone | 0.115 |
| 2 | (4-(oxazolo [4,5-b]pyridin-2-yl)piperazin-1-yl)(2-phenylbenzo[d]oxazol-6-yl)methanone | 0.194 |
| 3 | (4-(5-methyloxazolo[4,5-b]pyridin-2-yl)piperazin-1-yl)(2-(pyrrolidin-1-yl)benzo[d]oxazol-6-yl)methanone | 2.330 |
| 4 | (4-(5-(tert-butyl)-1,2,4-oxadiazol-3-yl)phenyl)(4-(5-methyloxazolo[4,5-b]pyridin-2-yl)piperazin-1-yl)methanone | 0.027 |
| 5 | (4-(5-methyloxazolo[4,5-b]pyridin-2-yl)piperazin-1-yl)(4-(5-neopentyl-1,2,4-oxadiazol-3-yl)phenyl)methanone | 0.009 |
| 6 | (2-(2-azaspiro[3.4]octan-2-yl)benzo[d]oxazol-6-yl)(4-(5-methyloxazolo[4,5-b]pyridin-2-yl)piperazin-1-yl)methanone | 0.025 |
| 7 | (4-(5-(1-hydroxy-2-methylpropan-2-yl)-1,2,4-oxadiazol-3-yl)phenyl)(4-(5-methyloxazolo[4,5-b]pyridin-2-yl)piperazin-1-yl)methanone | 0.262 |
| 8 | (4-(5-(tert-butyl)-1,2,4-oxadiazol-3-yl)phenyl)(4-(7-chlorooxazolo[4,5-b]pyridin-2-yl)piperazin-1-yl)methanone | 0.247 |
| 9 | (4-(7-chlorooxazolo[4,5-b]pyridin-2-yl)piperazin-1-yl)(4-(5-neopentyl-1,2,4-oxadiazol-3-yl)phenyl)methanone | 0.027 |
| 10 | (4-(5-chlorooxazolo[4,5-b]pyridin-2-yl)piperazin-1-yl)(4-(5-neopentyl-1,2,4-oxadiazol-3 -yl)phenyl)methanone | 0.002 |
| 11 | (4-(5-(1-fluoro-2-methylpropan-2-yl)-1,2,4-oxadiazol-3-yl)phenyl)(4-(5-methyloxazolo[4,5-b]pyridin-2-yl)piperazin-1-yl)methanone | 0.049 |
| 12 | (4-(5-(tert-butyl)-1,2,4-oxadiazol-3-yl)phenyl)(4-(5-chlorooxazolo[4,5-b]pyridin-2-yl)piperazin-1-yl)methanone | 0.008 |
| 13 | (4-(5-neopentyl-1,2,4-oxadiazol-3-yl)phenyl)(4-(oxazolo[4,5-b]pyridin-2-yl)piperazin-1-yl)methanone | 0.037 |
| 14 | (4-(5-(tert-butyl)-1,2,4-oxadiazol-3-yl)phenyl)(4-(oxazolo[4,5- b]pyridin-2-yl)piperazin-1-yl)methanone | 0.128 |
| 15 | [2-(1-ethylpropyl)-1,3-benzoxazol-6-yl]-[4-(5-methyloxazolo[4,5-b]pyridin-2-yl)piperazin-1-yl]methanone | 0.352 |
| 16 | [4-(5-chlorooxazolo[4,5-b]pyridin-2-yl)piperazin-1-yl]-[4-[5-(2,2-dimethylpropyl)-1,3,4-oxadiazol-2-yl]phenyl]methanone | 0.010 |
| 17 | (2-cyclohexyl-1,3-benzoxazol-6-yl)-[4-(5-methyloxazolo[4,5-b]pyridin-2-yl)piperazin-1-yl]methanone | 0.036 |
| 18 | [4-(5-methyloxazolo[4,5-b]pyridin-2-yl)piperazin-1-yl]-[2-(3,3,3-trifluoropropyl)-1,3-benzoxazol-6-yl]methanone | 0.434 |
| 19 | [4-(5-chlorooxazolo[4,5-b]pyridin-2-yl)piperazin-1-yl]-[4-[1-(2,2-dimethylpropyl)pyrazol-4-yl]phenyl]methanone | 0.002 |
| 20 | (4-(5-chlorooxazolo[4,5-b]pyridin-2-yl)piperazin-1-yl)(4-(5-(1-fluoro-2-methylpropan-2-yl)-1,2,4-oxadiazol-3-yl)phenyl)methanone | 0.019 |
| 21 | [2-[(E)-but-1-enyl]-1,3-benzoxazol-6-yl]-[4-(5-methyloxazolo[4,5-b]pyridin-2-yl)piperazin-1-yl]methanone | 0.184 |
| 22 | (2-cyclopropyl-1,3-benzoxazol-6-yl)-[4-(5-methyloxazolo[4,5-b]pyridin-2-yl)piperazin-1-yl]methanone | 1.254 |
| 23 | 1-[3-[4-[4-(5-chlorooxazolo[4,5-b]pyridin-2-yl)piperazine-1-carbonyl]phenyl]-1,2,4-oxadiazol-5-yl]propan-2-one | 1.547 |
| 24 | [4-(5-chlorooxazolo[4,5-b]pyridin-2-yl)piperazin-1-yl]-[4-[5-(1-fluoro-1-methyl-ethyl)-1,2,4-oxadiazol-3 -yl]phenyl]methanone | 0.074 |
| 25 | [4-(5-chlorooxazolo[4,5-b]pyridin-2-yl)piperazin-1-yl]-[4-[3-(2,2-dimethylpropyl)-1,2,4-oxadiazol-5-yl]phenyl]methanone | 0.003 |
| 26 | [4-(5-chlorooxazolo[4,5-b]pyridin-2-yl)piperazin-1-yl]-[4-[5-(1-fluorocyclopropyl)-1,2,4-oxadiazol-3-yl]phenyl]methanone | 0.206 |
| 27 | [4-(5-chlorooxazolo[4,5-b]pyridin-2-yl)piperazin-1-yl]-[4-[1-(2,2-dimethylpropyl)-1,2,4-triazol-3-yl]phenyl]methanone | 0.003 |
| 28 | [4-[1-(2,2-dimethylpropyl)-1,2,4-triazol-3-yl]phenyl]-[4-(5-methyloxazolo[4,5-b]pyridin-2-yl)piperazin-1-yl]methanone | 0.012 |
| 29 | [4-(5-chlorooxazolo[4,5-b]pyridin-2-yl)piperazin-1-yl]-[4-[1-(2,2-dimethylpropyl)imidazol-4-yl]phenyl]methanone | 0.003 |
| 30 | [4-(5-tert-butyl-1,3,4-oxadiazol-2-yl)phenyl]-[4-(5-chlorooxazolo[4,5-b]pyridin-2-yl)piperazin-1-yl]methanone; 2,2,2-trifluoroacetic acid | 0.068 |
| 31 | [4-(5-tert-butyl-1,3,4-oxadiazol-2-yl)phenyl]-[4-(5-methyloxazolo[4,5-b]pyridin-2-yl)piperazin-1-yl]methanone | 0.260 |
| 32 | [4-(5-chlorooxazolo[4,5-b]pyridin-2-yl)piperazin-1-yl]-[4-[5-(2-hydroxy-2-methyl-propyl)-1,2,4-oxadiazol-3-yl]phenyl]methanone | 0.653 |
| 33 | [4-[5-(2-fluoro-1,1-dimethyl-ethyl)-1,3,4-oxadiazol-2-yl]phenyl]-[4-(5-methyloxazolo[4,5-b]pyridin-2-yl)piperazin-1-yl]methanone | 0.498 |
| 34 | [4-(5-chlorooxazolo[4,5-b]pyridin-2-yl)piperazin-1-yl]-[4-[5-(2-fluoro-1,1-dimethyl-ethyl)-1,3,4-oxadiazol-2-yl]phenyl]methanone; 2,2,2-trifluoroacetic acid | 0.176 |
| 35 | [4-[5-(1-fluorocyclopropyl)-1,2,4-oxadiazol-3-yl]phenyl]-[4-(5-methyloxazolo[4,5-b]pyridin-2-yl)piperazin-1-yl]methanone | 0.369 |
| 36 | [4-[5-[1-(fluoromethyl)cyclopropyl]-1,2,4-oxadiazol-3-yl]phenyl]-[4-(5-methyloxazolo[4,5-b]pyridin-2-yl)piperazin-1-yl]methanone | 0.257 |
| 37 | [4-(5-chlorooxazolo[4,5-b]pyridin-2-yl)piperazin-1-yl]-[4-[5-[1-(fluoromethyl)cyclopropyl]-1,2,4-oxadiazol-3-yl]phenyl]methanone | 0.101 |
| 38 | [4-(3-tert-butyl-1,2,4-oxadiazol-5-yl)phenyl]-[4-(5-methyloxazolo[4,5-b]pyridin-2-yl)piperazin-1-yl]methanone | 0.045 |
| 39 | [4-(3-tert-butyl-1,2,4-oxadiazol-5-yl)phenyl]-[4-(5-chlorooxazolo[4,5-b]pyridin-2-yl)piperazin-1-yl]methanone | 0.019 |
| 40 | [4-(5-chlorooxazolo[4,5-b]pyridin-2-yl)piperazin-1-yl]-[4-[5-[(1-methylcyclopropyl)methyl]-1,2,4-oxadiazol-3- yl]phenyl]methanone | 0.006 |
| 41 | [4-(1-tert-butyl-1,2,4-triazol-3-yl)phenyl]-[4-(5-chlorooxazolo[4,5-b]pyridin-2-yl)piperazin-1-yl]methanone | 0.029 |
| 42 | [4-(1-tert-butyl-1,2,4-triazol-3-yl)phenyl]-[4-(5-methyloxazolo[4,5-b]pyridin-2-yl)piperazin-1-yl]methanone | 0.086 |
| 43 | [4-(5-chlorooxazolo[4,5-b]pyridin-2-yl)piperazin-1-yl]-[4-[1-(2,2-dimethylpropyl)triazol-4-yl]phenyl]methanone | 0.006 |
| 44 | [4-(5-chlorooxazolo[4,5-b]pyridin-2-yl)piperazin-1-yl]-[4-[2-(2,2-dimethylpropyl)triazol-4-yl]phenyl]methanone | 0.003 |
| 45 | [4-(5-tert-butyl-1,2,4-oxadiazol-3-yl)phenyl]-[4-(5-chlorooxazolo[4,5-b]pyridin-2-yl)-2-methyl-piperazin-1-yl]methanone | 0.072 |
| 46 | [4-(5-tert-butyl-1,2,4-oxadiazol-3-yl)phenyl]-[4-(5-chlorooxazolo[4,5-b]pyridin-2-yl)-3-methyl-piperazin-1-yl]methanone | 0.028 |
| 47 | [4-(1-tert-butylpyrazol-4-yl)phenyl]-[4-(5-methyloxazolo[4,5-b]pyridin-2-yl)piperazin-1-yl]methanone | 0.037 |
| 48 | [4-(1-tert-butylpyrazol-4-yl)phenyl]-[4-(5-chlorooxazolo[4,5-b]pyridin-2-yl)piperazin-1-yl]methanone | 0.015 |
| 49 | [4-(5-chlorooxazolo[4,5-b]pyridin-2-yl)piperazin-1-yl]-[4-[1-[(1-methylcyclopropyl)methyl]-1,2,4-triazol-3-yl]phenyl]methanone | 0.013 |
| 50 | [4-(5-chlorooxazolo[4,5-b]pyridin-2-yl)piperazin-1-yl]-[4-[2-[(1-methylcyclopropyl)methyl]-1,2,4-triazol-3-yl]phenyl]methanone | 4.230 |
| 51 | [4-[5-(1-methylcyclopropyl)-1,2,4-oxadiazol-3-yl]phenyl]-[4-(5-methyloxazolo[4,5-b]pyridin-2-yl)piperazin-1-yl]methanone | 0.067 |
| 52 | [4-(4-tert-butylpyrazol-1-yl)phenyl]-[4-(5-chlorooxazolo[4,5-b]pyridin-2-yl)piperazin-1-yl]methanone | 0.005 |
| 53 | [4-(4-tert-butylpyrazol-1-yl)phenyl]-[4-(5-methyloxazolo[4,5-b]pyridin-2-yl)piperazin-1-yl]methanone | 0.012 |
| 54 | [4-[1-(2,2-dimethylpropyl)pyrazol-4-yl]phenyl]-[4-(5-methyloxazolo[4,5-b]pyridin-2-yl)piperazin-1-yl] methanone | 0.006 |
| 55 | [4-(4-tert-butylimidazol-1-yl)phenyl]-[4-(5-methyloxazolo[4,5- b]pyridin-2-yl)piperazin-1-yl]methanone | 0.063 |
| 56 | [4-(5-tert-butyl-1,2,4-oxadiazol-3-yl)-3-methyl-phenyl]-[4-(5-methyloxazolo[4,5-b]pyridin-2-yl)piperazin-1-yl]methanone | 0.011 |
| 57 | [4-(5-tert-butyl-1,2,4-oxadiazol-3-yl)-3-methyl-phenyl]-[4-(5-chlorooxazolo[4,5-b]pyridin-2-yl)piperazin-1-yl]methanone | 0.005 |
| 58 | [4-(5-tert-butyl-1,2,4-oxadiazol-3-yl)-3-fluoro-phenyl]-[4-(5-methyloxazolo[4,5-b]pyridin-2-yl)piperazin-1-yl]methanone | 0.043 |
| 59 | [4-(5-tert-butyl-1,2,4-oxadiazol-3-yl)-3-fluoro-phenyl]-[4-(5-chlorooxazolo[4,5-b]pyridin-2-yl)piperazin-1-yl]methanone | 0.015 |
| 60 | [3-chloro-4-[(1-methylcyclopropyl)methoxy]phenyl]-[4-(5-chlorooxazolo[4,5-b]pyridin-2-yl)piperazin-1-yl]methanone | 0.046 |
| 61 | [6-(5-tert-butyl-1,2,4-oxadiazol-3-yl)-3-pyridyl]-[4-(5-chlorooxazolo[4,5-b]pyridin-2-yl)piperazin-1-yl]methanone | 0.272 |
| 62 | [6-(5-tert-butyl-1,2,4-oxadiazol-3-yl)-3-pyridyl]-[4-(5-methyloxazolo[4,5-b]pyridin-2-yl)piperazin-1-yl]methanone | 0.871 |
| 63 | [4-(4-tert-butylimidazol-1-yl)phenyl]-[4-(5-chlorooxazolo[4,5 - b]pyridin-2-yl)piperazin-1-yl]methanone | 0.020 |
| 64 | [4-(5-chlorooxazolo[4,5-b]pyridin-2-yl)piperazin-1-yl]-[4-(2,2-dimethylpropoxy)-3-methyl-phenyl]methanone | 0.005 |
| 65 | [4-(2,2-dimethylpropoxy)-3-methyl-phenyl]-[4-(5-methyloxazolo[4,5-b]pyridin-2-yl)piperazin-1-yl]methanone | 0.011 |
| 66 | [3-chloro-4-[(1-methylcyclopropyl)methoxy]phenyl]-[4-(5-methyloxazolo[4,5-b]pyridin-2-yl)piperazin-1-yl]methanone | 0.087 |
| 67 | (4-(5-methyloxazolo[4,5-b]pyridin-2-yl)piperazin-1-yl)(4-(3-methyloxetan-3-yl)phenyl)methanone | 0.367 |
| 68 | (4-(1-fluorocyclopropyl)phenyl)(4-(5-methyloxazolo[4,5- b]pyridin-2-yl)piperazin-1-yl)methanone | 4.181 |
| 69 | [4-(5-chlorooxazolo[4,5-b]pyridin-2-yl)piperazin-1-yl]-[3-fluoro-4-[(1-methylcyclopropyl)methoxy]phenyl]methanone | 0.108 |
| 70 | [4-(3-tert-butyl-1,2,4-triazol-1-yl)phenyl]-[4-(5 - methyloxazolo[4,5-b]pyridin-2-yl)piperazin-1-yl]methanone | 0.043 |
| 71 | [3-fluoro-4-[(1-methylcyclopropyl)methoxy]phenyl]-[4-(5-methyloxazolo[4,5-b]pyridin-2-yl)piperazin-1-yl]methanone | 0.382 |
| 72 | [4-(3-tert-butyl-1,2,4-triazol-1-yl)phenyl]-[4-(5- chlorooxazolo[4,5-b]pyridin-2-yl)piperazin-1-yl]methanone | 0.027 |
| 73 | [4-(5-chlorooxazolo[4,5-b]pyridin-2-yl)piperazin-1-yl]-[4-[5-(2,2-dimethylpropyl)-4-methyl-1,2,4-triazol-3-yl]phenyl]methanone | 0.409 |
| 74 | [4-[5-(2,2-dimethylpropyl)-4-methyl-1,2,4-triazol-3-yl]phenyl]-[4-(5-methyloxazolo[4,5-b]pyridin-2-yl)piperazin-1-yl] methanone | 0.327^{[b]} |
| 75 | [4-[1-(2,2-dimethylpropyl)triazol-4-yl]phenyl]-[4-(5-methyloxazolo[4,5-b]pyridin-2-yl)piperazin-1-yl]methanone | 0.019^{[b]} |
| 76 | [4-(5-tert-butyl-4-methyl-1,2,4-triazol-3-yl)phenyl]-[4-(5-chlorooxazolo[4,5-b]pyridin-2-yl)piperazin-1-yl]methanone | 1.310^{[b]} |
| 77 | [4-(5-tert-butyl-4-methyl-1,2,4-triazol-3-yl)phenyl]-[4-(5-methyloxazolo[4,5-b]pyridin-2-yl)piperazin-1-yl]methanone | 2.323^{[b]} |
| 78 | [4-(5-tert-butyl-1,2,4-oxadiazol-3-yl)-3-chloro-phenyl]-[4-(5-chlorooxazolo[4,5-b]pyridin-2-yl)piperazin-1-yl]methanone | 0.004^{[b]} |
| 79 | [4-(5-tert-butyl-1,2,4-oxadiazol-3-yl)-3-chloro-phenyl]-[4-(5-methyloxazolo[4,5-b]pyridin-2-yl)piperazin-1-yl]methanone | 0.024^{[b]} |
| 80 | [4-(5-tert-butyl-1,2,4-oxadiazol-3-yl)-3,5-difluoro-phenyl]-[4-(5-chlorooxazolo[4,5-b]pyridin-2-yl)piperazin-1-yl]methanone | 0.021^{[b]} |
| 81 | [4-(5-tert-butyl-1,2,4-oxadiazol-3-yl)-3-(trifluoromethyl)phenyl]-[4-(5-chlorooxazolo[4,5-b]pyridin-2-yl)piperazin-1-yl]methanone | 0.009^{[b]} |
| 82 | [4-(5-tert-butyl-1,3,4-oxadiazol-2-yl)-3-methyl-phenyl]-[4-(5-methyloxazolo[4,5-b]pyridin-2-yl)piperazin-1-yl]methanone | 0.037^{[b]} |
| 83 | [4-(5-tert-butyl-1,3,4-oxadiazol-2-yl)-3-methyl-phenyl]-[4-(5-chlorooxazolo[4,5-b]pyridin-2-yl)piperazin-1-yl]methanone | 0.011^{[b]} |
| 84 | (4-(5-chlorooxazolo[4,5-b]pyridin-2-yl)piperazin-1-yl)(3-fluoro-4-(2-neopentyl-2H-1,2,3-triazol-4-yl)phenyl)methanone | 0.002^{[b]} |
| 85 | (3-fluoro-4-(2-neopentyl-2H-1,2,3-triazol-4-yl)phenyl)(4-(5- methyloxazolo[4,5-b]pyridin-2-yl)piperazin-1-yl)methanone | 0.005^{[b]} |
| 86 | (4-(5-chlorooxazolo[4,5-b]pyridin-2-yl)piperazin-1-yl)(3-fluoro-4-(1-neopentyl-1H-1,2,3-triazol-4-yl)phenyl)methanone (4-(5-chlorooxazolo[4,5-b]pyridin-2-yl)piperazin-1-yl)(3-fluoro-4-(1-neopentyl-1H-1,2,3-triazol-4-yl)phenyl)methanone | 0.002^{[b]} |
| 87 | (4-(1-(tert-butyl)-1H-1,2,3-triazol-4-yl)phenyl)(4-(5-methyloxazolo[4,5-b]pyridin-2-yl)piperazin-1-yl)methanone | 0.151^{[b]} |
| 88 | (4-(1-(tert-butyl)-1H-1,2,3-triazol-4-yl)phenyl)(4-(5-chlorooxazolo[4,5-b]pyridin-2-yl)piperazin-1-yl)methanone | 0.071^{[b]} |
| 89 | (4-(1-(tert-butyl)-1H-1,2,4-triazol-3-yl)-3-chlorophenyl)(4-(5-methyloxazolo[4,5-b]pyridin-2-yl)piperazin-1-yl)methanone | 0.025^{[b]} |
| 90 | (4-(1-(tert-butyl)-1H-1,2,4-triazol-3-yl)-3-chlorophenyl)(4-(5-chlorooxazolo[4,5-b]pyridin-2-yl)piperazin-1-yl)methanone | 0.016^{[b]} |
| 91 | (4-(5-chlorooxazolo[4,5-b]pyridin-2-yl)piperazin-1-yl)(3,5-difluoro-4-(2-neopentyl-2H-1,2,3-triazol-4-yl)phenyl)methanone | 0.003^{[b]} |
| 92 | (3,5-difluoro-4-(2-neopentyl-2H-1,2,3-triazol-4-yl)phenyl)(4-(5-methyloxazolo[4,5-b]pyridin-2-yl)piperazin-1-yl)methanone | 0.010^{[b]} |
| 93 | (4-(5-chlorooxazolo[4,5-b]pyridin-2-yl)piperazin-1-yl)(3,5-difluoro-4-(1-neopentyl-1H-1,2,3-triazol-4-yl)phenyl)methanone | 0.007^{[b]} |
| 94 | (4-(1-(bicyclo[1.1.1]pentan-1-yl)-1H-1,2,3-triazol-4-yl)phenyl)(4-(5-methyloxazolo[4,5-b]pyridin-2-yl)piperazin-1-yl)methanone | 0.088^{[b]} |
| 95 | (4-(1-(bicyclo[1.1.1]pentan-1-yl)-1H-1,2,3-triazol-4-yl)phenyl)(4-(5-chlorooxazolo[4,5-b]pyridin-2-yl)piperazin-1-yl)methanone | 0.042^{[b]} |
| 96 | (4-(1-(tert-butyl)-1H-1,2,4-triazol-3-yl)-3-methylphenyl)(4-(5-methyloxazolo[4,5-b]pyridin-2-yl)piperazin-1-yl)methanone | 0.023^{[b]} |
| 97 | (4-(1-(tert-butyl)-1H-1,2,4-triazol-3-yl)-3-methylphenyl)(4-(5-chlorooxazolo[4,5-b]pyridin-2-yl)piperazin-1-yl)methanone | 0.012^{[b]} |
| 98 | (4-(5-chlorooxazolo[4,5-b]pyridin-2-yl)piperazin-1-yl)(4-(1-(2,2,2-trifluoroethyl)-1H-1,2,3-triazol-4-yl)phenyl)methanone | 0.144^{[b]} |
| 99 | (4-(5-methyloxazolo[4,5-b]pyridin-2-yl)piperazin-1-yl)(4-(1-(2,2,2-trifluoroethyl)-1H-1,2,3-triazol-4-yl)phenyl)methanone | 0.523^{[b]} |
| 100 | (4-(5-methyloxazolo[4,5-b]pyridin-2-yl)piperazin-1-yl)(4-(1-((3-methyloxetan-3-yl)methyl)-1H-1,2,3-triazol-4-yl)phenyl)methanone | 1.080^{[b]} |
| 101 | (4-(5-chlorooxazolo[4,5-b]pyridin-2-yl)piperazin-1-yl)(4-(1-((3-methyloxetan-3-yl)methyl)-1H-1,2,3-triazol-4-yl)phenyl)methanone | 0.609^{[b]} |
| 102 | 1-(4-(5-chlorooxazolo[4,5-b]pyridin-2-yl)piperazin-1-yl)-3-phenylprop-2-yn-1-one | 0.932^{[b]} |
| 103 | 1-(4-(5-chlorooxazolo[4,5-b]pyridin-2-yl)piperazin-1-yl)-3-(4-chlorophenyl)prop-2-yn-1-one | 0.452^{[b]} |
| 104 | (4-(5-chlorooxazolo[4,5-b]pyridin-2-yl)piperazin-1-yl)(4-ethynyl-3-methoxyphenyl)methanone | 0.109^{[b]} |
| 105 | [4-[5-(3-bicyclo[1.1.1]pentanyl)-1,2,4-oxadiazol-3-yl]phenyl]-[4-(5-chlorooxazolo[4,5-b]pyridin-2-yl)piperazin-1-yl]methanone | 0.009^{[b]} |
| 106 | [4-(1-tert-butyl-1,2,4-triazol-3-yl)-3-fluorophenyl]-[4-(5-methyl-[1,3]oxazolo[4,5-b]pyridin-2-yl)piperazin-1-yl]methanone | 0.036^{[b]} |
| 107 | [4-(1-tert-butyl-1,2,4-triazol-3-yl)-3-fluorophenyl]-[4-(5-chloro-[1,3]oxazolo[4,5-b]pyridin-2-yl)piperazin-1-yl]methanone | 0.015^{[b]} |
| 108 | [4-(5-methyl-[1,3]oxazolo[4,5-b]pyridin-2-yl)piperazin-1-yl]-[4-[1-[1-(trifluoromethyl)cyclopropyl]triazol-4-yl]phenyl]methanone | 0.154^{[b]} |
| 109 | [4-(5-methyl-[1,3]oxazolo[4,5-b]pyridin-2-yl)piperazin-1-yl]-[4-[1-(3-methyloxetan-3-yl)triazol-4-yl]phenyl]methanone | 2.525^{[b]} |
| 110 | [4-(5-chloro-[1,3]oxazolo[4,5-b]pyridin-2-yl)piperazin-1-yl]-[4-[1-(3 -methyloxetan-3 -yl)triazol-4-yl]phenyl]methanone | 0.859^{[b]} |
| 111 | [4-(1-tert-butyl-1,2,4-triazol-3-yl)-3-(trifluoromethyl)phenyl]-[4-(5-methyl-[1,3]oxazolo[4,5-b]pyridin-2-yl)piperazin-1-yl]methanone | 0.028^{[b]} |
| 112 | [4-(1-tert-butyl-1,2,4-triazol-3-yl)-3-(trifluoromethyl)phenyl]-[4-(5-chloro-[1,3]oxazolo[4,5-b]pyridin-2-yl)piperazin-1-yl]methanone | 0.012^{[b]} |
| 113 | [4-(5-methyl-[1,3]oxazolo [4,5-b]pyridin-2-yl)piperazin-1-yl]-[4-[1-(oxetan-3-ylmethyl)triazol-4-yl]phenyl]methanone | 4.269^{[b]} |
| 114 | [4-(5-chloro-[1,3]oxazolo[4,5-b]pyridin-2-yl)piperazin-1-yl]-[4-[1-(oxetan-3-ylmethyl)triazol-4-yl]phenyl]methanone | 5.553^{[b]} |
| 115 | (4-(5-(tert-butyl)-1,3,4-oxadiazol-2-yl)-3-chlorophenyl)(4-(5-chlorooxazolo[4,5-b]pyridin-2-yl)piperazin-1-yl)methanone | 0.008^{[b]} |
| 116 | (4-(5-(tert-butyl)-1,3,4-oxadiazol-2-yl)-3-fluoro-5-hydroxyphenyl)(4-(5-chlorooxazolo[4,5-b]pyridin-2-yl)piperazin-1-yl)methanone | N/A |
| 117 | (4-(5-(tert-butyl)-1,3,4-oxadiazol-2-yl)-3-chlorophenyl)(4-(5-methyloxazolo[4,5-b]pyridin-2-yl)piperazin-1-yl)methanone | 0.034^{[b]} |
| 118 | (4-(5-(tert-butyl)-1,3,4-oxadiazol-2-yl)-3-fluoro-5-hydroxyphenyl)(4-(5-methyloxazolo[4,5-b]pyridin-2-yl)piperazin-1-yl)methanone | 0.368^{[b]} |
| 119 | [4-(5-tert-butyl-2-methyl-1,2,4-triazol-3-yl)phenyl]-[4-(5-chlorooxazolo[4,5-b]pyridin-2-yl)piperazin-1-yl]methanone | 0.140^{[b]} |
| 120 | [6-(4-tert-butylpyrazol-1-yl)-5-methylpyridin-3-yl]-[4-(5-chloro-[1,3]oxazolo[4,5-b]pyridin-2-yl)piperazin-1-yl]methanone | 0.004^{[b]} |
| 121 | [4-(5-tert-butyl-1,3,4-oxadiazol-2-yl)-3-(trifluoromethyl)phenyl]-[4-(5-methyloxazolo[4,5-b]pyridin-2-yl)piperazin-1-yl]methanone | 0.013^{[b]} |
| 122 | [4-(5-tert-butyl-1,3,4-oxadiazol-2-yl)-3-(trifluoromethyl)phenyl]-[4-(5-chlorooxazolo[4,5-b]pyridin-2-yl)piperazin-1-yl]methanone | 0.003^{[b]} |
| 123 | (4-(1-(tert-butyl)-1H-1,2,4-triazol-3-yl)-3-methoxyphenyl)(4-(5- methyloxazolo[4,5-b]pyridin-2-yl)piperazin-1-yl)methanone | 0.174^{[b]} |
| 124 | (4-(1-(tert-butyl)-1H-1,2,4-triazol-3-yl)-3-methoxyphenyl)(4-(5- chlorooxazolo[4,5-b]pyridin-2-yl)piperazin-1-yl)methanone | 0.081^{[b]} |
| 125 | (4-(5-chlorooxazolo[4,5-b]pyridin-2-yl)piperazin-1-yl)(4-(1-((1-fluorocyclopropyl)methyl)-1H-1,2,3-triazol-4-yl)phenyl)methanone | 0.121^{[b]} |
| 126 | (4-(1-((1-fluorocyclopropyl)methyl)-1H-1,2,3-triazol-4-yl)phenyl)(4-(5-methyloxazolo[4,5-b]pyridin-2-yl)piperazin-1-yl)methanone | 0.472^{[b]} |
| 127 | [4-(5-tert-butyl-2-methyl-1,2,4-triazol-3-yl)phenyl]-[4-(5-methyloxazolo[4,5-b]pyridin-2-yl)piperazin-1-yl]methanone | 0.228^{[b]} |
| 128 | [6-(3-tert-butyl-1,2,4-triazol-1-yl)-5-methylpyridin-3-yl]-[4-(5-methyl-[1,3]oxazolo[4,5-b]pyridin-2-yl)piperazin-1-yl]methanone | 0.107^{[b]} |
| 129 | [6-(3-tert-butyl-1,2,4-triazol-1-yl)-5-methylpyridin-3-yl]-[4-(5-chloro-[1,3]oxazolo[4,5-b]pyridin-2-yl)piperazin-1-yl]methanone | 0.052^{[b]} |
| 130 | [6-(4-tert-butylpyrazol-1-yl)-5-methylpyridin-3-yl]-[4-(5-methyl-[1,3]oxazolo[4,5-b]pyridin-2-yl)piperazin-1-yl]methanone | 0.008^{[b]} |
| 131 | 3-[4-[4-[4-(5-chlorooxazolo[4,5-b]pyridin-2-yl)piperazine-1-carbonyl]phenyl]triazol-1-yl]-2,2-dimethyl-propanenitrile | 0.035^{[b]} |
| 132 | (4-(5-chlorooxazolo[4,5-b]pyridin-2-yl)piperazin-1-yl)(4-(1-(2-fluoro-2-methylpropyl)-1H-1,2,3-triazol-4-yl)phenyl)methanone | 0.049^{[b]} |
| 133 | (4-(1-(2-fluoro-2-methylpropyl)-1H-1,2,3-triazol-4-yl)phenyl)(4-(5-methyloxazolo[4,5-b]pyridin-2-yl)piperazin-1-yl)methanone | 0.094^{[b]} |
| 134 | (4-(5-chlorooxazolo[4,5-b]pyridin-2-yl)piperazin-1-yl)(4-(1-(1-fluoro-2-methylpropan-2-yl)-1H-1,2,3-triazol-4-yl)phenyl)methanone | 0.048^{[b]} |
| 135 | (4-(1-(1-fluoro-2-methylpropan-2-yl)-1H-1,2,3-triazol-4-yl)phenyl)(4-(5-methyloxazolo[4,5-b]pyridin-2-yl)piperazin-1-yl)methanone | 0.116^{[b]} |
| 136 | 3-[4-[4-[4-(5-chlorooxazolo[4,5-b]pyridin-2-yl)piperazine-1-carbonyl]phenyl]triazol-2-yl]-2,2-dimethyl-propanenitrile | 0.034^{[b]} |
| 137 | [4-(5-chlorooxazolo[4,5-b]pyridin-2-yl)piperazin-1-yl]-[6-methyl-5-[[1-(trifluoromethyl)cyclopropyl]methoxy]pyrazin-2-yl]methanone | 0.034^{[b]} |
| 138 | [4-(5-chlorooxazolo[4,5-b]pyridin-2-yl)piperazin-1-yl]-[5-methoxy-6-[[1-(trifluoromethyl)cyclopropyl]methoxy]-3-pyridyl]methanone | 0.183^{[b]} |
| 139 | [4-(5-methyloxazolo[4,5-b]pyridin-2-yl)piperazin-1-yl]-[6-methyl-5-[[1-(trifluoromethyl)cyclopropyl]methoxy]-2-pyridyl]methanone | 0.387^{[b]} |
| 140 | [4-(5-chlorooxazolo[4,5-b]pyridin-2-yl)piperazin-1-yl]-[6-methyl-5-[[1-(trifluoromethyl)cyclopropyl]methoxy]-2-pyridyl]methanone | 0.326^{[b]} |
| 141 | (4-(5-(tert-butyl)-1,2,4-oxadiazol-3-yl)phenyl)(4-(5-iodooxazolo[4,5-b]pyridin-2-yl)piperazin-1-yl)methanone | N/A |
| 142 | (5-methyl-6-((1-methylcyclopropyl)methoxy)pyridin-3-yl)(4-(5- methyloxazolo[4,5-b]pyridin-2-yl)piperazin-1-yl)methanone | 0.106 |
| 143 | (5-methyl-6-((1-(trifluoromethyl)cyclopropyl)methoxy)pyridin-3- yl)(4-(5-methyloxazolo[4,5-b]pyridin-2-yl)piperazin-1-yl)methanone | 0.077 |
| 144 | (4-(5-chlorooxazolo[4,5-b]pyridin-2-yl)piperazin-1-yl)(5-fluoro-6-((1-(trifluoromethyl)cyclopropyl)methoxy)pyridin-3-yl)methanone | 0.105 |
| 145 | (4-(5-chlorooxazolo[4,5-b]pyridin-2-yl)piperazin-1-yl)(5-fluoro-6-((1-methylcyclopropyl)methoxy)pyridin-3-yl)methanone | 0.216 |
| 146 | (5-chloro-6-((1-methylcyclopropyl)methoxy)pyridin-3-yl)(4-(5-methyloxazolo[4,5-b]pyridin-2-yl)piperazin-1-yl)methanone | 0.135 |
| 147 | (5-chloro-6-((1-(trifluoromethyl)cyclopropyl)methoxy)pyridin-3-yl)(4-(5-methyloxazolo[4,5-b]pyridin-2-yl)piperazin-1-yl)methanone | 0.128 |
| 148 | (5-fluoro-6-((1-methylcyclopropyl)methoxy)pyridin-3-yl)(4-(5- methyloxazolo[4,5-b]pyridin-2-yl)piperazin-1-yl)methanone | 0.226^{[b]} |
| 149 | (5-chloro-6-((1-methylcyclopropyl)methoxy)pyridin-3-yl)(4-(5- chlorooxazolo[4,5-b]pyridin-2-yl)piperazin-1-yl)methanone | 0.020^{[b]} |
| 150 | (5-fluoro-6-((1-(trifluoromethyl)cyclopropyl)methoxy)pyridin-3- yl)(4-(5-methyloxazolo[4,5-b]pyridin-2-yl)piperazin-1-yl)methanone | 0.046^{[b]} |
| 151 | (5,6-bis((1-(trifluoromethyl)cyclopropyl)methoxy)pyridin-3- yl)(4-(5-methyloxazolo[4,5-b]pyridin-2-yl)piperazin-1-yl)methanone | 0.055^{[b]} |
| 152 | (5-chloro-6-((1-(trifluoromethyl)cyclopropyl)methoxy)pyridin-3-yl)(4-(5-chlorooxazolo[4,5-b]pyridin-2-yl)piperazin-1-yl)methanone | 0.015^{[b]} |
| 153 | (4-(5-chlorooxazolo[4,5-b]pyridin-2-yl)piperazin-1-yl)(5-methyl-6-((1-(trifluoromethyl)cyclopropyl)methoxy)pyridin-3-yl)methanone | 0.010^{[b]} |
| 154 | (4-(5-chlorooxazolo[4,5-b]pyridin-2-yl)piperazin-1-yl)(5-(trifluoromethyl)-6-((1-(trifluoromethyl)cyclopropyl)methoxy)pyridin-3-yl)methanone | 0.008^{[b]} |
| 155 | (4-(5-chlorooxazolo[4,5-b]pyridin-2-yl)piperazin-1-yl)(6-(cyclopropylmethoxy)-5-methylpyridin-3-yl)methanone | 0.090^{[b]} |
| 156 | (4-(5-chlorooxazolo[4,5-b]pyridin-2-yl)piperazin-1-yl)(5-methyl-6-((1-methylcyclopropyl)methoxy)pyridin-3-yl)methanone | 0.021^{[b]} |
| 157 | (4-(5-chlorooxazolo[4,5-b]pyridin-2-yl)piperazin-1-yl)(5-methyl-6-((3-methyloxetan-3-yl)methoxy)pyridin-3-yl)methanone | 0.317^{[b]} |
| 158 | (4-(5-chlorooxazolo[4,5-b]pyridin-2-yl)piperazin-1-yl)(6-((2,2-difluoro-1-methylcyclopropyl)methoxy)-5-methylpyridin-3-yl)methanone | 0.011^{[b]} |
| 159 | (4-(5-chlorooxazolo[4,5-b]pyridin-2-yl)piperazin-1-yl)(6-((3-ethyloxetan-3-yl)methoxy)-5-methylpyridin-3-yl)methanone | 0.117^{[b]} |
| 160 | (4-(5-chlorooxazolo[4,5-b]pyridin-2-yl)piperazin-1-yl)(6-((1-(methoxymethyl)cyclopropyl)methoxy)-5-methylpyridin-3-yl)methanone | 0.295^{[b]} |
| 161 | (4-(5-chlorooxazolo[4,5-b]pyridin-2-yl)piperazin-1-yl)(6-((3-fluorooxetan-3-yl)methoxy)-5-methylpyridin-3-yl)methanone | 0.906^{[b]} |
| 162 | 2-(1-(((5-(4-(5-chlorooxazolo[4,5-b]pyridin-2-yl)piperazine-1- carbonyl)-3-methylpyridin-2-yl)oxy)methyl)cyclopropyl)acetonitrile | 0.230^{[b]} |
| 163 | 1-(((5-(4-(5-chlorooxazolo[4,5-b]pyridin-2-yl)piperazine-1-carbonyl)-3-methylpyridin-2-yl)oxy)methyl)cyclopropane-1-carbonitrile | 0.677^{[b]} |
| 164 | 3-(((5-(4-(5-chlorooxazolo[4,5-b]pyridin-2-yl)piperazine-1-carbonyl)-3-methylpyridin-2-yl)oxy)methyl)oxetane-3-carbonitrile | 1.544^{[b]} |
| 165 | 3-(((5-(4-(5-chlorooxazolo[4,5-b]pyridin-2-yl)piperazine-1-carbonyl)-3-methylpyridin-2-yl)oxy)methyl)-5-fluorobenzonitrile | 0.042^{[b]} |
| 166 | (4-(5-chlorooxazolo[4,5-b]pyridin-2-yl)piperazin-1-yl)(5-methyl-6-((tetrahydrofuran-2-yl)methoxy)pyridin-3-yl)methanone | 0.475^{[b]} |
| 167 | [4-(5-methyloxazolo[4,5-b]pyridin-2-yl)piperazin-1-yl]-[6-methyl-5-[[1-(trifluoromethyl)cyclopropyl]methoxy]pyrazin-2-yl]methanone | 0.063^{[b]} |
| 168 | (4-(5-chlorooxazolo[4,5-b]pyridin-2-yl)piperazin-1-yl)(5-methyl-6-(oxetan-2-ylmethoxy)pyridin-3-yl)methanone | 1.745^{[b]} |
| 169 | [5-methoxy-6-[[1-(trifluoromethyl)cyclopropyl]methoxy]-3-pyridyl]-[4-(5-methyloxazolo[4,5-b]pyridin-2-yl)piperazin-1-yl]methanone | 0.138^{[b]} |
| 170 | (4-(5-chlorooxazolo[4,5-b]pyridin-2-yl)piperazin-1-yl)(5-methyl-6-((tetrahydro-2H-pyran-2-yl)methoxy)pyridin-3-yl)methanone | 0.057^{[b]} |
| 171 | (4-(5-chlorooxazolo[4,5-b]pyridin-2-yl)piperazin-1-yl)(6-((1-methoxycyclopropyl)methoxy)-5-methylpyridin-3-yl)methanone | 0.188^{[b]} |
| 172 | (S)-(4-(5-chlorooxazolo[4,5-b]pyridin-2-yl)piperazin-1-yl)(6-(2,3-difluoropropoxy)-5-methylpyridin-3-yl)methanone | 0.978^{[b]} |
| 173 | (4-(5-chlorooxazolo[4,5-b]pyridin-2-yl)piperazin-1-yl)(5-methyl-6-(3,3,3-trifluoropropoxy)pyridin-3-yl)methanone | 0.135^{[b]} |
| 174 | (R)-(4-(5-chlorooxazolo[4,5-b]pyridin-2-yl)piperazin-1-yl)(6-(2,3-difluoropropoxy)-5-methylpyridin-3-yl)methanone | 0.706^{[b]} |
| 175 | [3-chloro-5-(1,1-dioxo-1,4-thiazinan-4-yl)phenyl]-[4-(5-methyl-[1,3]oxazolo[4,5-b]pyridin-2-yl)piperazin-1-yl]methanone | 1.519 |
| 176 | 1-[4-[3-chloro-5-[4-(5-methyl-[1,3]oxazolo[4,5-b]pyridin-2-yl)piperazine-1-carbonyl]phenyl]piperazin-1-yl]ethanone | 4.147 |
| 177 | [3-chloro-5-(4,4-difluoropiperidin-1-yl)phenyl]-[4-(5-methyl-[1,3]oxazolo[4,5-b]pyridin-2-yl)piperazin-1-yl]methanone | 3.160 |
| 178 | [3-chloro-5-[4-(hydroxymethyl)piperidin-1-yl]phenyl]-[4-(5-methyl-[1,3]oxazolo[4,5-b]pyridin-2-yl)piperazin-1-yl]methanone | 2.488 |
| 179 | [3-chloro-5-(6-hydroxy-2-azaspiro[3.3]heptan-2-yl)phenyl]-[4-(5-methyl-[1,3]oxazolo[4,5-b]pyridin-2-yl)piperazin-1-yl]methanone | 3.941 |
| 180 | [4-(2-azaspiro[3.4]octan-2-yl)-3-(1H-imidazol-2-yl)phenyl]-[4-(5-methyl-[1,3]oxazolo[4,5-b]pyridin-2-yl)piperazin-1-yl]methanone | 0.810 |
| 181 | [3-chloro-5-[4-(hydroxymethyl)phenyl]phenyl]-[4-(5-methyl-[1,3]oxazolo[4,5-b]pyridin-2-yl)piperazin-1-yl]methanone | 0.180 |
| 182 | [3-[4-(hydroxymethyl)phenyl]-5-(3,3,3-trifluoropropoxy)phenyl]-[4-(5-methyl-[1,3]oxazolo[4,5-b]pyridin-2-yl)piperazin-1-yl]methanone | 0.379 |
| 183 | [3-[4-(hydroxymethyl)phenyl]-4-[[1-(trifluoromethyl)cyclopropyl]methoxy]phenyl]-[4-(5-methyl-[1,3]oxazolo[4,5-b]pyridin-2-yl)piperazin-1-yl]methanone | 0.010 |
| 184 | [3-[4-(hydroxymethyl)phenyl]-4-(3,3,3-trifluoropropoxy)phenyl]-[4-(5-methyl-[1,3]oxazolo[4,5-b]pyridin-2-yl)piperazin-1-yl]methanone | 0.078 |
| 185 | [3-[4-(hydroxymethyl)phenyl]-5-[[1-(trifluoromethyl)cyclopropyl]methoxy]phenyl]-[4-(5-methyl-[1,3]oxazolo[4,5-b]pyridin-2-yl)piperazin-1-yl]methanone | 0.302 |
| 186 | [4-[5-(2,2-dimethylpropyl)-1,2,4-oxadiazol-3-yl]-3-[4-(hydroxymethyl)phenyl]phenyl]-[4-(5-methyl-[1,3]oxazolo[4,5-b]pyridin-2-yl)piperazin-1-yl]methanone | 0.012 |
| 187 | [3-[4-(hydroxymethyl)phenyl]-4-[(1-methylcyclopropyl)methoxy]phenyl]-[4-(5-methyl-[1,3]oxazolo[4,5-b]pyridin-2-yl)piperazin-1-yl]methanone | 0.009 |
| 188 | [3-[4-(hydroxymethyl)phenyl]-5-[(1-methylcyclopropyl)methoxy]phenyl]-[4-(5-methyl-[1,3]oxazolo[4,5-b]pyridin-2-yl)piperazin-1-yl]methanone | 0.165 |
| 189 | (4-(5-(tert-butyl)-1,2,4-oxadiazol-3-yl)phenyl)(4-(5-cyclopropyloxazolo[4,5-b]pyridin-2-yl)piperazin-1-yl)methanone | 2.491 |
| 190 | [6-(1-tert-butylpyrazol-4-yl)-5-methylpyridin-3-yl]-[4-(5-chloro-[1,3]oxazolo[4,5-b]pyridin-2-yl)piperazin-1-yl]methanone | 0.046^{[b]} |
| 191 | [6-(1-tert-butylpyrazol-4-yl)-5-methylpyridin-3-yl]-[4-(5-methyl-[1,3]oxazolo[4,5-b]pyridin-2-yl)piperazin-1-yl]methanone | 0.098^{[b]} |
| 192 | (4-(5-oxa-2-azaspiro[3.4]octan-2-yl)phenyl)(4-(oxazolo[4,5- b]pyridin-2-yl)piperazin-1-yl)methanone | 4.116 |
| 193 | (4-(oxazolo[4,5-b]pyridin-2-yl)piperazin-1-yl)(4-(3-((1,1,1-trifluoropropan-2-yl)oxy)azetidin-1-yl)phenyl)methanone | 0.355 |
| 194 | (4-(5-oxa-2-azaspiro[3.4]octan-2-yl)phenyl)(4-(5- methyloxazolo[4,5-b]pyridin-2-yl)piperazin-1-yl)methanone | 0.486 |
| 195 | (4-(5-methyloxazolo[4,5-b]pyridin-2-yl)piperazin-1-yl)(4-(3-((1,1,1-trifluoropropan-2-yl)oxy)azetidin-1-yl)phenyl)methanone | 0.039 |
| 196 | (4-(oxazolo[4,5-b]pyridin-2-yl)piperazin-1-yl)(4-(3-phenoxyazetidin-1-yl)phenyl)methanone | 0.441 |
| 197 | (4-(5-(tert-butyl)-1,2,4-oxadiazol-3-yl)phenyl)(4-(5-methoxyoxazolo[4,5-b]pyridin-2-yl)piperazin-1-yl)methanone | 0.471 |
| 198 | (4-(5-methyloxazolo[4,5-b]pyridin-2-yl)piperazin-1-yl)(4-(3-phenoxyazetidin-1-yl)phenyl)methanone | 0.046 |
| 199 | [4-[(3-chlorophenyl)methoxy]-3-(1H-imidazol-2-yl)phenyl]-[4-(5-methyl-[1,3]oxazolo[4,5-b]pyridin-2-yl)piperazin-1-yl]methanone | 0.625 |
| 200 | (S)-(4-(5-methyloxazolo[4,5-b]pyridin-2-yl)piperazin-1-yl)(4-(3-((1,1,1-trifluoropropan-2-yl)oxy)azetidin-1-yl)phenyl)methanone | 0.015 |
| 201 | (R)-(4-(5-methyloxazolo[4,5-b]pyridin-2-yl)piperazin-1-yl)(4-(3-((1,1,1-trifluoropropan-2-yl)oxy)azetidin-1-yl)phenyl)methanone | 0.038 |
| 202 | [4-(5-chloro-[1,3]oxazolo[4,5-b]pyridin-2-yl)piperazin-1-yl]-[4-(2,2-difluoro-5-azaspiro[2.4]heptan-5-yl)phenyl]methanone | 0.014 |
| 203 | [4-(5-chloro-[1,3]oxazolo[4,5-b]pyridin-2-yl)piperazin-1-yl]-[4-(7,7-difluoro-2-azaspiro[3.3]heptan-2-yl)phenyl]methanone | 0.035 |
| 204 | (4-(3-(tert-butoxy)azetidin-1-yl)phenyl)(4-(5-chlorooxazolo[4,5-b]pyridin-2-yl)piperazin-1-yl)methanone | 0.018 |
| 205 | (4-(5-chlorooxazolo[4,5-b]pyridin-2-yl)piperazin-1-yl)(4-(3-(tert-pentyloxy)azetidin-1-yl)phenyl)methanone | 0.005 |
| 206 | (4-(5,5-difluoro-2-azaspiro[3.3]heptan-2-yl)phenyl)(4-(5-methyloxazolo[4,5-b]pyridin-2-yl)piperazin-1-yl)methanone | 0.061 |
| 207 | (2-fluoro-4-(3-((1,1,1-trifluoropropan-2-yl)oxy)azetidin-1- yl)phenyl)(4-(5-methyloxazolo[4,5-b]pyridin-2-yl)piperazin-1-yl)methanone | 0.016 |
| 208 | (4-(5-chlorooxazolo[4,5-b]pyridin-2-yl)piperazin-1-yl)(4-(3-((1,1,1-trifluoropropan-2-yl)oxy)azetidin-1-yl)phenyl)methanone | 0.016 |
| 209 | (4-(5-chlorooxazolo[4,5-b]pyridin-2-yl)piperazin-1-yl)(4-(3-ethoxy-3-methylazetidin-1-yl)phenyl)methanone | 0.235 |
| 210 | (3-fluoro-4-(3-((1,1,1-trifluoropropan-2-yl)oxy)azetidin-1- yl)phenyl)(4-(5-methyloxazolo[4,5-b]pyridin-2-yl)piperazin-1-yl)methanone | 0.024 |
| 211 | (2-methyl-4-(3-((1,1,1-trifluoropropan-2-yl)oxy)azetidin-1- yl)phenyl)(4-(5-methyloxazolo[4,5-b]pyridin-2-yl)piperazin-1-yl)methanone | 0.346 |
| 212 | (2,6-difluoro-4-(3-((1,1,1-trifluoropropan-2-yl)oxy)azetidin-1-yl)phenyl)(4-(5-methyloxazolo[4,5-b]pyridin-2-yl)piperazin-1-yl)methanone | 1.126 |
| 213 | (2-chloro-4-(3-((1,1,1-trifluoropropan-2-yl)oxy)azetidin-1-yl)phenyl)(4-(5-methyloxazolo[4,5-b]pyridin-2-yl)piperazin-1-yl)methanone | 0.099 |
| 214 | (4-(5-methyloxazolo[4,5-b]pyridin-2-yl)piperazin-1-yl)(6-(3-((1,1,1-trifluoropropan-2-yl)oxy)azetidin-1-yl)pyridin-3- yl)methanone | 0.170 |
| 215 | (4-(5-methyloxazolo[4,5-b]pyridin-2-yl)piperazin-1-yl)(6-(3-(tert-pentyloxy)azetidin-1-yl)pyridin-3-yl)methanone | 0.070 |
| 216 | (5-methyl-6-(3-((1,1,1-trifluoropropan-2-yl)oxy)azetidin-1- yl)pyridin-3-yl)(4-(5-methyloxazolo[4,5-b]pyridin-2-yl)piperazin-1-yl)methanone | 0.113 |
| 217 | (4-chloro-5-fluoro-6-(3-((1,1,1-trifluoropropan-2-yl)oxy)azetidin-1-yl)pyridin-3-yl)(4-(5-methyloxazolo[4,5-b]pyridin-2-yl)piperazin-1-yl)methanone | 0.167 |
| 218 | (4-(5-methyloxazolo[4,5-b]pyridin-2-yl)piperazin-1-yl)(6-(3-((1,1,1-trifluoropropan-2-yl)oxy)azetidin-1-yl)pyridazin-3- yl)methanone | 1.541 |
| 219 | (4-(5-methyloxazolo[4,5-b]pyridin-2-yl)piperazin-1-yl)(2-(3-((1,1,1-trifluoropropan-2-yl)oxy)azetidin-1-yl)pyrimidin-5-yl)methanone | 0.220 |
| 220 | (4-methyl-6-(3-((1,1,1-trifluoropropan-2-yl)oxy)azetidin-1-yl)pyridin-3-yl)(4-(5-methyloxazolo[4,5-b]pyridin-2-yl)piperazin-1-yl)methanone | 4.002 |
| 221 | (6-(5,5-difluoro-2-azaspiro[3.3]heptan-2-yl)-4-methylpyridin-3-yl)(4-(5-methyloxazolo[4,5-b]pyridin-2-yl)piperazin-1-yl)methanone | 13.107 |
| 222 | (2-(1,1-difluoro-5-azaspiro[2.4]heptan-5-yl)pyrimidin-5-yl)(4-(5-methyloxazolo[4,5-b]pyridin-2-yl)piperazin-1-yl)methanone | 0.794 |
| 223 | (6-(1,1-difluoro-5-azaspiro[2.4]heptan-5-yl)-4-methylpyridin-3-yl)(4-(5-methyloxazolo[4,5-b]pyridin-2-yl)piperazin-1-yl)methanone | 4.456 |
| 224 | (5-fluoro-6-(3-((1,1,1-trifluoropropan-2-yl)oxy)azetidin-1- yl)pyridin-3-yl)(4-(5-methyloxazolo[4,5-b]pyridin-2-yl)piperazin-1-yl)methanone | 0.090 |
| 225 | (6-(1,1-difluoro-5-azaspiro[2.4]heptan-5-yl)-5-methylpyridin-3-yl)(4-(5-methyloxazolo[4,5-b]pyridin-2-yl)piperazin-1-yl)methanone | 0.244 |
| 226 | (5-chloro-6-(3-((1,1,1-trifluoropropan-2-yl)oxy)azetidin-1-yl)pyridin-3-yl)(4-(5-methyloxazolo[4,5-b]pyridin-2-yl)piperazin-1-yl)methanone | 0.038 |
| 227 | (5-chloro-6-(1,1-difluoro-5-azaspiro[2.4]heptan-5-yl)pyridin-3-yl)(4-(5-methyloxazolo[4,5-b]pyridin-2-yl)piperazin-1-yl)methanone | 0.062 |
| 228 | (6-(1,1-difluoro-5-azaspiro[2.4]heptan-5-yl)-5-fluoropyridin-3-yl)(4-(5-methyloxazolo[4,5-b]pyridin-2-yl)piperazin-1-yl)methanone | 0.152 |
| 229 | (4-(5-chlorooxazolo[4,5-b]pyridin-2-yl)piperazin-1-yl)(5-fluoro-6-(3-((1,1,1-trifluoropropan-2-yl)oxy)azetidin-1-yl)pyridin-3- yl)methanone | 0.035 |
| 230 | (4-(5-chlorooxazolo[4,5-b]pyridin-2-yl)piperazin-1-yl)(6-(1,1-difluoro-5-azaspiro[2.4]heptan-5-yl)-5-fluoropyridin-3-yl)methanone | 0.056 |
| 231 | (4-(5-chlorooxazolo[4,5-b]pyridin-2-yl)piperazin-1-yl)(3-methyl-4-(1-neopentyl-1H-1,2,3-triazol-4-yl)phenyl)methanone | 0.004^{[b]} |
| 232 | (4-(5-chlorooxazolo[4,5-b]pyridin-2-yl)piperazin-1-yl)(3-methyl-4-(2-neopentyl-2H-1,2,3-triazol-4-yl)phenyl)methanone | 0.001^{[b]} |
| 233 | 5-(4-(5-chlorooxazolo[4,5-b]pyridin-2-yl)piperazine-1-carbonyl)-2-(1-neopentyl-1H-1,2,3-triazol-4-yl)benzonitrile | 0.020^{[b]} |
| 234 | 5-(4-(5-chlorooxazolo[4,5-b]pyridin-2-yl)piperazine-1-carbonyl)-2-(2-neopentyl-2H-1,2,3-triazol-4-yl)benzonitrile | 0.010^{[b]} |
| 235 | 5-(4-(5-methyloxazolo[4,5-b]pyridin-2-yl)piperazine-1-carbonyl)-2-(1-neopentyl-1H-1,2,3-triazol-4-yl)benzonitrile | 0.104^{[b]} |
| 236 | 5-(4-(5-methyloxazolo[4,5-b]pyridin-2-yl)piperazine-1-carbonyl)-2-(2-neopentyl-2H-1,2,3-triazol-4-yl)benzonitrile | 0.033^{[b]} |
| 237 | (4-(5-chlorooxazolo[4,5-b]pyridin-2-yl)piperazin-1-yl)(3-methoxy-4-(1-neopentyl-1H-1,2,3-triazol-4-yl)phenyl)methanone | 0.007^{[b]} |
| 238 | (4-(5-chlorooxazolo[4,5-b]pyridin-2-yl)piperazin-1-yl)(3-methoxy-4-(2-neopentyl-2H-1,2,3-triazol-4-yl)phenyl)methanone | 0.001^{[b]} |
| 239 | (3-methoxy-4-(1-neopentyl-1H-1,2,3-triazol-4-yl)phenyl)(4-(5-methyloxazolo[4,5-b]pyridin-2-yl)piperazin-1-yl)methanone | 0.013^{[b]} |
| 240 | (3-methoxy-4-(2-neopentyl-2H-1,2,3-triazol-4-yl)phenyl)(4-(5-methyloxazolo[4,5-b]pyridin-2-yl)piperazin-1-yl)methanone | 0.002^{[b]} |
| 241 | (4-(5-chlorooxazolo[4,5-b]pyridin-2-yl)piperazin-1-yl)(5-fluoro-6-(1-neopentyl-1H-1,2,3-triazol-4-yl)pyridin-3-yl)methanone | 0.027^{[b]} |
| 242 | (4-(5-chlorooxazolo[4,5-b]pyridin-2-yl)piperazin-1-yl)(5-fluoro-6-(1-neopentyl-1H-1,2,3-triazol-5-yl)pyridin-3-yl)methanone | 8.406^{[b]} |
| 243 | (4-(5-chlorooxazolo[4,5-b]pyridin-2-yl)piperazin-1-yl)(5-fluoro-6-(2-neopentyl-2H-1,2,3-triazol-4-yl)pyridin-3 -yl)methanone | 0.025^{[b]} |
| 244 | (5-fluoro-6-(1-neopentyl-1H-1,2,3-triazol-4-yl)pyridin-3-yl)(4-(5- methyloxazolo[4,5-b]pyridin-2-yl)piperazin-1-yl)methanone | 0.050[b] |
| 245 | (5 -fluoro-6-(2-neopentyl-2H-1,2,3-triazol-4-yl)pyridin-3-yl)(4-(5- methyloxazolo[4,5-b]pyridin-2-yl)piperazin-1-yl)methanone | 0.057^{[b]} |
| 246 | (5-fluoro-6-(1-neopentyl-1H-1,2,3-triazol-5-yl)pyridin-3-yl)(4-(5- methyloxazolo[4,5-b]pyridin-2-yl)piperazin-1-yl)methanone | 1.304^{[b]} |
| 247 | [4-(5-chloro-[1,3]oxazolo[4,5-b]pyridin-2-yl)piperazin-1-yl]-[6-[1-(2,2-dimethylpropyl)pyrazol-4-yl]-5-methylpyridin-3-yl]methanone | 0.007^{[b]} |
| 248 | [6-[1-(2,2-dimethylpropyl)pyrazol-4-yl]-5-methylpyridin-3-yl]-[4-(5-methyl-[1,3]oxazolo[4,5-b]pyridin-2-yl)piperazin-1-yl]methanone | 0.014^{[b]} |
| 249 | (4-(5-chlorooxazolo[4,5-b]pyridin-2-yl)piperazin-1-yl)(3-((2,6-dichlorophenyl)ethynyl)azetidin-1-yl)methanone | 0.022^{[b]} |
| 250 | [3-[2-(2-chloro-4-fluorophenyl)ethynyl] azetidin-1-yl]-[4-(5-chloro-[1,3]oxazolo[4,5-b]pyridin-2-yl)piperazin-1-yl]methanone | 0.025^{[b]} |
| 251 | [3-[2-(2-chloro-4-fluorophenyl)ethynyl]azetidin-1-yl]-[4-(5-chloro-[1,3]oxazolo[4,5-b]pyridin-2-yl)piperazin-1-yl]methanone | 0.326^{[b]} |
| 252 | [3-[(2-chloro-4-fluorophenoxy)methyl]azetidin-1-yl]-[4-(5-methyl-[1,3]oxazolo[4,5-b]pyridin-2-yl)piperazin-1-yl]methanone | 1.239^{[b]} |
| 253 | 2-chloro-N-[1-[4-(5-chloro-[1,3]oxazolo[4,5-b]pyridin-2-yl)piperazine-1-carbonyl]azetidin-3-yl]-4-fluorobenzamide | 12.500^{[b]} |
| 254 | 2-chloro-4-fluoro-N-[1-[4-(5-methyl-[1,3]oxazolo[4,5-b]pyridin-2-yl)piperazine-1-carbonyl]azetidin-3-yl]benzamide | 12.500^{[b]} |
| 255 | N-(2-chloro-4-fluorophenyl)-1-[4-(5-chloro-[1,3]oxazolo[4,5-b]pyridin-2-yl)piperazine-1-carbonyl]azetidine-3-carboxamide | 12.500^{[b]} |
| 256 | N-(2-chloro-4-fluorophenyl)-1-[4-(5-methyl-[1,3]oxazolo[4,5-b]pyridin-2-yl)piperazine-1-carbonyl]azetidine-3-carboxamide | 12.500^{[b]} |
| 257 | (4-(1-(tert-butyl)-1H-1,2,3-triazol-4-yl)-3-methylphenyl)(4-(5- chlorooxazolo[4,5-b]pyridin-2-yl)piperazin-1-yl)methanone | 0.058^{[b]} |
| 258 | [4-(5-chlorooxazolo[4,5-b]pyridin-2-yl)piperazin-1-yl]-[4-[1-(2-methoxy-2-methyl-propyl)triazol-4-yl]phenyl]methanone | 0.031^{[b]} |
| 259 | (4-(5-iodooxazolo[4,5-b]pyridin-2-yl)piperazin-1-yl)(4-(5-neopentyl-1,2,4-oxadiazol-3-yl)phenyl)methanone | N/A |
| 260 | 2-(4-(4-(5-neopentyl-1,2,4-oxadiazol-3-yl)benzoyl)piperazin-1-yl)oxazolo[4,5-b]pyridine-5-carbonitrile | 0.301 |
| 261 | (4-(5-neopentyl-1,2,4-oxadiazol-3-yl)phenyl)(4-(5-(trifluoromethyl)oxazolo[4,5-b]pyridin-2-yl)piperazin-1-yl)methanone | 0.040 |
| 262 | [4-(5-chlorooxazolo[4,5-b]pyridin-2-yl)piperazin-1-yl]-[4-[5 - (2,2-difluoropropyl)-1,2,4-oxadiazol-3-yl]phenyl]methanone | 0.065 |
| 263 | (2-butyl-1,3-benzoxazol-6-yl)-[4-(5-methyloxazolo[4,5-b]pyridin-2-yl)piperazin-1-yl]methanone | 0.181 |
| 264 | [4-(5-chlorooxazolo[4,5-b]pyridin-2-yl)piperazin-1-yl]-[4-[5-(2-fluoro-2-methyl-propyl)-1,2,4-oxadiazol-3-yl]phenyl]methanone | 0.059 |
| 265 | [4-(5-chlorooxazolo[4,5-b]pyridin-2-yl)piperazin-1-yl]-[4-[5-(2-methylallyl)-1,2,4-oxadiazol-3-yl]phenyl]methanone | 0.030 |
| 266 | [4-(5-chlorooxazolo[4,5-b]pyridin-2-yl)piperazin-1-yl]-[4-[5-(2-methylprop-1-enyl)-1,2,4-oxadiazol-3-yl]phenyl]methanone | 0.101 |
| 267 | (4-(5-chlorooxazolo[4,5-b]pyridin-2-yl)piperazin-1-yl)(4-(5-isobutyl-1,3,4-oxadiazol-2-yl)phenyl)methanone | 0.069^{[b]} |
| 268 | (4-(5-chlorooxazolo[4,5-b]pyridin-2-yl)piperazin-1-yl)(4-(1-neopentyl-1H-1,2,3-triazol-4-yl)-3-(trifluoromethyl)phenyl)methanone | 0.004^{[b]} |
| 269 | (4-(5-chlorooxazolo[4,5-b]pyridin-2-yl)piperazin-1-yl)(4-(2-neopentyl-2H-1,2,3-triazol-4-yl)-3- (trifluoromethyl)phenyl)methanone | 0.006^{[b]} |
| 270 | (4-(5-chlorooxazolo[4,5-b]pyridin-2-yl)piperazin-1-yl)(4-(1-neopentyl-1H-1,2,3-triazol-5-yl)-3-(trifluoromethyl)phenyl)methanone | 0.323^{[b]} |
| 271 | (4-(5-chlorooxazolo[4,5-b]pyridin-2-yl)piperazin-1-yl)(5-methyl-6-(1-neopentyl-1H-1,2,3-triazol-4-yl)pyridin-3-yl)methanone | 0.074^{[b]} |
| 272 | (4-(5-chlorooxazolo[4,5-b]pyridin-2-yl)piperazin-1-yl)(5-methyl-6-(2-neopentyl-2H-1,2,3-triazol-4-yl)pyridin-3-yl)methanone | 0.009^{[b]} |
| 273 | [(3R)-4-(5-chlorooxazolo[4,5-b]pyridin-2-yl)-3-methyl-piperazin-1-yl]-[4-[1-(2,2-dimethylpropyl)triazol-4-yl]phenyl]methanone | 0.020^{[b]} |
| 274 | [(3S)-4-(5-chlorooxazolo[4,5-b]pyridin-2-yl)-3-methyl-piperazin-1-yl]-[4-[1-(2,2-dimethylpropyl)triazol-4-yl]phenyl]methanone | 0.137^{[b]} |
| 275 | [4-(5-chlorooxazolo[4,5-b]pyridin-2-yl)piperazin-1-yl]-[4-(5-isobutyl-1,3,4-oxadiazol-2-yl)-3-methyl-phenyl]methanone | 0.026^{[b]} |
| 276 | [4-(5-chlorooxazolo[4,5-b]pyridin-2-yl)piperazin-1-yl]-[2-chloro-6-[[1-(trifluoromethyl)cyclopropyl]methoxy]-3-pyridyl]methanone | 1.517^{[b]} |
| 277 | (4-(5-methyloxazolo[4,5-b]pyridin-2-yl)piperazin-1-yl)(4-(2-neopentyl-2H-tetrazol-5-yl)phenyl)methanone | 0.009^{[b]} |
| 278 | [6-(5-tert-butyl-1,3,4-oxadiazol-2-yl)-5-fluoro-3-pyridyl]-[4-(5-chlorooxazolo[4,5-b]pyridin-2-yl)piperazin-1-yl] methanone | 0.801^{[b]} |
| 279 | [4-(5-chlorooxazolo[4,5-b]pyridin-2-yl)piperazin-1-yl]-[6-[1-(2,2-dimethylpropyl)pyrazol-4-yl]-5-(trifluoromethyl)-3-pyridyl] methanone | 0.011^{[b]} |
| 280 | [4-(5-chlorooxazolo[4,5-b]pyridin-2-yl)piperazin-1-yl]-[3-fluoro-4-[5-(2-fluoro-1,1-dimethyl-ethyl)-1,3,4-oxadiazol-2-yl]phenyl]methanone | 0.119^{[b]} |
| 281 | [4-(5-tert-butyl-1,3,4-oxadiazol-2-yl)-3-fluoro-phenyl]-[4-(5-chlorooxazolo[4,5-b]pyridin-2-yl)piperazin-1-yl]methanone | 0.036^{[b]} |
| 282 | [6-[1-(2,2-dimethylpropyl)pyrazol-4-yl]-5-(trifluoromethyl)-3-pyridyl]-[4-(5-methyloxazolo[4,5-b]pyridin-2-yl)piperazin-1-yl]methanone | 0.011^{[b]} |
| 283 | (4-(5-chlorooxazolo[4,5-b]pyridin-2-yl)piperazin-1-yl)(4-(2-neopentyl-2H-tetrazol-5-yl)phenyl)methanone | 0.003^{[b]} |
| 284 | [4-(5-tert-butyl-1,2,4-oxadiazol-3-yl)phenyl]-[8-(5-chlorooxazolo[4,5-b]pyridin-2-yl)-3,8-diazabicyclo[3.2.1]octan-3-yl]methanone | 0.350^{[b]} |
| 285 | [4-(5-chlorooxazolo[4,5-b]pyridin-2-yl)-3-(hydroxymethyl)piperazin-1-yl]-[4-[1-(2,2-dimethylpropyl)triazol-4-yl]phenyl]methanone | 2.049^{[b]} |
| 286 | [4-[1-(2,2-dimethylpropyl)triazol-4-yl]phenyl]-[4-(5-fluorooxazolo[4,5-b]pyridin-2-yl)piperazin-1-yl]methanone | 0.045^{[b]} |
| 287 | [4-(3-tert-butyl-1,2,4-triazol-1-yl)-3-methyl-phenyl]-[4-(5-chlorooxazolo[4,5-b]pyridin-2-yl)piperazin-1-yl]methanone | 0.114^{[b]} |
| 288 | [4-(3-tert-butyl-1,2,4-triazol-1-yl)-3-methyl-phenyl]-[4-(5-methyloxazolo[4,5-b]pyridin-2-yl)piperazin-1-yl]methanone | 0.175^{[b]} |
| 289 | [(2S)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone | 0.063^{[b]} |
| 290 | [(2R)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone | 2.067^{[b]} |
| 291 | [4-(3-tert-butyl-1,2,4-triazol-1-yl)-3-chloro-phenyl]-[4-(5-methyloxazolo[4,5-b]pyridin-2-yl)piperazin-1-yl] methanone | 0.172^{[b]} |
| 292 | [4-(3-tert-butyl-1,2,4-triazol-1-yl)-3-chloro-phenyl]-[4-(5-chlorooxazolo[4,5-b]pyridin-2-yl)piperazin-1-yl]methanone | 0.075^{[b]} |
| 293 | [4-(5-chlorooxazolo[4,5-b]pyridin-2-yl)piperazin-1-yl]-[4-[1-(2,2-dimethylpropyl)-1,2,4-triazol-3-yl]-3-fluoro-phenyl]methanone | 0.020^{[b]} |
| 294 | [4-[1-(2,2-dimethylpropyl)-1,2,4-triazol-3-yl]-3-fluoro-phenyl]-[4-(5-methyloxazolo[4,5-b]pyridin-2-yl)piperazin-1-yl]methanone | 0.034^{[b]} |
| 295 | [4-(5-chlorooxazolo[4,5-b]pyridin-2-yl)-2-methyl-piperazin-1-yl]-[4-[1-(2,2-dimethylpropyl)triazol-4-yl]phenyl]methanone | 0.243^{[b]} |
| 296 | [2-chloro-6-[[1-(trifluoromethyl)cyclopropyl]methoxy]-3-pyridyl]-[4-(5-methyloxazolo[4,5-b]pyridin-2-yl)piperazin-1 - yl]methanone | 2.616^{[b]} |
| 297 | [4-(5-tert-butyl-1,2,4-oxadiazol-3-yl)phenyl]-[3-(5-chlorooxazolo[4,5-b]pyridin-2-yl)-3,8-diazabicyclo[3.2.1]octan-8-yl]methanone | 0.100^{[b]} |
| 298 | [4-(5-chlorooxazolo[4,5-b]pyridin-2-yl)piperazin-1-yl]-[3-fluoro-4-[5-(2-fluoro-2-methyl-propyl)-1,3,4-oxadiazol-2-yl]phenyl]methanone | 0.095^{[b]} |
| 299 | [4-[2-(2,2-dimethylpropyl)tetrazol-5-yl]-3,5-difluoro-phenyl]-[4-(5-methyloxazolo[4,5-b]pyridin-2-yl)piperazin-1-yl]methanone | 0.031^{[b]} |
| 300 | [4-(5-chlorooxazolo[4,5-b]pyridin-2-yl)piperazin-1-yl]-[4-[2-(2,2-dimethylpropyl)tetrazol-5-yl]-3,5-difluorophenyl]methanone | 0.010^{[b]} |
| 301 | [4-[2-(2,2-dimethylpropyl)tetrazol-5-yl]-3-methyl-phenyl]-[4-(5-methyloxazolo[4,5-b]pyridin-2-yl)piperazin-1-yl]methanone | 0.003^{[b]} |
| 302 | [4-(5-chlorooxazolo[4,5-b]pyridin-2-yl)piperazin-1-yl]-[4-[2-(2,2-dimethylpropyl)tetrazol-5-yl]-3-methyl-phenyl]methanone | 0.002^{[b]} |
| 303 | [4-[2-(2,2-dimethylpropyl)tetrazol-5-yl]-3-fluoro-phenyl]-[4-(5-methyloxazolo[4,5-b]pyridin-2-yl)piperazin-1-yl]methanone | 0.007^{[b]} |
| 304 | [4-(5-chlorooxazolo[4,5-b]pyridin-2-yl)piperazin-1-yl]-[4-[2-(2,2-dimethylpropyl)tetrazol-5-yl]-3-fluoro-phenyl]methanone | 0.004^{[b]} |
| 305 | [2-(3-chlorophenyl)sulfonyl-2,6-diazaspiro[3.3]heptan-6-yl]-[4-(5-methyloxazolo[4,5-b]pyridin-2-yl)piperazin-1-yl]methanone | 2.419 |
| 306 | [4-(5-chlorooxazolo[4,5-b]pyridin-2-yl)piperazin-1-yl]-[6-[2-(2,2-dimethylpropyl)tetrazol-5-yl]-5-fluoro-3-pyridyl]methanone | 0.363^{[b]} |
| 307 | [6-[2-(2,2-dimethylpropyl)tetrazol-5-yl]-5-fluoro-3-pyridyl]-[4-(5-methyloxazolo[4,5-b]pyridin-2-yl)piperazin-1-yl]methanone | 1.13^{[b]} |
| 308 | [4-(5-chlorooxazolo[4,5-b]pyridin-2-yl)piperazin-1-yl]-[6-[2-(2,2-dimethylpropyl)tetrazol-5-yl]-5-methyl-3-pyridyl]methanone | 0.111^{[b]} |
| 309 | [6-[2-(2,2-dimethylpropyl)tetrazol-5-yl]-5-methyl-3-pyridyl]-[4-(5-methyloxazolo[4,5-b]pyridin-2-yl)piperazin-1-yl]methanone | 0.051^{[b]} |
| 310 | [6-[2-(2,2-dimethylpropyl)tetrazol-5-yl]-5-methoxy-3-pyridyl]-[4-(5-methyloxazolo[4,5-b]pyridin-2-yl)piperazin-1-yl] methanone | 0.167^{[b]} |
| 311 | [4-(5-chlorooxazolo[4,5-b]pyridin-2-yl)piperazin-1-yl]-[6-[2-(2,2-dimethylpropyl)tetrazol-5-yl]-5-methoxy-3-pyridyl] methanone | 0.061^{[b]} |

| | | |
|---|---|---|
| *[a]: if not indicated otherwise (see [b]), the activity was measured in 4-NPA assay; [b]: measured in 2-AG assay.* | | |

In one aspect, the present invention provides compounds of formula (I) and their pharmaceutically acceptable salts or esters as described herein, wherein said compounds of formula (I) and their pharmaceutically acceptable salts or esters have IC₅₀'s for MAGL inhibition below 25 µM, preferably below 10 µM, more preferably below 5 µM as measured in the MAGL assay described herein.

In one embodiment, compounds of formula (I) and their pharmaceutically acceptable salts or esters as described herein have IC₅₀ (MAGL inhibition) values between 0.000001 µM and 25 µM, particular compounds have IC₅₀ values between 0.000005 µM and 10 µM, further particular compounds have IC₅₀ values between 0.00005 µM and 5 µM, as measured in the MAGL assay described herein.

In one embodiment, the present invention provides compounds of formula (I) and their pharmaceutically acceptable salts or esters as described herein, wherein said compounds of formula (I) and their pharmaceutically acceptable salts or esters have an IC₅₀ for MAGL below 25 µM, preferably below 10 µM, more preferably below 5 µM as measured in an assay comprising the steps of:
a) providing a solution of a compound formula (I), or a pharmaceutically acceptable salt or ester thereof, in DMSO;
b) providing a solution of MAGL (recombinant wild-type) in assay buffer (50 mM tris(hydroxymethyl)aminomethane; 1 mM ethylenediaminetetraacetic acid);
c) adding 1 µL of compound solution from step a) to 19 µL of MAGL solution from step b);
d) shaking the mixture for 1 min at 2000 rpm;
e) incubating for 15 min at RT;
f) adding 20 µL of a solution of 4-nitrophenlyacetate in assay buffer (50 mM tris(hydroxymethyl)aminomethane; 1 mM ethylenediaminetetraacetic acid, 6% EtOH);
g) shaking the mixture for 1 min at 2000 rpm;
h) incubating for 5 min at RT;
i) measuring the absorbance of the mixture at 405 nm a fist time;
j) incubating a further 80 min at RT;
k) measuring the absorbance of the mixture at 405 nm a second time;
l) substracting the absorbance measured under i) from the absorbance measured under k) and calculating the slope of absorbance;
wherein:
i) the concentration of the compound of formula (I), or the pharmaceutically acceptable salt or ester thereof in the assay after step f) is in the range of 25 µM to 1.7 nM;
ii) the concentration of MAGL in the assay after step f) is 1 nM;
iii) the concentration of 4-nitrophenylacetate in the assay after step f) is 300 µM; and
iv) steps a) to l) are repeated for at least 3 times, each time with a different concentration of the compound of formula (I), or the pharmaceutically acceptable salt or ester thereof.

### Using the Compounds of the Invention

In one aspect, the present invention provides compounds of formula (I) as described herein for use as therapeutically active substance.

In a further aspect, the present invention provides the use of compounds of formula (I) as described herein for the treatment or prophylaxis of neuroinflammation, neurodegenerative diseases, pain, cancer and/or mental disorders in a mammal.

In one embodiment, the present invention provides the use of compounds of formula (I) as described herein for the treatment or prophylaxis of neuroinflammation and/or neurodegenerative diseases in a mammal.

In one embodiment, the present invention provides the use of compounds of formula (I) as described herein for the treatment or prophylaxis of neurodegenerative diseases in a mammal.

In one embodiment, the present invention provides the use of compounds of formula (I) as described herein for the treatment or prophylaxis of cancer in a mammal.

In one aspect, the present invention provides the use of compounds of formula (I) as described herein for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides the use of compounds of formula (I) as described herein for the treatment or prophylaxis of multiple sclerosis, Alzheimer's disease and/or Parkinson's disease in a mammal.

In a particularly preferred embodiment, the present invention provides the use of compounds of formula (I) as described herein for the treatment or prophylaxis of multiple sclerosis in a mammal.

In one aspect, the present invention provides compounds of formula (I) as described herein for use in the treatment or prophylaxis of neuroinflammation, neurodegenerative diseases, pain, cancer and/or mental disorders in a mammal.

In one embodiment, the present invention provides compounds of formula (I) as described herein for use in the treatment or prophylaxis of neuroinflammation and/or neurodegenerative diseases in a mammal.

In one embodiment, the present invention provides compounds of formula (I) as described herein for use in the treatment or prophylaxis of cancer in a mammal.

In one embodiment, the present invention provides compounds of formula (I) as described herein for use in the treatment or prophylaxis of neurodegenerative diseases in a mammal.

In one aspect, the present invention provides compounds of formula (I) as described herein for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides compounds of formula (I) as described herein for use in the treatment or prophylaxis of multiple sclerosis, Alzheimer's disease and/or Parkinson's disease in a mammal.

In a particularly preferred embodiment, the present invention provides compounds of formula (I) as described herein for use in the treatment or prophylaxis of multiple sclerosis in a mammal.

In one aspect, the present invention provides the use of compounds of formula (I) as described herein for the preparation of a medicament for the treatment or prophylaxis of neuroinflammation, neurodegenerative diseases, pain, cancer and/or mental disorders in a mammal.

In one embodiment, the present invention provides the use of compounds of formula (I) as described herein for the preparation of a medicament for the treatment or prophylaxis of neuroinflammation and/or neurodegenerative diseases in a mammal.

In one embodiment, the present invention provides the use of compounds of formula (I) as described herein for the preparation of a medicament for the treatment or prophylaxis of neurodegenerative diseases in a mammal.

In one embodiment, the present invention provides the use of compounds of formula (I) as described herein for the preparation of a medicament for the treatment or prophylaxis of cancer in a mammal.

In a further aspect, the present invention provides the use of compounds of formula (I) as described herein for the preparation of a medicament for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

In a preferred embodiment, the present invention provides the use of compounds of formula (I) as described herein for the preparation of a medicament for the treatment or prophylaxis of multiple sclerosis, Alzheimer's disease and/or Parkinson's disease in a mammal.

In a particularly preferred embodiment, the present invention provides the use of compounds of formula (I) as described herein for the preparation of a medicament for the treatment or prophylaxis of multiple sclerosis in a mammal.

In one aspect, the present invention provides a method for the treatment or prophylaxis of neuroinflammation, neurodegenerative diseases, pain, cancer and/or mental disorders in a mammal, which method comprises administering an effective amount of a compound of formula (I) as described herein to the mammal.

In one embodiment, the present invention provides a method for the treatment or prophylaxis of neuroinflammation and/or neurodegenerative diseases in a mammal, which method comprises administering an effective amount of a compound of formula (I) as described herein to the mammal.

In one embodiment, the present invention provides a method for the treatment or prophylaxis of neurodegenerative diseases in a mammal, which method comprises administering an effective amount of a compound of formula (I) as described herein to the mammal.

In one aspect, the present invention provides a method for the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression and/or pain in a mammal, which method comprises administering an effective amount of a compound of formula (I) as described herein to the mammal.

In a preferred embodiment, the present invention provides a method for the treatment or prophylaxis of multiple sclerosis, Alzheimer's disease and/or Parkinson's disease in a mammal, which method comprises administering an effective amount of a compound of formula (I) as described herein to the mammal.

In a particularly preferred embodiment, the present invention provides a method for the treatment or prophylaxis of multiple sclerosis in a mammal, which method comprises administering an effective amount of a compound of formula (I) as described herein to the mammal.

### Pharmaceutical Compositions and Administration

In one aspect, the present invention provides a pharmaceutical composition comprising a compound of formula (I) as described herein and a therapeutically inert carrier.

The compounds of formula (I) and their pharmaceutically acceptable salts and esters can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions).

The compounds of formula (I) and their pharmaceutically acceptable salts and esters can be processed with pharmaceutically inert, inorganic or organic adjuvants for the production of tablets, coated tablets, dragées and hard gelatin capsules. Lactose, corn starch or derivatives thereof, talc, stearic acid or its salts etc. can be used, for example, as such adjuvants for tablets, dragées and hard gelatin capsules.

Suitable adjuvants for soft gelatin capsules are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Abbreviations

AcOH = acetic acid, ACN = acetonitrile , Bn = benzyl, BINAP = (2,2'-bis(diphenylphosphino)-1,1'-binaphthyl), Boc = tert-butyloxycarbonyl, CAS RN = chemical abstracts registration number, Cbz = benzyloxycarbonyl, Cs₂CO₃ = cesium carbonate, CO = carbon monoxide, CuCl = copper(I) chloride, CuCN = copper(I) cyanide, CuI = copper(I) iodide, DAST = (diethylamino)sulfur trifluoride, DBU = 1,8-diazabicyclo[5,4,0]undec-7-ene, DEAD = diethyl azodicarboxylate, DIAD = diisopropyl azodicarboxylate, DMAP = 4-dimethylaminopyridine, DME = dimethoxyethane, DMEDA = N,N'-dimethylethylenediamine, DMF = N,N-dimethylformamide, DIPEA = N,N-diisopropylethylamine, dppf = 1,1 bis(diphenyl phosphino)ferrocene, EDC·HCl = N-(3-dimethylaminopropyl)-N'-ethylcarbodiimide hydrochloride, EI = electron impact, ESI = electrospray ionization, EtOAc = ethyl acetate, EtOH = ethanol, h = hour(s), FA = formic acid, H₂O = water, H₂SO₄= sulfuric acid, HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluoro-phosphate, HCl = hydrogen chloride, HOBt = 1-hydroxy-1H-benzotriazole, HPLC = high performance liquid chromatography, iPrMgCl = isopropylmagnesium chloride, I₂ = iodine, IPA = 2-propanol, ISP = ion spray positive (mode), ISN = ion spray negative (mode), K₂CO₃ = potassium carbonate, KHCO₃ = potassium bicarbonate, KI = potassium iodide, KOH = potassium hydroxide, K₃PO₄= potassium phosphate tribasic, LiAlH₄ or LAH = lithium aluminium hydride, LiHMDS = lithium bis(trimethylsilyl)amide, LiOH = lithium hydroxide, mCPBA = meta-chloroperoxybenzoic acid, MgSO₄ = magnesium sulfate, min = minute(s), mL = milliliter, MPLC = medium pressure liquid chromatography, MS = mass spectrum, nBuLi = n-butyllithium, NaBH₃CN = sodium cyanoborohydride, NaH = sodium hydride, NBS = N-bromosuccinimide, NaHCOs = sodium hydrogen carbonate, NaNO₂ = sodium nitrite, NaBH(OAc)₃ = sodium triacetoxyborohydride, NaOH = sodium hydroxide, Na₂CO₃ = sodium carbonate, Na₂SO₄ = sodium sulfate, Na₂S₂O₃ = sodium thiosulfate, NBS = N-bromosuccinimide, nBuLi = n-butyllithium, NEt₃ = triethylamine (TEA), NH₄Cl = ammonium chloride, NMP = N-methyl-2-pyrrolidone, OAc = Acetoxy, T₃P = propylphosphonic anhydride, PE = petroleum ether, PG = protective group, Pd-C = palladium on activated carbon, PdCl₂(dppf)-CH₂Cl₂ = 1,1'-bis(diphenylphosphino)ferrocene-palladium(II)dichloride dichloromethane complex, Pd₂(dba)₃ = tris(dibenzylideneacetone)dipalladium(0), Pd(OAc)₂ = palladium(II) acetate, Pd(OH)₂ = palladium hydroxide, Pd(PPh₃)₄ = tetrakis(triphenylphosphine)palladium(0), PTSA = p-toluenesulfonic acid, R = any group, RP = reverse phase, RT = room temperature, SFC = Supercritical Fluid Chromatography, S-PHOS = 2-dicyclohexylphosphino-2',6'-dimethoxybiphenyl, TBAI = tetra butyl ammonium iodine, TEA = triethylamine, TFA = trifluoroacetic acid, THF = tetrahydrofuran, TMEDA = N,N,N',N'-tetramethylethylenediamine, ZnCl₂ = zinc chloride, Hal = halogen.

### Example 1

### (4-(5-Methyloxazolo [4,5-b] pyridin-2-yl)piperazin-1-yl)(2-phenylbenzo [d] oxazol-6-yl)methanone

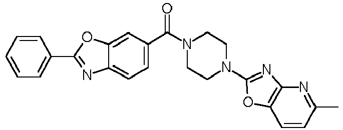

To a solution of 2-phenylbenzo[d]oxazole-6-carboxylic acid (54.8 mg, 229 µmol), HATU (95.8 mg, 252 µmol) and DIPEA (118 mg, 160 µL, 916 µmol) in DMF (0.5 mL) was added 5-methyl-2-(piperazin-1-yl)oxazolo[4,5-b]pyridine (50 mg, 229 µmol) and the mixture was stirred at RT overnight. To the light brown suspension was added dropwise water (1 mL) and the suspension was filtered. The filter cake was washed with plenty of water and dried to provide the title compound (70 mg, 69.5%) as a light brown solid. MS (ESI): m/z = 440.2 [M+H]⁺.

In analogy to example 1, example 2 to 141, 259, 273 and 274 of the following table were prepared by coupling an acid derivative with an amine using HATU or T₃P as coupling reagent and a suitable amine base such as DIPEA or TEA. Where necessary, the crude reaction mixtures were purified by HPLC (FA) and lyophilized to yield the title compounds.

| **Ex.** | **Structure** | **Systematic Name** | **Building Blocks** | **MS, ESI: m/z** |
|---|---|---|---|---|
| 2 | 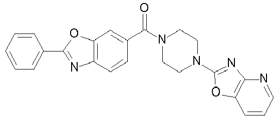 | (4-(oxazolo[4,5-b]pyridin-2-yl)piperazin-1-yl)(2-phenylbenzo[d]oxazol-6-yl)methanone | CAS 594839-90-4 | 426.3 [M+H]⁺ |
| | | | and | |
| | | | CAS 113520-22-2 | |
| 3 | 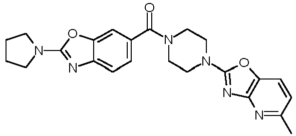 | 4-(5-methyloxazolo[4,5-b]pyridin-2-yl)piperazin-1-yl)(2-(pyrrolidin-1-yl)benzo[d]oxazol-6-yl)methanone | CAS 1393746-51-4 | 433.2 [M+H]⁺ |
| | | | and | |
| | | | CAS 1035840-99-3 | |
| 4 | 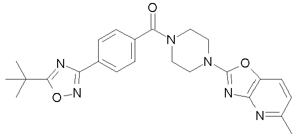 | (4-(5-(tert-butyl)-1,2,4-oxadiazol-3-yl)phenyl)(4-(5-methyloxazolo[4,5-b]pyridin-2-yl)piperazin-1-yl)methanone | CAS 1119452-72-0 | 447.2 [M+H]⁺ |
| | | | and | |
| | | | CAS 1035840-99-3 | |
| 5 | 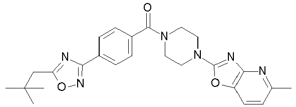 | (4-(5-methyloxazolo [4,5-b]pyridin-2-yl)piperazin-1-yl)(4-(5-neopentyl-1,2,4-oxadiazol-3-yl)phenyl)methanone | CAS 1305938-51-5 | 461.2 [M+H]⁺ |
| | | | and | |
| | | | CAS 1035840-99-3 | |
| 6 | 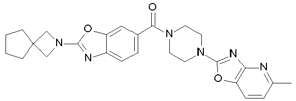 | ((2-(2-azaspiro[3.4]octan-2-yl)benzo[d]oxazol-6-yl)(4-(5-methyloxazolo[4,5-b]pyridin-2-yl)piperazin-1-yl)methanone | A.1 | 473.2 [M+H]⁺ |
| | | | and | |
| | | | CAS 1035840-99-3 | |
| 7 | 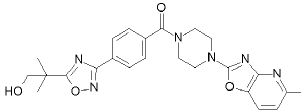 | (4-(5-(1-hydroxy-2-methylpropan-2-yl)-1,2,4-oxadiazol-3-yl)phenyl)(4-(5-methyloxazolo[4,5-b]pyridin-2-yl)piperazin-1-yl)methanone | A.2 | 463.2 [M+H]⁺ |
| | | | and | |
| | | | CAS 1035840-99-3 | |
| 8 | 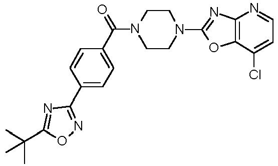 | (4-(5-(tert-butyl)-1,2,4-oxadiazol-3-yl)phenyl)(4-(7-chlorooxazolo[4,5- b]pyridin-2-yl)piperazin-1-yl)methanone | CAS 1119452-72-0 | 467.3 [M+H]⁺ |
| | | | and | |
| | | | B.1 | |
| 9 | 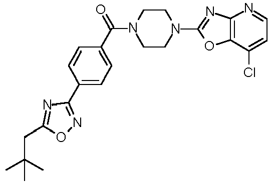 | (4-(7-chlorooxazolo[4,5-b]pyridin-2-yl)piperazin-1-yl)(4-(5- neopentyl-1,2,4-oxadiazol-3-yl)phenyl)methanone | CAS 1305938-51-5 | 481.3 [M+H]⁺ |
| | | | and | |
| | | | B.1 | |
| 10 | 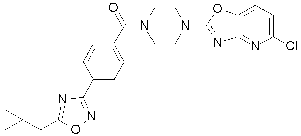 | (4-(5-chlorooxazolo[4,5-b]pyridin-2-yl)piperazin-1-yl)(4-(5- neopentyl-1,2,4-oxadiazol-3-yl)phenyl)methanone | CAS 1305938-51-5 | 481.3 [M+H]⁺ |
| | | | and | |
| | | | B.2 | |
| 11 | 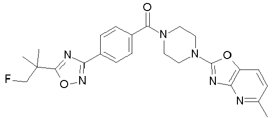 | (4-(5-(1-fluoro-2-methylpropan-2-yl)-1,2,4-oxadiazol-3-yl)phenyl)(4-(5-methyloxazolo[4,5-b]pyridin-2-yl)piperazin-1-yl)methanone | A.3 | 465.3 [M+H]⁺ |
| | | | and | |
| | | | CAS 1035840-99-3 | |
| 12 | 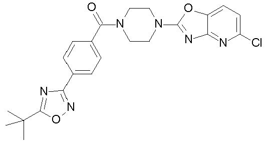 | [4-(5-tert-butyl-1,2,4-oxadiazol-3-yl)phenyl]- [4-(5-chlorooxazolo[4,5-b]pyridin-2-yl)piperazin-1-yl]methanone | CAS 1119452-72-0 | 467.2 [M+H]⁺ |
| | | | and | |
| | | | B.2 | |
| 13 | 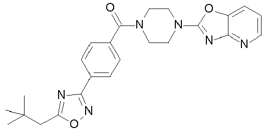 | (4-(5-neopentyl-1,2,4-oxadiazol-3-yl)phenyl)(4-(oxazolo[4,5-b]pyridin-2-yl)piperazin-1-yl)methanone | CAS 1305938-51-5 | 447.3 [M+H]⁺ |
| | | | and | |
| | | | CAS 113520-22-2 | |
| 14 | 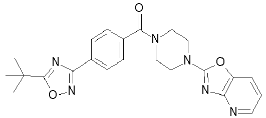 | (4-(5-(tert-butyl)-1,2,4-oxadiazol-3-yl)phenyl)(4-(oxazolo[4,5-b]pyridin-2-yl)piperazin-1-yl)methanone | CAS 1119452-72-0 | 433.3 [M+H]⁺ |
| | | | and | |
| | | | CAS 113520-22-2 | |
| 15 | 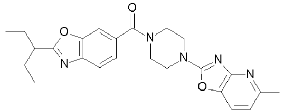 | [2-(1-ethylpropyl)-1,3-benzoxazol-6-yl]-[4-(5-methyloxazolo[4,5-b]pyridin-2-yl)piperazin-1-yl]methanone | CAS 1528651-83-3 | 434.4 [M+H]⁺ |
| | | | and | |
| | | | CAS 1035840-99-3 | |
| 16 | 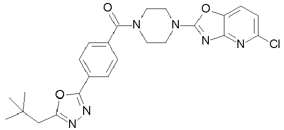 | [4-(5-chlorooxazolo[4,5-b]pyridin-2-yl)piperazin-1-yl]-[4-[5-(2,2-dimethylpropyl)-1,3,4-oxadiazol-2-yl]phenyl]methanone | CAS 1275943-26-4 | 481.2 [M+H]⁺ |
| | | | and | |
| | | | B.2 | |
| 17 | 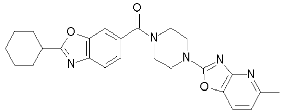 | (2-cyclohexyl-1,3-benzoxazol-6-yl)-[4-(5-methyloxazolo[4,5-b]pyridin-2-yl)piperazin-1-yl]methanone | CAS 1181353-33-2 | 446.2 [M+H]⁺ |
| | | | and CAS 1035840-99-3 | |
| 18 | 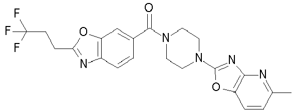 | [4-(5-methyloxazolo[4,5-b]pyridin-2-yl)piperazin-1-yl]-[2-(3,3,3-trifluoropropyl)-1,3-benzoxazol-6-yl]methanone | CAS 1542602-23-2 | 460.0 [M+H]⁺ |
| | | | and | |
| | | | CAS 1035840-99-3 | |
| 19 | 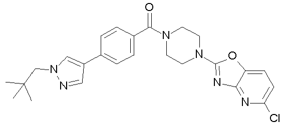 | [4-(5-chlorooxazolo[4,5-b]pyridin-2-yl)piperazin-1-yl]-[4-[1-(2,2-dimethylpropyl)pyrazol -4-yl]phenyl]methanone | A.4 | 479.3 [M+H]⁺ |
| | | | and | |
| | | | B.2 | |
| 20 | 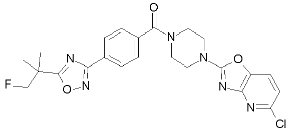 | (4-(5-chlorooxazolo[4,5-b]pyridin-2-yl)piperazin-1-yl)(4-(5- (1-fluoro-2-methylpropan-2-yl)-1,2,4-oxadiazol-3-yl)phenyl)methanone | A.3 | 485.2 [M+H]⁺ |
| | | | and | |
| | | | B.2 | |
| 21 | 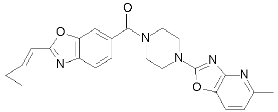 | [2-[(E)-but-1-enyl]-1,3-benzoxazol-6-yl]-[4-(5-methyloxazolo[4,5-b]pyridin-2-yl)piperazin-1-yl]methanone | A.5 | 418.2 [M+H]⁺ |
| | | | and CAS 1035840-99-3 | |
| 22 | 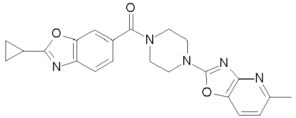 | (2-cyclopropyl-1,3-benzoxazol-6-yl)-[4-(5-methyloxazolo [4,5-b]pyridin-2-yl)piperazin-1-yl] methanone | A. 6 | 404.2 [M+H]⁺ |
| | | | and | |
| | | | CAS 1035840-99-3 | |
| 23 | 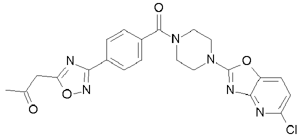 | 1-[3-[4-[4-(5-chlorooxazolo[4,5-b]pyridin-2-yl)piperazine-1-carbonyl]phenyl]-1,2,4-oxadiazol-5-yl]propan-2-one | A. 7 | 467.2 [M+H]⁺ |
| | | | and | |
| | | | B.2 | |
| 24 | 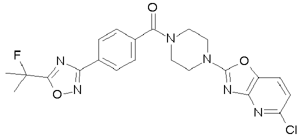 | [4-(5-chlorooxazolo [4,5- b]pyridin-2-yl)piperazin-1-yl]-[4-[5-(1-fluoro-1-methylethyl)-1,2,4-oxadiazol-3 -yl]phenyl] methanone | A. 8 | 471.2 [M+H]⁺ |
| | | | and | |
| | | | B.2 | |
| 25 | 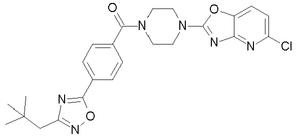 | (4-(5-chlorooxazolo[4,5- b]pyridin-2-yl)piperazin-1-yl)(4-(3- neopentyl-1,2,4-oxadiazol-5-yl)phenyl)methanone | CAS 1488565-99-6 | 481.1 [M+H]⁺ |
| | | | and | |
| | | | B.2 | |
| 26 | 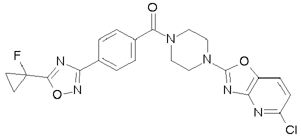 | [4-(5-chlorooxazolo[4,5-b]pyridin-2-yl)piperazin-1-yl]-[4-[5-(1-fluorocyclopropyl)-1,2,4-oxadiazol-3-yl]phenyl]methanone | A.9 | 469.1 [M+H]⁺ |
| | | | and | |
| | | | B.2 | |
| 27 | 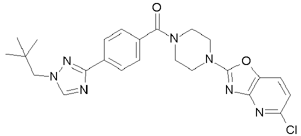 | [4-(5-chlorooxazolo[4,5-b]pyridin-2-yl)piperazin-1-yl]-[4-[1-(2,2-dimethylpropyl)-1,2,4-triazol-3-yl]phenyl]methanone | A.10 | 480.2 [M+H]⁺ |
| | | | and | |
| | | | B.2 | |
| 28 | 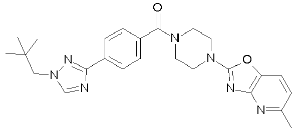 | [4-[1-(2,2-dimethylpropyl)-1,2,4-triazol-3-yl]phenyl]-[4-(5-methyloxazolo[4,5-b]pyridin-2-yl)piperazin-1-yl]methanone | A.10 | 460.2 [M+H]⁺ |
| | | | and | |
| | | | CAS 1035840-99-3 | |
| 29 | 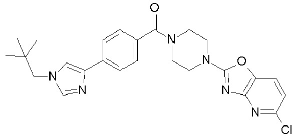 | [4-(5-chlorooxazolo[4,5-b]pyridin-2-yl)piperazin-1-yl]-[4-[1-(2,2-dimethylpropyl)imidazo 1-4-yl]phenyl]methanone | A.11 | 479.3 [M+H]⁺ |
| | | | and | |
| | | | B.2 | |
| 30 | 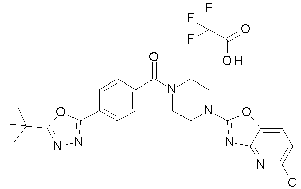 | [4-(5-tert-butyl-1,3,4-oxadiazol-2-yl)phenyl]- [4-(5-chlorooxazolo[4,5-b]pyridin-2-yl)piperazin-1-yl]methanone; 2,2,2-trifluoroacetic acid salt | CAS 1406499-71-5 | 467.1 [M+H]⁺ |
| | | | and | |
| | | | B.2 | |
| 31 | 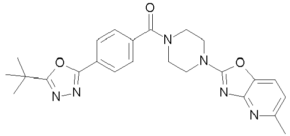 | [4-(5-tert-butyl-1,3,4-oxadiazol-2-yl)phenyl]- [4-(5-methyloxazolo [4,5-b]pyridin-2-yl)piperazin-1-yl]methanone | CAS 1406499-71-5 | 447.3 [M+H]⁺ |
| | | | and | |
| | | | CAS 1035840-99-3 | |
| 32 | 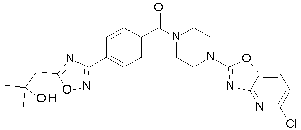 | [4-(5-chlorooxazolo[4,5-b]pyridin-2-yl)piperazin-1-yl]-[4-[5-(2-hydroxy-2-methyl-propyl)-1,2,4-oxadiazol-3-yl]phenyl]methanone | A. 12 | 483.2 [M+H]⁺ |
| | | | and | |
| | | | B.2 | |
| 33 | 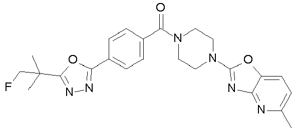 | [4-[5-(2-fluoro-1,1-dimethyl-ethyl)-1,3,4-oxadiazol-2-yl]phenyl]-[4-(5-methyloxazolo[4,5-b]pyridin-2-yl)piperazin-1-yl] methanone | A. 13 | 465.3 [M+H]⁺ |
| | | | and | |
| | | | CAS 1035840-99-3 | |
| 34 | 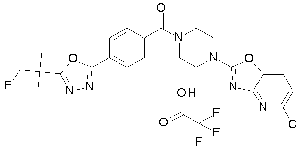 | [4-(5-chlorooxazolo[4,5-b]pyridin-2-yl)piperazin-1-yl]-[4-[5-(2-fluoro-1,1-dimethyl-ethyl)-1,3,4-oxadiazol-2-yl]phenyl]methanone; 2,2,2-trifluoroacetic acid salt | A. 13 | 485.2 [M+H]⁺ |
| | | | and | |
| | | | B.2 | |
| 35 | 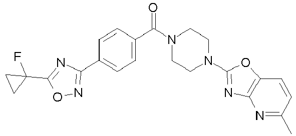 | [4-[5-(1-fluorocyclopropyl)-1,2,4-oxadiazol-3-yl]phenyl]-[4-(5-methyloxazolo[4,5-b]pyridin-2-yl)piperazin-1-yl]methanone | A.9 | 449.1 [M+H]⁺ |
| | | | and | |
| | | | CAS 1035840-99-3 | |
| 36 | 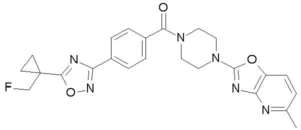 | [4-[5-[1-(fluoromethyl)cyclopro pyl]-1,2,4-oxadiazol-3-yl]phenyl]-[4-(5-methyloxazolo[4,5-b]pyridin-2-yl)piperazin-1-yl]methanone | A. 16 | 463.1 [M+H]⁺ |
| | | | and | |
| | | | CAS 1035840-99-3 | |
| 37 | 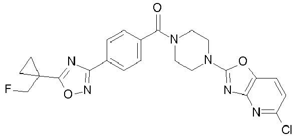 | [4-(5-chlorooxazolo[4,5-b]pyridin-2-yl)piperazin-1-yl]-[4-[5-[1-(fluoromethyl)cyclopro pyl]-1,2,4-oxadiazol-3-yl]phenyl]methanone | A. 16 | 483.1 [M+H]⁺ |
| | | | and | |
| | | | B.2 | |
| 38 | 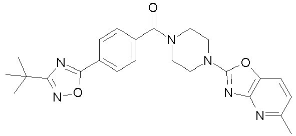 | [4-(3-tert-butyl-1,2,4-oxadiazol-5-yl)phenyl]-[4-(5-methyloxazolo [4,5-b]pyridin-2-yl)piperazin-1-yl]methanone | CAS 1281761-55-4 | 447.3 [M+H]⁺ |
| | | | and | |
| | | | CAS 1035840-99-3 | |
| 39 | 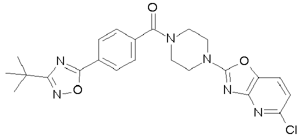 | [4-(3-tert-butyl-1,2,4-oxadiazol-5-yl)phenyl]-[4-(5-chlorooxazolo[4,5-b]pyridin-2-yl)piperazin-1-yl]methanone | CAS 1281761-55-4 | 467.3 [M+H]⁺ |
| | | | and | |
| | | | B.2 | |
| 40 | 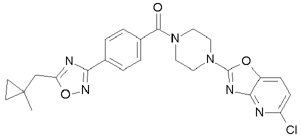 | [4-(5-chlorooxazolo[4,5-b]pyridin-2-yl)piperazin-1-yl]-[4-[5-[(1-methylcyclopropyl)met hyl]-1,2,4-oxadiazol-3-yl]phenyl]methanone | A. 17 | 479.1 [M+H]⁺ |
| | | | and | |
| | | | B.2 | |
| 41 | 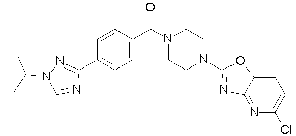 | [4-(1-tert-butyl-1,2,4-triazol-3-yl)phenyl]-[4-(5-chlorooxazolo[4,5- b]pyridin-2-yl)piperazin-1-yl] methanone | A.18 | 466.2 [M+H]⁺ |
| | | | and | |
| | | | B.2 | |
| 42 | 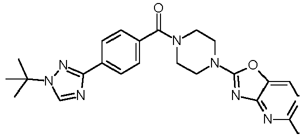 | [4-(1-tert-butyl-1,2,4-triazol-3-yl)phenyl]-[4-(5-methyloxazolo[4,5-b]pyridin-2-yl)piperazin-1-yl] methanone | A.18 | 446.2 [M+H]⁺ |
| | | | and | |
| | | | CAS 1035840-99-3 | |
| 43 | 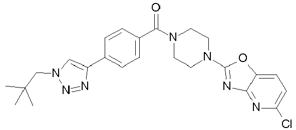 | (4-(5-chlorooxazolo[4,5-b]pyridin-2-yl)piperazin-1-yl)(4-(1- neopentyl-1H-1,2,3-triazol-4-yl)phenyl)methanone | A. 19 | 480.1 [M+H]⁺ |
| | | | and | |
| | | | B.2 | |
| 44 | 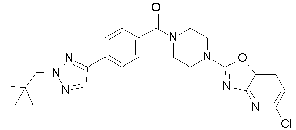 | (4-(5-chlorooxazolo[4,5-b]pyridin-2-yl)piperazin-1-yl)(4-(2-neopentyl-2H-1,2,3-triazol-4-yl)phenyl)methanone | A.20 | 480.1 [M+H]⁺ |
| | | | and | |
| | | | B.2 | |
| 45 | 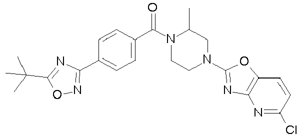 | [4-(5-tert-butyl-1,2,4-oxadiazol-3-yl)phenyl]-[4-(5-chlorooxazolo[4,5-b]pyridin-2-yl)-2-methyl-piperazin-1-yl]methanone | CAS 1119452-72-0 | 481.1 [M+H]⁺ |
| | | | and | |
| | | | B.4 | |
| 46 | 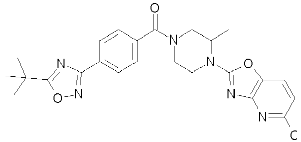 | [4-(5-tert-butyl-1,2,4-oxadiazol-3-yl)phenyl]-[4-(5-chlorooxazolo[4,5-b]pyridin-2-yl)-3-methyl-piperazin-1-yl]methanone | CAS 1119452-72-0 | 481.1 [M+H]⁺ |
| | | | and | |
| | | | B.5 | |
| 47 | 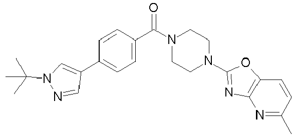 | [4-(1-tert-butylpyrazol-4-yl)phenyl]-[4-(5-methyloxazolo [4,5-b]pyridin-2-yl)piperazin-1-yl] methanone | A.21 | 445.4 [M+H]⁺ |
| | | | and | |
| | | | CAS 1035840-99-3 | |
| 48 | 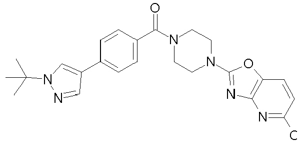 | [4-(1-tert-butylpyrazol-4-yl)phenyl]-[4-(5-chlorooxazolo[4,5-b]pyridin-2-yl)piperazin-1-yl] methanone | A.21 | 465.3 [M+H]⁺ |
| | | | and | |
| | | | B.2 | |
| 49 | 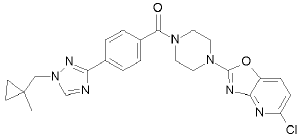 | [4-(5-chlorooxazolo[4,5-b]pyridin-2-yl)piperazin-1-yl]-[4-[1-[(1-methylcyclopropyl)met hyl]-1,2,4-triazol-3-yl]phenyl]methanone | A.22 | 478.3 [M+H]⁺ |
| | | | and | |
| | | | B.2 | |
| 50 | 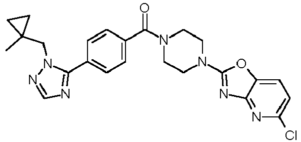 | [4-(5-chlorooxazolo[4,5-b]pyridin-2-yl)piperazin-1-yl]-[4-[2-[(1-methylcyclopropyl)met hyl]-1,2,4-triazol-3-yl]phenyl]methanone | A.22 | 478.3 [M+H]⁺ |
| | | | and | |
| | | | B.2 | |
| 51 | 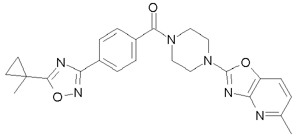 | [4-[5-(1-methylcyclopropyl)-1,2,4-oxadiazol-3-yl]phenyl]-[4-(5-methyloxazolo[4,5-b]pyridin-2-yl)piperazin-1-yl] methanone | A.23 | 445.2 [M+H]⁺ |
| | | | and | |
| | | | CAS 1035840-99-3 | |
| 52 | 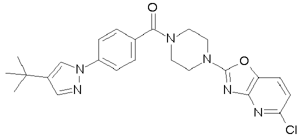 | [4-(4-tert-butylpyrazol-1-yl)phenyl]-[4-(5-chlorooxazolo[4,5- b]pyridin-2-yl)piperazin-1-yl]methanone | CAS 2105841-23-2 | 465.3 [M+H]⁺ |
| | | | and | |
| | | | B.2 | |
| 53 | 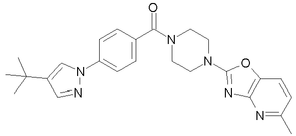 | [4-(4-tert-butylpyrazol-1-yl)phenyl]-[4-(5-methyloxazolo[4,5-b]pyridin-2-yl)piperazin-1-yl]methanone | CAS 2105841-23-2 | 445.4 [M+H]⁺ |
| | | | and | |
| | | | CAS 1035840-99-3 | |
| 54 | 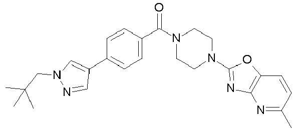 | [4-[1-(2,2-dimethylpropyl)pyrazol -4-yl]phenyl]-[4-(5-methyloxazolo[4,5-b]pyridin-2-yl)piperazin-1-yl] methanone | A.4 | 459.4 [M+H]⁺ |
| | | | and | |
| | | | CAS 1035840-99-3 | |
| 55 | 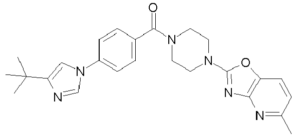 | [4-(4-tert-butylimidazol-1-yl)phenyl]-[4-(5-methyloxazolo[4,5-b]pyridin-2-yl)piperazin-1-yl]methanone | CAS 1368435-89-5 | 445.2 [M+H]⁺ |
| | | | and | |
| | | | CAS 1035840-99-3 | |
| 56 | 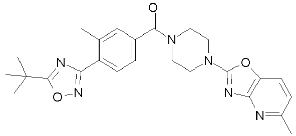 | [4-(5-tert-butyl-1,2,4-oxadiazol-3-yl)-3-methyl-phenyl]-[4-(5- methyloxazolo[4,5-b]pyridin-2-yl)piperazin-1-yl]methanone | A.24 | 461.2 [M+H]⁺ |
| | | | and | |
| | | | CAS 1035840-99-3 | |
| 57 | 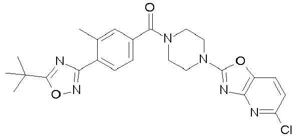 | [4-(5-tert-butyl-1,2,4-oxadiazol-3-yl)-3-methyl-phenyl]-[4-(5-chlorooxazolo[4,5-b]pyridin-2-yl)piperazin-1-yl]methanone | A.24 | 481.1 [M+H]⁺ |
| | | | and | |
| | | | B.2 | |
| 58 | 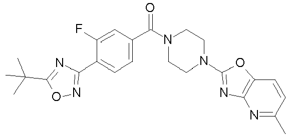 | [4-(5-tert-butyl-1,2,4-oxadiazol-3-yl)-3-fluoro-phenyl]-[4-(5-methyloxazolo[4,5-b]pyridin-2-yl)piperazin-1-yl] methanone | A.25 | 465.4 [M+H]⁺ |
| | | | and | |
| | | | CAS 1035840-99-3 | |
| 59 | 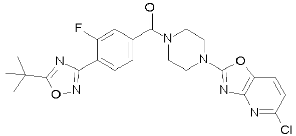 | [4-(5-tert-butyl-1,2,4-oxadiazol-3-yl)-3-fluoro-phenyl]-[4-(5-chlorooxazolo[4,5-b]pyridin-2-yl)piperazin-1-yl]methanone | A.25 | 485.2 [M+H]⁺ |
| | | | and | |
| | | | B.2 | |
| 60 | 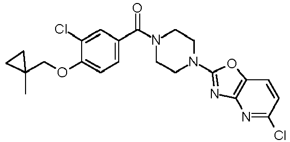 | [3-chloro-4-[(1-methylcyclopropyl)met hoxy]phenyl]-[4-(5-chlorooxazolo[4,5-b]pyridin-2-yl)piperazin-1-yl]methanone | A.26 | 461.2 [M+H]⁺ |
| | | | and | |
| | | | B.2 | |
| 61 | 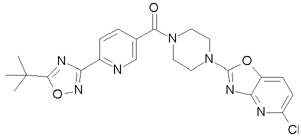 | [6-(5-tert-butyl-1,2,4-oxadiazol-3-yl)-3-pyridyl]-[4-(5-chlorooxazolo[4,5-b]pyridin-2-yl)piperazin-1-yl]methanone | A. 27 | 468.2 [M+H]⁺ |
| | | | and | |
| | | | B.2 | |
| 62 | 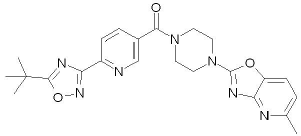 | [6-(5-tert-butyl-1,2,4-oxadiazol-3-yl)-3-pyridyl]-[4-(5-methyloxazolo[4,5-b]pyridin-2-yl)piperazin-1-yl] methanone | A.27 | 448.3 [M+H]⁺ |
| | | | and | |
| | | | CAS 1035840-99-3 | |
| 63 | 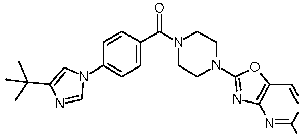 | [4-(4-tert-butylimidazol-1-yl)phenyl]-[4-(5-chlorooxazolo[4,5-b]pyridin-2-yl)piperazin-1-yl]methanone | CAS 1368435-89-5 | 465.1 [M+H]⁺ |
| | | | and | |
| | | | B.2 | |
| 64 | 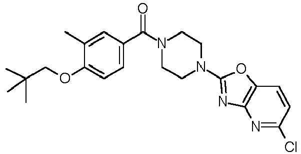 | [4-(5-chlorooxazolo[4,5-b]pyridin-2-yl)piperazin-1-yl]-[4-(2,2-dimethylpropoxy)-3-methyl-phenyl] methanone | A.28 | 443.2 [M+H]⁺ |
| | | | and | |
| | | | B.2 | |
| 65 | 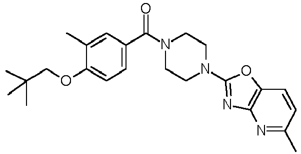 | [4-(2,2-dimethylpropoxy)-3- methyl-phenyl]-[4-(5-methyloxazolo[4,5-b]pyridin-2-yl)piperazin-1-yl]methanone | A.28 | 423.2 [M+H]⁺ |
| | | | and | |
| | | | CAS 1035840-99-3 | |
| 66 | 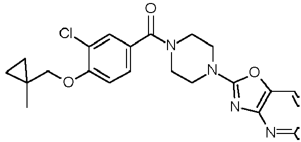 | [3-chloro-4-[(1-methylcyclopropyl)met hoxy]phenyl]-[4-(5-methyloxazolo[4,5-b]pyridin-2-yl)piperazin-1-yl]methanone | A.26 | 441.1 [M+H]⁺ |
| | | | and | |
| | | | CAS 1035840-99-3 | |
| 67 | 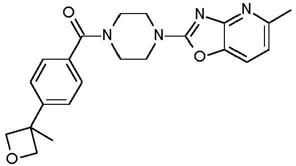 | (4-(5-methyloxazolo[4,5-b]pyridin-2-yl)piperazin-1-yl)(4-(3- methyloxetan-3-yl)phenyl)methanone | CAS 1315567-78-2 | 393.3 [M+H]⁺ |
| | | | and | |
| | | | CAS 1035840-99-3 | |
| 68 | 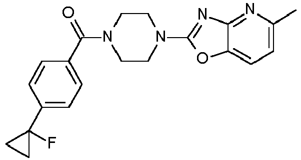 | (4-(1-fluorocyclopropyl)phen yl)(4-(5-methyloxazolo[4,5-b]pyridin-2-yl)piperazin-1-yl)methanone | CAS 946118-80-5 | 381.3 [M+H]⁺ |
| | | | and | |
| | | | CAS 1035840-99-3 | |
| 69 | 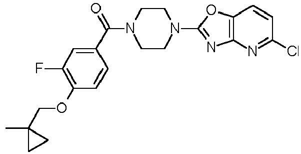 | [4-(5-chlorooxazolo[4,5-b]pyridin-2-yl)piperazin-1-yl]-[3-fluoro-4-[(1-methylcyclopropyl)met hoxy]phenyl]methanon e | A.29 | 445.3 [M+H]⁺ |
| | | | and | |
| | | | B.2 | |
| 70 | 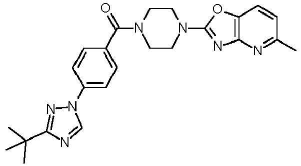 | [4-(3-tert-butyl-1,2,4-triazol-1-yl)phenyl]-[4-(5-methyloxazolo[4,5-b]pyridin-2-yl)piperazin-1-yl]methanone | A.30 | 446.3 [M+H]⁺ |
| | | | and | |
| | | | CAS 1035840-99-3 | |
| 71 | 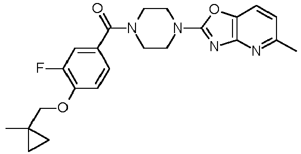 | [3-fluoro-4-[(1-methylcyclopropyl)met hoxy]phenyl]-[4-(5-methyloxazolo[4,5-b]pyridin-2-yl)piperazin-1-yl]methanone | A.29 | 425.2 [M+H]⁺ |
| | | | and | |
| | | | CAS 1035840-99-3 | |
| 72 | 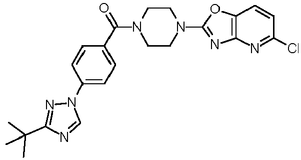 | [4-(3-tert-butyl-1,2,4-triazol-1-yl)phenyl]-[4-(5-chlorooxazolo[4,5- b]pyridin-2-yl)piperazin-1-yl]methanone | A.30 | 466.3 [M+H]⁺ |
| | | | and | |
| | | | B.2 | |
| 73 | 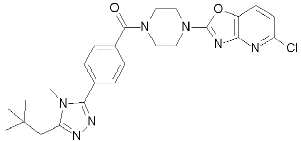 | [4-(5-chlorooxazolo[4,5-b]pyridin-2-yl)piperazin-1-yl]-[4-[5-(2,2-dimethylpropyl)-4-methyl-1,2,4-triazol-3- yl]phenyl]methanone | A.31 | 494.3 [M+H]⁺ |
| | | | and | |
| | | | B.2 | |
| 74 | 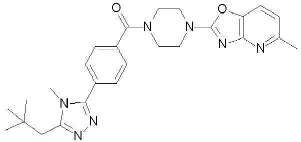 | [4-[5-(2,2-dimethylpropyl)-4-methyl-1,2,4-triazol-3- yl]phenyl]-[4-(5-methyloxazolo[4,5-b]pyridin-2-yl)piperazin-1-yl]methanone | A.31 | 474.2 [M+H]⁺ |
| | | | and | |
| | | | CAS 1035840-99-3 | |
| 75 | 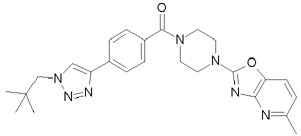 | [4-[1-(2,2-dimethylpropyl)triazol-4-yl]phenyl]-[4-(5-methyloxazolo[4,5-b]pyridin-2-yl)piperazin-1-yl]methanone | A.19 | 460.3 [M+H]⁺ |
| | | | and CAS 1035840-99-3 | |
| 76 | 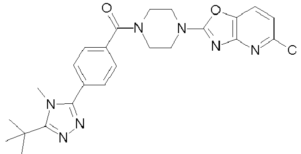 | [4-(5-tert-butyl-4-methyl-1,2,4-triazol-3- yl)phenyl]-[4-(5-chlorooxazolo[4,5- b]pyridin-2-yl)piperazin-1-yl] methanone | A.32 | 480.1 [M+H]⁺ |
| | | | and | |
| | | | B.2 | |
| 77 | 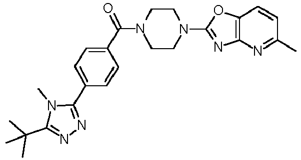 | [4-(5-tert-butyl-4-methyl-1,2,4-triazol-3- yl)phenyl]-[4-(5-methyloxazolo[4,5-b]pyridin-2-yl)piperazin-1-yl]methanone | A.32 | 460.3 [M+H]⁺ |
| | | | and | |
| | | | CAS 1035840-99-3 | |
| 78 | 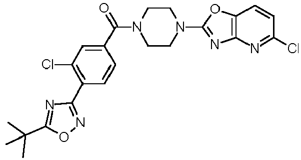 | [4-(5-tert-butyl-1,2,4-oxadiazol-3-yl)-3-chloro-phenyl]-[4-(5- chlorooxazolo[4,5- b]pyridin-2-yl)piperazin-1-yl]methanone | A.33 | 501.1 [M+H]⁺ |
| | | | and | |
| | | | B.2 | |
| 79 | 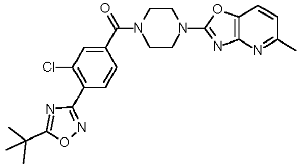 | [4-(5-tert-butyl-1,2,4-oxadiazol-3-yl)-3-chloro-phenyl]-[4-(5- methyloxazolo[4,5-b]pyridin-2-yl)piperazin-1-yl]methanone | A.33 | 481.2 [M+H]⁺ |
| | | | and CAS 1035840-99-3 | |
| 80 | 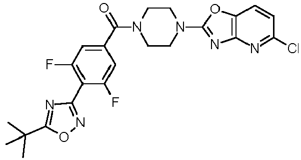 | [4-(5-tert-butyl-1,2,4-oxadiazol-3-yl)-3,5-difluoro-phenyl]-[4-(5-chlorooxazolo[4,5-b]pyridin-2-yl)piperazin-1-yl]methanone | A.34 | 503.2 [M+H]⁺ |
| | | | and | |
| | | | B.2 | |
| 81 | 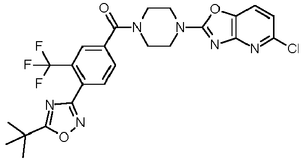 | [4-(5-tert-butyl-1,2,4-oxadiazol-3-yl)-3-(trifluoromethyl)phenyl ]-[4-(5-chlorooxazolo[4,5- b]pyridin-2-yl)piperazin-1-yl]methanone | A.35 | 535.2 [M+H]⁺ |
| | | | and | |
| | | | B.2 | |
| 82 | 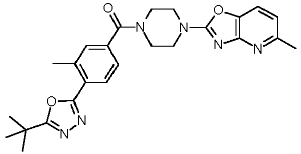 | [4-(5-tert-butyl-1,3,4-oxadiazol-2-yl)-3-methyl-phenyl]-[4-(5-methyloxazolo[4,5-b]pyridin-2-yl)piperazin-1-yl] methanone | A.36 | 461.1 [M+H]⁺ |
| | | | and | |
| | | | CAS 1035840-99-3 | |
| 83 | 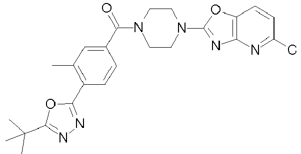 | [4-(5-tert-butyl-1,3,4-oxadiazol-2-yl)-3-methyl-phenyl]-[4-(5-chlorooxazolo[4,5-b]pyridin-2-yl)piperazin-1-yl] methanone | A.36 | 481.2 [M+H]⁺ |
| | | | and | |
| | | | B.2 | |
| 84 | 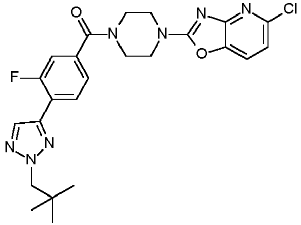 | (4-(5-chlorooxazolo[4,5-b]pyridin-2-yl)piperazin-1-yl)(3-fluoro-4-(2-neopentyl-2H-1,2,3-triazol-4-yl)phenyl)methanone | A.38 | 498.3 [M+H]⁺ |
| | | | and | |
| | | | B.2 | |
| 85 | 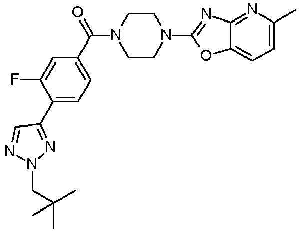 | (3-fluoro-4-(2-neopentyl-2H-1,2,3-triazol-4-yl)phenyl)(4-(5-methyloxazolo[4,5-b]pyridin-2-yl)piperazin-1-yl)methanone | A.38 | 478.4 [M+H]⁺ |
| | | | and | |
| | | | CAS 1035840-99-3 | |
| 86 | 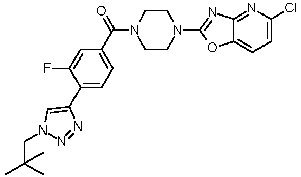 | (4-(5-chlorooxazolo[4,5-b]pyridin-2-yl)piperazin-1-yl)(3-fluoro-4-(1-neopentyl-1H-1,2,3-triazol-4-yl)phenyl)methanone | A.37 | 498.3 [M+H]⁺ |
| | | | and | |
| | | | B.2 | |
| 87 | 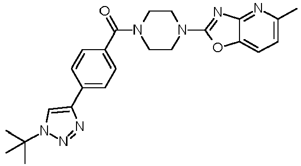 | (4-(1-(tert-butyl)-1H-1,2,3-triazol-4-yl)phenyl)(4-(5-methyloxazolo[4,5-b]pyridin-2-yl)piperazin-1-yl)methanone | A.39 | 446.4 [M+H]⁺ |
| | | | and CAS 1035840-99-3 | |
| 88 | 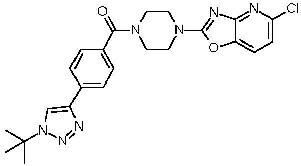 | (4-(1-(tert-butyl)-1H-1,2,3-triazol-4-yl)phenyl)(4-(5-chlorooxazolo[4,5-b]pyridin-2-yl)piperazin-1-yl)methanone | A.39 | 466.3 [M+H]⁺ |
| | | | and | |
| | | | B.2 | |
| 89 | 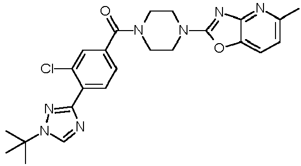 | (4-(1-(tert-butyl)-1H-1,2,4-triazol-3-yl)-3- chlorophenyl)(4-(5- methyloxazolo[4,5-b]pyridin-2-yl)piperazin-1-yl)methanone | A.40 | 480.3 [M+H]⁺ |
| | | | and | |
| | | | CAS 1035840-99-3 | |
| 90 | 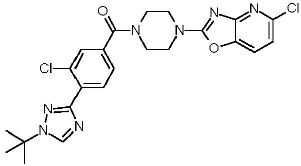 | (4-(1-(tert-butyl)-1H-1,2,4-triazol-3-yl)-3-chlorophenyl)(4-(5- chlorooxazolo[4,5-b]pyridin-2-yl)piperazin-1-yl)methanone | A.40 | 500.3 [M+H]⁺ |
| | | | and | |
| | | | B.2 | |
| 91 | 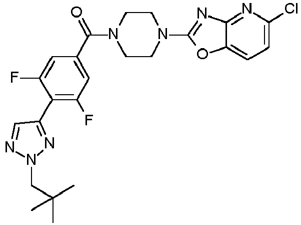 | (4-(5-chlorooxazolo[4,5-b]pyridin-2-yl)piperazin-1-yl)(3,5- difluoro-4-(2-neopentyl-2H-1,2,3-triazol-4-yl)phenyl)methanone | A. 42 | 516.3 [M+H]⁺ |
| | | | and | |
| | | | B.2 | |
| 92 | 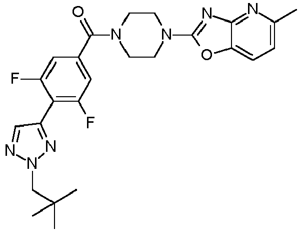 | (3,5-difluoro-4-(2-neopentyl-2H-1,2,3-triazol-4-yl)phenyl)(4-(5-methyloxazolo[4,5-b]pyridin-2-yl)piperazin-1-yl)methanone | A.42 | 496.4 [M+H]⁺ |
| | | | and | |
| | | | CAS 1035840-99-3 | |
| 93 | 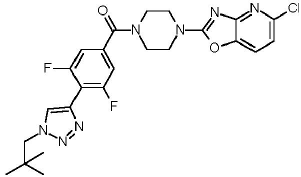 | (4-(5-chlorooxazolo[4,5-b]pyridin-2-yl)piperazin-1-yl)(3,5-difluoro-4-(1-neopentyl-1H-1,2,3-triazol-4-yl)phenyl)methanone | A.41 | 516.3 [M+H]⁺ |
| | | | and | |
| | | | B.2 | |
| 94 | 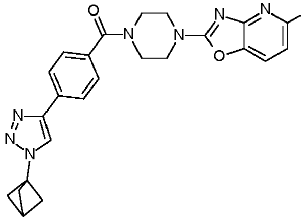 | (4-(1-(bicyclo[1.1.1]pentan-1-yl)-1H-1,2,3-triazol-4-yl)phenyl)(4-(5-methyloxazolo [4,5-b]pyridin-2-yl)piperazin-1-yl)methanone | A.43 | 456.4 [M+H]⁺ |
| | | | and | |
| | | | CAS 1035840-99-3 | |
| 95 | 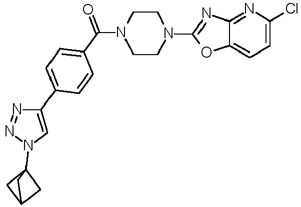 | (4-(1-(bicyclo[1.1.1]pentan-1-yl)-1H-1,2,3-triazol-4-yl)phenyl)(4-(5-chlorooxazolo[4,5-b]pyridin-2-yl)piperazin-1-yl)methanone | A.43 | 476.3 [M+H]⁺ |
| | | | and | |
| | | | B.2 | |
| 96 | 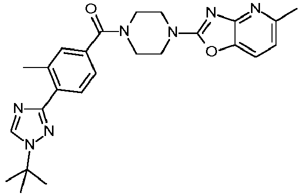 | (4-(1-(tert-butyl)-1H-1,2,4-triazol-3-yl)-3- methylphenyl)(4-(5-methyloxazolo[4,5-b]pyridin-2-yl)piperazin-1-yl)methanone | A. 44 | 460.4 [M+H]⁺ |
| | | | and | |
| | | | CAS 1035840-99-3 | |
| 97 | 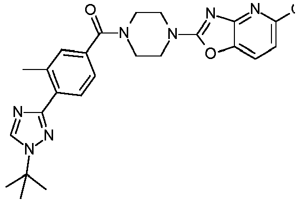 | (4-(1-(tert-butyl)-1H-1,2,4-triazol-3-yl)-3- methylphenyl)(4-(5-chlorooxazolo[4,5- b]pyridin-2-yl)piperazin-1-yl)methanone | A. 44 | 480.4 [M+H]⁺ |
| | | | and | |
| | | | B.2 | |
| 98 | 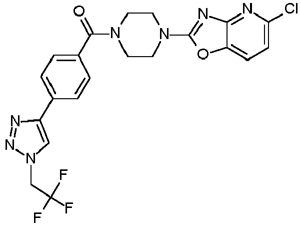 | (4-(5-chlorooxazolo[4,5-b]pyridin-2-yl)piperazin-1-yl)(4-(1- (2,2,2-trifluoroethyl)-1H-1,2,3-triazol-4-yl)phenyl)methanone | A.45 | 492.2 [M+H]⁺ |
| | | | and | |
| | | | B.2 | |
| 99 | 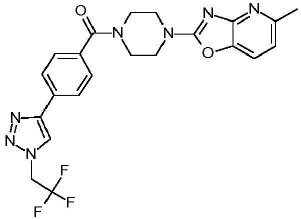 | (4-(5-methyloxazolo[4,5-b]pyridin-2-yl)piperazin-1-yl)(4-(1-(2,2,2-trifluoroethyl)-1H-1,2,3-triazol-4-yl)phenyl)methanone | A.45 | 472.3 [M+H]⁺ |
| | | | and | |
| | | | CAS 1035840-99-3 | |
| 100 | 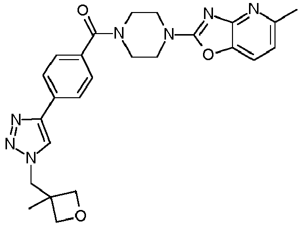 | (4-(5-methyloxazolo[4,5-b]pyridin-2-yl)piperazin-1-yl)(4-(1-((3-methyloxetan-3-yl)methyl)-1H-1,2,3-triazol-4-yl)phenyl)methanone | A.46 | 474.4 [M+H]⁺ |
| | | | and | |
| | | | CAS 1035840-99-3 | |
| 101 | 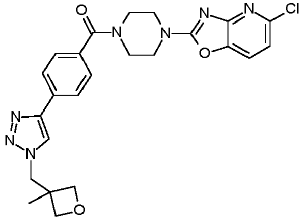 | (4-(5-chlorooxazolo[4,5- b]pyridin-2-yl)piperazin-1-yl)(4-(1- ((3-methyloxetan-3- yl)methyl)-1H-1,2,3-triazol-4-yl)phenyl)methanone | A.46 | 494.3 [M+H]⁺ |
| | | | and | |
| | | | B.2 | |
| 102 | 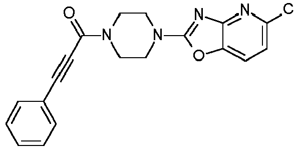 | 1-(4-(5-chlorooxazolo[4,5-b]pyridin-2-yl)piperazin-1-yl)-3-phenylprop-2-yn-1-one | CAS 637-44-5 | 367.2 [M+H]⁺ |
| | | | and | |
| | | | B.2 | |
| 103 | 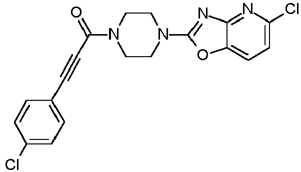 | 1-(4-(5-chlorooxazolo[4,5-b]pyridin-2-yl)piperazin-1-yl)-3-(4-chlorophenyl)prop-2-yn-1-one | CAS 3240-10-6 | 401.2 [M+H]⁺ |
| | | | and | |
| | | | B.2 | |
| 104 | 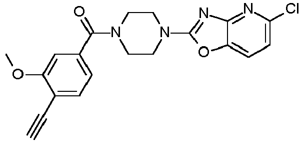 | (4-(5-chlorooxazolo[4,5- b]pyridin-2-yl)piperazin-1-yl)(4-ethynyl-3-methoxyphenyl)methan one | CAS 1270964-89-0 | 397.2 [M+H]⁺ |
| | | | and | |
| | | | B.2 | |
| 105 | 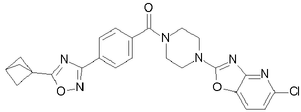 | [4-[5-(3-bicyclo[1.1.1]pentanyl)-1,2,4-oxadiazol-3-yl]phenyl]-[4-(5-chlorooxazolo[4,5-b]pyridin-2-yl)piperazin-1-yl]methanone | A.49 | 477.1 [M+H]⁺ |
| | | | and | |
| | | | B.2 | |
| 106 | 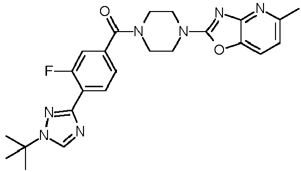 | [4-(1-*tert-*butyl-1,2,4-triazol-3-yl)-3-fluorophenyl]-[4-(5-methyl-[1,3]oxazolo[4,5-b]pyridin-2-yl)piperazin-1-yl]methanone | A.50 | 464.4 |
| | | | and | [M+H]⁺ |
| | | | CAS 1035840-99-3 | |
| 107 | 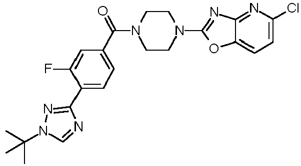 | [4-(1-*tert*-butyl-1,2,4-triazol-3-yl)-3-fluorophenyl]-[4-(5-chloro-[1,3]oxazolo[4,5-b]pyridin-2-yl)piperazin-1-yl]methanone | A.50 | 484.4 [M+H]⁺ |
| | | | and | |
| | | | B.2 | |
| 108 | 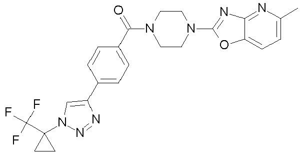 | [4-(5-methyl-[1,3]oxazolo[4,5-b]pyridin-2-yl)piperazin-1-yl]-[4-[1-[1-(trifluoromethyl)cyclop ropyl]triazol-4-yl]phenyl]methanone | A.51 | 498.4 [M+H]⁺ |
| | | | and | |
| | | | CAS 1035840-99-3 | |
| 109 | 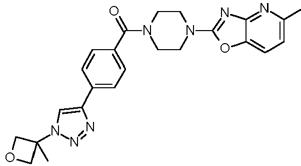 | [4-(5-methyl-[1,3]oxazolo[4,5-b]pyridin-2-yl)piperazin-1-yl]-[4-[1-(3-methyloxetan-3-yl)triazol-4-yl]phenyl]methanone | A.52 | 460.4 [M+H]⁺ |
| | | | and | |
| | | | CAS 1035840-99-3 | |
| 110 | 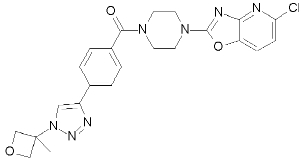 | [4-(5-chloro-[1,3]oxazolo[4,5-b]pyridin-2-yl)piperazin-1-yl]-[4-[1-(3-methyloxetan-3- yl)triazol-4-yl]phenyl]methanone | A.52 | 480.4 [M+H]⁺ |
| | | | and | |
| | | | B.2 | |
| 111 | 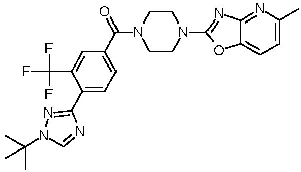 | [4-(1-*tert*-butyl-1,2,4-triazol-3-yl)-3-(trifluoromethyl)phenyl ]-[4-(5-methyl-[1,3]oxazolo[4,5-b]pyridin-2-yl)piperazin-1-yl] methanone | A.53 | 514.4 [M+H]⁺ |
| | | | and | |
| | | | CAS 1035840-99-3 | |
| 112 | 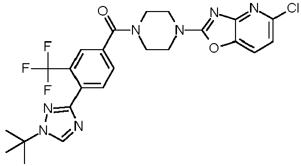 | [4-(1-*tert*-butyl-1,2,4-triazol-3-yl)-3-(trifluoromethyl)phenyl ]-[4-(5-chloro-[1,3]oxazolo[4,5-b]pyridin-2-yl)piperazin-1-yl] methanone | A.53 | 534.4 [M+H]⁺ |
| | | | and | |
| | | | B.2 | |
| 113 | 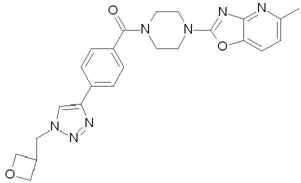 | [4-(5-methyl-[1,3]oxazolo[4,5-b]pyridin-2-yl)piperazin-1-yl]-[4-[1-(oxetan-3-ylmethyl)triazol-4-yl]phenyl]methanone | A.54 | 460.4 [M+H]⁺ |
| | | | and | |
| | | | CAS 1035840-99-3 | |
| 114 | 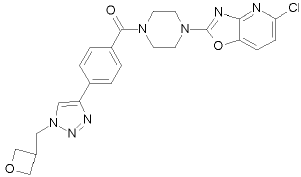 | [4-(5-chloro-[1,3]oxazolo[4,5-b]pyridin-2-yl)piperazin-1-yl]-[4-[1-(oxetan-3-ylmethyl)triazol-4-yl]phenyl]methanone | A.54 | 480.4 [M+H]⁺ |
| | | | and | |
| | | | B.2 | |
| 115 | 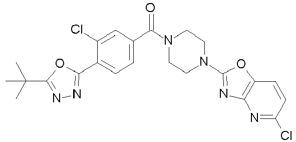 | (4-(5-(tert-butyl)-1,3,4-oxadiazol-2-yl)-3-chlorophenyl)(4-(5- chlorooxazolo[4,5-b]pyridin-2-yl)piperazin-1-yl)methanone | A.55 | 501.1 [M+H]⁺ |
| | | | and | |
| | | | B.2 | |
| 116 | 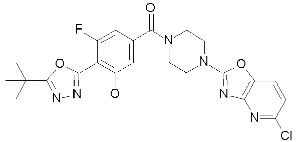 | (4-(5-(tert-butyl)-1,3,4-oxadiazol-2-yl)-3-fluoro-5-hydroxyphenyl)(4-(5-chlorooxazolo[4,5-b]pyridin-2-yl)piperazin-1-yl)methanone | A.56 | 501.1 [M+H]⁺ |
| | | | and | |
| | | | B.2 | |
| 117 | 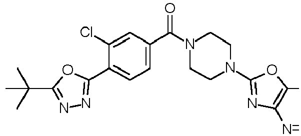 | (4-(5-(tert-butyl)-1,3,4-oxadiazol-2-yl)-3-chlorophenyl)(4-(5- methyloxazolo[4,5-b]pyridin-2-yl)piperazin-1-yl)methanone | A.55 | 481.2 [M+H]⁺ |
| | | | and | |
| | | | CAS 1035840-99-3 | |
| 118 | 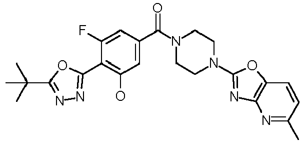 | (4-(5-(tert-butyl)-1,3,4-oxadiazol-2-yl)-3-fluoro-5-hydroxyphenyl)(4-(5-methyloxazolo[4,5-b]pyridin-2-yl)piperazin-1-yl)methanone | A.56 | 481.2 [M+H]⁺ |
| | | | and | |
| | | | CAS 1035840-99-3 | |
| 119 | 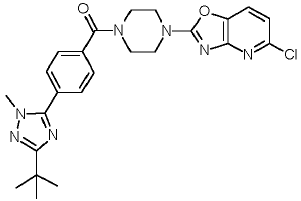 | [4-(5-tert-butyl-2-methyl-1,2,4-triazol-3- yl)phenyl]-[4-(5-chlorooxazolo[4,5-b]pyridin-2-yl)piperazin-1-yl]methanone | CAS 1411685-08-9 | 480.1 [M+H]⁺ |
| | | | and | |
| | | | B.2 | |
| 120 | 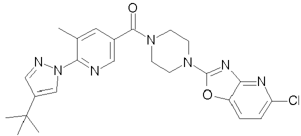 | [6-(4-*tert*-butylpyrazol-1-yl)-5-methylpyridin-3-yl]-[4-(5-chloro-[1,3]oxazolo[4,5-b]pyridin-2-yl)piperazin-1-yl]methanone | A. 62 | 480.1 [M+H]⁺ |
| | | | and | |
| | | | B.2 | |
| 121 | 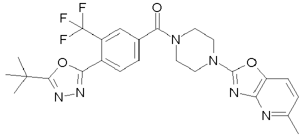 | [4-(5-tert-butyl-1,3,4-oxadiazol-2-yl)-3-(trifluoromethyl)phenyl ]-[4-(5-methyloxazolo[4,5-b]pyridin-2-yl)piperazin-1-yl]methanone | A.58 | 515.1 [M+H]⁺ |
| | | | and | |
| | | | CAS 1035840-99-3 | |
| 122 | 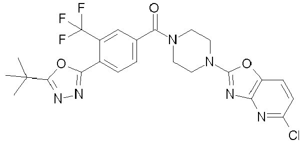 | [4-(5-tert-butyl-1,3,4-oxadiazol-2-yl)-3-(trifluoromethyl)phenyl ]-[4-(5-chlorooxazolo[4,5- b]pyridin-2-yl)piperazin-1-yl]methanone | A.58 | 535.1 [M+H]⁺ |
| | | | and | |
| | | | B.2 | |
| 123 | 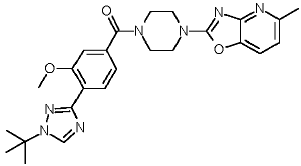 | (4-(1-(tert-butyl)-1H-1,2,4-triazol-3-yl)-3- methoxyphenyl)(4-(5-methyloxazolo[4,5-b]pyridin-2-yl)piperazin-1-yl)methanone | A.59 | 476.4 [M+H]⁺ |
| | | | and | |
| | | | CAS 1035840-99-3 | |
| 124 | 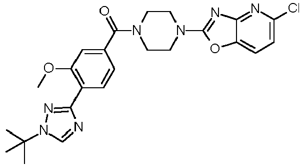 | (4-(1-(tert-butyl)-1H-1,2,4-triazol-3-yl)-3- methoxyphenyl)(4-(5-chlorooxazolo[4,5-b]pyridin-2-yl)piperazin-1-yl)methanone | A. 59 | 496.4 [M+H]⁺ |
| | | | and | |
| | | | B.2 | |
| 125 | 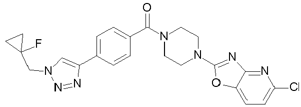 | (4-(5-chlorooxazolo[4,5-b]pyridin-2-yl)piperazin-1-yl)(4-(1-((1-fluorocyclopropyl)meth yl)-1H-1,2,3-triazol-4-yl)phenyl)methanone | A.60 | 482.2 [M+H]⁺ |
| | | | and | |
| | | | B.2 | |
| 126 | 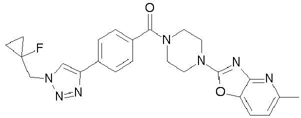 | (4-(1-((1-fluorocyclopropyl)meth yl)-1H-1,2,3-triazol-4-yl)phenyl)(4-(5-methyloxazolo[4,5-b]pyridin-2-yl)piperazin-1-yl)methanone | A.60 | 462.2 [M+H]⁺ |
| | | | and | |
| | | | CAS 1035840-99-3 | |
| 127 | 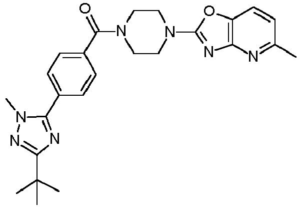 | [4-(5-tert-butyl-2-methyl-1,2,4-triazol-3- yl)phenyl]-[4-(5-methyloxazolo[4,5-b]pyridin-2-yl)piperazin-1-yl]methanone | CAS 1411685-08-9 | 460.3 [M+H]⁺ |
| | | | and | |
| | | | CAS 1035840-99-3 | |
| 128 | 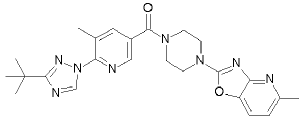 | [6-(3-*tert*-butyl-1,2,4-triazol-1-yl)-5-methylpyridin-3-yl]-[4-(5-methyl-[1,3]oxazolo[4,5-b]pyridin-2-yl)piperazin-1-yl]methanone | A. 63 | 461.1 [M+H]⁺ |
| | | | and | |
| | | | CAS 1035840-99-3 | |
| 129 | 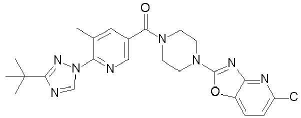 | [6-(3-*tert*-butyl-1,2,4-triazol-1-yl)-5-methylpyridin-3-yl]-[4-(5-chloro-[1,3]oxazolo[4,5-b]pyridin-2-yl)piperazin-1-yl]methanone | A. 63 | 481.1 [M+H^{]+} |
| | | | and | |
| | | | B.2 | |
| 130 | 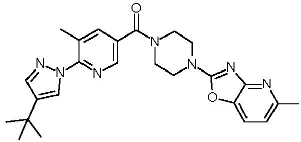 | [6-(4-*tert*-butylpyrazol-1-yl)-5-methylpyridin-3-yl]-[4-(5-methyl-[1,3]oxazolo[4,5-b]pyridin-2-yl)piperazin-1-yl]methanone | A.62 | 460.2 [M+H]⁺ |
| | | | and | |
| | | | CAS 1035840-99-3 | |
| 131 | 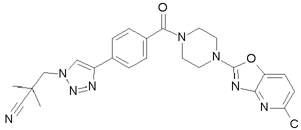 | 3-[4-[4-[4-(5-chlorooxazolo[4,5-b]pyridin-2-yl)piperazine-1-carbonyl]phenyl]triazol -1-yl]-2,2-dimethyl-propanenitrile | A. 64 | 491.2 [M+H]⁺ |
| | | | and | |
| | | | B.2 | |
| 132 | 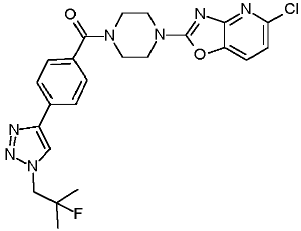 | (4-(5-chlorooxazolo[4,5-b]pyridin-2-yl)piperazin-1-yl)(4-(1-(2-fluoro-2-methylpropyl)-1H-1,2,3-triazol-4-yl)phenyl)methanone | A. 67 | 484.4 [M+H]⁺ |
| | | | and | |
| | | | B.2 | |
| 133 | 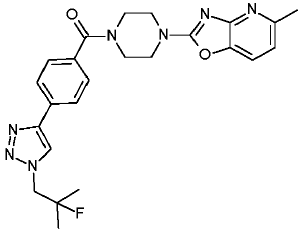 | (4-(1-(2-fluoro-2-methylpropyl)-1H-1,2,3-triazol-4-yl)phenyl)(4-(5-methyloxazolo[4,5-b]pyridin-2-yl)piperazin-1-yl)methanone | A.67 | 464.4 [M+H]⁺ |
| | | | and | |
| | | | CAS 1035840-99-3 | |
| 134 | 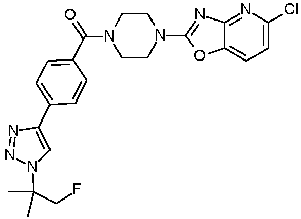 | (4-(5-chlorooxazolo[4,5-b]pyridin-2-yl)piperazin-1-yl)(4-(1-(1-fluoro-2-methylpropan-2-yl)-1H-1,2,3-triazol-4-yl)phenyl)methanone | A.68 | 484.4 [M+H]⁺ |
| | | | and | |
| | | | B.2 | |
| 135 | 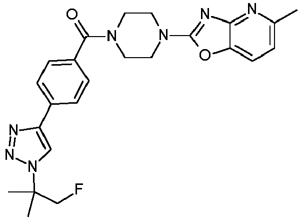 | (4-(1-(1-fluoro-2-methylpropan-2-yl)-1H-1,2,3-triazol-4-yl)phenyl)(4-(5-methyloxazolo [4,5-b]pyridin-2-yl)piperazin-1-yl)methanone | A.68 | 464.4 [M+H]⁺ |
| | | | and | |
| | | | CAS 1035840-99-3 | |
| 136 | 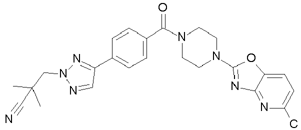 | 3-[4-[4-[4-(5-chlorooxazolo[4,5- b]pyridin-2-yl)piperazine-1-carbonyl]phenyl]triazol -2-yl]-2,2-dimethyl-propanenitrile | A.65 | 491.2 [M+H]⁺ |
| | | | and | |
| | | | B.2 | |
| 137 | 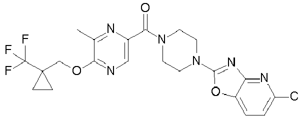 | [4-(5-chlorooxazolo[4,5-b]pyridin-2-yl)piperazin-1-yl]-[6-methyl-5-[[1-(trifluoromethyl)cyclop ropyl]methoxy]pyrazin-2-yl]methanone | A. 66 | 497.1 [M+H]⁺ |
| | | | and | |
| | | | B.2 | |
| 138 | 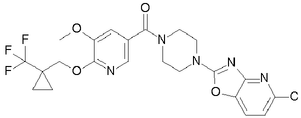 | [4-(5-chlorooxazolo[4,5-b]pyridin-2-yl)piperazin-1-yl]-[5-methoxy-6-[[1-(trifluoromethyl)cyclop ropyl]methoxy]-3-pyridyl]methanone | A.69 | 512.2 [M+H]⁺ |
| | | | and | |
| | | | B.2 | |
| 139 | 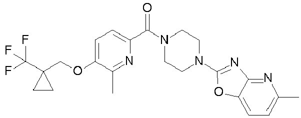 | [4-(5-methyloxazolo[4,5-b]pyridin-2-yl)piperazin-1-yl]-[6-methyl-5-[[1-(trifluoromethyl)cyclop ropyl]methoxy]-2-pyridyl]methanone | A.70 | 476.2 [M+H]⁺ |
| | | | and | |
| | | | CAS 1035840-99-3 | |
| 140 | 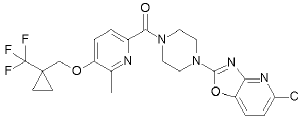 | [4-(5-chlorooxazolo[4,5-b]pyridin-2-yl)piperazin-1-yl]-[6-methyl-5-[[1-(trifluoromethyl)cyclop ropyl]methoxy]-2-pyridyl]methanone | A.70 | 496.2 [M+H]⁺ |
| | | | and | |
| | | | B.2 | |
| 141 | 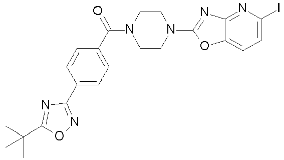 | (4-(5-(tert-butyl)-1,2,4-oxadiazol-3-yl)phenyl)(4-(5-iodooxazolo[4,5-b]pyridin-2-yl)piperazin-1-yl)methanone | CAS 1119452-72-0 | 559.2 [M+H]⁺ |
| | | | and | |
| | | | B.3 | |
| 259 | 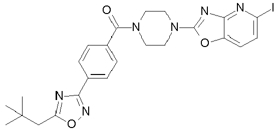 | (4-(5-iodooxazolo[4,5-b]pyridin-2-yl)piperazin-1-yl)(4-(5-neopentyl-1,2,4-oxadiazol-3-yl)phenyl)methanone | CAS 1305938-51-5 | 573.3 [M+H]⁺ |
| | | | and | |
| | | | B.3 | |
| 273 | 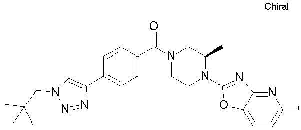 | [(3R)-4-(5-chlorooxazolo[4,5-b]pyridin-2-yl)-3-methyl-piperazin-1-yl]- [4-[1-(2,2-dimethylpropyl)triazol-4-yl]phenyl]methanone | A.19 | 494.1 [M+H]⁺ |
| | | | and | |
| | | | B.5 | |
| 274 | 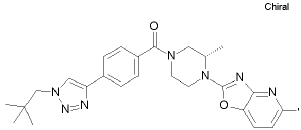 | [(3S)-4-(5-chlorooxazolo[4,5- b]pyridin-2-yl)-3-methyl-piperazin-1-yl]- [4-[1-(2,2-dimethylpropyl)triazol-4-yl]phenyl]methanone | A.19 | 494.1 [M+H]⁺ |
| | | | and | |
| | | | B.5 | |
| 277 | 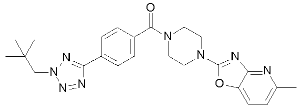 | [4-[2-(2,2-dimethylpropyl)tetrazol -5-yl]phenyl]-[4-(5-methyloxazolo [4,5-b]pyridin-2-yl)piperazin-1-yl] methanone | A.73 | 461.3 [M+H]⁺ |
| | | | and | |
| | | | CAS 1035840-99-3 | |
| 278 | 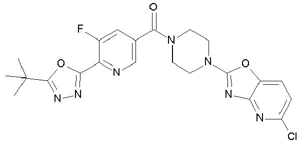 | [6-(5-tert-Butyl-1,3,4-oxadiazol-2-yl)-5-fluoro-3-pyridyl]-[4-(5-chlorooxazolo[4,5- b]pyridin-2-yl)piperazin-1-yl]methanone | A.82 | 486.2 [M+H]⁺ |
| | | | and | |
| | | | B.2 | |
| 280 | 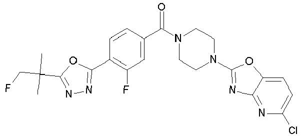 | [4-(5-Chlorooxazolo[4,5-b]pyridin-2-yl)piperazin-1-yl]-[3-fluoro-4-[5-(2-fluoro-1,1-dimethyl-ethyl)-1,3,4-oxadiazol-2-yl]phenyl]methanone | A.81 | 503.0 [M+H]⁺ |
| | | | and | |
| | | | B.2 | |
| 281 | 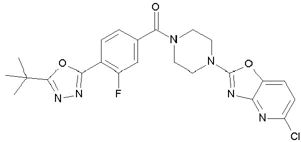 | [4-(5-tert-Butyl-1,3,4-oxadiazol-2-yl)-3-fluoro-phenyl]-[4-(5-chlorooxazolo[4,5-b]pyridin-2-yl)piperazin-1-yl] methanone | A.80 | 485.1 [M+H]⁺ |
| | | | and | |
| | | | B.2 | |
| 283 | 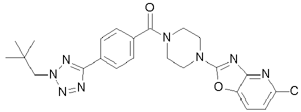 | (4-(5-chlorooxazolo[4,5-b]pyridin-2-yl)piperazin-1-yl)(4-(2-neopentyl-2H-tetrazol-5-yl)phenyl)methanone | A.73 | 481.2 [M+H]⁺ |
| | | | and | |
| | | | B.2 | |
| 284 | 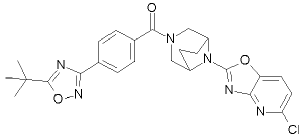 | [4-(5-tert-butyl-1,2,4-oxadiazol-3-yl)phenyl]- [8-(5-chlorooxazolo[4,5- b]pyridin-2-yl)-3,8-diazabicyclo[3.2.1]octa n-3-yl]methanone | CAS 1119452-72-0 | 493.0 [M+H]⁺ |
| | | | and | |
| | | | B.9 | |
| 285 | 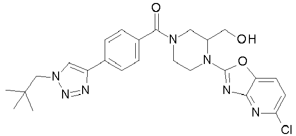 | [4-(5-chlorooxazolo[4,5-b]pyridin-2-yl)-3-(hydroxymethyl)piperaz in-1-yl]-[4-[1-(2,2-dimethylpropyl)triazol-4-yl]phenyl]methanone | A.19 | 510.3 [M+H]⁺ |
| | | | and | |
| | | | B.11 | |
| 286 | 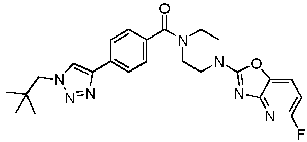 | [4-[1-(2,2-dimethylpropyl)triazol-4-yl]phenyl]-[4-(5-fluorooxazolo[4,5- b]pyridin-2-yl)piperazin-1-yl]methanone | A.19 | 464.3 [M+H]⁺ |
| | | | and | |
| | | | B.13 | |
| 287 | 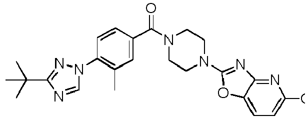 | (4-(3-(tert-butyl)-1H-1,2,4-triazol-1-yl)-3-methylphenyl)(4-(5-chlorooxazolo[4,5-b]pyridin-2-yl)piperazin-1-yl)methanone | A.78 | 480.1 [M+H]⁺ |
| | | | and | |
| | | | B.2 | |
| 288 | 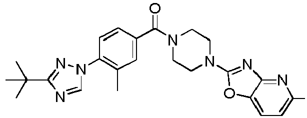 | (4-(3-(tert-butyl)-1H-1,2,4-triazol-1-yl)-3-methylphenyl)(4-(5-methyloxazolo[4,5-b]pyridin-2-yl)piperazin-1-yl)methanone | A.78 | 460.2 [M+H]⁺ |
| | | | and | |
| | | | CAS 1035840-99-3 | |
| 289 | 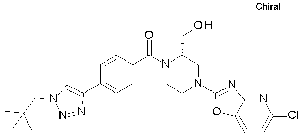 | (S)-(4-(5-chlorooxazolo[4,5-b]pyridin-2-yl)-2-(hydroxymethyl)piperaz in-1-yl)(4-(1-neopentyl-1H-1,2,3-triazol-4-yl)phenyl)methanone | A.19 | 510.2 [M+H]⁺ |
| | | | and | |
| | | | B.12 | |
| | | | (chiral separation of racemate) | |
| 290 | 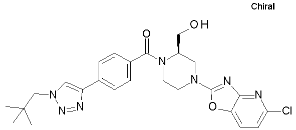 | (R)-(4-(5-chlorooxazolo[4,5-b]pyridin-2-yl)-2-(hydroxymethyl)piperaz in-1-yl)(4-(1-neopentyl-1H-1,2,3-triazol-4-yl)phenyl)methanone | A.19 | 510.2 [M+H]⁺ |
| | | | and | |
| | | | B.12 | |
| | | | (chiral separation of racemate) | |
| 291 | 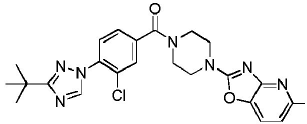 | (4-(3-(tert-butyl)-1H-1,2,4-triazol-1-yl)-3-chlorophenyl)(4-(5- methyloxazolo[4,5-b]pyridin-2-yl)piperazin-1-yl)methanone | A.79 | 480.2 [M+H]⁺ |
| | | | and | |
| | | | CAS 1035840-99-3 | |
| 292 | 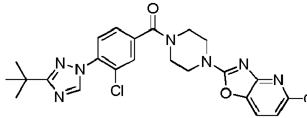 | (4-(3-(tert-butyl)-1H-1,2,4-triazol-1-yl)-3-chlorophenyl)(4-(5- chlorooxazolo[4,5-b]pyridin-2-yl)piperazin-1-yl)methanone | A.79 | 500.2 [M+H]⁺ |
| | | | and | |
| | | | B.2 | |
| 293 | 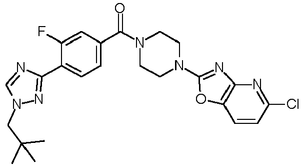 | (4-(5-chlorooxazolo[4,5-b]pyridin-2-yl)piperazin-1-yl)(3-fluoro-4-(1-neopentyl-1H-1,2,4-triazol-3-yl)phenyl)methanone | A.77 | 498.2 [M+H]⁺ |
| | | | and | |
| | | | B.2 | |
| 294 | 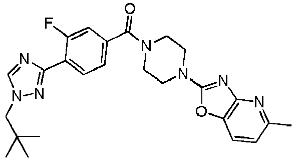 | [4-[1-(2,2-dimethylpropyl)-1,2,4-triazol-3 -yl]-3-fluoro-phenyl]-[4-(5-methyloxazolo[4,5-b]pyridin-2-yl)piperazin-1-yl] methanone | A.77 | 478.3 [M+H]⁺ |
| | | | and | |
| | | | CAS 1035840-99-3 | |
| 295 | 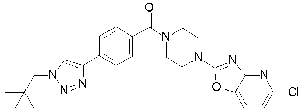 | [4-(5-chlorooxazolo[4,5-b]pyridin-2-yl)-2-methyl-piperazin-1-yl]- [4-[1-(2,2-dimethylpropyl)triazol-4-yl]phenyl]methanone | A.19 | 494.2 [M+H]⁺ |
| | | | and | |
| | | | B.4 | |
| 297 | 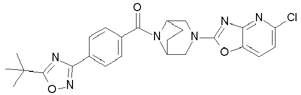 | [4-(5-tert-butyl-1,2,4-oxadiazol-3-yl)phenyl]- [3-(5-chlorooxazolo[4,5- b]pyridin-2-yl)-3,8-diazabicyclo[3.2.1]octa n-8-yl]methanone | CAS 1119452-72-0 | 493.2 [M+H]⁺ |
| | | | and | |
| | | | B.10 | |
| 298 | 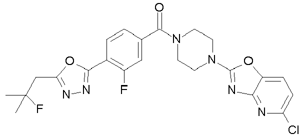 | [4-(5-chlorooxazolo[4,5-b]pyridin-2-yl)piperazin-1-yl]-[3-fluoro-4-[5-(2-fluoro-2-methyl-propyl)-1,3,4-oxadiazol-2-yl]phenyl]methanone | A.83 | 503.2 [M+H]⁺ |
| | | | and | |
| | | | B.2 | |
| 299 | 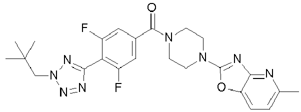 | [4-[2-(2,2-dimethylpropyl)tetrazol -5-yl]-3,5-difluoro-phenyl]-[4-(5-methyloxazolo[4,5-b]pyridin-2-yl)piperazin-1-yl]methanone | A.74 | 497.2 [M+H]⁺ |
| | | | and | |
| | | | CAS 1035840-99-3 | |
| 300 | 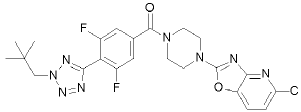 | [4-(5-chlorooxazolo[4,5-b]pyridin-2-yl)piperazin-1-yl]-[4-[2-(2,2-dimethylpropyl)tetrazol -5-yl]-3,5-difluoro-phenyl] methanone | A.74 | 517.2 [M+H]⁺ |
| | | | and | |
| | | | B.2 | |
| 301 | 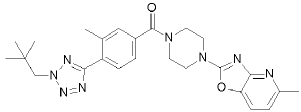 | [4-[2-(2,2-dimethylpropyl)tetrazol -5-yl]-3-methyl-phenyl]-[4-(5-methyloxazolo[4,5-b]pyridin-2-yl)piperazin-1-yl] methanone | A.75 | 475.3 [M+H]⁺ |
| | | | and | |
| | | | CAS 1035840-99-3 | |
| 302 | 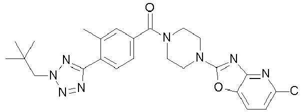 | [4-(5-chlorooxazolo[4,5-b]pyridin-2-yl)piperazin-1-yl]-[4-[2-(2,2-dimethylpropyl)tetrazol -5-yl]-3-methyl-phenyl]methanone | A.75 | 495.2 [M+H]⁺ |
| | | | and | |
| | | | B.2 | |
| 303 | 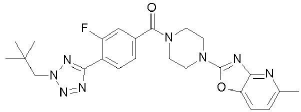 | [4-[2-(2,2-dimethylpropyl)tetrazol -5-yl]-3-fluoro-phenyl]-[4-(5-methyloxazolo[4,5-b]pyridin-2-yl)piperazin-1-yl]methanone | A.76 | 479.3 [M+H]⁺ |
| | | | and | |
| | | | CAS 1035840-99-3 | |
| 304 | 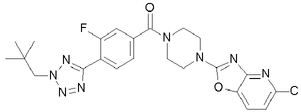 | [4-(5-chlorooxazolo[4,5-b]pyridin-2-yl)piperazin-1-yl]-[4-[2-(2,2-dimethylpropyl)tetrazol -5-yl]-3-fluoro-phenyl]methanone | A.76 | 499.2 [M+H]⁺ |
| | | | and | |
| | | | B.2 | |
| 306 | 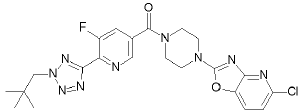 | [4-(5-chlorooxazolo[4,5-b]pyridin-2-yl)piperazin-1-yl]-[6-[2-(2,2-dimethylpropyl)tetrazol -5-yl]-5-fluoro-3-pyridyl] methanone | A.84 | 500.2 [M+H]⁺ |
| | | | and | |
| | | | B.2 | |
| 307 | 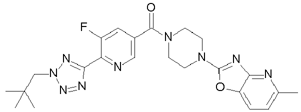 | [6-[2-(2,2-dimethylpropyl)tetrazol -5-yl]-5-fluoro-3-pyridyl]-[4-(5-methyloxazolo [4,5-b]pyridin-2-yl)piperazin-1-yl] methanone | A.84 | 480.3 [M+H]⁺ |
| | | | and | |
| | | | CAS 1035840-99-3 | |
| 308 | 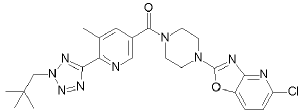 | [4-(5-chlorooxazolo[4,5-b]pyridin-2-yl)piperazin-1-yl]-[6-[2-(2,2-dimethylpropyl)tetrazol -5-yl]-5-methyl-3-pyridyl] methanone | A.85 | 496.2 [M+H]⁺ |
| | | | and | |
| | | | B.2 | |
| 309 | 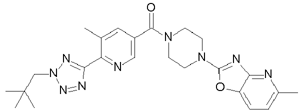 | [6-[2-(2,2-dimethylpropyl)tetrazol -5-yl]-5-methyl-3-pyridyl]-[4-(5-methyloxazolo [4,5-b]pyridin-2-yl)piperazin-1-yl] methanone | A.85 | 476.3 [M+H]⁺ |
| | | | and | |
| | | | CAS 1035840-99-3 | |
| 310 | 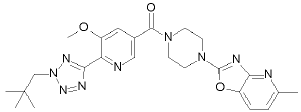 | [6-[2-(2,2-dimethylpropyl)tetrazol -5-yl]-5-methoxy-3-pyridyl]-[4-(5-methyloxazolo [4,5-b]pyridin-2-yl)piperazin-1-yl] methanone | A. 86 | 492.3 [M+H]⁺ |
| | | | and | |
| | | | CAS 1035840-99-3 | |
| 311 | 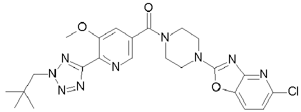 | [4-(5-chlorooxazolo[4,5-b]pyridin-2-yl)piperazin-1-yl]-[6-[2-(2,2-dimethylpropyl)tetrazol -5-yl]-5-methoxy-3-pyridyl] methanone | A. 86 | 512.3 |
| | | | and | [M+H]⁺ |
| | | | B.2 | |

### Example 142

### (5-Methyl-6-((1-methylcyclopropyl)methoxy)pyridin-3-yl)(4-(5-methyloxazolo[4,5-b]pyridin-2-yl)piperazin-1-yl)methanone

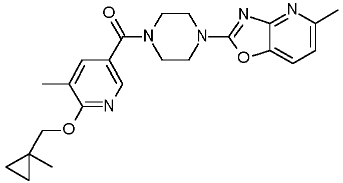

To a stirred suspension of Sodium hydride 60% dispersion in mineral oil (9.72 mg, 154 µmol) at r.t. in DMF (1 mL) under an argon atmosphere was added (1-methylcyclopropyl)methanol (13.2 mg, 14.9 µL, 154 µmol). The mixture was stirred for 15 min. (6-fluoro-5-methylpyridin-3-yl)(4-(5-methyloxazolo[4,5-b]pyridin-2-yl)piperazin-1-yl)methanone (30 mg, 76.8 µmol) (C.14) was added in one portion and stirring at r.t. was continued for 30 min. The mixture was diluted with EtOAc and washed with water. The aqueous phase was back extracted with EtOAc. The combined organics were washed with water and brine, dried (MgSO₄), filtered and concentrated to leave the crude product as a brown oil. The crude product was purified by silica column flash chomatography with an eluent mixture of MeOH and DCM (0% - 10% gradient) to provide the title compound (29 mg, 89.6%) as a white foam. MS (ESI): m/z = 422.3 [M+H]⁺.

In analogy to example 142, example 143 to 174 of the following table were prepared from a building block C.X and an alcohol.

| **Ex.** | **Structure** | **Systematic Name** | **Building Blocks** | **MS (ESI): m/z** |
|---|---|---|---|---|
| 143 | 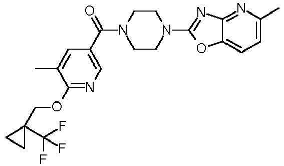 | (5-methyl-6-((1-(trifluoromethyl)cycl opropyl)methoxy)py ridin-3-yl)(4-(5-methyloxazolo[4,5- b]pyridin-2-yl)piperazin-1-yl)methanone | C.14 | 476.3 [M+H]⁺ |
| | | | and | |
| | | | CAS 371917-17-8 | |
| 144 | 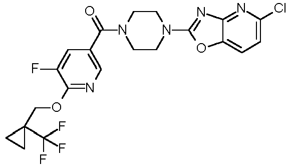 | (4-(5-chlorooxazolo[4,5-b]pyridin-2-yl)piperazin-1-yl)(5-fluoro-6-((1-(trifluoromethyl)cycl opropyl)methoxy)pyridin-3-yl)methanone | C.26 | 500.2 [M+H]⁺ |
| | | | and | |
| | | | CAS 371917-17-8 | |
| 145 | 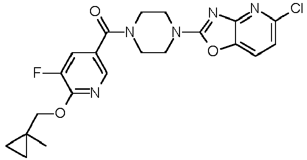 | (4-(5-chlorooxazolo[4,5-b]pyridin-2-yl)piperazin-1-yl)(5-fluoro-6-((1-methylcyclopropyl) methoxy)pyridin-3-yl)methanone | C.26 | 446.2 [M+H]⁺ |
| | | | and | |
| | | | CAS 2746-14-7 | |
| 146 | 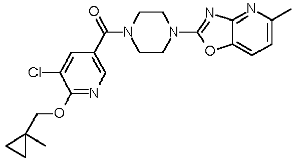 | (5-chloro-6-((1-methylcyclopropyl) methoxy)pyridin-3-yl)(4-(5-methyloxazolo[4,5- b]pyridin-2-yl)piperazin-1-yl)methanone | C.25 | 442.3 [M+H]⁺ |
| | | | and | |
| | | | CAS 2746-14-7 | |
| 147 | 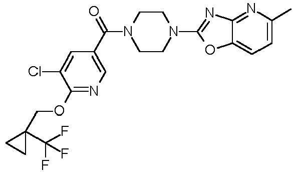 | (5-chloro-6-((1-(trifluoromethyl)cycl opropyl)methoxy)py ridin-3-yl)(4-(5-methyloxazolo[4,5- b]pyridin-2-yl)piperazin-1-yl)methanone | C.25 | 496.4 [M+H]⁺ |
| | | | and | |
| | | | CAS 371917-17-8 | |
| 148 | 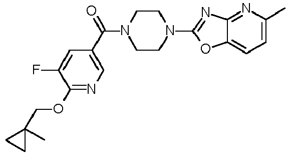 | (5-fluoro-6-((1-methylcyclopropyl) methoxy)pyridin-3-yl)(4-(5-methyloxazolo[4,5-b]pyridin-2-yl)piperazin-1-yl)methanone | C.21 | 426.3 [M+H]⁺ |
| | | | and | |
| | | | CAS 2746-14-7 | |
| 149 | 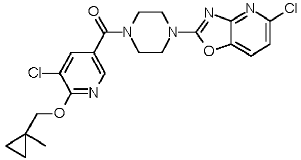 | (5-chloro-6-((1-methylcyclopropyl) methoxy)pyridin-3-yl)(4-(5-chlorooxazolo[4,5- b]pyridin-2-yl)piperazin-1-yl)methanone | C.28 | 462.3 [M+H]⁺ |
| | | | and | |
| | | | CAS 2746-14-7 | |
| 150 | 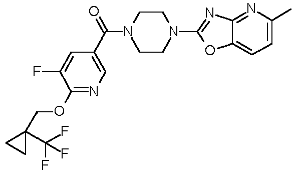 | (5-fluoro-6-((1-(trifluoromethyl)cycl opropyl)methoxy)py ridin-3-yl)(4-(5-methyloxazolo[4,5- b]pyridin-2-yl)piperazin-1-yl)methanone | C.21 | 480.2 [M+H]⁺ |
| | | | and | |
| | | | CAS 371917-17-8 | |
| 151 | 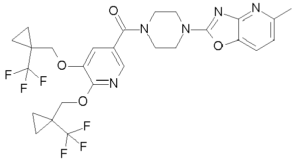 | (5,6-bis((1-(trifluoromethyl)cycl opropyl)methoxy)py ridin-3-yl)(4-(5-methyloxazolo[4,5- b]pyridin-2-yl)piperazin-1-yl)methanone | C.21 | 600.4 [M+H]⁺ |
| | | | and | |
| | | | CAS 371917-17-8 | |
| 152 | 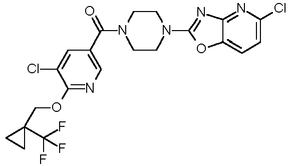 | (5-chloro-6-((1-(trifluoromethyl)cycl opropyl)methoxy)py ridin-3-yl)(4-(5-chlorooxazolo[4,5-b]pyridin-2-yl)piperazin-1-yl)methanone | C.28 | 516.3 [M+H]⁺ |
| | | | and | |
| | | | CAS 371917-17-8 | |
| 153 | 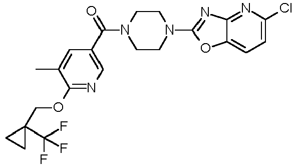 | (4-(5-chlorooxazolo[4,5-b]pyridin-2-yl)piperazin-1-yl)(5-methyl-6-((1-(trifluoromethyl)cycl opropyl)methoxy)py ridin-3-yl)methanone | C.29 | 496.3 [M+H]⁺ |
| | | | and | |
| | | | CAS 371917-17-8 | |
| 154 | 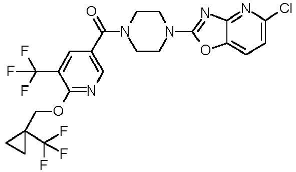 | (4-(5-chlorooxazolo[4,5-b]pyridin-2-yl)piperazin-1-yl)(5-(trifluoromethyl)-6-((1-(trifluoromethyl)cycl opropyl)methoxy)py ridin-3-yl)methanone | C.30 | 550.3 [M+H]⁺ |
| | | | and | |
| | | | CAS 371917-17-8 | |
| 155 | 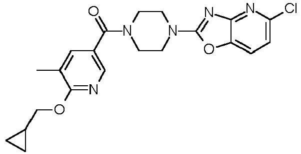 | (4-(5-chlorooxazolo[4,5-b]pyridin-2-yl)piperazin-1-yl)(6-(cyclopropylmethoxy)-5-methylpyridin-3-yl)methanone | C.29 | 428.3 [M+H]⁺ |
| | | | and CAS 2516-33-8 | |
| 156 | 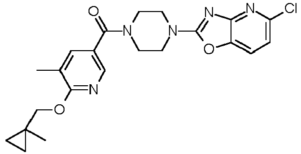 | (4-(5-chlorooxazolo[4,5-b]pyridin-2-yl)piperazin-1-yl)(5-methyl-6-((1-methylcyclopropyl) methoxy)pyridin-3-yl)methanone | C.29 | 442.3 [M+H]⁺ |
| | | | and | |
| | | | CAS 2746-14-7 | |
| 157 | 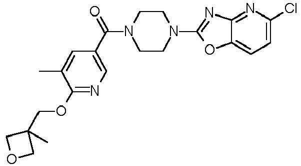 | (4-(5-chlorooxazolo[4,5-b]pyridin-2-yl)piperazin-1-yl)(5-methyl-6-((3-methyloxetan-3-yl)methoxy)pyridin-3-yl)methanone | C.29 | 458.3 [M+H]⁺ |
| | | | and | |
| | | | CAS 3143-02-0 | |
| 158 | 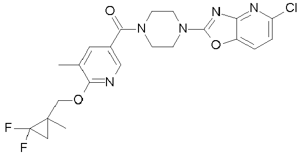 | (4-(5-chlorooxazolo[4,5-b]pyridin-2-yl)piperazin-1-yl)(6-((2,2-difluoro-1-methylcyclopropyl) methoxy)-5-methylpyridin-3-yl)methanone | C.29 | 478.4 [M+H]⁺ |
| | | | and | |
| | | | CAS 128230-72-8 | |
| 159 | 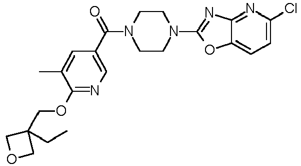 | (4-(5-chlorooxazolo [4,5 - b]pyridin-2-yl)piperazin-1-yl)(6-((3 -ethyloxetan-3-yl)methoxy)-5-methylpyridin-3-yl)methanone | C.29 | 472.3 [M+H]⁺ |
| | | | and CAS 3047-32-3 | |
| 160 | 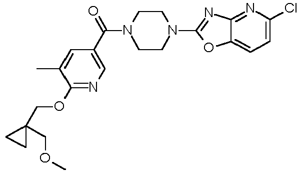 | (4-(5-chlorooxazolo[4,5-b]pyridin-2-yl)piperazin-1-yl)(6-((1-(methoxymethyl)cyc lopropyl)methoxy)-5-methylpyridin-3-yl)methanone | C.29 | 472.4 [M+H]⁺ |
| | | | and | |
| | | | CAS 338455-22-4 | |
| 161 | 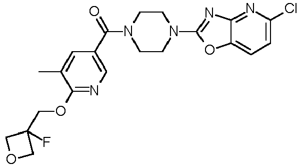 | (4-(5-chlorooxazolo[4,5- b]pyridin-2-yl)piperazin-1-yl)(6-((3-fluorooxetan-3- yl)methoxy)-5-methylpyridin-3-yl)methanone | C.29 | 462.3 [M+H]⁺ |
| | | | and | |
| | | | CAS 865451-85-0 | |
| 162 | 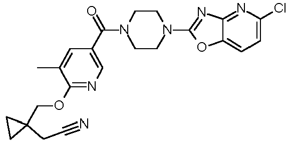 | 2-(1-(((5-(4-(5-chlorooxazolo[4,5-b]pyridin-2-yl)piperazine-1-carbonyl)-3-methylpyridin-2-yl)oxy)methyl)cyclo propyl)acetonitrile | C.29 | 467.4 [M+H]⁺ |
| | | | and | |
| | | | CAS 152922-71-9 | |
| 163 | 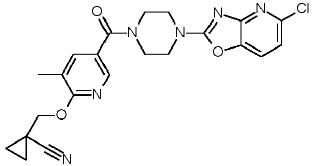 | 1-(((5-(4-(5-chlorooxazolo[4,5-b]pyridin-2-yl)piperazine-1-carbonyl)-3-methylpyridin-2-yl)oxy)methyl)cyclo propane-1-carbonitrile | C.29 | 453.3 [M+H]⁺ |
| | | | and | |
| | | | CAS 98730-77-9 | |
| 164 | 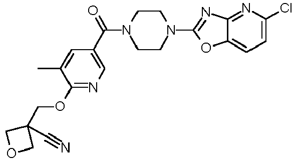 | 3-(((5-(4-(5-chlorooxazolo[4,5-b]pyridin-2-yl)piperazine-1-carbonyl)-3-methylpyridin-2-yl)oxy)methyl)oxeta ne-3-carbonitrile | C.29 | 469.3 [M+H]⁺ |
| | | | and | |
| | | | CAS 1374657-44-9 | |
| 165 | 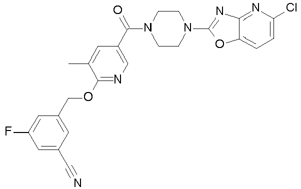 | 3-(((5-(4-(5-chlorooxazolo[4,5-b]pyridin-2-yl)piperazine-1-carbonyl)-3-methylpyridin-2-yl)oxy)methyl)-5-fluorobenzonitrile | C.29 | 507.3 [M+H]⁺ |
| | | | and | |
| | | | CAS 1021871-34-0 | |
| 166 | 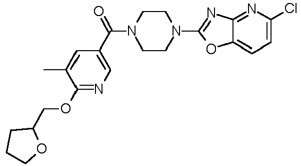 | (4-(5-chlorooxazolo[4,5-b]pyridin-2-yl)piperazin-1-yl)(5-methyl-6-((tetrahydrofuran-2-yl)methoxy)pyridin-3-yl)methanone | C.29 | 458.4 [M+H]⁺ |
| | | | and | |
| | | | CAS 97-99-4 | |
| 167 | 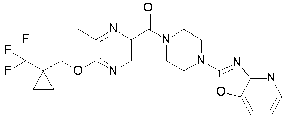 | [4-(5-methyloxazolo[4,5-b]pyridin-2-yl)piperazin-1-yl]-[6-methyl-5-[[1-(trifluoromethyl)cycl opropyl]methoxy]py razin-2-yl] methanone | C.33 | 477.1 [M+H]⁺ |
| | | | and | |
| | | | CAS 371917-17-8 | |
| 168 | 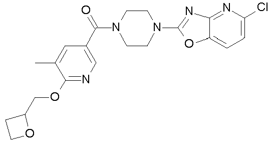 | (4-(5-chlorooxazolo[4,5-b]pyridin-2-yl)piperazin-1-yl)(5-methyl-6-(oxetan-2-ylmethoxy)pyridin-3-yl)methanone | C.29 | 444.3 [M+H]⁺ |
| | | | and | |
| | | | CAS 61266-70-4 | |
| 169 | 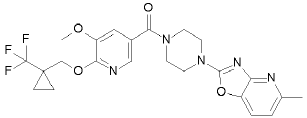 | [5-methoxy-6-[[1-(trifluoromethyl)cycl opropyl]methoxy]-3-pyridyl]-[4-(5-methyloxazolo[4,5-b]pyridin-2-yl)piperazin-1-yl] methanone | C.40 | 492.1 [M+H]⁺ |
| | | | and | |
| | | | CAS 371917-17-8 | |
| 170 | 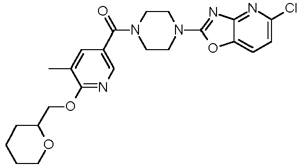 | (4-(5-chlorooxazolo[4,5-b]pyridin-2-yl)piperazin-1-yl)(5-methyl-6-((tetrahydro-2H-pyran-2-yl)methoxy)pyridin-3-yl)methanone | C.29 | 472.4 [M+H]⁺ |
| | | | and | |
| | | | CAS 100-72-1 | |
| 171 | 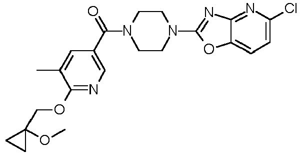 | (4-(5-chlorooxazolo[4,5-b]pyridin-2-yl)piperazin-1-yl)(6-((1-methoxycyclopropyl )methoxy)-5-methylpyridin-3-yl)methanone | C.29 | 458.3 [M+H]⁺ |
| | | | and | |
| | | | CAS 912346-81-7 | |
| 172 | 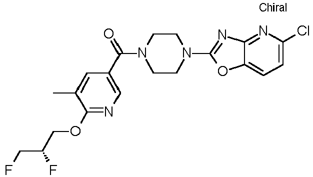 | (S)-(4-(5-chlorooxazolo[4,5-b]pyridin-2-yl)piperazin-1-yl)(6-(2,3-difluoropropoxy)-5-methylpyridin-3-yl)methanone | C.29 | 452.3 [M+H]⁺ |
| | | | and | |
| | | | CAS 869670-34-8 | |
| 173 | 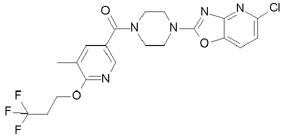 | (4-(5-chlorooxazolo[4,5-b]pyridin-2-yl)piperazin-1-yl)(5-methyl-6-(3,3,3-trifluoropropoxy)pyr idin-3-yl)methanone | C.29 | 470.3 [M+H]⁺ |
| | | | and | |
| | | | CAS 2240-88-2 | |
| 174 | 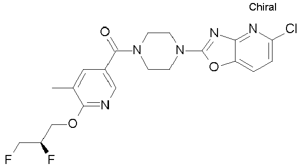 | (R)-(4-(5-chlorooxazolo[4,5-b]pyridin-2-yl)piperazin-1-yl)(6-(2,3-difluoropropoxy)-5-methylpyridin-3-yl)methanone | C.29 | 452.4 [M+H]⁺ |
| | | | and | |
| | | | CAS 869670-33-7 | |

### Example 175

### [3-Chloro-5-(1,1-dioxo-1,4-thiazinan-4-yl)phenyl]-[4-(5-methyl-[1,3]oxazolo[4,5-b]pyridin-2-yl)piperazin-1-yl]methanone

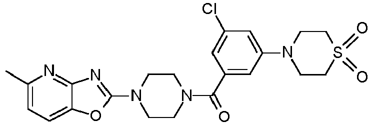

A stirred solution of 1-[(3-chloro-5-iodophenyl)carbonyl]-4-{5-methyl-[1,3]oxazolo[4,5-b]pyridin-2-yl}piperazine (50 mg, 0.10 mmol) (C.1) in toluene (2 mL) was degassed with argon for 5 minutes followed by the addition of Cs₂CO₃ (101 mg, 0.31 mmol), 1,4-thiazinane 1,1-dioxide (21 mg, 0.16 mmol), BINAP (19 mg, 0.03 mmol) and Pd(OAc)₂ (4.7 mg, 0.02 mmol) while degassing continued. The tube was sealed and heated at 120 °C for 6 h. The reaction mass was filtered through a pad of Celite and washed with 10% methanol in dichloromethane. The filtrate was concentrated in vacuo to dryness. The crude product was purified by silica column flash chomatography with an eluent mixture of MeOH and DCM (2%) providing the title compound (26 mg, 51 %) as an off-white solid. MS (ESI): m/z = 489.8 [M+H]⁺.

In analogy to example 175, example 176 to 180 of the following table were prepared from building block C.1 or C.11 and an suitable amine.

| **Ex.** | **Structure** | **Systematic Name** | **Building Blocks** | **MS (ESI): m/z** |
|---|---|---|---|---|
| 176 | 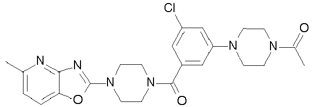 | 1-[4-[3-chloro-5-[4-(5-methyl-[1,3]oxazolo[4,5-b]pyridin-2-yl)piperazine-1-carbonyl]phenyl]pi perazin-1-yl]ethanone | C.1 | 458.3 [M+H]⁺ |
| | | | and | |
| | | | 1-(piperazin-1-yl)ethan-1-one | |
| 177 | 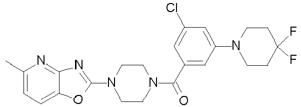 | [3-chloro-5-(4,4-difluoropiperidin-1-yl)phenyl]-[4-(5-methyl-[1,3]oxazolo[4,5-b]pyridin-2-yl)piperazin-1-yl]methanone | C.1 | 475.9 [M+H]⁺ |
| | | | and | |
| | | | 4,4-difluoropiperi dine | |
| 178 | 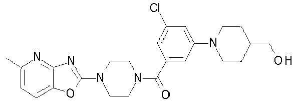 | [3-chloro-5-[4-(hydroxymethyl)pi peridin-1-yl]phenyl]-[4-(5-methyl-[1,3]oxazolo[4,5-b]pyridin-2-yl)piperazin-1-yl]methanone | C.1 | 469.7 [M+H]⁺ |
| | | | and | |
| | | | piperidin-4-ylmethanol | |
| 179 | 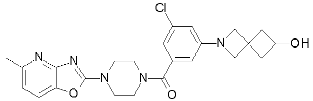 | [3-chloro-5-(6-hydroxy-2-azaspiro[3.3]heptan-2-yl)phenyl]-[4-(5-methyl-[1,3]oxazolo[4,5-b]pyridin-2-yl)piperazin-1-yl]methanone | C.1 | 467.8 [M+H]⁺ |
| | | | and 2-azaspiro[3.3] heptan-6-ol | |
| 180 | 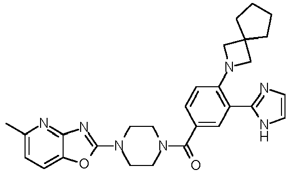 | [4-(2-azaspiro[3.4]octan-2-yl)-3-(1H-imidazol-2-yl)phenyl]-[4-(5-methyl-[1,3]oxazolo[4,5-b]pyridin-2-yl)piperazin-1-yl]methanone | C.11 | 497.9 [M+H]⁺ |
| | | | and | |
| | | | 2-azaspiro[3.4] octane hydrochloride | |

### Example 181

### [3-Chloro-5-[4-(hydroxymethyl)phenyl]phenyl]-[4-(5-methyl-[1,3]oxazolo[4,5-b]pyridin-2-yl)piperazin-1-yl]methanone

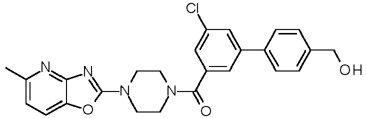

A stirred solution of 1-[(3-chloro-5-iodophenyl)carbonyl]-4-{5-methyl-[1,3]oxazolo[4,5-b]pyridin-2-yl}piperazine (100 mg, 0.21 mmol) (C.1) in 1,4-dioxane (4.5 mL) and water (0.5 mL) was degassed with argon for 5 minutes, followed by the addition of potassium phosphate tri-basic (97 mg, 0.46 mmol), [4-(hydroxymethyl)phenyl]boronic acid (31 mg, 0.21 mmol) and Pd(dppf)Cl₂·CH₂Cl₂ (8.5 mg, 0.01 mmol) while degassing continued. The tube was sealed and heated at 115 °C for 17 h. The reaction mass was filtered through a pad of Celite and concentrated in vacuo. The crude was purified by preparative HPLC to provide the title compound (46 mg, 48%) as an off white solid. MS (ESI): m/z = 463.2 [M+H]⁺.

In analogy to example 181, example 182 to 191 of the following table were prepared from a halide (example C.X or example 141) and a boronic acid derivative with K₃PO₄ or Cs₂CO₃.

| **Ex.** | **Structure** | **Systematic Name** | **Starting materials** | **Base** | **MS (ESI) m/z** |
|---|---|---|---|---|---|
| 182 | 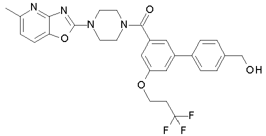 | [3-[4-(hydroxymethyl)ph enyl]-5-(3,3,3-trifluoropropoxy)p henyl]-[4-(5-methyl-[1,3]oxazolo[4,5-b]pyridin-2-yl)piperazin-1-yl]methanone | C.2 | K₃PO₄ | 541.4 [M+H]⁺ |
| | | | and | | |
| | | | [4-(hydroxy methyl)ph enyl]boron ic acid | | |
| 183 | 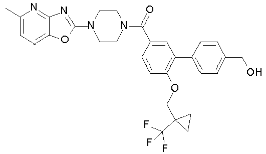 | [3-[4-(hydroxymethyl)ph enyl]-4-[[1-(trifluoromethyl)cy clopropyl]methoxy ]phenyl]-[4-(5-methyl-[1,3]oxazolo[4,5-b]pyridin-2-yl)piperazin-1-yl]methanone | C.3 | K₃PO₄ | 567.2 [M+H]⁺ |
| | | | and | | |
| | | | [4-(hydroxy methyl)ph enyl]boron ic acid | | |
| 184 | 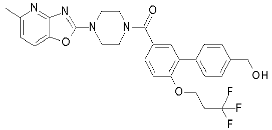 | [3-[4-(hydroxymethyl)ph enyl]-4-(3,3,3-trifluoropropoxy)p henyl]-[4-(5-methyl-[1,3]oxazolo[4,5-b]pyridin-2-yl)piperazin-1-yl]methanone | C.4 | K₃PO₄ | 541.3 [M+H]⁺ |
| | | | and | | |
| | | | [4-(hydroxy methyl)ph enyl]boron ic acid | | |
| 185 | 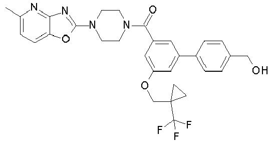 | [3-[4-(hydroxymethyl)ph enyl]-5-[[1-(trifluoromethyl)cy clopropyl]methoxy ]phenyl]-[4-(5-methyl-[1,3]oxazolo[4,5-b]pyridin-2-yl)piperazin-1-yl]methanone | C.5 | K₃PO₄ | 567.2 [M+H]⁺ |
| | | | and | | |
| | | | [4-(hydroxy methyl)ph enyl]boron ic acid | | |
| 186 | 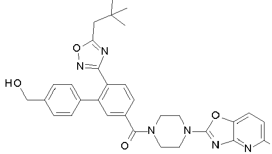 | [4-[5-(2,2-dimethylpropyl)-1,2,4-oxadiazol-3-yl]-3-[4-(hydroxymethyl)ph enyl]phenyl]-[4-(5-methyl-[1,3]oxazolo[4,5-b]pyridin-2-yl)piperazin-1-yl]methanone | C.8 | K₃PO₄ | 567.2 [M+H]⁺ |
| | | | and | | |
| | | | [4-(hydroxy methyl)ph enyl]boron ic acid | | |
| 187 | 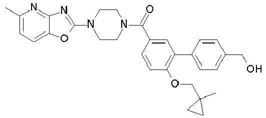 | [3-[4-(hydroxymethyl)ph enyl]-4-[(1-methylcyclopropyl) methoxy]phenyl]-[4-(5-methyl-[1,3]oxazolo[4,5-b]pyridin-2-yl)piperazin-1-yl]methanone | C.9 | K₃PO₄ | 513.2 [M+H]⁺ |
| | | | and | | |
| | | | [4-(hydroxy methyl)ph enyl]boron ic acid | | |
| 188 | 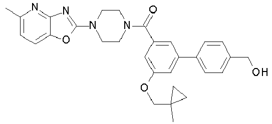 | [3-[4-(hydroxymethyl)ph enyl]-5-[(1-methylcyclopropyl) methoxy]phenyl]-[4-(5-methyl-[1,3]oxazolo[4,5-b]pyridin-2-yl)piperazin-1-yl]methanone | C.10 | K₃PO₄ | 513.9 [M+H]⁺ |
| | | | and | | |
| | | | [4-(hydroxy methyl)ph enyl]boron ic acid | | |
| 189 | 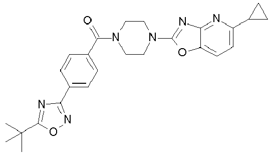 | (4-(5-(tert-butyl)-1,2,4-oxadiazol-3-yl)phenyl)(4-(5-cyclopropyloxazolo [4,5-b]pyridin-2-yl)piperazin-1-yl)methanone | Example 141 | Cs₂CO₃ | 473.3 [M+H]⁺ |
| | | | and | | |
| | | | cycloprop ylboronic acid | | |
| 190 | 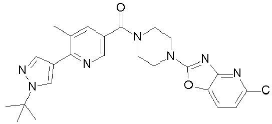 | [6-(1-tert-butylpyrazol-4-yl)-5-methylpyridin-3-yl]-[4-(5-chloro-[1,3]oxazolo[4,5-b]pyridin-2-yl)piperazin-1-yl]methanone | C.46 | K₃PO₄ | 480.1 [M+H]⁺ |
| | | | and CAS 1256359-15-5 | | |
| 191 | 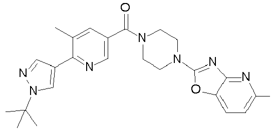 | [6-(1-tert-butylpyrazol-4-yl)-5-methylpyridin-3-yl]-[4-(5-methyl-[1,3]oxazolo[4,5-b]pyridin-2-yl)piperazin-1-yl]methanone | C.47 | K₃PO₄ | 460.2 [M+H]⁺ |
| | | | and | | |
| | | | CAS 1256359-15-5 | | |

### Example 192

### (4-(5-Oxa-2-azaspiro [3.4] octan-2-yl)phenyl)(4-(oxazolo [4,5-b] pyridin-2-yl)piperazin-1-yl)methanone

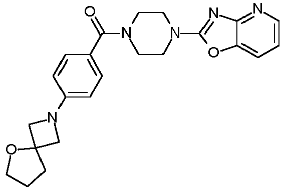

A mixture of (4-iodophenyl)(4-(oxazolo[4,5-b]pyridin-2-yl)piperazin-1-yl)methanone (60.3 mg, 125 µmol) (C.6), 5-oxa-2-azaspiro[3.4]octane hemioxalate (39.5 mg, 250 µmol) and potassium carbonate (69.1 mg, 500 µmol) in DMSO (0.75 mL) was purged with argon for 5 min. Then N,N-dimethylglycine (5.16 mg, 50 µmol) and copper (I) iodide (4.76 mg, 25 µmol) was added, the tube sealed and heated to 95°C overnight. The reaction mixture was diluted with water, filtrated, the solid solved in EtOAc, dried with MgSO₄ and evaporated to dryness. The crude product was purified by silica column flash chomatography with an eluent mixture of MeOH and DCM (0% - 8% gradient) providing the title compound (24.2 mg, 46.2%) as an off-white solid. MS (ESI): m/z = 420.2 [M+H]⁺.

In analogy to example 192, example 193 to 213 of the following table were prepared from a halide building block (Example C.X or example 141) and an amine derivative with K₂CO₃ or Cs₂CO₃.

| **Ex.** | **Structure** | **Systematic Name** | **Starting materials** | **Base** | **MS (ESI): m/z** |
|---|---|---|---|---|---|
| 193 | 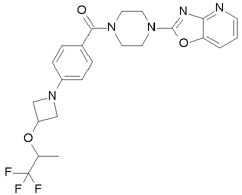 | (4-(oxazolo[4,5-b]pyridin-2-yl)piperazin-1-yl)(4-(3-((1,1,1-trifluoropropan-2-yl)oxy)azetidin-1-yl)phenyl)methanon e | C.6 | K₂CO₃ | 476.3 [M+H]⁺ |
| | | | and | | |
| | | | CAS 1502257-94-4 | | |
| 194 | 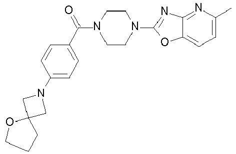 | (4-(5-oxa-2-azaspiro[3.4]octan-2-yl)phenyl)(4-(5-methyloxazolo [4,5- b]pyridin-2-yl)piperazin-1-yl)methanone | C.7 | K₂CO₃ | 434.3 [M+H]⁺ |
| | | | and | | |
| | | | CAS 145309-24-6 | | |
| 195 | 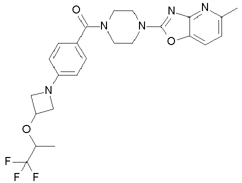 | (4-(5-methyloxazolo[4,5-b]pyridin-2-yl)piperazin-1-yl)(4-(3-((1,1,1-trifluoropropan-2-yl)oxy)azetidin-1-yl)phenyl)methanon e | C.7 | K₂CO₃ | 490.3 [M+H]⁺ |
| | | | and | | |
| | | | CAS 1502257-94-4 | | |
| 196 | 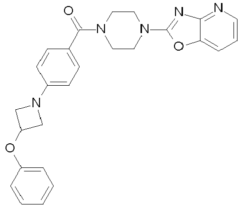 | (4-(oxazolo[4,5-b]pyridin-2-yl)piperazin-1-yl)(4-(3-phenoxyazetidin-1- yl)phenyl)methanon e | C.6 | K₂CO₃ | 456.3 [M+H]⁺ |
| | | | and | | |
| | | | CAS 301335-39-7 | | |
| 197 | 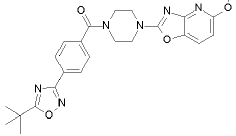 | (4-(5-(tert-butyl)-1,2,4-oxadiazol-3-yl)phenyl)(4-(5-methoxyoxazolo[4, 5-b]pyridin-2-yl)piperazin-1-yl)methanone | Example 141 | Cs₂CO₃ | 463.3 [M+H]⁺ |
| 198 | 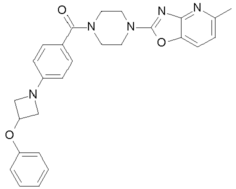 | (4-(5-methyloxazolo[4,5- b]pyridin-2-yl)piperazin-1-yl)(4-(3-phenoxyazetidin-1-yl)phenyl)methanon e | C.7 | K₂CO₃ | 470.3 [M+H]⁺ |
| | | | and | | |
| | | | CAS 301335-39-7 | | |
| 199 | 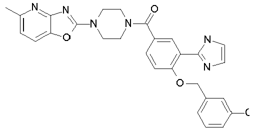 | [4-[(3-chlorophenyl)metho xy]-3-(1H-imidazol-2-yl)phenyl]-[4-(5-methyl-[1,3]oxazolo[4,5-b]pyridin-2-yl)piperazin-1-yl]methanone | C.11 | Cs₂CO₃ | 529.1 [M+H]⁺ |
| | | | and | | |
| | | | CAS 873-63-2 | | |
| 200 | 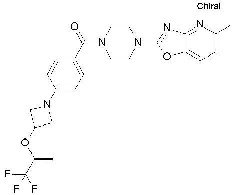 | (S)-(4-(5-methyloxazolo[4,5- b]pyridin-2-yl)piperazin-1-yl)(4-(3-((1,1,1-trifluoropropan-2-yl)oxy)azetidin-1-yl)phenyl)methanon e | C.7 | K₂CO₃ | 490.3 [M+H]⁺ |
| | | | and | | |
| | | | CAS 1803585-68-3 | | |
| 201 | 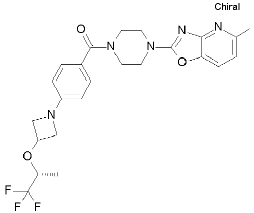 | (R)-(4-(5-methyloxazolo[4,5- b]pyridin-2-yl)piperazin-1-yl)(4-(3-((1,1,1-trifluoropropan-2-yl)oxy)azetidin-1-yl)phenyl)methanon e | C.7 | K₂CO₃ | 490.3 [M+H]⁺ |
| | | | and | | |
| | | | CAS 1803585-68-3 | | |
| 202 | 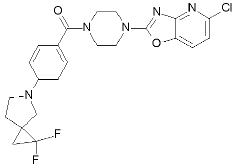 | [4-(5-chloro-[1,3]oxazolo[4,5-b]pyridin-2-yl)piperazin-1-yl]-[4-(2,2-difluoro-5-azaspiro[2.4]heptan -5-yl)phenyl]methanon e | C.12 | K₂CO₃ | 474.3 [M+H]⁺ |
| | | | and | | |
| | | | CAS 1215071-12-7 | | |
| 203 | 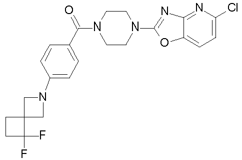 | [4-(5-chloro-[1,3]oxazolo[4,5-b]pyridin-2-yl)piperazin-1-yl]-[4-(7,7-difluoro-2-azaspiro[3.3]heptan -2-yl)phenyl]methanon e | C.12 | K₂CO₃ | 474.3 [M+H]⁺ |
| | | | and | | |
| | | | CAS 1523617-85-7 | | |
| 204 | 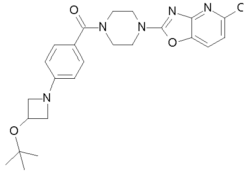 | (4-(3-(tert-butoxy)azetidin-1-yl)phenyl)(4-(5-chlorooxazolo[4,5-b]pyridin-2-yl)piperazin-1-yl)methanone | C.12 | K₂CO₃ | 470.3 [M+H]⁺ |
| | | | and | | |
| | | | CAS 1147530-63-9 | | |
| 205 | 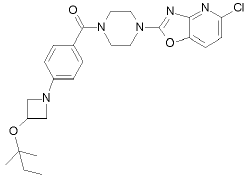 | (4-(5-chlorooxazolo[4,5-b]pyridin-2-yl)piperazin-1-yl)(4-(3-(tert-pentyloxy)azetidin-1-yl)phenyl)methanon e | C.12 | K₂CO₃ | 484.3 [M+H]⁺ |
| | | | and | | |
| | | | CAS 1343048-51-0 | | |
| 206 | 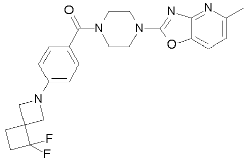 | (4-(5,5-difluoro-2-azaspiro[3.3]heptan -2-yl)phenyl)(4-(5 - methyloxazolo[4,5- b]pyridin-2-yl)piperazin-1-yl)methanone | C.7 | K₂CO₃ | 454.3 [M+H]⁺ |
| | | | and | | |
| | | | CAS 1523617-85-7 | | |
| 207 | 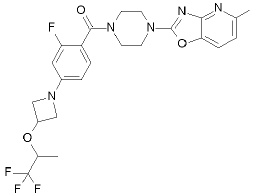 | (2-fluoro-4-(3-((1,1,1-trifluoropropan-2-yl)oxy)azetidin-1-yl)phenyl)(4-(5-methyloxazolo[4,5- b]pyridin-2-yl)piperazin-1-yl)methanone | C.15 | K₂CO₃ | 508.3 [M+H]⁺ |
| | | | and | | |
| | | | CAS 1803585-68-3 | | |
| 208 | 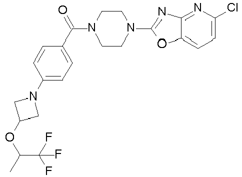 | (4-(5-chlorooxazolo[4,5- b]pyridin-2-yl)piperazin-1-yl)(4-(3-((1,1,1-trifluoropropan-2-yl)oxy)azetidin-1-yl)phenyl)methanon e | C.12 | K₂CO₃ | 510.2 [M+H]⁺ |
| | | | and | | |
| | | | CAS 1803585-68-3 | | |
| 209 | 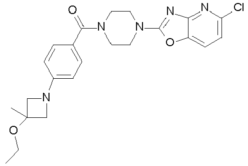 | (4-(5-chlorooxazolo[4,5- b]pyridin-2-yl)piperazin-1-yl)(4-(3-ethoxy-3-methylazetidin-1-yl)phenyl)methanon e | C.12 | K₂CO₃ | 456.3 [M+H]⁺ |
| | | | and | | |
| | | | CAS 1416586-63-4 | | |
| 210 | 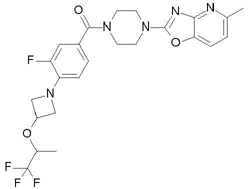 | (3-fluoro-4-(3-((1,1,1-trifluoropropan-2-yl)oxy)azetidin-1-yl)phenyl)(4-(5-methyloxazolo[4,5- b]pyridin-2-yl)piperazin-1-yl)methanone | C.16 | K₂CO₃ | 508.3 [M+H]⁺ |
| | | | and | | |
| | | | CAS 1803585-68-3 | | |
| 211 | 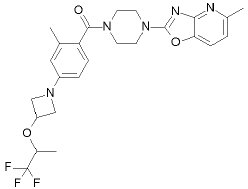 | (2-methyl-4-(3-((1,1,1-trifluoropropan-2-yl)oxy)azetidin-1-yl)phenyl)(4-(5-methyloxazolo[4,5- b]pyridin-2-yl)piperazin-1-yl)methanone | C.22 | K₂CO₃ | 504.3 [M+H]⁺ |
| | | | and | | |
| | | | CAS 1803585-68-3 | | |
| 212 | 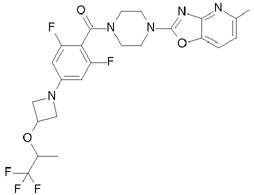 | (2,6-difluoro-4-(3 - ((1,1,1-trifluoropropan-2-yl)oxy)azetidin-1-yl)phenyl)(4-(5-methyloxazolo[4,5- b]pyridin-2-yl)piperazin-1-yl)methanone | C.23 | K₂CO₃ | 526.3 [M+H]⁺ |
| | | | and | | |
| | | | CAS 1803585-68-3 | | |
| 213 | 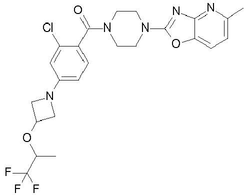 | (2-chloro-4-(3-((1,1,1-trifluoropropan-2-yl)oxy)azetidin-1-yl)phenyl)(4-(5-methyloxazolo[4,5- b]pyridin-2-yl)piperazin-1-yl)methanone | C.24 | K₂CO₃ | 524.3 [M+H]⁺ |
| | | | and | | |
| | | | CAS 1803585-68-3 | | |

### Example 214

### (4-(5-Methyloxazolo [4,5-b] pyridin-2-yl)piperazin-1-yl)(6-(3-((1,1,1-trifluoropropan-2-yl)oxy)azetidin-1-yl)pyridin-3-yl)methanone

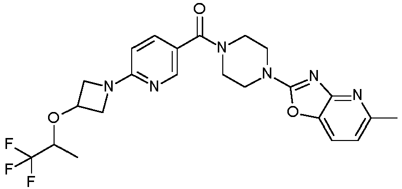

In a vial suitable for microwave chemistry, to a stirred solution of (6-fluoropyridin-3-yl)(4-(5-methyloxazolo[4,5-b]pyridin-2-yl)piperazin-1-yl)methanone (50 mg, 132 µmol) (C.13) in NMP (1 mL) at r.t., 3-((1,1,1-trifluoropropan-2-yl)oxy)azetidine hydrochloride (54.2 mg, 0.164 mmol) and N-ethyldisopropylamine (85.2 mg, 115 µL, 659 µmol) were added. The vial was sealed. The mixture was heated to 100°C (oil bath temperature) and stirring was continued for 1.5 h. The mixture was cooled to r.t., diluted with EtOAc and washed with water. The aqueous phase was back-extracted with EtOAc. The combined organic layers were washed with water and brine, dried (MgSO₄), filtered and concentrated to leave the crude product as a light brown sticky solid. The crude product was purified by silica column flash chomatography with an eluent mixture of MeOH and DCM (0% - 10% gradient) to provide the title compound (55 mg, 82.5%) as a light yellow solid. MS (ESI): m/z = 491.3 [M+H]⁺.

In analogy to example 214, example 215 to 230 of the following table were prepared from an example C.X and an amine derivative.

| **Ex.** | **Structure** | **Systematic Name** | **Starting material** | **MS (ESI): m/z** |
|---|---|---|---|---|
| 215 | 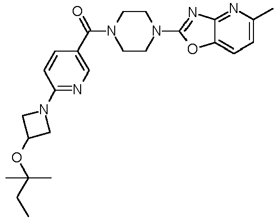 | (4-(5-methyloxazolo[4,5 -b]pyridin-2-yl)piperazin-1-yl)(6-(3-(tert-pentyloxy)azetidin -1-yl)pyridin-3-yl)methanone | C.13 | 465.4 [M+H]⁺ |
| | | | and | |
| | | | CAS 1343048-51-0 | |
| 216 | 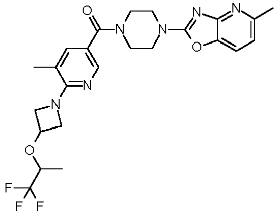 | (5-methyl-6-(3-((1,1,1-trifluoropropan-2-yl)oxy)azetidin-1-yl)pyridin-3-yl)(4-(5-methyloxazolo[4,5 -b]pyridin-2-yl)piperazin-1-yl)methanone | C.14 | 505.3 [M+H]⁺ |
| | | | and | |
| | | | CAS 1803585-68-3 | |
| 217 | 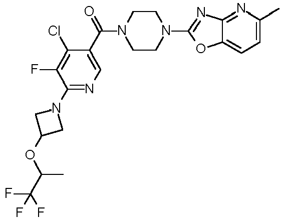 | (4-chloro-5-fluoro-6-(3-((1,1,1-trifluoropropan-2-yl)oxy)azetidin-1-yl)pyridin-3-yl)(4-(5-methyloxazolo[4,5 -b]pyridin-2-yl)piperazin-1-yl)methanone | C.17 | 543.3 [M+H]⁺ |
| | | | and | |
| | | | CAS 1803585-68-3 | |
| 218 | 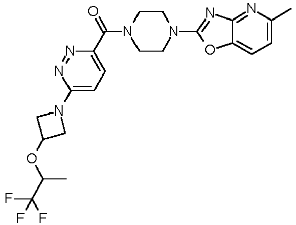 | (4-(5-methyloxazolo[4,5 -b]pyridin-2-yl)piperazin-1-yl)(6-(3-((1,1,1-trifluoropropan-2-yl)oxy)azetidin-1-yl)pyridazin-3-yl)methanone | C.18 | 492.3 [M+H]⁺ |
| | | | and | |
| | | | CAS 1803585-68-3 | |
| 219 | 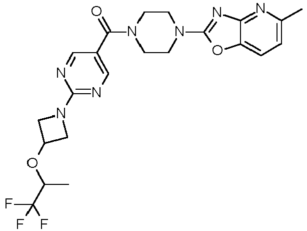 | (4-(5-methyloxazolo[4,5 -b]pyridin-2-yl)piperazin-1-yl)(2-(3-((1,1,1-trifluoropropan-2-yl)oxy)azetidin-1-yl)pyrimidin-5-yl)methanone | C.19 | 492.3 [M+H]⁺ |
| | | | and | |
| | | | CAS 1803585-68-3 | |
| 220 | 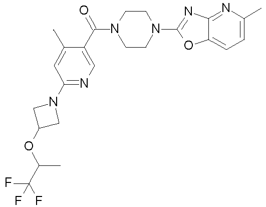 | (4-methyl-6-(3 - ((1,1,1-trifluoropropan-2-yl)oxy)azetidin-1-yl)pyridin-3 -yl)(4-(5-methyloxazolo[4,5 -b]pyridin-2-yl)piperazin-1-yl)methanone | C.20 | 505.4 [M+H]⁺ |
| | | | and | |
| | | | CAS 1803585-68-3 | |
| 221 | 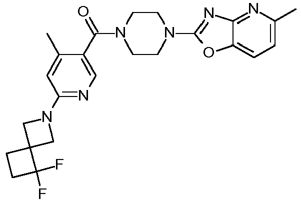 | (6-(5,5-difluoro-2-azaspiro[3.3]hepta n-2-yl)-4-methylpyridin-3-yl)(4-(5-methyloxazolo[4,5 -b]pyridin-2-yl)piperazin-1-yl)methanone | C.20 | 469.4 [M+H]⁺ |
| | | | and | |
| | | | CAS 1523617-85-7 | |
| 222 | 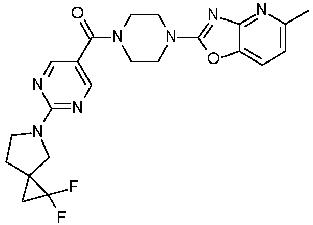 | (2-(1,1-difluoro-5-azaspiro[2.4]hepta n-5-yl)pyrimidin-5-yl)(4-(5-methyloxazolo[4,5 -b]pyridin-2-yl)piperazin-1-yl)methanone | C.19 | 456.3 [M+H]⁺ |
| | | | and | |
| | | | CAS 1215071-12-7 | |
| 223 | 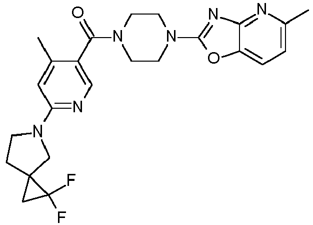 | (6-(1,1-difluoro-5-azaspiro[2.4]hepta n-5-yl)-4-methylpyridin-3-yl)(4-(5-methyloxazolo[4,5 -b]pyridin-2-yl)piperazin-1-yl)methanone | C.20 | 469.4 [M+H]⁺ |
| | | | and | |
| | | | CAS121507 1-12-7 | |
| 224 | 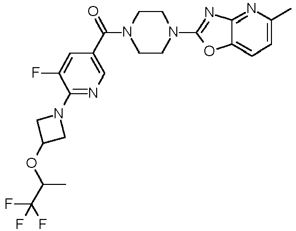 | (5-fluoro-6-(3-((1,1,1-trifluoropropan-2-yl)oxy)azetidin-1-yl)pyridin-3-yl)(4-(5-methyloxazolo[4,5 -b]pyridin-2-yl)piperazin-1-yl)methanone | C.21 | 509.3 [M+H]⁺ |
| | | | and | |
| | | | CAS 1803585-68-3 | |
| 225 | 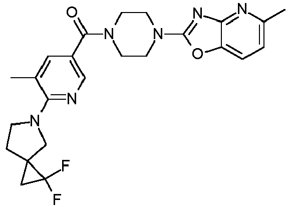 | (6-(1,1-difluoro-5-azaspiro[2.4]hepta n-5-yl)-5-methylpyridin-3-yl)(4-(5-methyloxazolo[4,5 -b]pyridin-2-yl)piperazin-1-yl)methanone | C.14 | 469.3 [M+H]⁺ |
| | | | and | |
| | | | CAS121507 1-12-7 | |
| 226 | 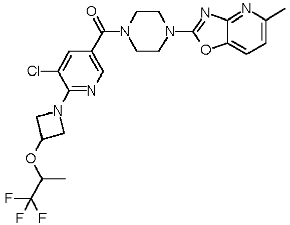 | (5-chloro-6-(3-((1,1,1-trifluoropropan-2-yl)oxy)azetidin-1-yl)pyridin-3 -yl)(4-(5-methyloxazolo[4,5 -b]pyridin-2-yl)piperazin-1-yl)methanone | C.25 | 525.2 [M+H]⁺ |
| | | | and | |
| | | | CAS 1803585-68-3 | |
| 227 | 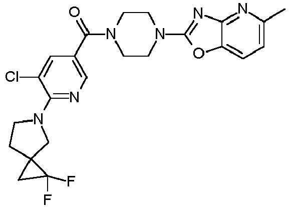 | (5-chloro-6-(1,1-difluoro-5-azaspiro[2.4]hepta n-5-yl)pyridin-3-yl)(4-(5-methyloxazolo[4,5 -b]pyridin-2-yl)piperazin-1-yl)methanone | C.25 | 489.2 [M+H]⁺ |
| | | | and | |
| | | | CAS 1215071-12-7 | |
| 228 | 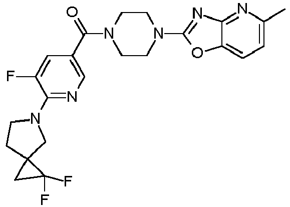 | (6-(1,1-difluoro-5-azaspiro[2.4]hepta n-5-yl)-5-fluoropyridin-3-yl)(4-(5-methyloxazolo[4,5 -b]pyridin-2-yl)piperazin-1-yl)methanone | C.21 | 473.2 [M+H]⁺ |
| | | | and | |
| | | | CAS 1215071-12-7 | |
| 229 | 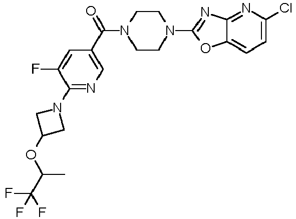 | (4-(5-chlorooxazolo[4,5-b]pyridin-2-yl)piperazin-1-yl)(5-fluoro-6-(3-((1,1,1-trifluoropropan-2-yl)oxy)azetidin-1-yl)pyridin-3-yl)methanone | C.26 | 529.2 [M+H]⁺ |
| | | | and | |
| | | | CAS 1803585-68-3 | |
| 230 | 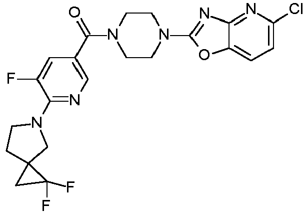 | (4-(5-chlorooxazolo[4,5-b]pyridin-2-yl)piperazin-1-yl)(6-(1,1-difluoro-5-azaspiro[2.4]hepta n-5-yl)-5-fluoropyridin-3-yl)methanone | C.26 | 493.2 [M+H]⁺ |
| | | | and | |
| | | | CAS 1215071-12-7 | |

### Example 231

### (4-(5-Chlorooxazolo[4,5-b] pyridin-2-yl)piperazin-1-yl)(3-methyl-4-(1-neopentyl-1H-1,2,3-triazol-4-yl)phenyl)methanone

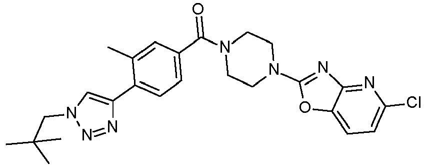

To a solution of (4-(5-chlorooxazolo[4,5-b]pyridin-2-yl)piperazin-1-yl)(3-methyl-4-(1H-1,2,3-triazol-4-yl)phenyl)methanone (65 mg, 153 µmol) (C.27) in DMF (0.7 mL) in a vial suitable for microwave chemistry was added potassium carbonate (42.4 mg, 307 µmol) and 1-iodo-2,2-dimethylpropane (40.3 mg, 27 µL, 199 µmol). The vial was closed and the mixture was stirred at 83 °C overnight. The mixture was diluted with EtOAc and washed with water. The aqueous phase was back extracted with EtOAc. The combined organics were washed with water and brine, dried (MgSO₄), filtered and concentrated to leave the crude product as a light yellow liquid. The crude product was purified by preparative HPLC to provide the title compound (10.5 mg, 13.9%) as a white lyophilized powder. MS (ESI): m/z = 494.2 [M+H]⁺

Examples 232 to 248 of the following table were prepared in analogy to Example 231.

| **Ex.** | **Structure** | **Systematic Name** | **Starting material** | **MS (ESI): m/z** |
|---|---|---|---|---|
| 232 | 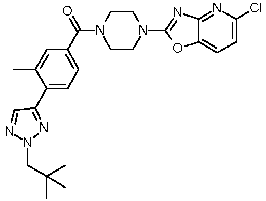 | (4-(5-chlorooxazolo[4,5-b]pyridin-2-yl)piperazin-1-yl)(3-methyl-4-(2-neopentyl-2H-1,2,3-triazol-4-yl)phenyl)methanone | C.27 | 494.2 [M+H]⁺ |
| | | | and | |
| | | | CAS 15501-33-4 | |
| 233 | 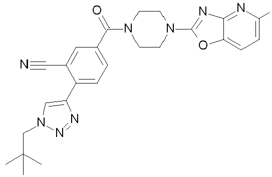 | 5-(4-(5-chlorooxazolo[4,5-b]pyridin-2-yl)piperazine-1-carbonyl)-2-(1-neopentyl-1H-1,2,3-triazol-4-yl)benzonitrile | C.32 | 505.2 [M+H]⁺ |
| | | | and | |
| | | | CAS 15501-33-4 | |
| 234 | 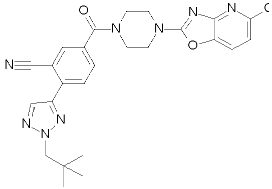 | 5-(4-(5-chlorooxazolo[4,5-b]pyridin-2-yl)piperazine-1-carbonyl)-2-(2-neopentyl-2H-1,2,3-triazol-4-yl)benzonitrile | C.32 | 505.2 [M+H]⁺ |
| | | | and | |
| | | | CAS 15501-33-4 | |
| 235 | 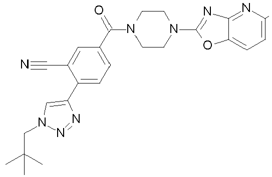 | 5-(4-(5-methyloxazolo[4,5-b]pyridin-2-yl)piperazine-1-carbonyl)-2-(1-neopentyl-1H-1,2,3-triazol-4-yl)benzonitrile | C.35 | 485.2 [M+H]⁺ |
| | | | and | |
| | | | CAS 15501-33-4 | |
| 236 | 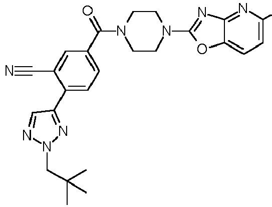 | 5-(4-(5-methyloxazolo[4,5-b]pyridin-2-yl)piperazine-1-carbonyl)-2-(2-neopentyl-2H-1,2,3-triazol-4-yl)benzonitrile | C.35 | 485.2 [M+H]⁺ |
| | | | and | |
| | | | CAS 15501-33-4 | |
| 237 | 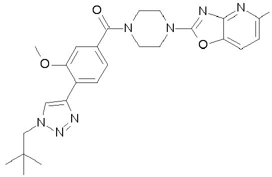 | (4-(5-chlorooxazolo[4,5-b]pyridin-2-yl)piperazin-1-yl)(3-methoxy-4-(1-neopentyl-1H-1,2,3-triazol-4-yl)phenyl)methanone | C.37 | 510.2 [M+H]⁺ |
| | | | and | |
| | | | CAS 15501-33-4 | |
| 238 | 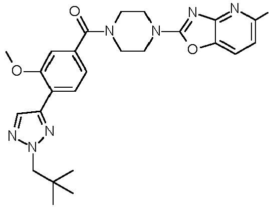 | (4-(5-chlorooxazolo[4,5-b]pyridin-2-yl)piperazin-1-yl)(3-methoxy-4-(2-neopentyl-2H-1,2,3-triazol-4-yl)phenyl)methanone | C.37 | 510.2 [M+H]⁺ |
| | | | and | |
| | | | CAS 15501-33-4 | |
| 239 | 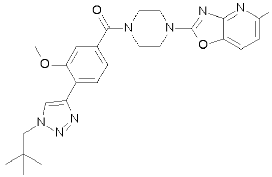 | (3-methoxy-4-(1-neopentyl-1H-1,2,3-triazol-4-yl)phenyl)(4-(5 -methyloxazolo [4,5-b]pyridin-2-yl)piperazin-1-yl)methanone | C.39 | 490.2 [M+H]⁺ |
| | | | and | |
| | | | CAS 15501-33-4 | |
| 240 | 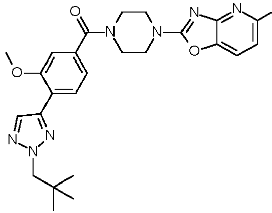 | (3-methoxy-4-(2-neopentyl-2H-1,2,3-triazol-4-yl)phenyl)(4-(5-methyloxazolo [4,5-b]pyridin-2-yl)piperazin-1-yl)methanone | C.39 | 490.2 [M+H]⁺ |
| | | | and | |
| | | | CAS 15501-33-4 | |
| 241 | 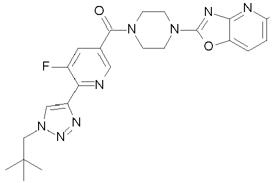 | (4-(5-chlorooxazolo[4,5-b]pyridin-2-yl)piperazin-1-yl)(5-fluoro-6-(1-neopentyl-1H-1,2,3-triazol-4-yl)pyridin-3-yl)methanone | C.42 | 499.2 [M+H]⁺ |
| | | | and | |
| | | | CAS 15501-33-4 | |
| 242 | 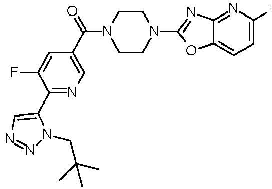 | (4-(5-chlorooxazolo[4,5-b]pyridin-2-yl)piperazin-1-yl)(5-fluoro-6-(1-neopentyl-1H-1,2,3-triazol-5-yl)pyridin-3-yl)methanone | C.42 | 499.2 [M+H]⁺ |
| | | | and | |
| | | | CAS 15501-33-4 | |
| 243 | 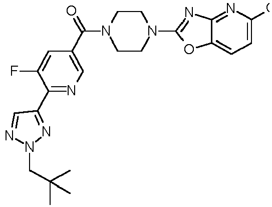 | (4-(5-chlorooxazolo[4,5-b]pyridin-2-yl)piperazin-1-yl)(5-fluoro-6-(2-neopentyl-2H-1,2,3-triazol-4-yl)pyridin-3-yl)methanone | C.42 | 499.2 [M+H]⁺ |
| | | | and | |
| | | | CAS 15501-33-4 | |
| 244 | 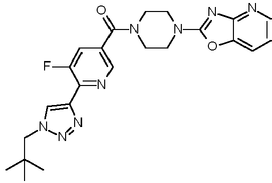 | (5-fluoro-6-(1-neopentyl-1H-1,2,3-triazol-4-yl)pyridin-3- yl)(4-(5-methyloxazolo[4,5-b]pyridin-2-yl)piperazin-1-yl)methanone | C.45 | 479.2 [M+H]⁺ |
| | | | and | |
| | | | CAS 15501-33-4 | |
| 245 | 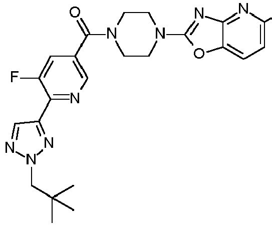 | (5-fluoro-6-(2-neopentyl-2H-1,2,3-triazol-4-yl)pyridin-3- yl)(4-(5-methyloxazolo[4,5-b]pyridin-2-yl)piperazin-1-yl)methanone | C.45 | 479.2 [M+H]⁺ |
| | | | and | |
| | | | CAS 15501-33-4 | |
| 246 | 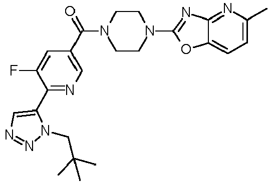 | (5-fluoro-6-(1-neopentyl-1H-1,2,3-triazol-5-yl)pyridin-3-yl)(4-(5-methyloxazolo[4,5-b]pyridin-2-yl)piperazin-1-yl)methanone | C.45 | 479.2 [M+H]⁺ |
| | | | and | |
| | | | CAS 15501-33-4 | |
| 247 | 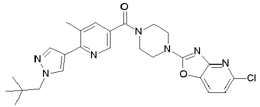 | [4-(5-chloro-[1,3]oxazolo[4,5-b]pyridin-2-yl)piperazin-1-yl]-[6-[1-(2,2-dimethylpropyl)pyraz ol-4-yl]-5-methylpyridin-3-yl]methanone | C.48 | 494.2 [M+H]⁺ |
| | | | and | |
| | | | CAS 630-17-1 | |
| 248 | 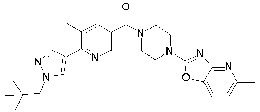 | [6-[1-(2,2-dimethylpropyl)pyraz ol-4-yl]-5-methylpyridin-3-yl]-[4-(5-methyl-[1,3]oxazolo[4,5-b]pyridin-2-yl)piperazin-1-yl] methanone | C.49 | 474.4 [M+H]⁺ |
| | | | and | |
| | | | CAS 630-17-1 | |
| 268 | 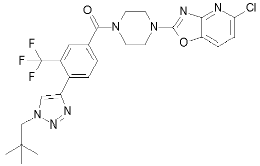 | (4-(5-chlorooxazolo[4,5- b]pyridin-2-yl)piperazin-1-yl)(4-(1-neopentyl-1H-1,2,3-triazol-4-yl)-3- (trifluoromethyl)phen yl)methanone | C.52 | 548.2 [M+H]⁺ |
| | | | and | |
| | | | CAS 15501-33-4 | |
| 269 | 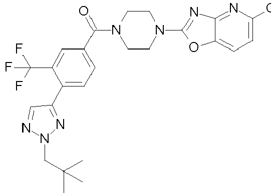 | (4-(5-chlorooxazolo[4,5-b]pyridin-2-yl)piperazin-1-yl)(4-(2-neopentyl-2H-1,2,3-triazol-4-yl)-3- (trifluoromethyl)phen yl)methanone | C.52 | 548.2 [M+H]⁺ |
| | | | and | |
| | | | CAS 15501-33-4 | |
| 270 | 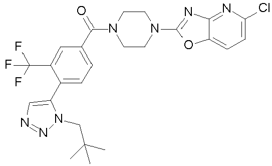 | (4-(5-chlorooxazolo[4,5-b]pyridin-2-yl)piperazin-1-yl)(4-(1-neopentyl-1H-1,2,3-triazol-5-yl)-3-(trifluoromethyl)phen yl)methanone | C.52 | 548.2 [M+H]⁺ |
| | | | and | |
| | | | CAS 15501-33-4 | |
| 271 | 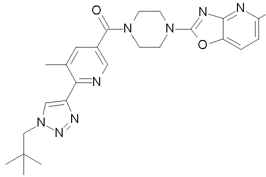 | (4-(5-chlorooxazolo[4,5-b]pyridin-2-yl)piperazin-1-yl)(5-methyl-6-(1-neopentyl-1H-1,2,3-triazol-4-yl)pyridin-3- yl)methanone | C.54 | 495.2 [M+H]⁺ |
| | | | and | |
| | | | CAS 15501-33-4 | |
| 272 | 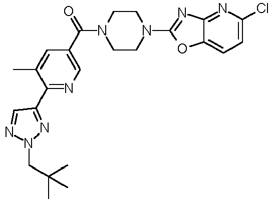 | (4-(5-chlorooxazolo[4,5-b]pyridin-2-yl)piperazin-1-yl)(5-methyl-6-(2-neopentyl-2H-1,2,3-triazol-4-yl)pyridin-3- yl)methanone | C.54 | 495.2 [M+H]⁺ |
| | | | and | |
| | | | CAS 15501-33-4 | |

### Example 249

### (4-(5-Chlorooxazolo[4,5-b]pyridin-2-yl)piperazin-1-yl)(3-((2,6-dichlorophenyl)ethynyl)azetidin-1-yl)methanone

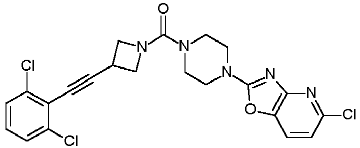

In a sealed tube, 3-((2,6-dichlorophenyl)ethynyl)azetidine (0.010 g, 44.2 µmol) (A.47) and 4-nitrophenyl 4-(5-chlorooxazolo[4,5-b]pyridin-2-yl)piperazine-1-carboxylate (17.9 mg, 44.2 µmol) (B.6) were mixed in acetonitrile (500 µL). Then DIPEA (22.9 mg, 30.9 µL, 177 µmol) and DMAP (2.7 mg, 22.1 µmol) were added. The mixture was heated to 90 °C and stirred overnight. The mixture was evaporated to dryness and the crude brown residue purified by reverse phase chromatography to provide the title compound (8 mg, 36.8%) as a light brown solid. MS (ESI): m/z = 492.0 [M+H]⁺.

Examples 250 to 256 of the following table were prepared in analogy to example 249.

| **Ex.** | **Structure** | **Systematic Name** | **Starting material** | **MS (ESI): m/z** |
|---|---|---|---|---|
| 250 | 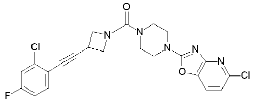 | [3-[2-(2-chloro-4-fluorophenyl)ethynyl ]azetidin-1-yl]-[4-(5-chloro-[1,3]oxazolo[4,5-b]pyridin-2-yl)piperazin-1-yl] methanone | B.6 | 474.2 [M+H]⁺ |
| | | | and | |
| | | | A.48 | |
| 251 | 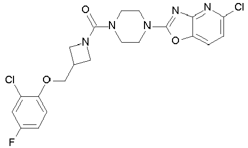 | [3-[(2-chloro-4-fluorophenoxy)methy l]azetidin-1-yl]-[4-(5-chloro-[1,3]oxazolo[4,5-b]pyridin-2-yl)piperazin-1-yl] methanone | B.6 | 480.3 [M+H]⁺ |
| | | | and | |
| | | | CAS 1332301-43-5 | |
| 252 | 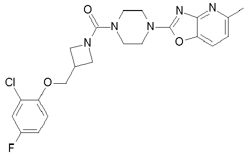 | [3-[(2-chloro-4-fluorophenoxy)methy l]azetidin-1-yl]-[4-(5-methyl-[1,3]oxazolo[4,5-b]pyridin-2-yl)piperazin-1-yl] methanone | B.7 | 460.4 [M+H]⁺ |
| | | | and | |
| | | | CAS 1332301-43-5 | |
| 253 | 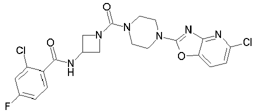 | 2-chloro-N-[1-[4-(5-chloro-[1,3]oxazolo[4,5-b]pyridin-2-yl)piperazine-1-carbonyl]azetidin-3-yl]-4-fluorobenzamide | B.6 | 493.3 [M+H]⁺ |
| | | | and | |
| | | | CAS 1490358-43-4 | |
| 254 | 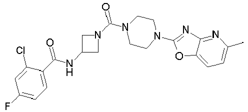 | 2-chloro-4-fluoro-A-[1-[4-(5-methyl-[1,3]oxazolo[4,5-b]pyridin-2-yl)piperazine-1-carbonyl]azetidin-3-yl]benzamide | B.7 | 473.3 [M+H]⁺ |
| | | | and | |
| | | | CAS 1490358-43-4 | |
| 255 | 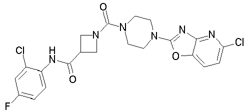 | N-(2-chloro-4-fluorophenyl)-1-[4-(5-chloro-[1,3]oxazolo[4,5-b]pyridin-2-yl)piperazine-1-carbonyl]azetidine-3-carboxamide | B.2 | 493.3 [M+H]⁺ |
| | | | and | |
| | | | CAS 1490358-43-4 | |
| 256 | 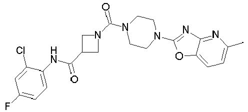 | N-(2-chloro-4-fluorophenyl)-1-[4-(5-methyl-[1,3]oxazolo[4,5-b]pyridin-2-yl)piperazine-1-carbonyl]azetidine-3-carboxamide | B.7 | 473.3 [M+H]⁺ |
| | | | and | |
| | | | CAS 1490358-43-4 | |

### Example 257

### (4-(1-(tert-Butyl)-1H-1,2,3-triazol-4-yl)-3-methylphenyl)(4-(5-chlorooxazolo[4,5-b]pyridin-2-yl)piperazin-1-yl)methanone

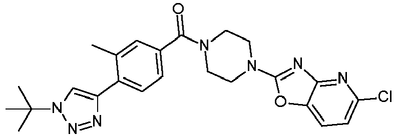

To a stirred solution of 2-methylpropan-2-amine (112 mg, 162 µL, 1.53 mmol) at r.t. in acetonitrile anhydrous (5 mL) under an argon atmosphere were added DMAP (224 mg, 1.84 mmol) and 2-azido-1,3-dimethyl-4,5-dihydro-1H-imidazol-3-ium hexafluorophosphate(V) (524 mg, 1.84 mmol). The mixture was heated to 30 °C and stirring was continued for 1 h. (4-(5-chlorooxazolo[4,5-b]pyridin-2-yl)piperazin-1-yl)(4-ethynyl-3-methylphenyl)methanone (700 mg, 1.84 mmol) (C.43), copper(II) sulfate pentahydrate (19.1 mg, 76.6 µmol) and (+)-sodium L-ascorbate 1.25M aq. solution (613 µL, 766 µmol) were added. The was heated to 70 °C and stirring was continued for 16 h. The mixture was cooled to r.t., diluted with EtOAc and washed with sat. aq. NaHCO₃. The aqueous phase was back-extracted with EtOAc. The combined organics were washed with brine, dried (MgSO4), filtered and concentrated to leave the crude product as a brown viscous oil. The crude was purified by preparative HPLC to provide the title compound (59 mg, 8.03%) as a white lyophilized solid. MS (ESI): m/z = 480.4 [M+H]⁺.

In analogy to example 257, example 258 of the following table was prepared.

| **Ex.** | **Structure** | **Systematic Name** | **Building Blocks** | **MS (ESI): m/z** |
|---|---|---|---|---|
| 258 | 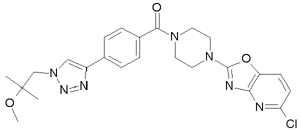 | [4-(5-chlorooxazolo [4,5 - b]pyridin-2-yl)piperazin-1-yl]-[4-[1-(2-methoxy-2-methylpropyl)triazol-4-yl]phenyl]methano ne | C.50 | 496.2 [M+H]⁺ |
| | | | and | |
| | | | CAS 1020743-95-6 | |

### Example 260

### 2-(4-(4-(5-Neopentyl-1,2,4-oxadiazol-3-yl)benzoyl)piperazin-1-yl)oxazolo [4,5-b]pyridine-5-carbonitrile

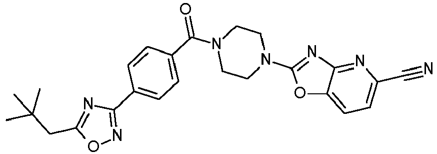

To a solution of (4-(5-iodooxazolo[4,5-b]pyridin-2-yl)piperazin-1-yl)(4-(5-neopentyl-1,2,4-oxadiazol-3-yl)phenyl)methanone (100 mg, 175 µmol) (example 259) in dry DMF (1 mL) was added dicyanozinc (20.5 mg, 175 µmol) and Pd(PPh₃)₄ (10.1 mg, 8.74 µmol), the reaction mixture was then stirred at 100 °C for 16 h. Volatiles were removed in vacuo. The crude product was purified by silica column flash chomatography with an eluent mixture of EtOAc and heptane (10% - 90% gradient) providing the title compound (57 mg, 69.2%). MS (ESI): m/z = 472.3 [M+H]⁺.

### Example 261

### (4-(5-Neopentyl-1,2,4-oxadiazol-3-yl)phenyl)(4-(5-(trifluoromethyl)oxazolo[4,5-b]pyridin-2-yl)piperazin-1-yl)methanone

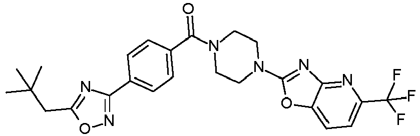

To a solution of (4-(5-iodooxazolo[4,5-b]pyridin-2-yl)piperazin-1-yl)(4-(5-neopentyl-1,2,4-oxadiazol-3-yl)phenyl)methanone (180 mg, 314 µmol) (example 259) in dry DMF (1 mL) under an inert atmosphere was added (1,10-Phenanthroline)(trifluoromethyl)copper(I) (148 mg, 472 µmol). The reaction was then stirred at 60 °C for 42 hours. Insolubles were removed by filtration over a pad of Celite, the filtrate was concentrated in vacuo to dryness. The crude product was purified by RP chromatography providing the title compound (17.2 mg, 10.6%). MS (ESI): m/z = 515.3 [M+H]⁺.

### Example 262

### [4-(5-Chlorooxazolo[4,5-b]pyridin-2-yl)piperazin-1-yl]-[4-[5-(2,2-difluoropropyl)-1,2,4-oxadiazol-3-yl]phenyl]methanone

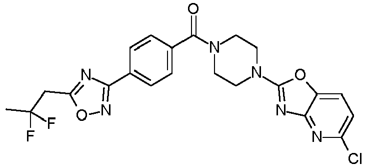

To a mixture of 1-[3-[4-[4-(5-chlorooxazolo[4,5-b]pyridin-2-yl)piperazine-1-carbonyl]phenyl]-1,2,4-oxadiazol-5-yl]propan-2-one (100 mg, 0.210 mmol) (example 23) in DCM (15 mL) was added (diethylamino)sulfur trifluoride (69.1 mg, 0.430 mmol) at 20 °C. Then the mixture was stirred at 20 °C for 15 h. To the mixture was added 15 mL water and extracted with DCM. Combined the organic layer to dry over anhydrous sodium sulfate, filter and concentrate in vacuo. The crude product was purified by prep-HPLC to provide the title compound (11.8 mg, 0.020 mmol, 10.89%) as a white solid. MS (ESI): m/z = 489.2 [M+H]⁺.

### Example 263

### (2-Butyl-1,3-benzoxazol-6-yl)-[4-(5-methyloxazolo[4,5-b]pyridin-2-yl)piperazin-1-yl]methanone

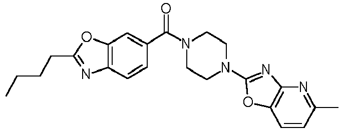

A solution of [2-[(E)-but-1-enyl]-1,3-benzoxazol-6-yl]-[4-(5-methyloxazolo[4,5-b]pyridin-2-yl)piperazin-1-yl]methanone (70.0 mg, 0.170 mmol) (example 21) in methanol (7 mL) was added Pd/C (20.0 mg, 0.170 mmol) and the mixture was stirred at 20 °C for 2 h under a hydrogen atmosphere. The mixture was filtered and concentrated under vacuum, purified by Prep-HPLC and reversed phase column (ammonium hydroxide) to provide the title compound (3.8 mg, 0.010 mmol, 4.92 %) as an off-white solid. MS (ESI): m/z = 420.3 [M+H]⁺.

### Example 264

### [4-(5-Chlorooxazolo[4,5-b]pyridin-2-yl)piperazin-1-yl]-[4-[5-(2-fluoro-2-methylpropyl)-1,2,4-oxadiazol-3-yl]phenyl]methanone

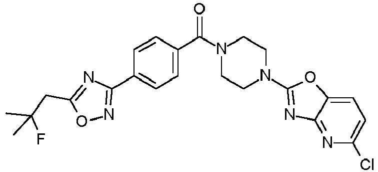

To a mixture of [4-(5-chlorooxazolo[4,5-b]pyridin-2-yl)piperazin-1-yl]-[4-[5-(2-hydroxy-2-methyl-propyl)-1,2,4-oxadiazol-3-yl]phenyl]methanone (100 mg, 0.210 mmol) (example 32) in DCM (20 mL) was added FLUOLEAD^{™} (4-tert-butyl-2,6-dimethylphenylsulfur trifluoride) (55.5 mg, 0.250 mmol) at 20 °C. Then the mixture was stirred at 20 °C for 2 h. The mixture was added to 20 mL water and extracted with DCM. Combined the organic layer to dry over anhydrous sodium sulfate, filter and concentrate in vacuo to dryness. The crude mixture was purified by Prep-HPLC to provide the title compound (12.6 mg, 8.34%) as a colorless oil. MS (ESI): m/z = 485.2 [M+H]⁺.

Two further compounds were isolated as side products of this reaction:

| **Ex.** | **Structure** | **Systematic Name** | **MS (ESI): m/z** |
|---|---|---|---|
| 265 | 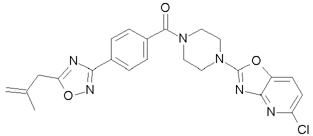 | [4-(5-chlorooxazolo[4,5-b]pyridin-2-yl)piperazin-1-yl]-[4-[5-(2-methylallyl)-1,2,4-oxadiazol-3-yl]phenyl]methanone | 465.2 [M+H]⁺ |
| 266 | 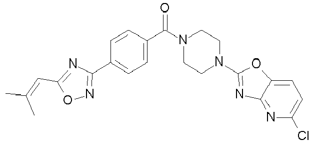 | [4-(5-chlorooxazolo[4,5-b]pyridin-2-yl)piperazin-1-yl]-[4-[5-(2-methylprop-1-enyl)-1,2,4-oxadiazol-3-yl]phenyl]methanone | 465.2 [M+H]⁺ |

### Example 267

### (4-(5-Chlorooxazolo[4,5-b]pyridin-2-yl)piperazin-1-yl)(4-(5-isobutyl-1,3,4-oxadiazol-2-yl)phenyl)methanone

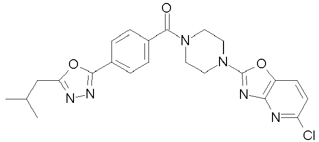

The title compound was isolated by achiral SFC chromatography as a side product from the synthesis of example 30 (39 mg, 6.26%) as an off-white solid. MS (ESI): m/z = 467.2 [M+H]⁺.

Isomer originates from regioisomeric impurity in commercial trimethylacetaldehyde used in synthesis of A.36.

### Example 275

### [4-(5-Chlorooxazolo[4,5-b]pyridin-2-yl)piperazin-1-yl]-[4-(5-isobutyl-1,3,4-oxadiazol-2-yl)-3-methyl-phenyl] methanone

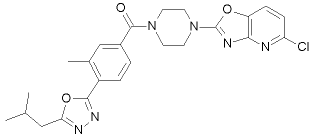

The title compound was isolated by achiral SFC chromatography as a side product from the synthesis of example 83 (58.3 mg, 4.78%) as a white solid. MS (ESI): m/z = 481.2 [M+H]⁺.

Isomer originates from regioisomeric impurity in commercial trimethylacetaldehyde used in synthesis of A.36.

### Example 276

### [4-(5-Chlorooxazolo[4,5-b]pyridin-2-yl)piperazin-1-yl]-[2-chloro-6-[[1-(trifluoromethyl)cyclopropyl]methoxy]-3-pyridyl]methanone

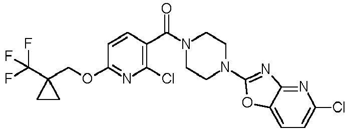

To a solution of (2-chloro-6-hydroxy-3-pyridyl)-[4-(5-chlorooxazolo[4,5-b]pyridin-2-yl)piperazin-1-yl]methanone (100 mg, 0.250 mmol) (C.55) and [1-(trifluoromethyl)cyclopropyl]methanol (42.7 mg, 0.300 mmol) in THF (3.5 mL) was added cyanomethyl tributylphosphorane (122 mg, 0.510 mmol) and the mixture was stirred at 25 °C for 12 h. The mixture was purified by prep-HPLC and lyophilized to provide the title compound (33.3 mg, 0.060 mmol, 25.4%) as a light yellow solid. MS (ESI): m/z = 516.1 [M+H]⁺.

### Example 279

### [4-(5-Chlorooxazolo[4,5-b]pyridin-2-yl)piperazin-1-yl]-[6-[1-(2,2-dimethylpropyl)pyrazol-4-yl]-5-(trifluoromethyl)-3-pyridyl]methanone

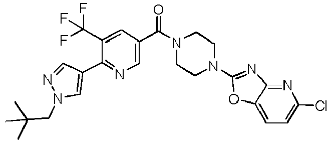

A mixture of [6-bromo-5-(trifluoromethyl)-3-pyridyl]-[4-(5-chlorooxazolo[4,5-b]pyridin-2-yl)piperazin-1-yl]methanone (150 mg, 0.310 mmol), 1-(2,2-dimethylpropyl)-4-(4,4,5,5-tetramethyl-1,3,2-dioxaborolan-2-yl)pyrazole (96.9 mg, 0.370 mmol) (CAS-RN: 1430751-26-0), K₂CO₃ (105 mg, 0.760 mmol), Pd(PPh₃)₄ (35.3 mg, 0.030 mmol) in 1,4-dioxane (7.5 mL) was stirred at 60 °C for 12 h. The mixture was filtered, purified by Prep-HPLC (0.225% v/v FA) for twice to give the desired product (43.9 mg, 0.080 mmol, 95.76% purity, 25.1% yield) as off-white solid. MS (ESI): m/z = 548.2 [M+H]⁺.

### Step a) [6-Bromo-5-(trifluoromethyl)-3-pyridyl]-[4-(5-chlorooxazolo[4,5-b]pyridin-2-yl)piperazin-1-yl]methanone

A mixture of 6-bromo-5-(trifluoromethyl)pyridine-3-carboxylic acid (200 mg, 0.740 mmol) (Example 280, Step b), 5-chloro-2-piperazin-1-yl-oxazolo[4,5-b]pyridine (177 mg, 0.740 mmol) and T₃P (707 mg, 50 wt.% in EtOAc, 1.11 mmol) in DMF (5 mL) was added DIEA (287 mg, 2.22 mmol). The mixture was stirred at 20 °C for 12 h. The mixture was poured into water and extracted three times with EtOAc. The combined organic layer was washed with brine, dried over Na₂SO₄ and filtered. The filtrate was concentrated under vacuum, purified by Prep-HPLC (0.225% v/v FA) and concentrated under vacuum to give the desired product (220 mg, 0.450 mmol, 60.53% yield) as light yellow solid. MS (ESI): m/z = 492.1 [M+H]⁺.

### Example 282

### [6-[1-(2,2-Dimethylpropyl)pyrazol-4-yl]-5-(trifluoromethyl)-3-pyridyl]-[4-(5-methyloxazolo[4,5-b]pyridin-2-yl)piperazin-1-yl]methanone

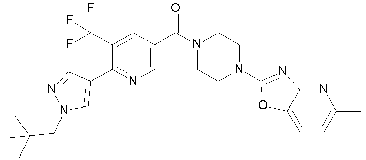

A mixture of [6-bromo-5-(trifluoromethyl)-3-pyridyl]-[4-(5-methyloxazolo[4,5-b]pyridin-2-yl)piperazin-1-yl]methanone (150 mg, 0.320 mmol), 1-(2,2-dimethylpropyl)-4-(4,4,5,5-tetramethyl-1,3,2-dioxaborolan-2-yl)pyrazole (101 mg, 0.380 mmol), potassium carbonate (110 mg, 0.800 mmol), Pd(PPh₃)₄ (36.8 mg, 0.030 mmol) in DMF (8 mL) was stirred at 60 °C for 12 h. The mixture was filtered and the filtrate was purified by Prep-HPLC (0.225% v/v FA) to give the desired product (30 mg, 0.060 mmol, 17.6% yield, 98.6% purity) as off-white solid. MS (ESI): m/z = 528.3 [M+H]⁺.

### Step a) 6-Bromo-5-(trifluoromethyl)pyridine-3-carboxylic acid

To a solution of 2,5-dibromo-3-(trifluoromethyl)pyridine (4400 mg, 14.4 mmol, CAS RN 79623-39-5) in THF (50 mL) was added i-PrMgCl-LiCl/toluene (12.1 mL, 1.3 M) at -20 °C. The mixture was stirred at 25 °C for 30 mins, and then cooled to -78 °C. To the above mixture was added dry carbon dioxide (1905 mg, 43.3 mmol) at -78 °C. The mixture was stirred at 25 °C for 10 h. The mixture was poured into water and adjusted to pH = 4 with 1 N HCl. The mixture was extracted three times with EtOAc. The combined organic layer was washed with brine, dried over Na₂SO₄, filtered and concentrated under vacuum. The residue was diluted with PE/EtOAc (3:1, 20 mL) and water (30 mL). The mixture was adjusted to pH=10 with 1 N NaOH and extracted with PE/EtOAc (10:1, 20 mL three times). The water layer was adjusted to pH=4 with 1 N HCl and extracted three times with EtOAc. The combined organic layer was washed with brine, dried over Na₂SO₄ and filtered. The filtrate was concentrated under vacuum to give desired product (900 mg, 3.33 mmol, 23.1% yield) as light yellow solid.

### Step b) [6-Bromo-5-(trifluoromethyl)-3-pyridyl]-[4-(5-methyloxazolo[4,5-b]pyridin-2-yl)piperazin-1-yl]methanone

A mixture of 6-bromo-5-(trifluoromethyl)pyridine-3-carboxylic acid (200.0 mg, 0.740 mmol), 5-methyl-2-piperazin-1-yl-oxazolo[4,5-b]pyridine (161.67 mg, 0.740 mmol), DIEA (286.7 mg, 2.22 mmol) and T₃P (707.07 mg, 1.11 mmol, 50 wt.% in EtOAc) in DMF (5 mL) was stirred at 20 °C for 12 h. The mixture was poured into water and extracted three times with EtOAc. The combined organic layer was washed with brine, dried over Na₂SO₄ and filtered. The filtrate was concentrated under vacuum. The residue purified by Prep-HPLC (0.225% v/v FA) and concentrated under vacuum to give desired product (190 mg, 0.400 mmol, 54.55% yield) as light yellow gum. MS (ESI): m/z = 472.1 [M+H]⁺.

### Example 296

### [2-Chloro-6-[[1-(trifluoromethyl)cyclopropyl]methoxy]-3-pyridyl]-[4-(5-methyloxazolo[4,5-b]pyridin-2-yl)piperazin-1-yl]methanone

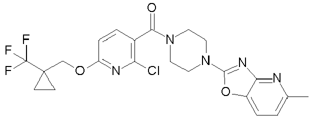

Prepared in analogy to Example 276, using (2-chloro-6-hydroxy-3-pyridyl)-[4-(5-methyloxazolo[4,5-b]pyridin-2-yl)piperazin-1-yl]methanone (C.56). MS (ESI): m/z = 496.2 [M+H]⁺.

### Example 305

### [2-(3-Chlorophenyl)sulfonyl-2,6-diazaspiro[3.3]heptan-6-yl]-[4-(5-methyloxazolo[4,5-b]pyridin-2-yl)piperazin-1-yl]methanone

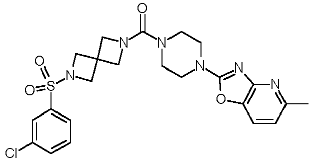

To a solution of 2-(3-chlorophenyl)sulfonyl-2,6-diazaspiro[3.3]heptane (100 mg, 0.370 mmol) (A.72) and triethylamine (0.15 mL, 1.1 mmol) in DCM (2 mL) was added 4-(5-methyloxazolo[4,5-b]pyridin-2-yl)piperazine-1-carbonyl chloride (123.5 mg, 0.440 mmol) (B.8) at 0 °C and the mixture was stirred at 20 °C for 2 h. The mixture was washed with water (2 mL), the organic phase was concentrated and purified by prep-HPLC and lyophilized to provide the title compound (24.3 mg, 0.050 mmol, 12.4%) as a white solid. MS (ESI): m/z = 517.1 [M+H]⁺.

### Synthesis of Building Blocks

### Example A.1

### 2-(2-azaspiro[3.4]octan-2-yl)benzo[d]oxazole-6-carboxylic acid

To a suspension of methyl 2-(2-azaspiro[3.4]octan-2-yl)benzo[d]oxazole-6-carboxylate (121 mg, 423 µmol) in dioxane (1 mL) and water (1 mL) was added lithium hydroxide monohydrate (26.6 mg, 634 µmol) and the suspension was stirred overnight at r.t. to give a clear, colorless solution. The dioxane was evaporated and the residue was diluted with water (approx. 2 mL). To the solution was added dropwise HCl (1M in water, 634 µL, 634 µmol) and the resulting suspension was filtered. The filter cake was washed with water and dried at HV to get the title compound (0.104 g, 90.4%) as a white solid. MS (ESI): m/z = 273.3 [M+H]⁺

### Step a) methyl 2-(2-azaspiro[3.4]octan-2-yl)benzo[d]oxazole-6-carboxylate

A solution of methyl 2-(methylthio)benzo[d]oxazole-6-carboxylate (100 mg, 448 µmol) and 2-azaspiro[3.4]octane (49.8 mg, 448 µmol) in THF (1.5 mL) was stirred at 70 °C for 21 hours. Another batch of 2-azaspiro[3.4]octane (24.9 mg, 224 µmol) was added and stirring was continued at 70 °C for total of 48 hours. The mixture was taken up in water and ethyl acetate and the layers were separated. The aqueous layer was extracted once with ethyl acetate. The organic layers were dried over MgSO₄, filtered and evaporated to get the title compound (0.121 g, 94.3%) as a light brown solid. MS (ESI): m/z = 287.3 [M+H]⁺

### Example A.2

### 4-(5-(1-hydroxy-2-methylpropan-2-yl)-1,2,4-oxadiazol-3-yl)benzoic acid

In analogy to the procedure described for example A.1 (hydrolysis step), the title compound was prepared from methyl 4-(5-(1-hydroxy-2-methylpropan-2-yl)-1,2,4-oxadiazol-3-yl)benzoate (126 mg, 456 µmol) as a white solid (99 mg, 82.8%). MS (ESI): m/z = 263.2 [M+H]⁺

### Step a) methyl 4-(5-(1-hydroxy-2-methylpropan-2-yl)-1,2,4-oxadiazol-3-yl)benzoate

To a solution of 3-hydroxy-2,2-dimethylpropanoic acid (125 mg, 1.06 mmol) in DMF (5 mL) were added HOBT.H₂O (162 mg, 1.06 mmol) and EDC (406 mg, 2.12 mmol) and the suspension was stirred at r.t. for 15 minutes to give a clear and colorless solution. To this was added methyl (Z)-4-(N'-hydroxycarbamimidoyl)benzoate (205 mg, 1.06 mmol) and stirring was continued at r.t. for 15 minutes followed by stirring at 90 °C for 3 days. The reaction mixture was poured on water and ethyl acetate and the layers were separated. The aqueous layer was extracted twice with ethyl acetate. The organic layers were washed once with water, dried over MgSO4, filtered and evaporated to dryness. The crude material was purified by flash chromatography with an eluent mixture of ethyl acetate and heptane (0% to 50%) providing the title compound (0.128 g, 43.8%) as a white solid. MS (ESI): m/z = 277.2 [M+H]⁺

### Example A.3

### 4-(5-(1-fluoro-2-methylpropan-2-yl)-1,2,4-oxadiazol-3-yl)benzoic acid

In analogy to the procedure described for example A.1 (hydrolysis step), the title compound was prepared from methyl 4-(5-(1-fluoro-2-methylpropan-2-yl)-1,2,4-oxadiazol-3-yl)benzoate (103 mg, 370 µmol) as a white solid (92 mg, 84.7%). MS (ESI): m/z = 265.2 [M+H]⁺

### Step a) methyl 4-(5-(1-fluoro-2-methylpropan-2-yl)-1,2,4-oxadiazol-3-yl)benzoate

To a solution of 3-fluoro-2,2-dimethylpropanoic acid (47.6 mg, 397 µmol) in dry DMF (1.8 mL) was added 1,1'-Carbonyldiimidazole (64.3 mg, 397 µmol) and the reaction mixture was stirred for 15 min. Then, methyl (Z)-4-(N'-hydroxycarbamimidoyl)benzoate (100 mg, 360 µmol) was added in one portion. The reaction mixture was heated to 90 °C for 16 hours. The reaction was allowed to reach r.t. and partitioned between ethyl acetate and water, the organic phase was collected and the aqueous phase was back-extracted with ethyl acetate. The combined organic phases were washed with brine, dried over sodium sulfate and evaporated down to dryness. The crude material was purified by flash chromatography with an eluent mixture of ethyl acetate and heptane (0% to 25%) providing the title compound (0.103 g, 77%) as a colorless oil. MS (ESI): m/z = 279.2 [M+H]⁺

### Example A.4

### 4-[1-(2,2-Dimethylpropyl)pyrazol-4-yl]benzoic acid

A solution of methyl 4-[1-(2,2-dimethylpropyl)pyrazol-4-yl]benzoate (940 mg, 3.45 mmol) and NaOH (6.9 mL, 6.9 mmol, 1 M aqueous solution) in methanol (10 mL) was stirred at 20 °C for 15 h. The reaction mixture was concentrated under reduced pressure to give a crude product and 1 M HCl (10 mL) aqueous solution was added dropwise into the reaction mixture until pH = 3. The reaction mixture was filtered and the filter cake was washed with 10 mL water, dried in vacuum to give the title compound (850 mg, 95.3%) as a white solid. MS (ESI): m/z = 259.2 [M+H]⁺

### Step a) 4-(4-Bromophenyl)-1-neopentyl-1H-pyrazole

A solution of 4-(4-bromophenyl)-1H-pyrazole (3.00 g, 13.5 mmol), 1-bromo-2,2-dimethylpropane (3.05 g, 20.17 mmol) and potassium carbonate (3.72 g, 26.9 mmol) in DMF (30 mL) was stirred at 80 °C for 15 h. The mixture was diluted with water and extracted with EtOAc three times. The combined organic phase was washed with brine, dried over Na₂SO₄, filtered and concentrated under reduced pressure to give a residue. The residue was purified by silica gel chromatography providing the title compound (1.34 g, 34.0%) as a light yellow solid. MS (ESI): m/z = 293.0, 295.0 [M+H]⁺

### Step b) Methyl 4-[1-(2,2-dimethylpropyl)pyrazol-4-yl]benzoate

To a mixture of 4-(4-bromophenyl)-1-(2,2-dimethylpropyl)pyrazole (1.34 g, 4.57 mmol) and TEA (1.27 mL, 9.14 mmol) in DMF (20 mL) and methanol (20 mL) were added Pd(dppf)Cl₂ (167.2 mg, 0.230 mmol) at 20 °C. Then the mixture was purged with CO (50 psi) and stirred at 80 °C for 15 h. The mixture was concentrated in vacuo. To the residue was added water and extracted with EtOAc three times. Combined the organic layer to dry over anhydrous sodium sulfate, filtered and concentrated in vacuo. The residue was purified by silica gel chromatography providing the title compound (940 mg, 75.5%) as a light yellow solid. MS (ESI): m/z = 273.3 [M+H]⁺

Examples A.10 and A.11 were prepared in analogy to A.4

| **Example** | **Systematic Name** | **Starting material** | **Base** | **MS (ESI): m/z** |
|---|---|---|---|---|
| A. 10 | 4-[1-(2,2-Dimethylpropyl)-1,2,4-triazol-3-yl]benzoic acid | 3-(4-bromophenyl)-1H-1,2,4-triazole | NaH | 260.3 [M+H]⁺ |
| A.11 | 4-[1-(2,2-Dimethylpropyl)imidazol-4-yl]benzoic acid | 4-(4-bromophenyl)-1H-imidazole | NaH | 259.2 [M+H]⁺ |

### Example A.5

### 2-[(E)-But-1-enyl]-1,3-benzoxazole-6-carboxylic acid

To a solution of methyl 4-[(2-cyclopropylacetyl) amino]-3-hydroxy-benzoate (1.0 g, 4.01 mmol) and polyphosphoric acid (0.96 g, 4.01 mmol) was stirred at 180 °C for 2 h. The mixture was poured into ice-cooled concentrated ammonia solution and extracted with ethyl acetate three times. The combined organic layer was washed with brine, dried over sodium sulfate, filtered and concentrated. The residue was purified by silica gel chromatography with an eluent mixture of ethyl acetate and petroleum ether (0% - 30%) to give the title compound (276 mg, 1.27 mmol, 27.5%) as a yellow solid. MS (ESI): m/z = 218.1 [M+H]⁺

### Step a) Methyl 4-[(2-cyclopropylacetyl)amino]-3-hydroxy-benzoate

To a solution of methyl 4-amino-3-hydroxybenzoate (2.0 g, 12.0 mmol) and cyclopropylacetic acid (1.56 mL, 14.4 mmol), EDCI (2.79 g, 18.0 mmol), HOBt (2.75 g, 18.0 mmol) in DCM (40 mL) was added triethylamine (5.0 mL, 35.9 mmol), the mixture was stirred at 20 °C for 15 h. The mixture was diluted with water and extracted with DCM three times. The combined organic phase was washed with brine, dried over Na₂SO₄ and concentrated. The residue was washed with methyl tert-butyl ether and dried to give the title compound (1.94 g, 7.80 mmol, 61.4%) as a yellow solid. MS (ESI): m/z = 250.1 [M+H]⁺

### Example A.6

### 2-Cyclopropyl-1,3-benzoxazole-6-carboxylic acid

In analogy to the procedure described for example A.5 the title compound was prepared from methyl 4-amino-3-hydroxybenzoate (2.00 g, 12.0 mmol) and cyclopropanecarboxylic acid (1.34 mL, 14.4 mmol), as a yellow solid (263 mg, 1.29 mmol, 27.5%). MS (ESI): m/z = 204.0 [M+H]⁺

### Example A.7

### 4-(5-Acetonyl-1,2,4-oxadiazol-3-yl)benzoic acid

In analogy to the procedure described for Example A.4 (Step b onwards) the title compound was prepared from 1-[3-(4-Bromophenyl)-1,2,4-oxadiazol-5-yl]propan-2-one (CAS: 1204296-19-4) (6.00 g, 21.3 mmol), as a white solid (1.00 g, 64.1%). MS (ESI): m/z = 247.1 [M+H]⁺

### Example A. 8

### 4-[5-(1-Fluoro-1-methyl-ethyl)-1,2,4-oxadiazol-3-yl]benzoic acid

In analogy to the procedure described for example A.4 (Step b onwards) the title compound was prepared from 3-(4-Bromophenyl)-5-(1-fluoro-1-methyl-ethyl)-1,2,4-oxadiazole (200 mg, 0.7 mmol), as a white solid (70 mg, 73.92%). MS (ESI): m/z = 251.0 [M+H]⁺

### Step a) 3-(4-Bromophenyl)-5-(1-fluoro-1-methyl-ethyl)-1,2,4-oxadiazole

A solution of 4-bromo-N-hydroxy-benzamidine (0.8 g, 3.72 mmol), 2-fluoroisobutyric acid (395 mg, 3.72 mmol) and dicyclohexylcarbodiimide (1.15 g, 5.58 mmol) in DMF (10 mL) was stirred at 110 °C for 15 h. The mixture was diluted with water and extracted with EtOAc three times. The combined organic phase was washed with brine, dried over sodium sulfate, filtered and concentrated under reduced pressure to give a residue. The residue was purified by silica gel chromatography providing the title compound (640 mg, 60.3%) as a colorless liquid. MS (ESI): m/z = 284.9, 286.9 [M+H]⁺

Examples A.9, A.12, A.16, A.17 and A.23 were prepared in analogy to A.8.

| **Example** | **Systematic Name** | **Acid** | **MS (ESI): m/z** |
|---|---|---|---|
| A.9 | 4-[5-(1-Fluorocyclopropyl)-1,2,4-oxadiazol-3-yl]benzoic acid | 1-fluorocyclopropanecarboxylic acid, CAS: 137081-41-5 | 249.2 [M+H]⁺ |
| A.12 | 4-[5-(2-Hydroxy-2-methyl-propyl)-1,2,4-oxadiazol-3-yl]benzoic acid | beta-hydroxyisovaleric acid, CAS: 625-08-1 | 261.1 [M-H]⁻ |
| A.16 | 4-[5-[1-(Fluoromethyl)cyclopropyl ]-1,2,4-oxadiazol-3-yl]benzoic acid | 1-(Fluoromethyl)cyclopropaneca rboxylic acid, CAS: 1273565-75-5 | 263.0 [M+H]⁺ |
| A.17 | 4-[5-[(1-Methylcyclopropyl)methyl ]-1,2,4-oxadiazol-3-yl]benzoic acid | 2-(1-methylcyclopropyl)acetic acid, CAS: 71199-15-0 | 257.1 [M-H]⁻ |
| A.23 | 4-[5-(1-Methylcyclopropyl)-1,2,4-oxadiazol-3-yl]benzoic acid | 1-methylcyclopropane-1-carboxylic acid, CAS: 6914-76-7 | 245.1 [M+H]⁺ |

### Example A. 49

### 4-[5-(3-bicyclo[1.1.1]pentanyl)-1,2,4-oxadiazol-3-yl]benzoic acid

In analogy to the procedure described for example A.4 (Step b onwards) the title compound was prepared from 5-(3-bicyclo[1.1.1]pentanyl)-3-(4-bromophenyl)-1,2,4-oxadiazole (400 mg, 1.37 mmol), as a white solid (127 mg, 77.4%). MS (ESI): m/z = 257.1 [M+H]⁺

### Step a) 5-(3-bicyclo[1.1.1]pentanyl)-3-(4-bromophenyl)-1,2,4-oxadiazole

To a solution of 4-bromo-N'-hydroxy-benzamidine (780 mg, 3.63 mmol) and triethylamine (1.52 mL, 10.9 mmol) in toluene (8.32 mL) was added bicyclo[1.1.1]pentane-3-carbonyl chloride (568 mg, 4.35 mmol) at 0 °C and the mixture was stirred at 25 °C for 10 min. and at 80 °C for 12 h. The mixture was concentrated, diluted with water (50 mL), the water phase washed with EtOAc, then the organic phase washed with brine, dried over with Na₂SO₄ and concentrated. The crude product was purified by reversed phase chromatography and lyophilized to give the title compound (400 mg, 1.37 mmol, 36.0%) as a yellow solid. MS (ESI): m/z = 291.0 [M+H]⁺

### Example A. 13

### 4-[5-(2-Fluoro-1,1-dimethyl-ethyl)-1,3,4-oxadiazol-2-yl]benzoic acid

In analogy to the procedure described for example A.4 (Step b onwards) the title compound was prepared from 2-(4-Bromophenyl)-5-(2-fluoro-1,1-dimethyl-ethyl)-1,3,4-oxadiazole (150 mg, crude). MS (ESI): m/z = 263.1 [M+H]⁺

### Step a) 3-Fluoro-2,2-dimethyl-propanoyl chloride

To a solution of 3-fluoro-2,2-dimethyl-propanoic acid (500 mg, 4.16 mmol) in toluene (5 mL), cooled to 0 °C, phosphorus oxychloride (1.0 mL, 11.4 mmol) was added and the mixture was stirred at 80 °C for 2 h. The solvent was removed under reduced pressure and the title compound (0.660 g, crude) was directly used in the next step without any purification.

### Step b) 4-Bromo-N'-(3-fluoro-2,2-dimethyl-propanoyl)benzohydrazide

To a solution of 3-fluoro-2,2-dimethyl-propanoyl chloride (644 mg, 4.65 mmol) and TEA (1.4 g, 14.0 mmol) in DCM (30 mL) was added 4-bromobenzhydrazide (1.0 g, 4.65 mmol) at 0 °C, then the mixture was stirred at 0 °C for 2 h. The solvent was removed by reduced pressure. The residue was purified by silica gel chromatography with an eluent mixture of ethyl acetate and petroleum ether providing the title compound (500.0 mg, 1.58 mmol) as a white solid. MS (ESI): m/z = 317.1, 319.1 [M+H]

### Step c) 2-(4-Bromophenyl)-5-(2-fluoro-1,1-dimethyl-ethyl)-1,3,4-oxadiazole

4-bromo-N'-(3-fluoro-2,2-dimethyl-propanoyl)benzohydrazide (500 mg, 1.58 mmol) was added to phosphorus oxychloride (5.0 mL, 57.1 mmol, 36.2 eq) at 0 °C, the reaction mixture was stirred at 0 °C for 2 h. The reaction mixture was quenched by the addition of the saturated aqueous sodium bicarbonate solution, and extracted with DCM three times. The combined organic phase was washed with saturated brine and concentrated in vacuum to dryness. The residue was purified by silica gel chromatography with en eluent mixture of ethyl acetate and petroleum ether providing the title compound (360 mg, 76.3%) as a yellow oil. MS (ESI): m/z = 299.0, 301.0 [M+H]

### Example A. 14

### 3-Bromo-4-[5-(2,2-dimethylpropyl)-1,2,4-oxadiazol-3-yl]benzoic acid

To a solution of methyl 3-bromo-4-[5-(2,2-dimethylpropyl)-1,2,4-oxadiazol-3-yl]benzoate (15 mg, 0.042 mmol) in THF (1.5 ml), methanol (1.2 ml) and water (0.3 ml) was added LiOH, H₂O (9 mg, 0.21 mmol) at -5 °C and stirred the reaction mixture at 0 °C for 2 h. The reaction mixture was acidified with 0.5 N aq.HCl. The volatiles were removed in vacuo to give the title compound (25 mg, crude mass). MS: m/z = 339.0 [M-H]⁻

### Step a) Methyl 3-bromo-4-[(1E)-(hydroxyimino)methyl]benzoate

Methyl 3-bromo-4-formylbenzoate (300 mg, 1.23 mmol) was taken up in ethanol (6 mL) followed by the addition of triethylamine (0.34 mL, 2.47 mmol), hydroxylammonium chloride (129 mg, 1.85 mmol) and water (3 mL) and the mixture was heated at 70 °C for 1 h. Ethanol was removed in vacuo and the white solid filtered through sintered glass, washed with water and dried in vacuo to give the title compound (270 mg, 85%) which was carried to the next step without further purification.

### Step b) Methyl 3-bromo-4-[(Z)-N'-hydroxycarbamimidoyl]benzoate

A solution ofN-chlorosuccinimide (141 mg, 1.06 mmol) in DMF (1.5 mL) was added slowly to a solution of methyl 3-bromo-4-[(1E)-(hydroxyimino)methyl]benzoate (260 mg, 1.01 mmol) in DMF (3.5 mL) at 50 °C. After complete addition, the reaction mixture was allowed to stir for 30 min. at the same temperature. The reaction mixture was then cooled to 5 °C and ammonium hydroxide (0.1 mL) was added dropwise. During addition the temperature was maintained between 0 - 10 °C. The reaction mixture was allowed to stir for 15 min. at the same temperature. Ethyl acetate was added to the cooled reaction mixture, followed by brine solution and the aqueous layer was extracted with ethyl acetate. The combined organic part was dried, filtered and evaporated under reduced pressure to give the title compound (325 mg, crude) as a brown liquid. MS (ESI): m/z = 273.1 [M+H]⁺

### Step c) Methyl 3-bromo-4-[5-(2,2-dimethylpropyl)-1,2,4-oxadiazol-3-yl]benzoate

To the solution of 3,3-dimethylbutanoic acid (136 mg, 1.17 mmol) in DMF (5 mL) at 25 °C was added CDI (228 mg, 1.41 mmol) and the reaction mixture was stirred at 50 °C for 45 min. Then methyl 3-bromo-4-[(Z)-N'-hydroxycarbamimidoyl]benzoate (320 mg, 1.17 mmol) was added and reaction mixture was heated at 100 °C for 1 h. Reaction mixture was cooled to 25 °C; water was added and the mixture was extracted with EtOAc. The organic layer was washed with brine, dried, filtered and concentrated under reduced pressure to dryness. The residue was purified by silica gel chromatography with an eluent mixture of ethyl acetate and hexane (2% - 5%) providing the title compound (25 mg, 6%) as a colorless gum.

### Example A. 15

### 4-Bromo-3-(1H-imidazol-2-yl)benzoic acid

To a mixture of methyl 4-bromo-3-(1H-imidazol-2-yl)benzoate (640 mg, 2.28 mmol) in THF (15 mL), methanol (12 mL) and water (3 mL) was added LiOH, H₂O (96 mg, 2.28 mmol) and heated for 1 h at 70 °C. Volatiles were removed in vacuo and the residue was acidified with aq. HCl (0.5N). The mixture was concentrated in vacuo to dryness to get the title compound (yield was considered to be quantitative) as a light yellow solid. MS: m/z = 265.0 [M-H]

### Step a) Methyl 4-bromo-3-(1H-imidazol-2-yl)benzoate

To a pre-heated mixture of triethylammonium acetate (1.2 mL) and ammonium acetate (4.44 g, 57.6 mmol) at 50 °C, was added a mixture of methyl 4-bromo-3-formylbenzoate (2.0 g, 8.23 mmol) and oxaldehyde (2.4 mL, 16.5 mmol, 40% aqueous solution), taken in ethanol (10 ml), via syringe pump over 1 h. After the addition was complete the heating was continued for another 30 minutes. The reaction mixture was poured into ice and stirred for 10 minutes and extracted with EtOAc. The combined organic part was washed with brine, dried, filtered and concentrated in vacuo. The residue was purified by amine silica gel chromatography with an eluent mixture of ethyl acetate and hexane (5% to 15%) providing the title compound (1.12 g, 48% g) as a light yellow solid. MS (ESI): m/z = 282.8 [M+H]

### Example A. 18

### 4-(1-tert-Butyl-1,2,4-triazol-3-yl)benzoic acid and 4-(2-tert-butyl-1,2,4-triazol-3-yl)benzoic acid

In analogy to the procedure described for example A.4 (Step b onwards) the title compound was prepared from 5-(4-bromophenyl)-1-tert-butyl-1,2,4-triazole & 3-(4-bromophenyl)-1-tert-butyl-1,2,4-triazole (500 mg), as a white solid (400 mg). MS (ESI): m/z = 244.1 [M-H]⁻

### Step a) 5-(4-Bromophenyl)-1-tert-butyl-1,2,4-triazole and 3-(4-bromophenyl)-1-tert-butyl-1,2,4-triazole

To a mixture of 3-(4-bromophenyl)-1H-1,2,4-triazole (2.0 g, 8.93 mmol) in DMF (30 mL) was added N,N-dimethylformamide di-tert-butyl acetal (9.07 g, 44.63 mmol) at 20 °C. Then the mixture was stirred at 110 °C for 15 h. The mixture was concentrated in vacuo to dryness. The residue was purified by silica gel chromatography with an eluent mixture of ethyl acetate and petroleum ether (20%) providing the title compound (500 mg) as a white solid. MS (ESI): m/z = 280.0, 282.0 [M+H]⁻

### Example A. 19

### 4-[1-(2,2-Dimethylpropyl)triazol-4-yl]benzoic acid

To a mixture of methyl 4-[1-(2,2-dimethylpropyl)triazol-4-yl]benzoate (400 mg, 1.46 mmol) in methanol (20 mL) was added 2 M sodium hydroxide aqueous solution (7.3 mL, 14.6 mmol) at 20 °C. Then the mixture was stirred at 20 °C for 15 h. The mixture was concentrated in vacuo. To the residue was added 1 M aqueous HCl solution until pH = 3 and extracted with ethyl acetate three times. The combined organic layer was dried over anhydrous sodium sulfate, filtered and concentrated in vacuo to get the title compound (350 mg, 91.3%) as a yellow solid. MS (ESI): m/z = 258.1 [M-H]⁻

### Step a) Methyl 4-(1H-triazol-4-yl)benzoate

To a mixture of methyl 4-ethynylbenzoate (5.0 g, 31.2 mmol) and azidotrimethylsilane (6.16 mL, 46.8 mmol) in DMF (27 mL) and methanol (3 mL) was added CuI (297 mg, 1.56 mmol) at 20 °C. Then the mixture was stirred at 100 °C for 15 h. To the mixture was added 50 mL water and extracted with 50 mL ethyl acetate three times. The combined organic layer was washed with 20 mL brine three times, dried over anhydrous sodium sulfate, filtered and concentrated in vacuo to dryness. The crude was purified by flash chromatography with an eluent mixture of ethyl acetate and petroleum ether (40%) providing the title compound (2.00 g, 31.0%) as a white solid. MS (ESI+): m/z = 204.1 [M+H]⁺

### Step b) Methyl 4-[2-(2,2-dimethylpropyl)triazol-4-yl]benzoate and methyl 4-[1-(2,2-dimethylpropyl)triazol-4-yl]benzoate

To a mixture of methyl 4-(1H-triazol-4-yl)benzoate (2.0 g, 9.84 mmol) and potassium carbonate (2.72 g, 19.7 mmol) in DMF (20 mL) was added 1-bromo-2,2-dimethylpropane (2.23 g, 14.8 mmol) at 20 °C. Then the mixture was stirred at 80 °C for 15 h. To the mixture was added 30 mL water and the mixture was extracted with 30 mL ethyl acetate three times. Combined organic layer was dried over anhydrous sodium sulfate, filtered and concentrated in vacuo. The residue was purified by flash eluting with an eluent mixture of ethyl acetate and petroleum ether (10% - 50%) providing the title compound methyl 4-[2-(2,2-dimethylpropyl)triazol-4-yl]benzoate (1.00 g, 36.4%) as a white solid. MS (ESI): m/z = 274.1 [M+H]⁺

As byproduct was isolated the title compound methyl 4-[1-(2,2-dimethylpropyl)triazol-4-yl]benzoate (400 mg, 13.8%) as a yellow solid. MS (ESI): m/z = 274.1 [M+H]⁺

### Example A.20

### 4-[2-(2,2-Dimethylpropyl)triazol-4-yl]benzoic acid

In analogy to the procedure described for example A.19 the title compound was prepared from methyl 4-[2-(2,2-dimethylpropyl)triazol-4-yl]benzoate (1.00 g, A. 19, Step b), as a white solid (900 mg, 94.8%). MS (ESI): m/z = 258.2 [M-H]⁻

### Example A.37

### 3-Fluoro-4-(1-neopentyl-1H-1,2,3-triazol-4-yl)benzoic acid

In analogy to the procedure described for example A.19 the title compound was prepared from methyl 4-ethynyl-3-fluorobenzoate (0.1 g, 0.561 mmol), as a white solid (21.4 mg, 74.7%). MS (ESI): m/z = 278.2 [M+H]⁺

### Example A.38

### 3-Fluoro-4-(2-neopentyl-2H-1,2,3-triazol-4-yl)benzoic acid

In analogy to the procedure described for example A.19 (step 3) the title compound was prepared from methyl 3-fluoro-4-(2-neopentyl-2H-1,2,3-triazol-4-yl)benzoate (1 g, A.37), as a white solid (45.5 mg, 74.1%). MS (ESI): m/z = 278.2 [M+H]⁺

### Example A.64

### 3-Fluoro-4-(1-neopentyl-1H-1,2,3-triazol-4-yl)benzoic acid

In analogy to the procedure described for example A.19 the title compound was prepared from methyl 4-(1H-triazol-4-yl)benzoate (A.19, Step a) (1.00 g, 4.9 mmol) and (2-cyano-2-methyl-propyl) trifluoromethanesulfonate (2.28 g, 9.8 mmol), as an off-white solid (20 mg, 70.1%). MS (ESI): m/z = 271.1 [M+H]⁺

### Example A.65

### 3-Fluoro-4-(2-neopentyl-2H-1,2,3-triazol-4-yl)benzoic acid

In analogy to the procedure described for example A.19 the title compound was prepared from methyl 4-[2-(2-cyano-2-methyl-propyl)triazol-4-yl]benzoate (85 mg, A.64), as an off-white solid (65 mg, 80.4%). MS (ESI): m/z = 271.1 [M+H]⁺

### Example A.39

### 4-(1-(tert-Butyl)-1H-1,2,3-triazol-4-yl)benzoic acid

In analogy to the procedure described for example A.19 the title compound was prepared from methyl 4-(1-(tert-butyl)-1H-1,2,3-triazol-4-yl)benzoate (110 mg, 0.424 mmol), (99 mg, 95.1%). MS (ESI): m/z = 246.2 [M-H]⁺

### Step a) Methyl 4-(1-(tert-butyl)-1H-1,2,3-triazol-4-yl)benzoate

To a solution of 2-methylpropan-2-amine (200 mg, 290 µL, 2.73 mmol) in dry ACN (9 mL) was added DMAP (401 mg, 3.28 mmol) and 2-azido-1,3-dimethylimidazolinium hexafluorophosphate (936 mg, 3.28 mmol), the reaction mixture was stirred at 30 °C for 1h. Methyl 4-ethynylbenzoate (657 mg, 4.1 mmol) and copper (II) sulfate pentahydrate (68.3 mg, 273 µmol) was added and the reaction mixture was then stirred at r.t for 2 hours. The reaction mixture was diluted with ethyl acetate and extracted with aq. sol. NH₃ 4 M, the organic phase was collected and the aqueous phase was back-extracted with ethyl acetate. The combined organic phases were dried over sodium sulfate and evaporated down to dryness. The residue was purified by silica gel chromatography with an eluent mixture of ethyl acetate and heptane (5% to 30%) providing the title compound (112 mg, 15.8%) as a white solid. MS (ESI): m/z = 260.3 [M+H]⁺

Examples A.43, A.45, A.46, A.51, A.52 and A.54 were prepared in analogy to A.39

| **Ex.** | **Systematic Name** | **Starting material 1** | **Starting material 2** | **MS (ESI): m/z** |
|---|---|---|---|---|
| A.43 | 4-(1-(bicyclo[1.1.1]pentan-1-yl)-1H-1,2,3-triazol-4-yl)benzoic acid | methyl 4-ethynylbenzoat e | bicyclo[1.1.1]pent an-1-amine hydrochloride | 256.2 [M+H]⁺ |
| | | | CAS: 22287-35-0 | |
| A.45 | 4-(1-(2,2,2-trifluoroethyl)-1H-1,2,3-triazol-4-yl)benzoic acid | methyl 4-ethynylbenzoat e | 2,2,2-trifluoroethan-1-amine | 272.2 [M+H]⁺ |
| | | | CAS: 753-90-2 | |
| A.46 | 4-(1-((3-methyloxetan-3-yl)methyl)-1H-1,2,3-triazol-4-yl)benzoic acid | methyl 4-ethynylbenzoat e | (3 -methyloxetan-3-yl)methanamine | 274.2 [M+H]⁺ |
| | | | CAS: 153209-97-3 | |
| A.51 | 4-(1-(1-(trifluoromethyl)cyclopropyl) -1H-1,2,3 -triazol-4-yl)benzoic acid | methyl 4-ethynylbenzoat e | 1-(trifluoromethyl)c yclopropan-1-amine | 298.2 [M+H]⁺ |
| | | | CAS: 112738-68-8 | |
| A.52 | 4-(1-(3-methyloxetan-3-yl)-1H-1,2,3-triazol-4-yl)benzoic acid | methyl 4-ethynylbenzoat e | 3-methyloxetan-3-amine hydrochloride | 260.2 [M+H]⁺ |
| | | | CAS: 1363404-87-8 | |
| A.54 | 4-(1-(oxetan-3-ylmethyl)-1H-1,2,3-triazol-4-yl)benzoic acid | methyl 4-ethynylbenzoat e | oxetan-3-ylmethanamine | 260.2 [M+H]⁺ |
| | | | CAS: 6246-05-5 | |

### Example A. 60

### 4-(1-((1-Fluorocyclopropyl)methyl)-1H-1,2,3-triazol-4-yl)benzoic acid

To a solution of tert-butyl 4-(1-((1-fluorocyclopropyl)methyl)-1H-1,2,3-triazol-4-yl)benzoate (157 mg, 496 µmol) in DCM (2.48 mL) was added 2,2,2-trifluoroacetic acid (452 mg, 306 µL, 3.97 mmol) and the reaction was stirred at r.t. for 3 days. The reaction mixture was concentrated to provide the crude title compound (178 mg, 131%) as a light brown solid. MS (ESI): m/z = 262.2 [M+H]⁺

### Step a) tert-Butyl 4-(1-((1-hydroxycyclopropyl)methyl)-1H-1,2,3-triazol-4-yl)benzoate

In analogy to the procedure described for example A.39 the title compound was prepared from 1-(aminomethyl)cyclopropan-1-ol (384 mg, 4.41 mmol) and tert-butyl 4-ethynylbenzoate (1.07g, 5.29 mmol), (809 mg, 55.3%). MS (ESI): m/z = 316.3 [M+H]⁺

### Step b) tert-Butyl 4-(1-((1-fluorocyclopropyl)methyl)-1H-1,2,3-triazol-4yl)benzoate

To a solution of triethylamine trihydrofluoride (51.1 mg, 51.6 µL, 317 µmol) in DCM (317 µL) under argon at 0 °C was added XtalFluor-E (54.5 mg, 238 µmol) followed by tert-butyl 4-(1-((1-hydroxycyclopropyl)methyl)-1H-1,2,3-triazol-4-yl)benzoate (50 mg, 159 µmol) and the mixture was stirred at 0 °C for 50 min. and r.t. for 22 h. The reaction was diluted with DCM and quenched with aq. sat. NaHCO₃ sol. The aqueous was extracted with DCM. The combined layers were washed with brine, dried over Na₂SO₄ and concentrated to give a brown oil. The residue was purified by column chromatography with an eluent mixture of ethyl acetate and heptane (0% to 40%) providing the title compound (157.4 mg, 29.7%) as a white solid. MS (ESI): m/z = 318.3 [M+H]⁺

### Example A.67

### 4-(1-(2-Fluoro-2-methylpropyl)-1H-1,2,3-triazol-4-yl)benzoic acid

In analogy to the procedure described for example A.19, the title compound was prepared from methyl 4-[1-(2-fluoro-2-methyl-propyl)triazol-4-yl]benzoate (200 mg, 0.721 mmol), (188 mg, 99%). MS (ESI): m/z = 264.2 [M+H]⁺

### Step a) Methyl 4-[1-(2-hydroxy-2-methyl-propyl)triazol-4-yl]benzoate

In analogy to the procedure described for example A.39 the title compound was prepared from methyl 4-ethynylbenzoate (647 mg, 4.04 mmol) and 1-amino-2-methylpropan-2-ol (300 mg, 3.37 mmol), as a light orange solid (657 mg, 70.9 %). MS (ESI): m/z = 276.2 [M+H]⁺

### Step b) Methyl 4-[1-(2-fluoro-2-methyl-propyl)triazol-4-yl]benzoate

To a solution of (diethylamino)difluorosulfonium tetrafluoroborate (817 mg, 3.57 mmol) in dry DCM (6 mL) cooled down to 0 °C was added triethylamine trihydrofluoride (767 mg, 4.76 mmol), followed by addition of methyl 4-(1-(2-hydroxy-2-methylpropyl)-1H-1,2,3-triazol-4-yl)benzoate (655 mg, 2.38 mmol). The reaction mixture was stirred at r.t. for 1 h and then at r.t. for 14 h. The reaction mixture was diluted with dichloromethane and extracted with aq. Na₂CO₃ 2M. The organic phase was collected and the aqueous phase was back-extracted with dichloromethane. The combined organic phases were dried over sodium sulfate and evaporated down to dryness. The crude material was purified by silica flash chromatography with an eluent mixture of ethyl acetate and heptane (5% - 75%) providing the title compound (618 mg, 93.7%). MS (ESI): m/z = 278.2 [M+H]⁺

### Example A. 68

### 4-(1-(1-Fluoro-2-methylpropan-2-yl)-1H-1,2,3-triazol-4-yl)benzoic acid

In analogy to the procedure described for example A. 19 the title compound was prepared from methyl 4-(1-(1-fluoro-2-methylpropan-2-yl)-1H-1,2,3-triazol-4-yl)benzoate (245 mg, 0.884 mmol), (241 mg, 104%). MS (ESI): m/z = 264.2 [M+H]⁺

### Step a) Methyl 4-(1-(1-hydroxy-2-methylpropan-2-yl)-1H-1,2,3-triazol-4-yl)benzoate

In analogy to the procedure described for example A.39 (Step a) the title compound was prepared from methyl 4-ethynylbenzoate (647 mg, 4.04 mmol) and 2-amino-2-methylpropan-1-ol (300 mg, 3.37 mmol), as a light yellow powder (722 mg, 77.9%). MS (ESI): m/z = 276.2 [M+H]⁺

### Step b) Methyl 4-(1-(1-fluoro-2-methylpropan-2-yl)-1H-1,2,3-triazol-4-yl)benzoate

To a solution of (diethylamino)difluorosulfonium tertrafluoroborate (898 mg, 3.92 mmol) in dry CH₂Cl₂ (8 mL) cooled down to 0 °C was added triethylamine trihydrofluoride (843 mg, 5.23 mmol), followed by addition of methyl 4-(1-(1-hydroxy-2-methylpropan-2-yl)-1H-1,2,3-triazol-4-yl)benzoate (720 mg, 2.62 mmol). The reaction mixture was stirred at r.t. for 15 h. The reaction was cooled down to 0 °C followed by addition of triethylamine (265 mg, 365 µL, 2.62 mmol), was stirred at 0 °C for 30 min. and at r.t. for 14 h. The reaction was quenched by slow addition of 2 M aq. Na₂CO₃ and the bi-phasic mixture was stirred at r.t. for 10 min., the mixture was transferred into a separating funnel for extraction. The organic phase was collected and the aqueous phase was back-extracted with dichloromethane. The combined organic phases were dried over sodium sulfate, and evaporated down to dryness. The crude oily product was dissolved in dry CH₂Cl₂ (8 mL), cooled down to 0 °C and DAST (5.23 mL, 5.23 mmol) was then added. The reaction mixture was stirred at 0 °C for 30 min. and 16 h at r.t. The reaction mixture was diluted with dichloromethane and extracted with aq. Na₂CO₃ 2M, the organic phase was collected and the aqueous phase was back-extracted with dichloromethane. The combined organic phases were dried over sodium sulfate and evaporated down to dryness. The crude material was purified by silica flash chromatography with an eluent mixture of ethyl acetate and heptane (5% to 50% gradient) providing the title compound (249 mg, 34.3%). MS (ESI): m/z = 278.2 [M+H]⁺

### Example A. 21

### 4-(1-tert-Butylpyrazol-4-yl)benzoic acid

To a solution of methyl 4-(1-tert-butylpyrazol-4-yl) benzoate (850 mg, 3.29 mmol) in THF (10 mL) was added sodium hydroxide (526 mg, 13.2 mmol) in water (10 mL). The mixture was stirred at 20 °C for 1 h. The mixture was concentrated to remove THF, and the residue water phase was washed with EtOAc three times. The water phase was acidified with 1M HCl aq. to pH 3, then water phase was extracted with EtOAc three times, the organic phase washed with brine, dried over Na₂SO₄ and concentrated to give the title compound (780 mg, 3.19 mmol, 92.9%) as a white solid. MS (ESI): m/z =245.1 [M+H]⁺

### Step a) Methyl 4-(1-tert-butylpyrazol-4-yl)benzoate

To a solution of 4-bromo-1-tert-butyl-pyrazole (1.00 g, 4.92 mmol) and 4-methoxycarbonylphenylboronic acid (1.06 g, 5.91 mmol), Na₂CO₃ (1.57 g, 14.8 mmol) in 1,4-dioxane (50 mL) and water (5 mL) was added Pd(Ph₃)₄ (569 mg, 0.490 mmol). The mixture was stirred at 110 °C under a nitrogen atmosphere for 12 h. The reaction mixture was filtered and the filtrate was concentrated. The residue was triturated with a mixture of EtOAc:PE = 1:3, filtered to collect the solid, and dried in vacuum to afford the title compound (950 mg, 3.68 mmol, 74.7%) as a grey solid. MS (ESI): m/z = 259.1 [M+H]⁺

### Example A.22

### 4-[1-[(1-Methylcyclopropyl)methyl]-1,2,4-triazol-3-yl]benzoic acid and 4-[2-[(1-methylcyclopropyl)methyl]-1,2,4-triazol-3-yl]benzoic acid

To a solution of the mixed methyl 4-[1-[(1-methylcyclopropyl)methyl]-1,2,4-triazol-3-yl]benzoate and methyl 4-[2-[(1-methylcyclopropyl)methyl]-1,2,4-triazol-3-yl]benzoate (150 mg, 0.550 mmol) in methanol (20 mL) was added 2 M aqueous NaOH solution (2.0 mL, 4 mmol) at 20 °C. Then the mixture was stirred at 20 °C for 15 h. The mixture was concentrated in vacuo, the residue was adjusted with 1 M HCl solution until pH = 3 and the solid product was precipitated. Then the mixture was filtered and the solid was dried under reduced pressure to afford the title compounds (200 mg, crude) as a white solid, which was carried directly to the next step.

### Step a) Methyl 4-[1-[(1-methylcyclopropyl)methyl]-1,2,4-triazol-3-yl]benzoate and methyl 4-[2-[(1-methylcyclopropyl)methyl]-1,2,4-triazol-3-yl]benzoate

To a mixture of 1-methylcyclopropanemethanol (127 mg, 1.48 mmol) and methyl 4-(1H-1,2,4-triazol-3-yl)benzoate (300 mg, 1.48 mmol) in THF (15 mL) were added triphenylphosphine (581 mg, 2.21 mmol) and diisopropyl azodicarboxylate (448 mg, 2.21 mmol) at 20 °C. The mixture was stirred at 60 °C for 15 h. To the mixture was added 20 mL water and extracted with 20 mL ethyl acetate three times. The combined organic layer was dried over anhydrous sodium sulfate, filtered and concentrated in vacuo. The residue was purified by flash eluting with an eluent mixture of ethyl acetate 20% in petroleum ether providing the title compounds (150 mg, 37.4%) as a colorless oil. MS (ESI): m/z = 272.3 [M+H]⁺

Examples A.26, A.29 and A.70 were prepared in analogy to A.22

| **Ex.** | **Systematic Name** | **Starting material 1** | **Starting material 2** | **MS (ESI): m/z** |
|---|---|---|---|---|
| A.26 | 3-Chloro-4-[(1-methylcyclopropyl)metho xy]benzoic acid | methyl 3-chloro-4-hydroxyben zoate | 1-methylcyclo propanemet hanol | 239.1 [M+H] + |
| A.29 | 3-Fluoro-4-((1-methylcyclopropyl)metho xy)benzoic acid | methyl 3-fluoro-4-hydroxyben zoate | 1-methylcyclo propanemet hanol | 225.1 [M+H] + |
| A.70 | 6-methyl-5-[[1-(trifluoromethyl)cyclopropyl] methoxy] pyridine-2-carboxylic acid | methyl 5-hydroxy-6-methyl-pyridine-2-carboxylate | 1-(trifluorome thyl)cyclopropyl]metha nol | 276.1 [M+H] + |

### Example A. 24

### 4-(5-tert-Butyl-1,2,4-oxadiazol-3-yl)-3-methyl-benzoic acid

In analogy to the procedure described for example A.4 (Step b onwards) the title compound was prepared from 3-(4-Bromo-2-methyl-phenyl)-5-tert-butyl-1,2,4-oxadiazole (300 mg), as a white solid (60 mg, 37.2%). MS (ESI): m/z = 261.1 [M+H]⁺

### Step a) 4-Bromo-N-hydroxy-2-methyl-benzamidine

A solution of 4-bromo-2-methylbenzonitrile (1.0 g, 5.1 mmol), hydroxylamine hydrochloride (0.71 g, 10.2 mmol) and NaOH (408 mg, 10.2 mmol) in ethanol (10 mL) and water (1 mL) was stirred at 80 °C for 12 h. The reaction mixture was concentrated under reduced pressure to give a crude product and water (10 mL) was added dropwise into the reaction mixture. The reaction mixture was filtered and the filter cake was washed with 10 mL water and dried under vacuum to give the title compound (900 mg, 77.0%) as a white solid. MS (ESI): m/z = 229.1[M+H]⁺

### Step b) 3-(4-Bromo-2-methyl-phenyl)-5-tert-butyl-1,2,4-oxadiazole

A solution of 4-bromo-N-hydroxy-2-methyl-benzamidine (0.9 g, 3.93 mmol), pivalic acid (401 mg, 3.93 mmol) and N.N'-cyclohexylcarbodiimide (0.98 mL, 5.89 mmol) in DMF (20 mL) was stirred at 110 °C for 12 h. The reaction mixture was concentrated to give crude product. This was purified by chromatography on silica gel with an eluent mixture of ethyl acetate and petroleum ether (1:5 to 1:3 gradient) to give the title compound (300 mg, 25.8%) as colorless oil. MS (ESI): m/z =295.0, 297.0 [M+H]⁺

Examples A.25, A.27 and A.33 to A.35 were prepared in analogy to A.24

| **Ex.** | **Systematic Name** | **Starting material** | **Base** | **MS (ESI): m/z** |
|---|---|---|---|---|
| A.25 | 4-(5-tert-butyl-1,2,4-oxadiazol-3 -yl)-3 -fluoro-benzoic acid | 4-bromo-2-fluorobenzonitrile | NaOH | 265.1 [M+H]⁺ |
| A.27 | 6-(5-tert-butyl-1,2,4-oxadiazol-3 -yl)pyridine-3-carboxylic acid | 5-bromo-2-cyanopyridine | NaOH | 246.1 [M-H]⁻ |
| A.33 | 4-(5-tert-butyl-1,2,4-oxadiazol-3 -yl)-3 -chlorobenzoic acid | 4-bromo-2-chlorobenzonitrile | Na₂CO₃ | 281.0 [M+H]⁺ |
| A.34 | 4-(5-tert-butyl-1,2,4-oxadiazol-3-yl)-3,5-difluoro-benzoic acid | 4-bromo-2,6-difluorobenzonitrile | Na₂CO₃ | 283.0 [M+H]⁺ |
| A.35 | 4-(5-tert-butyl-1,2,4-oxadiazol-3-yl)-3-(trifluoromethyl)benzoic acid | 4-bromo-2-(trifluoromethyl)ben zonitrile | Na₂CO₃ | 315.0 [M+H]⁺ |

### Example A.28

### 4-(2,2-Dimethylpropoxy)-3-methyl-benzoic acid

A solution of methyl 4-(2,2-dimethylpropoxy)-3-methyl-benzoate (310.0 mg, 1.31 mmol) and NaOH (2.62 mL, 2.62 mmol, 1M) in methanol (10 mL) was stirred at 20 °C for 15 h. The reaction mixture was concentrated under reduced pressure to give a crude product and 1 mol/L HCl (10 mL) aqueous solution was added dropwise into the reaction mixture The reaction mixture was filtered and the filter cake was washed with 10 mL water, dried in vacuum to give the title compound (130 mg, 44.5%). MS (ESI): m/z = 223.3 [M+H]⁺

### Step a) Methyl 4-(2,2-dimethylpropoxy)-3-methyl-benzoate

A solution of methyl 4-hydroxy-3-methyl-benzoate (2.0 g, 12.04 mmol), 1-bromo-2,2-dimethylpropane (2.73 g, 18.1 mmol) and K₂CO₃ (3.33 g, 24.1 mmol) in DMF (30 mL) was stirred at 80 °C for 15 h. The mixture was diluted with water (100 mL) and extracted with 100 mL EtOAc three times. The combined organic phase was washed with brine (50 mL), dried over Na₂SO₄, filtered and concentrated under reduced pressure. The residue was purified by silica gel chromatography with an eluent mixture of ethyl acetate and petroleum ether (1:10 to 1:5 gradient) providing the title compound (310 mg, 10.9%) as a light yellow solid. MS (ESI): m/z = 237.1[M+H]⁺

### Example A.30

### 4-(3-(tert-Butyl)-1H-1,2,4-triazol-1-yl)benzoic acid

A solution of 3-tert-butyl-1H-1,2,4-triazole (500 mg, 3.99 mmol), methyl 4-bromobenzoate (1.29 g, 5.99 mmol), Cs₂CO₃ (3.25 g, 9.99 mmol), CuI (152 mg, 0.800 mmol) and 8-hydroxyquinoline (46.4 mg, 0.320 mmol) in DMF (10 mL) and water (1 mL), the reaction was purged with nitrogen for three times and stirred at 130 °C for 12 h. The reaction was diluted with water (30 mL) and extracted with EtOAc for three times, the combined organic layer was washed with water three times and brine, dried over sodium sulfate and concentrated in vacuum to get a green solid. The residue was triturated with EtOAc (1 mL) to get the title compound (900 mg, 29.6%) as a light green solid. MS (ESI): m/z = 246.1 [M+H]⁺

### Example A.31

### 4-[5-(2,2-Dimethylpropyl)-4-methyl-1,2,4-triazol-3-yl]benzoic acid

To a solution ofN,3,3-trimethylbutanamide (1200 mg, 9.29 mmol) and 2,6-lutidine (3.25 mL, 27.9 mmol) in DCM (20 mL) was added oxalylchloride (1415 mg, 11.2 mmol) at 0 °C. The mixture was stirred at 0 °C for 30 mins, then methyl 4-(hydrazinecarbonyl)benzoate (1804 mg, 9.29 mmol) was added. The mixture was stirred at 20 °C for 12 h. The mixture was concentrated and sat. aq. NaHCO₃ solution (100 mL, 9.29 mmol) was added. The mixture was stirred at 100 °C for 3.5 h. The mixture was extracted with EtOAc, the water phase was acidified with HCl (37% aq.) to pH 3, and then purified by reversed flash column chromatography to give the title compound (240 mg, 0.880 mmol, 9.45%) as a light yellow solid. MS (ESI): m/z = 274.1 [M+H]⁺

### Example A.32

### 4-(5-tert-Butyl-4-methyl-1,2,4-triazol-3-yl)benzoic acid

To a solution of N,2,2-trimethylpropanamide (1000 mg, 8.68 mmol) and DMF (0.05 mL, 8.68 mmol) in DCM (15 mL) was added oxalylchloride (1322 mg, 10.4 mmol) at 0 °C. The mixture was stirred at 20 °C for 1 h. The mixture was added to another solution of methyl 4-(hydrazinecarbonyl)benzoate (1686 mg, 8.68 mmol) and triethylamine (3.63 mL, 26.05 mmol) in DCM (15 mL) at 0 °C and stirred at 20 °C for 12 h. The mixture was concentrated and the residue was dissolved in sat. aq. NaHCO₃ solution (50.0 mL, 8.68 mmol) and heated at 100 °C for 3 h. The mixture was washed with EtOAc (30 mL) and the water phase was acidified with conc. HCl to pH=3. The solution was purified by reversed flash column chromatography and lyophilized to give the title compound (320 mg, 1.23 mmol, 14.2%) as a light yellow solid. MS (ESI): m/z = 260.1 [M+H]⁺

### Example A.36

### 4-(5-tert-butyl-1,3,4-oxadiazol-2-yl)-3-methyl-benzoic acid

In analogy to the procedure described for example A.4 (Step b onwards) the title compound was prepared from 2-(4-bromo-2-methyl-phenyl)-5-tert-butyl-1,3,4-oxadiazole (100 mg, 0.34 mmol), as a white solid (30 mg, 63.2%). MS (ESI): m/z = 261.1 [M+H]⁺

### Step a) 4-Bromo-N-[(E)-2,2-dimethylpropylideneamino]-2-methyl-benzamide

A mixture of 4-bromo-2-methyl-benzohydrazide (100.0 mg, 0.440 mmol) and trimethylacetaldehyde (41.4 mg, 0.480 mmol) in ethanol (3 mL) was stirred at 80 °C for 12 h. The mixture was concentrated to give the title compound (120 mg, 0.400 mmol, 92.5%) as an off-white solid. MS (ESI): m/z = 297.0 [M+H]⁺

### Step b) 2-(4-Bromo-2-methyl-phenyl)-5-tert-butyl-1,3,4-oxadiazole

To a solution of 4-bromo-N-[(E)-2,2-dimethylpropylideneamino]-2-methyl-benzamide (100 mg, 0.340 mmol) and potassium carbonate (140 mg, 1.01 mmol) in DMSO (2 mL) was added iodine (128 mg, 0.500 mmol). The mixture was stirred at 120 °C for 12 h. The mixture was diluted with water (10 mL) and extracted with EtOAc. The organic phase was washed with brine, dried over Na₂SO₄ and concentrated to give the crude title compound (100 mg, 0.340 mmol, 101%) as a yellow oil. MS (ESI): m/z = 295.0 [M+H]⁺

Examples A.55, A.56 and A.58 were prepared in analogy to A.36

| **Ex.** | **Systematic Name** | **Starting material** | **MS (ESI): m/z** |
|---|---|---|---|
| A.55 | 4-(5-(tert-butyl)-1,3,4-oxadiazol-2-yl)-3-chlorobenzoic acid | 4-bromo-2-chlorobenzohydrazide | 281.1 [M+H]⁺ |
| A.56 | 4-(5-(tert-butyl)-1,3,4-oxadiazol-2-yl)-3-fluoro-5-hydroxybenzoic acid | 4-bromo-2,6-difluorobenzohydrazide | 281.1 [M+H]⁺ |
| A.58 | 4-(5-tert-butyl-1,3,4-oxadiazol-2-yl)-3-(trifluoromethyl)benzoic acid | 4-bromo-2-(trifluoromethyl)benzoh ydrazide | 315.1 [M+H]⁺ |

### Example A.40

### 4-(1-(tert-Butyl)-1H-1,2,4-triazol-3-yl)-3-chlorobenzoic acid

In analogy to the procedure described for example A.4 (hydrolysis step) the title compound was prepared from methyl 4-(1-(tert-butyl)-1H-1,2,4-triazol-3-yl)-3-chlorobenzoate (200 mg, 0.681 mmol), (195 mg, 100%). MS (ESI): m/z = 280.2 [M+H]⁺

### Step a) 1-(tert-Butyl)-1H-1,2,4-triazol-3-amine

To a solution of 4H-1,2,4-triazol-3-amine (3 g, 35.7 mmol) in perchloric acid 70% aq. Solution (15 mL) was added 2-methylpropan-2-ol (2.78 g, 3.58 mL, 37.5 mmol) and the reaction mixture was stirred at r.t. for 14 h. The reaction medium was then quenched by slow addition of 4 M aqueous NaOH solution until pH 6-7 was obtained, and then extracted three times with DCM. The combined organic phases were dried over sodium sulfate and evaporated down to dryness to provide the title compound (5.08 g, 100%) as a light yellow solid. MS (ESI): m/z = 141.1 [M+H]⁺

### Step b) 3-Bromo-1-(tert-butyl)-1H-1,2,4-triazole

To a solution of 1-(tert-butyl)-1H-1,2,4-triazol-3-amine (1.5 g, 10.7 mmol) in ACN (50 mL) cooled down to 0 °C was added copper (II) bromide (3.11 g, 13.9 mmol) followed by addition of tert-butyl nitrite (1.59 g, 1.84 mL, 13.9 mmol), the reaction mixture was stirred at 0 °C for 10 min and at 70 °C for 16 h. The reaction mixture was concentrated in vacuo and then partitioned between 100 mL ethyl acetate and 80 mL aq. sol. NH₃ 4M. The organic phase was collected and the aqueous phase was back-extracted with 100 mL ethyl acetate. The combined organic phases were dried over sodium sulfate and evaporated down to dryness. The crude material was purified by silica gel flash chromatography with an eluent mixture of EtOAc and heptane (5% to 40% gradient) providing the title compound (1.65g, 8 mmol, 75.3%) as a colorless oil. MS (ESI): m/z = 204.1 [M+H]⁺

### Step c) Methyl 4-(1-(tert-butyl)-1H-1,2,4-triazol-3-yl)-3-chlorobenzoate

To a solution of 3-bromo-1-(tert-butyl)-1H-1,2,4-triazole (300 mg, 1.47 mmol) in DME (7 mL) was added (2-chloro-4-(methoxycarbonyl)phenyl)boronic acid (394 mg, 1.84 mmol), PdCl₂(dppf) (60 mg, 73.5 µmol) and Cs₂CO₃ 2.5M aqueous solution (1.76 mL, 4.41 mmol). The reaction mixture was then stirred at 100 °C for 30 min. under microwave irradiation. The reaction mixture was diluted with ethyl acetate and extracted with sat. aq. NaHCO₃ solution, the organic phase was collected and the aqueous phase was back-extracted with ethyl acetate. The combined organic phases were dried over sodium sulfate and evaporated down to dryness. The residue was purified by silica gel chromatography with an eluent mixture of EtOAc and heptane (5% to 40% gradient) to give the title compound (175 mg, 40.5%) as a white solid. MS (ESI): m/z = 294.2 [M+H]⁺

Examples A.44, A.50, A.53 and A.59 were prepared in analogy to A.40

| **Ex.** | **Systematic Name** | **Starting material** | **MS (ESI): m/z** |
|---|---|---|---|
| A.44 | 4-(1-(tert-butyl)-1H-1,2,4-triazol-3-yl)-3-methylbenzoic acid | (4-(methoxycarbonyl)-2-methylphenyl)boronic acid | 260.2 [M+H] ⁺ |
| A.50 | 4-(1-(tert-butyl)-1H-1,2,4-triazol-3-yl)-3-fluorobenzoic acid | (2-fluoro-4-(methoxycarbonyl)phenyl) boronic acid | 264.2 [M+H] ⁺ |

| | | | |
|---|---|---|---|
| A.53 | 4-(1-(tert-butyl)-1H-1,2,4-triazol-3-yl)-3-(trifluoromethyl)benzoic acid | methyl 4-(4,4,5,5-tetramethyl-1,3,2-dioxaborolan-2-yl)-3- (trifluoromethyl)benzoate | 314.2 [M+H] ⁺ |
| A.59 | 4-(1-(tert-butyl)-1H-1,2,4-triazol-3-yl)-3-methoxybenzoic acid | methyl 3-methoxy-4-(4,4,5,5-tetramethyl-1,3,2-dioxaborolan-2-yl)benzoate | 276.2 [M+H] ⁺ |

### Example A.41

### 3,5-Difluoro-4-(1-neopentyl-1H-1,2,3-triazol-4-yl)benzoic acid

In analogy to the procedure described for example A.19 the title compound was prepared from methyl 4-ethynyl-3,5-difluorobenzoate (404 mg 2.06 mmol) as an off-white solid (25.7 mg, 70.8%). MS (ESI): m/z = 296.2 [M+H]⁺

### Step a) Methyl 3,5-difluoro-4-((trimethylsilyl)ethynyl)benzoate

A mixture of methyl 4-bromo-3,5-difluorobenzoate (999 mg, 3.98 mmol) in THF (20.6 mL) was purged with argon in a vial suitable for microwave chemistry. Then ethynyltrimethylsilane (782 mg, 1.1 mL, 7.96 mmol), bis(triphenylphosphine)palladium (II) chloride (140 mg, 199 µmol), triphenylphosphine (20.9 mg, 79.6 µmol), copper (I) iodide (22.7 mg, 119 µmol) and triethylamine (1.21 g, 1.66 mL, 11.9 mmol) were added. The mixture was heated to 75 °C (oil bath temp.) for 2 h. The reaction mixture was filtered and the filter washed well with EtOAc. The organic solution was washed with water. The aqueous phase was back extracted with EtOAc. The combined organics were washed with brine, dried (MgSO₄), filtered and concentrated to leave the crude product as a orange liquid. The crude product was purified by silica gel flash chromatography with an eluent mixture of EtOAc and heptane (0% to 20% gradient) providing the title compound (0.96 g, 89.9%) as an orange liquid. MS (ESI): m/z = 269.2 [M+H]⁺

### Step b) Methyl 4-ethynyl-3,5-difluorobenzoate

The solution of methyl 3,5-difluoro-4-((trimethylsilyl)ethynyl)benzoate (0.94 g, 3.5 mmol) in THF (14.5 mL) was cooled to 0 °C and tetrabutylammonium fluoride solution (1 M in THF) (4.2 mL, 4.2 mmol) was added dropwise. The reaction was stirred at 0 °C for 0.5 h. The reaction solution was poured into sat. aq. NH₄Cl solution and extracted with EtOAc. The organic layer was dried over MgSO₄ and concentrated to leave the crude product as a yellow liquid. The crude product was purified by silica gel flash chromatography with an eluent mixture of EtOAc and heptane (0% to 30% gradient) providing the title compound (0.41 g, 59.7%) as a light yellow solid.

### Example A.42

### 3,5-Difluoro-4-(2-neopentyl-2H-1,2,3-triazol-4-yl)benzoic acid

In analogy to the procedure described for example A.20 the title compound was prepared from methyl 3,5-difluoro-4-(2-neopentyl-2H-1,2,3-triazol-4-yl)benzoate (62.8 mg, isomeric side product from A.41), as a white solid (61.9 mg, 100%). MS (ESI): m/z = 296.2 [M+H]⁺

### Example A.47

### 3-((2,6-Dichlorophenyl)ethynyl)azetidine

To a light yellow solution of tert-butyl 3-((2,6-dichlorophenyl)ethynyl)azetidine-1-carboxylate (134 mg, 411 µmol) in DCM (1 mL) at r.t. trifluoroacetic acid (468 mg, 316 µL, 4.11 mmol) was added and stirred for 30 min. The reaction mixture was diluted with DCM and extracted with ice-cold aq. 1N Na₂CO₃ solution to pH 8-9. The aqueous was back-extracted with DCM. The combined organic layers were washed with brine, dried over Na₂SO₄, filtered and evaporated providing the title compound (89 mg, 95.8%) as a colorless gum. MS (ESI): m/z = 226.1 [M+H]⁺

### Step a) tert-Butyl 3-((2,6-dichlorophenyl)ethynyl)azetidine-1-carboxylate

In a sealed tube, a mixture of tert-butyl 3-ethynylazetidine-1-carboxylate (0.150 g, 828 µmol), 1,3-dichloro-2-iodobenzene (294 mg, 1.08 mmol), copper (I) iodide (3.15 mg, 16.6 µmol), TEA (838 mg, 1.15 mL, 8.28 mmol) in THF (2.5mL) was degassed for 10 min under an argon atmosphere. Bis(triphenylphosphine)palladium (II) chloride (46.5 mg, 66.2 µmol) was added to above solution. The solution was heated to 70 °C and stirred for 18 h. The mixture was filtered over a glass fiber filter, the filter was washed with EtOAc and the mother liquor was evaporated to dryness. The crude product was purified by silica gel flash chromatography with an eluent mixture of EtOAc and heptane (0% to 25% gradient) providing the title compound (0.134 g, 49.6%) as a light yellow oil.
MS (ESI): m/z = 270.1 [M+H-tBu]⁺

Example A.48 was prepared in analogy to A.47

| **Ex.** | **Structure** | **Starting material 1** | **Starting material 2** | **MS (ESI): m/z** |
|---|---|---|---|---|
| A.48 | 3-((2-chloro-4-fluorophenyl)ethynyl)azetidi ne | tert-butyl 3-ethynylazetidin e-1-carboxylate | 2-chloro-4-fluoro-1-iodobenzene | 210.1 [M+H]⁺ |

### Example A.57

### 3-Cyano-4-ethynylbenzoic acid

To a stirred solution of methyl 3-cyano-4-((trimethylsilyl)ethynyl)benzoate (0.985 g, 3.83 mmol) at r.t. in methanol (10 mL) under an argon atmosphere was added dropwise 1 N NaOH (5.74 mL, 5.74 mmol). The mixture turned to a grey slurry, then to an almost clear solution. Stirring at r.t. was continued for 2 h. The mixture was treated with 1 N HCl (5.8 mL). The mixture soon turned to a thick slurry. The mixture was filtrated, and the filter cake was washed with plenty of H₂O and with n-heptane, providing the title compound (594 mg, 90.7%) as an off-white solid. MS (ESI): m/z = 170.1 [M-H]⁻

### Step a) Methyl 3-cyano-4-((trimethylsilyl)ethynyl)benzoate

In a vial suitable for microwave chemistry, argon was bubbled through a solution of methyl 4-bromo-3-cyanobenzoate (1 g, 4.17 mmol) at r.t. in THF (10 mL) for 5 min. Then ethynyltrimethylsilane (818 mg, 1.15 mL, 8.33 mmol), bis(triphenylphosphine)palladium (II) chloride (146 mg, 208 µmol), triphenylphosphine (21.9 mg, 83.3 µmol), copper(I) iodide (23.8 mg, 125 µmol) and triethylamine (1.26 g, 1.74 ml, 12.5 mmol,) were added. The vial was sealed. The mixture was heated to 75 °C and stirring was continued for 3 h. The reaction mixture was cooled to r.t., filtered and the cake was washed well with EtOAc. The filtrate was concentrated. The crude product was purified by silica gel flash chromatography with an eluent mixture of EtOAc and heptane (0% to 25%) to give the title compound (0.992 g, 92.5%) as a light brown solid.

### Example A.61

### 6-Ethynyl-5-fluoronicotinic acid

In analogy to the procedure described for example A.57 the title compound was prepared from methyl 6-bromo-5-fluoronicotinate (1 g, 4.27 mmol) as an off-white solid (828 mg, 101%). MS (ESI): m/z = 166.1 [M+H]⁺

### Example A.71

### 4-Ethynyl-3-(trifluoromethyl)benzoic acid

In analogy to the procedure described for example A.57 the title compound was prepared from methyl 4-bromo-3-(trifluoromethyl)benzoate (1 g 3.53 mmol) as a white solid (693 mg, 87.5%). MS (ESI): m/z = 213.2 [M+H]⁺

### Example A.62

### 6-(4-tert-Butylpyrazol-1-yl)-5-methyl-pyridine-3-carboxylic acid

Methyl 6-(4-tert-butylpyrazol-1-yl)-5-methyl-pyridine-3-carboxylate (750 mg, 2.74 mmol) was dissolved in a mixture of THF (4 mL) and MeOH (4 mL), then NaOH (2 M, 4mL) was added. The resulting solution was stirred at 25 °C for 1 h. The reaction mixture was adjusted to pH = 3 - 4 with HCl (1 M) and extracted with ethyl acetate. The combined organic layer was washed with brine, dried over Na₂SO₄, filtered and concentrated providing the title compound (700.0 mg, 2.70 mmol, 98.4%) as a white solid. MS (ESI): m/z = 260.0 [M+H]⁺

### Step a) Methyl 6-(4-tert-butylpyrazol-1-yl)-5-methyl-pyridine-3-carboxylate

Methyl 6-fluoro-5-methylnicotinate (370 mg, 2.98 mmol) and K₂CO₃ (1.03 g, 7.42 mmol) were combined and stirred in DMF (2 mL) for 10 minutes, then 4-tert-butylpyrazole (500 mg, 2.96 mmol) was added and the reaction was stirred for 1 h at 100 °C. The reaction mixture was adjusted to pH 3-4 with HCl (1 M), extracted with MTBE. The combined organic layer was washed with HCl (1M) and brine, dried over Na₂SO₄, filtered and concentrated providing the title compound (555 mg, 2.03 mmol, 68.7%) and hydrolysed side product (6-(4-tert-butylpyrazol-1-yl)-5-methyl-pyridine-3-carboxylic acid, A.62) (195.0 mg, 752.0 µmol, 25.4%) as white solid.
MS (ESI): m/z = 274.3 [M+H]⁺ for title compound
MS (ESI): m/z = 260.2 [M+H]⁺ for hydrolysed side product A.62

### Example A.63

### 6-(3-tert-Butyl-1,2,4-triazol-1-yl)-5-methyl-pyridine-3-carboxylic acid

In analogy to the procedure described for example A.62 the title compound was prepared from methyl 6-fluoro-5-methylnicotinate (200 mg, 1.18mmol) and 3-tert-butyl-1H-1,2,4-triazole (160 mg, 1.28 mmol), as a white solid (250 mg, 99.4%). MS (ESI): m/z = 261.1 [M+H]+

### Example A.66

### 6-Methyl-5-[[1-(trifluoromethyl)cyclopropyl]methoxy]pyrazine-2-carboxylic acid

To a solution of NaOH (300 mg, 7.5 mmol) in water (5 mL), Methanol (5 mL), and THF (5 mL) was added methyl 6-methyl-5-[[1-(trifluoromethyl)cyclopropyl]methoxy]pyrazine-2-carboxylate (170.0 mg, 0.590 mmol) and the mixture was stirred at 25 °C for 12 h. The reaction was acidized with 1 M HCl to pH 7 and concentrated under vacuum to remove the solvent. The residue was re-dissolved in EtOAc (20 mL) and washed with water and brine, dried over sodium sulfate and concentrated in vacuum to provide the title compound (120 mg, 0.430 mmol, 74.2%) as a yellow oil. MS (ESI): m/z = 277.0 [M+H]⁺

### Step a) Methyl 6-methyl-5-[[1-(trifluoromethyl)cyclopropyl]methoxy]pyrazine-2-carboxylate

To a solution of NaH (129 mg, 3.22 mmol) in THF (6 mL) was added [1-(trifluoromethyl)cyclopropyl]methanol (450 mg, 3.22 mmol). The mixture was stirred at 0 °C for 15 min, then methyl 5-chloro-6-methyl-pyrazine-2-carboxylate (300 mg, 1.61 mmol) was added and the mixture was stirred at 25 °C for 12 h. The reaction was poured into sat. aq. NH₄Cl solution (30 mL) and extracted with EtOAc. The combined organic layer was washed with water and brine, dried over sodium sulfate and concentrated in vacuum to get a yellow residue. The crude product was purified by reverse phase chromatography providing the title compound (170 mg, 36.43%), MS (ESI): m/z = 291.1 [M+H]⁺. The hydrolysed side product 6-methyl-5-[[1-(trifluoromethyl)cyclopropyl]methoxy]pyrazine-2-carboxylic acid (A.66) (170 mg, 0.620 mmol, 38.28%) was also isolated as a yellow oil. MS (ESI): m/z = 277.0 [M+H]⁺

### Example A.69

### 5-Methoxy-6-[[1-(trifluoromethyl)cyclopropyl]methoxy]pyridine-3-carboxylic acid

To a solution of [1-(trifluoromethyl)cyclopropyl]methanol (417 mg, 2.98 mmol) in THF (6 mL) was added t-BuOK (2.98 mL, 2.98 mmol) under a nitrogen atmosphere. The mixture was stirred at 25 °C for 5 min, then methyl 6-chloro-5-methoxy-pyridine-3-carboxylate (300 mg, 1.49 mmol) was added and the reaction was stirred for 12 h. The reaction was poured into sat. NH₄Cl (aq.) and extracted with EtOAc. The combined organic layer was washed with water and brine, dried over sodium sulfate and concentrated under vacuum to the crude product, which was purified by reverse phase chromatography providing the title compound (100 mg, 0.340 mmol, 23.1%) as a yellow oil. MS (ESI): m/z = 292.1 [M+H]⁺

### Example A.72

### 2-(3-Chlorophenyl)sulfonyl-2,6-diazaspiro [3.3] heptane

A solution of tert-butyl 2-(3-chlorophenyl)sulfonyl-2,6-diazaspiro[3.3]heptane-6-carboxylate (350 mg, 0.940 mmol) and TFA (20 mmol) in DCM (10 mL) (10.0 mL, 20 mmol) was stirred at 20 °C for 12 h. The mixture was concentrated and diluted with DCM (30 mL), washed with aq. Na₂CO₃ and brine. The organic phase was dried over Na₂SO₄, filtered and the filtrate was concentrated to provide the title compound (250 mg, 0.920 mmol, 97.7%) as a yellow solid. MS (ESI): m/z = 273.0 [M+H]⁺

### Step a) tert-Butyl 2-(3-chlorophenyl)sulfonyl-2,6-diazaspiro[3.3]heptane-6-carboxylate

To a solution of tert-butyl 2,6-diazaspiro[3.3]heptane-2-carboxylate, oxalic acid (300 mg, 1.04 mmol) and triethylamine (0.44 mL, 3.12 mmol) in DCM (10 mL) was added 3-chlorobenzenesulfonyl chloride (231 mg, 1.09 mmol) at 0 °C, the mixture was stirred at 20 °C for 2 h. The mixture was diluted with DCM (20 mL) washed with aq. Na₂CO₃ (2 M) solution and brine. The organic phase was dried over Na₂SO₄, filtered and the filtrate was concentrated to provide the title compound (350 mg, 0.940 mmol, 90.21%) as an off-white solid. MS (ESI): m/z = 316.9 [M-tBu+H]⁺

### Example A.73

### 4-[2-(2,2-Dimethylpropyl)tetrazol-5-yl]benzoic acid

To a solution of ethyl 4-(2-neopentyl-2H-tetrazol-5-yl)benzoate (410 mg, 1.42 mmol) in methanol (10 mL) was added NaOH 5 M aqueous solution (569 µL, 2.84 mmol) and the reaction mixture was stirred at 55 °C for 16 h. Volatiles were removed in vacuo and the crude residue was partitioned between ethyl acetate and an aqueous solution of HCl 0.5 M, the organic phase was collected and the aqueous phase was back-extracted with ethyl acetate. The combined organic phases were dried over sodium sulfate and evaporated down to dryness to give 348m g of the crude desired product. MS (ESI): m/z = 261.2 [M+H]⁺

### Step a) Ethyl 4-(2H-tetrazol-5-yl)benzoate

To a solution of ethyl 4-cyanobenzoate (500 mg, 2.85 mmol) in DMF (13 mL) was added sodium azide (232 mg, 3.57 mmol) and L-proline (9.86 mg, 85.6 µmol), the reaction mixture was then stirred at 110 °C for 18.5 h and then at 125°C for 41.5 h. Reaction mixture was concentrated in vacuo and the crude residue was suspended in dichloromethane. To the stirred suspension was added 20 mL of 0.2 M aq. HCl solution, the bi-phasic mixture was vigorously stirred for 10 min and transferred into a separating funnel for extraction. The organic phase was collected and the aqueous phase was back-extracted with dichloromethane. The combined organic phases were dried over sodium sulfate and evaporated down to dryness to yield 765 mg of a crude product as a light yellow oil (contains DMF). The crude was used for the next without further purification. MS (ESI): m/z = 219.1 [M+H]⁺

### Step b) Ethyl 4-(2-neopentyl-2H-tetrazol-5yl)benzoate

To a solution of ethyl 4-(2H-tetrazol-5-yl)benzoate (765 mg, 2.8 mmol) in dry DMF (15 mL) cooled down to 0 °C was added NaH (118 mg, 2.94 mmol) and the reaction mixture was stirred at 0 °C for 15 min followed by addition of 1-bromo-2,2-dimethylpropane (445 mg, 2.94 mmol). The reaction was stirred at r.t for 1 h and then for 1 h at 70°C, then at 90°C for 1 h, then at 125 °C for 1 h, then at 125°C in a closed vial for another 64 h. A further addition of 1-iodo-2,2-dimethylpropane (583 mg, 2.94 mmol) was made and the reaction mixture was stirred at 125 °C for another 16 h. The reaction mixture was quenched by addition of few drops of sat. aq. NH₄Cl, volatiles were removed in vacuo and the crude residue was partitioned between ethyl acetate and aq. NaHCO₃ 1 M. The organic phase was collected and the aqueous phase was back-extracted with ethyl acetate. The combined organic phases were dried over sodium sulfate and evaporated down to dryness. The crude material was purified by flash chromatography with an eluent mixture of heptane and ethyl acetate (5% to 20%) to yield 489 mg of the title compound as a white solid. MS (ESI): m/z = 289.2 [M+H]⁺

### Example A.74

### 3,5-Difluoro-4-(2-neopentyl-2H-tetrazol-5-yl)benzoic acid

To a solution of methyl 3,5-difluoro-4-(2-neopentyl-2H-tetrazol-5-yl)benzoate (172 mg, 499 µmol) in methanol (4 mL) was added NaOH 5.0 M aqueous solution (249 µL, 1.25 mmol) and the reaction mixture was stirred at 55 °C for 16 h. Volatiles were removed in vacuo, the obtained crude residue was then partitioned between ethyl acetate and a mixture of 1:1 brine HCl 1 M (aq.). The organic phase was collected and the aqueous phase was back-extracted with ethyl acetate. The combined organic phases were dried over sodium sulfate and evaporated down to dryness to yield 139 mg of the crude desired product which was used without further purification. MS (ESI): m/z = 297.1 [M+H]⁺

### Step a) 2-Neopentyl-2H-tetrazol-5-amine

To a solution of 1H-tetrazol-5-amine (3 g, 35.3 mmol) in DMF (90 mL) cooled down to 0 °C was added NaH (1.48 g, 37 mmol) and the reaction was stirred at 0 °C for 15 min followed by addition of 1-iodo-2,2-dimethylpropane (7.33 g, 4.91 mL, 37.0 mmol), the reaction mixture was then stirred at 120 °C for 24 h. Reaction was quenched by addition of a few drops or sat. aq. NH₄Cl solution, volatiles were then removed in vacuo, the crude residue was then partitioned between 150 mL ethyl acetate and 50 mL of a mixture 1:1 (brine: aq. NaHCO₃ 1 M solution). The organic phase was collected and the aqueous phase was back-extracted with 150 mL ethyl acetate. The combined organic phases were dried over sodium sulfate and evaporated down to dryness. The crude material loaded onto a silica column, but due to precipitation, was finally purified by washing the SiO₂ phase with a mixture CH₂Cl₂:MeOH (4:1), the insolubilities were removed by filtration, the filter pad was washed twice with mixture CH₂Cl₂:MeOH (4:1) and the filtrate was evaporated down to dryness to 5.85 g of a crude product. This was submitted for purification by SFC to yield the title compound (1.73 g). (The regioisomer (1.21 g) was also isolated.) MS (ESI): m/z = 156.1 [M+H]⁺

### Step b) 5-Iodo-2-neopentyl-2H-tetrazole

To a solution of 2-neopentyl-2H-tetrazol-5-amine (545 mg, 3.51 mmol) in dry CH₃CN (20 mL) was added copper (I) iodide (1 g, 5.27 mmol), isopentyl nitrite (617 mg, 708 µL, 5.27 mmol) and the reaction mixture was stirred at 65 °C for 20 h. A further addition of copper (I) iodide (334 mg, 1.76 mmol) was made and the reaction mixture was then stirred at 65°C for another 4 h, then at 80°C for 3 h. Reaction mixture was concentrated in vacuo and the crude residue was suspended in ethyl acetate followed by extraction with a 3.0 M aq. NH₃ solution, the organic phase was collected and the aqueous phase was back-extracted with ethyl acetate. The combined organic phases were dried over sodium sulfate and evaporated down to dryness. The crude material was purified by flash chromatography with an eluent mixture of heptane and ethyl acetate (5% to 50% gradient) to yield 607 mg of the title compound. MS (ESI): m/z = 267.1 [M+H]⁺

### Step c) 2-Neopentyl-5-(tributylstannyl)-2H-tetrazole

To a solution of 5-iodo-2-neopentyl-2H-tetrazole (1.53 g, 5.75 mmol) in dry THF (25 mL) cooled down to -78 °C under an inert atmosphere was added slowly BuLi 1.6M solution in hexanes (3.95 mL, 6.33 mmol) and the reaction was then stirred at -78 °C for 30 min followed by addition of tributylchlorostannane (2.06 g, 1.72 mL, 6.33 mmol). The reaction was stirred at -78 °C for 30 min and quenched by addition of a few drops of sat. aq NH₄Cl solution and the reaction was let to warm up to r.t. The reaction mixture was then diluted with ethyl acetate and extracted with aq. NaHCO₃ 1 M, the organic phase was collected and the aqueous phase was back-extracted with ethyl acetate. The combined organic phases were dried over sodium sulfate and evaporated down to dryness to yield 2.71 g of the title compound. The crude material was used without further purification. MS (ESI): m/z = 431.3 [M+H]⁺

### Step d) Methyl 3,5-difZuoro-4-(2-neopentyl-2H tetrazol-S yl)benzoate

To a solution of 2-neopentyl-5-(tributylstannyl)-2H-tetrazole (290 mg, 676 µmol) in dry DMF (4 mL) was added methyl 4-bromo-3,5-difluorobenzoate (195 mg, 777 µmol), Pd(Ph₃P)₄ (39 mg, 33.8 µmol) and copper (I) iodide (6.43 mg, 33.8 µmol). The reaction mixture was then stirred at 110 °C for 16 h. Volatiles were removed in vacuo and the crude residue was directly purified by flash chromatography with an eluent mixture of heptane and ethyl acetate (5% to 30%) to yield 172mg of the title compound (which was slightly contaminated with the tin by-product) but was carried into the next step without further purification. MS (ESI): m/z = 311.2 [M+H]⁺

Examples A.75 to A.76 were prepared in analogy to A.74 (Step d onwards) from the stannane building block and the commercially available halo-benzoates.

| **Ex.** | **Systematic Name** | **Starting material** | **MS (ESI): m/z** |
|---|---|---|---|
| A.75 | 4-[2-(2,2-dimethylpropyl)tetrazol-5-yl]-3-methyl-benzoic acid | 4-bromo-3-methylbenzoate | 275.2 [M+H]⁺ |
| A.76 | 4-[2-(2,2-dimethylpropyl)tetrazol-5-yl]-3-fluoro-benzoic acid | methyl 3-fluoro-4-iodobenzoate | 279.1 [M+H]⁺ |

### Example A.77

### 3-Fluoro-4-(1-neopentyl-1H-1,2,4-triazol-3-yl)benzoic acid

To a solution of (2-fluoro-4-methoxycarbonyl-phenyl)boronic acid (3.55 g, 17.9 mmol) and 3-bromo-1-(2,2-dimethylpropyl)-1,2,4-triazole (2.30 g, 10.6 mmol) in 1,4-Dioxane (115 mL) was added PdCl₂(dppf) (739 mg, 1.05 mmol, CAS RN 95408-45-0) and Cs₂CO₃ (10.3 g, 31.6 mmol). The reaction mixture was purged with nitrogen and stirred at 100 °C for 12 h. The reaction mixture was concentrated to remove the solvent and the residue was re-dissolved in EtOAc, the organic layer was washed with water and brine, dried over sodium sulfate and concentrated in vacuum to get yellow residue, which was purified with prep-HPLC (0.225% v/v FA) to get desired product (450 mg, 1.62 mmol, 15.4% yield) as light brown solid. MS (ESI): m/z = 278.6 [M+H]⁺.

### Step a) Neopentyl methanesulfonate

To a solution of TEA (6.89 g, 68.1 mmol) and neopentyl alcohol (2.00 g, 22.7 mmol, CAS RN 75-84-3) in DCM (20 mL) was added methanesulfonyl chloride (2.11 mL, 27.2 mmol, CAS RN 124-63-0) with the temperature was kept at 0 °C. The reaction mixture was stirred at 20 °C for 2 hrs. The reaction was quenched with sat. Na₂CO₃ (30 mL) and aqueous phase was extracted with DCM twice. The combined organic layer was washed with water twice and brine, dried over sodium sulfate and concentrated in vacuum to get crude product, which was purified with silica gel column (EtOAc in PE from 0% to 10%) to get desired product (3.5 g, 21.1 mmol, 92.8% yield) as colorless oil.

### Step b) 3-Bromo-1-neopentyl-1H-1,2,4-triazole

To a solution of NaH (837 mg, 20.93 mmol, 60 wt.%) in DMF (20.7 mL) was added 3-bromo-1H-1,2,4-triazole (2.58 g, 17.4 mmol) with the temperature was kept at 0 °C. The mixture was stirred for 15 min, then 2,2-dimethylpropyl methane sulfonate (2.90 g, 17.4 mmol) was added and stirred at 20 °C for 12hrs. The reaction mixture was poured into water and extracted with EtOAc twice, the combined organic layer was washed with water and brine, dried over sodium sulfate and concentrated in vacuum to get crude product (3.00 g) as yellow oil. MS (ESI): m/z = 218.0 [M+H]⁺.

### Example A.78

### 4-(3-(tert-Butyl)-1H-1,2,4-triazol-1-yl)-3-methylbenzoic acid

To a solution of 3-tert-butyl-1H-1,2,4-triazole (160.0 mg, 1.28 mmol, CAS RN 96440-78-7) and methyl 4-fluoro-3-methylbenzoate (258 mg, 1.53 mmol, CAS RN 180636-50-4) in DMF (8 mL) was added Cs₂CO₃ (1.25 g, 3.83 mmol). The mixture was stirred at 120 °C for 12 h. The reaction mixture was poured into water and extracted with EtOAc for three times. The combined organic layer was washed with water and brine, dried over sodium sulfate and concentrated in vacuum to get crude product (250 mg) as yellow solid. MS (ESI): m/z = 260.1 [M+H]⁺.

### Example A.79

### 4-(3-(tert-Butyl)-1H-1,2,4-triazol-1-yl)-3-chlorobenzoic acid

To a solution of methyl 4-(3-tert-butyl-1,2,4-triazol-1-yl)-3-chloro-benzoate (130 mg, 0.44 mmol) in THF (5 mL) was added NaOH (70 mg, 1.77 mmol), methanol (5 mL) and water (5 mL). The mixture was stirred at 20 °C for 12 h. The reaction mixture was acidized with 1 M HCl to pH ~6 and extracted with EtOAc for three times. The combined organic layer was washed with water and brine, dried over sodium sulfate and concentrated in vacuum to get crude product (150 mg) as a brown solid. MS (ESI): m/z = 280.1 [M+H]⁺.

### Step a) Methyl 4-(3-(tert-butyl)-1H-1,2,4-triazol-1-yl)-3-chlorobenzoate

To a solution of 3-tert-butyl-1H-1,2,4-triazole (300 mg, 2.4 mmol, CAS: 96440-78-7) and methyl 3-chloro-4-fluoro-benzoate (1.36 g, 7.19 mmol) in DMF (15 mL) was added Na₂CO₃ (762 mg, 7.19 mmol). The mixture was stirred at 120 °C for 12 h. The reaction mixture was filtered and purified by reverse phase chromatograph (0.05% v/v FA) to get desired product (150 mg, 0.510 mmol, 22.4% yield) as light yellow solid. MS (ESI): m/z = 294.5 [M+H]⁺.

### Example A.80

### 4-(5-tert-Butyl-1,3,4-oxadiazol-2-yl)-3-fluoro-benzoic acid

To a solution of methyl 4-(5-tert-butyl-1,3,4-oxadiazol-2-yl)-3-fluoro-benzoate (900 mg, 3.23 mmol) in methanol (9 mL), THF (9 mL) and water (9 mL) was added NaOH (517 mg, 12.9 mmol), the mixture was stirred at 20 °C for 12 h, the mixture was concentrated to remove the organic solvent, the residue was acidified by HCl (4M aq.) to pH-4, then extracted with EtOAc (30 mL three times). The combined organic phase was dried over Na₂SO₄, concentrated to give the desired product (830 mg, 3.14 mmol, 97.1% yield) as white solid. MS (ESI): m/z = 265.1 [M +H]⁺.

### Step a) 2-(4-Bromo-2-fluoro-phenyl)-5-tert-butyl-1,3,4-oxadiazole

A solution of 4-bromo-2-fluorobenzoic acid (2.2 g, 10.05 mmol, CAS RN 112704-79-7) and 2,2-dimethylpropanehydrazide (1.17 g, 10.1 mmol, CAS RN 42826-42-6) in POCl₃ (50.0 mL, 10.1 mmol) was stirred at 80 °C for 12 h. The mixture was concentrated to remove most of POCl₃ first, and then the solution was poured into cold saturated sodium bicarbonate (300 mL), extracted with ethyl acetate (50 mL three times). The combined organic layers were dried over sodium sulfate. After concentration, the crude mixture was purified by silica gel column (PE: EtOAc=20:1) to give the desired product (1.3 g, 4.35 mmol, 43.3% yield) as off-white solid. MS (ESI): m/z = 298.9 [M+H]⁺.

### Step b) Methyl 4-(5-tert-butyl-1,3,4-oxadiazol-2-yl)-3-fluoro-benzoate

To a mixture of 2-(4-bromo-2-fluoro-phenyl)-5-tert-butyl-1,3,4-oxadiazole (1300 mg, 4.35 mmol) and triethylamine (1.21 mL, 8.69 mmol) in methanol (80 mL) was added Pd(dppf)Cl₂ (318 mg, 0.430 mmol) at 20 °C. The mixture was purged with carbon monoxide (50 psi) and stirred at 80 °C for 15 h. The mixture was filtered and the filtrate was concentrated in vacuo. The residue was purified by silica gel column (PE: EtOAc=20:1-10:1) to give the title compound (900 mg, 3.23 mmol, 74.4% yield) as off-white solid. MS (ESI): m/z = 279.1 [M +H]⁺.

### Example A.81

### 3-Fluoro-4-[5-(2-fluoro-1,1-dimethyl-ethyl)-1,3,4-oxadiazol-2-yl]benzoic acid

To a solution of methyl 3-fluoro-4-[5-(2-fluoro-1,1-dimethyl-ethyl)-1,3,4-oxadiazol-2-yl]benzoate (800 mg, 2.7 mmol) in methanol (10 mL), THF (10 mL) and water (10 mL) was added NaOH (432 mg, 10.8 mmol). The mixture was stirred at 20 °C for 12 h. The mixture was concentrated to remove the organic solvent, the residue was acidified by HCl (4 M) to pH-4, then extracted with EtOAc (30 mL three times), the combined organic phase was dried over Na₂SO₄, concentrated to give the desired product (750 mg, 2.66 mmol, 98.4% yield) as white solid. MS (ESI): m/z = 283.1 [M +H]⁺.

### Step a) tert-Butyl N-[(4-bromo-2-fluoro-benzoyl)amino]carbamate

To a solution of 4-bromo-2-fluorobenzoic acid (10.0 g, 45.7 mmol, CAS RN 112704-79-7), DIEA (23.9 mL, 136.98 mmol), HOBT (10.5 g, 68.5 mmol, 1.5 eq) and EDCI (10.6 g, 68.5 mmol) in DCM (150 mL) was added tert-butyl carbazate (6.34 g, 47.94 mmol, CAS RN 870-46-2). The mixture was stirred at 20 °C for 12 h, the mixture was washed with aq. Na₂CO₃ solution and brine, dried over Na₂SO₄, concentrated to give crude product (15 g, 45.02 mmol, 98.61% yield) as yellow oil. MS (ESI): m/z = 278.9 [M+H]⁺.

### Step b) 4-Bromo-2-fluoro-benzohydrazide

To a solution of tert-butyl N-[(4-bromo-2-fluoro-benzoyl)amino]carbamate (15.0 g, 45.0 mmol) in EtOAc (100 mL) was added HCl/Dioxane (100 mL, 4 M), the mixture was stirred at 20 °C for 2 h. The mixture was filtered and the filter cake was dissolved in water (100 mL), then basified by Na₂CO₃ to pH-9, white precipitate formed, filtered and dried the filter cake to give desired product (7.5 g, 32.2 mmol, 71.5% yield) as white solid. MS (ESI): m/z = 235.0 [M+H]⁺.

### Step c) 2-(4-Bromo-2-fluoro-phenyl)-5-(2-fluoro-1,1-dimethyl-ethyl)-1,3, 4-oxadiazole

A solution of 4-bromo-2-fluoro-benzohydrazide (2.3 g, 9.87 mmol) and 3-fluoro-2,2-dimethyl-propanoic acid (1.24 g, 10.4 mmol) in POCl₃ (50.0 mL, 9.87 mmol) was stirred at 80 °C for 12 h. The mixture was concentrated to remove most of the POCl₃, the residue was diluted with EtOAc (100 mL), washed with aq. Na₂CO₃ solution (100 mL) then brine, dried over Na₂SO₄ and concentrated to give the desired product (1.8 g, 5.68 mmol, 57.5% yield) as light yellow solid.

### Step d) Methyl 3-fluoro-4-[5-(2fluoro-1,1-dimethyl-ethyl)-1,3, 4-oxadiazol-2yl]benzoate

To a mixture of 2-(4-bromo-2-fluoro-phenyl)-5-(2-fluoro-1,1-dimethyl-ethyl)-1,3,4-oxadiazole (1700 mg, 5.36 mmol) and triethylamine (1.49 mL, 10.7 mmol) in methanol (80 mL) was added Pd(dppf)Cl₂ (392 mg, 0.540 mmol) at 20 °C. The mixture was purged with CO gas (50 psi) and stirred at 80 °C for 15 h. The mixture was filtered and the filtrate was concentrated in vacuo. The residue was purified by silica gel column (PE: EtOAc=20:1-10:1) to give the desired product (1.4 g, 4.73 mmol, 88.2% yield) as a white solid. MS (ESI): m/z = 297.0 [M +H]⁺.

### Example A.82

### 6-(5-tert-Butyl-1,3,4-oxadiazol-2-yl)-5-fluoro-pyridine-3-carboxylic acid

A mixture of methyl 6-(5-tert-butyl-1,3,4-oxadiazol-2-yl)-5-fluoro-pyridine-3-carboxylate (860 mg, 3.08 mmol) in sodium hydroxide/water (20.0 mL, 2 M) and THF (20 mL) was stirred at 20 °C for 16 h. The mixture was concentrated under vacuum to removed THF. The residue was acidified to pH~4 with 1 N HCl, white precipitate formed, filtered and the filter cake was washed with water and dried under vacuum to give desired product (650 mg, 2.45 mmol, 79.58% yield) as off-white solid. MS (ESI): m/z = 266.1 [M +H]⁺.

### Step a) 2-(5-Bromo-3-fluoro-2-pyridyl)-5-tert-butyl-1,3,4-oxadiazole

A solution of 5-bromo-3-fluoro-pyridine-2-carboxylic acid (2.0 g, 9.09 mmol, CAS RN 669066-91-5) and 2,2-dimethylpropanehydrazide (1056.0 mg, 9.09 mmol, CAS RN 42826-42-6) in POCl₃ (50.0 mL) was stirred at 90 °C for 12 h. The mixture was concentrated under vacuum to remove the POCl₃. The residue was diluted with EtOAc (100 mL), washed with aq. Na₂CO₃ (200 mL) and brine, dried over Na₂SO₄ and filtered. The filtrate was concentrated and purified by column chromatography (PE:EtOAc=20:1~10:1) to give desired product (1.14 g, 3.8 mmol, 41.8% yield) as off-white solid. MS (ESI): m/z = 301.9 [M+H]⁺.

### Step b) Methyl 6-(5-tert-butyl-1,3,4-oxadiazol-2-yl)-5-fluoro-pyridine-3-carboxylate

To a mixture of 2-(5-bromo-3-fluoro-2-pyridyl)-5-tert-butyl-1,3,4-oxadiazole (1.04 g, 3.47 mmol) and triethylamine (0.97 mL, 6.93 mmol) in methanol (30 mL) was added Pd(dppf)Cl₂ (254 mg, 0.350 mmol) at 20 °C. The mixture was purged with carbon monoxide (50 psi) and stirred at 80 °C for 15 h. The mixture was filtered and the filtrate was concentrated in vacuum. The residue was purified by silica gel column (PE:EtOAc=5:1) to give the desired product (910 mg, 3.26 mmol, 94% yield) as off-white solid. MS (ESI): m/z = 280.0 [M+H]⁺.

### Example A.83

### 3-Fluoro-4-[5-(2-fluoro-2-methyl-propyl)-1,3,4-oxadiazol-2-yl]benzoic acid

Generated in analogy to A.81, using 3-fluoro-3-methyl-butanoic acid as the acid in Step c (condensation-cyclization can be carried out stepwise if required, using standard techniques.)

### Example A.84

### 6-[2-(2,2-dimethylpropyl)tetrazol-5-yl]-5-fluoro-pyridine-3-carboxylic acid

To a solution of methyl 5-fluoro-6-(2-neopentyl-2H-tetrazol-5-yl)nicotinate (160 mg, 546 µmol) in methanol (4 mL) was added NaOH 5.0 M aqueous solution (218 µl, 1.09 mmol) and the reaction mixture was stirred at 55 °C for 16 h. Volatiles were removed in vacuo, the obtained crude residue was then partitioned between ethyl acetate and a mixture of 1: 1 brine: HCl 1M (aq.). The organic phase was collected and the aqueous phase was back-extracted with ethyl acetate. The combined organic phases were dried over sodium sulfate and evaporated down to dryness to yield 161 mg of the crude title compound, which was used without further purification. MS (ESI): m/z = 280.1 [M+H]+.

### Step a) methyl 5-fluoro-6-(2-neopentyl-2H-tetrazol-5-yl)nicotinate

To a solution of 2-neopentyl-5-(tributylstannyl)-2H-tetrazole (Example 74, Step c) (350 mg, 734 µmol) in dry DMF (5 mL) was added methyl 6-chloro-5-fluoronicotinate (CAS: 78686-78-9) (160 mg, 844 µmol), Pd(Ph₃P)₄ (42.4 mg, 36.7 µmol) and copper (I) iodide (6.99 mg, 36.7 µmol). The reaction mixture was then stirred at 110 °C for 16 h. Volatiles were removed in vacuo and the crude residue was directly purified by flash chromatography eluting with ethylacetate in heptane (5% to 30% gradient) to yield 168 mg of the title compound (contaminated with tin by-product), which was carried directly to the next step without further purification. MS (ESI): m/z = 294.2 [M+H]+.

In analogy to Example A.84, the following building blocks were generated from the commercially available nitriles (hydrolysis carried out in EtOH/NaOH 5M (aq.), 85 °C, 16 h).

| **Exam ple** | **Structure** | **Systematic Name** | **Starting material** | **MS (ESI): m/z** |
|---|---|---|---|---|
| A.85 | 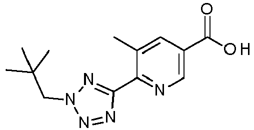 | 6-[2-(2,2-dimethylpropyl)tetr azol-5-yl]-5-methylpyridine-3-carboxylic acid | 6-chloro-5-methylnicotinonitril e (CAS: 66909-33-9) | 276.2 [M+H]⁺ |
| A. 86 | 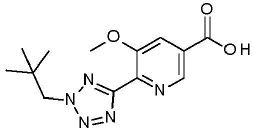 | 6-[2-(2,2-dimethylpropyl)tetr azol-5-yl]-5-methoxy-pyridine-3-carboxylic acid | 6-chloro-5-methoxynicotinonitr ile (CAS: 1256835-79-6) | 292.1 [M+H]⁺ |

### Example B.1

### 7-Chloro-2-(piperazin-1-yl)oxazolo[4,5-b] pyridine hydrochloride

A solution of tert-butyl 4-(7-chlorooxazolo[4,5-b]pyridin-2-yl)piperazine-1-carboxylate (135 mg, 398 µmol) in HCl (4.0 M solution in dioxane, 1.49 mL, 5.98 mmol) was stirred at r.t. for 2 h. The solvent was removed in vacuo to get the title compound (114 mg, 100%) as a white solid. MS (ESI): m/z = 239.1 [M+H]⁺

### Step a) 2-(4-(tert-Butoxycarbonyl)piperazin-1-yl)oxazolo[4,5-b]pyridine 4-oxide

To a solution of tert-butyl 4-(oxazolo[4,5-b]pyridin-2-yl)piperazine-1-carboxylate (610 mg, 2 mmol, CAS: 1255391-05-9) in CHCl₃ (6 mL) was added mCPBA (584 mg, 2.61 mmol) and the reaction mixture was stirred at r.t. for 14 h. Another portion of mCPBA (584 mg, 2.61 mmol) was added, and the reaction mixture was stirred at r.t. for another 18 h. The reaction was diluted with dichloromethane and extracted with aq. NaHCO₃ 1M solution, the organic phase was collected and the aqueous phase was back-extracted with dichloromethane. The combined organic phases were dried over sodium sulfate and evaporated down to dryness. The crude material was purified by flash chromatography with an eluent mixture of MeOH and dichloromethane (0% to 5% gradient) providing the title compound (212 mg, 33%). MS (ESI): m/z = 321.3 [M+H]⁺

### Step b) tert-Butyl 4-(7-chlorooxazolo[4,5-b]pyridin-2-yl)piperazine-1-carboxylate

To a solution of 2-(4-(tert-butoxycarbonyl)piperazin-1-yl)oxazolo[4,5-b]pyridine 4-oxide (200 mg, 624 µmol) in dry DMF (2 mL) cooled down to 0 °C was added oxalyl chloride (87.2 mg, 60.1 µL, 687 µmol) and the reaction mixture was stirred at 0 °C for 20 min. followed by 14 h at room temperature. Addition of a few drops of oxalyl chloride and the reaction was stirred at r.t. for another 14 h. The reaction was quenched by addition of a few drops of sat. aq. NH₄Cl solution, volatiles were removed in vacuo and the crude residue was partitioned between ethyl acetate and sat. aq. NaHCO₃. The organic phase was collected and the aqueous phase was back-extracted with ethyl acetate. The combined organic phases were dried over sodium sulfate and evaporated down to dryness. The crude material was purified by flash chromatography with an eluent mixture of methanol and dichloromethane (0% to 5%) providing the title compound (152 mg, 71.9%) as a white solid. MS (ESI): m/z = 339.1 [M+H]⁺

### Example B.2

### 5-Chloro-2-(piperazin-1-yl)oxazolo[4,5-b]pyridine hydrochloride

In analogy to the procedure described for example B.1 the title compound was prepared from tert-butyl 4-(5-chlorooxazolo[4,5-b]pyridin-2-yl)piperazine-1-carboxylate (1.16 g, 3.42 mmol) as a white solid (1.07 g, 100%). MS (ESI): m/z = 239.1 [M+H]⁺

### Step a) tert-Butyl 4-(5-chlorooxazolo[4,5-b]pyridin-2-yl)piperazine-1-carboxylate

To a solution of 5-chloro-2-(methylthio)oxazolo[4,5-b]pyridine (CAS 1783370-92-2, Aurora Building Blocks) (50 mg, 249 µmol) in 1,4-dioxane (0.5 mL) was added tert-butyl piperazine-1-carboxylate (46.4 mg, 249 µmol) and triethylamine (27.7 mg, 38.2 µL, 274 µmol). The reaction mixture was stirred at reflux for 16 h in a sealed vial. The reaction mixture was stirred at reflux for further 48 h. The mixture was quenched with 25 mL sat. aq. sodium thiosulfate sol. and extracted with EtOAc. The combined organics were washed with brine, dried (MgSO₄), filtered and concentrated to leave the crude product as an off-white solid. The crude product was purified by flash chromatography with an eluent mixture of EtOAc and heptane (0% - 80% gradient) providing the title compound (76.3 mg, 90.4%) as an off-white solid. MS (ESI): m/z = 339.2 [M+H]⁺

### Example B.3

### 5-Iodo-2-(piperazin-1-yl)oxazolo[4,5-b]pyridine hydrochloride

In analogy to the procedure described for example B.1, the title compound was prepared from tert-butyl 4-(5-iodooxazolo[4,5-b]pyridin-2-yl)piperazine-1-carboxylate (960 mg, 2.23 mmol) as solid (818 mg, 100%). MS (ESI): m/z = 331.1 [M+H]⁺

### Step a) 2-(4-(tert-Butoxycarbonyl)piperazin-1-yl)-5-iodooxazolo[4,5-b]pyridine 4-oxide

A suspension of 2-(4-(tert-butoxycarbonyl)piperazin-1-yl)oxazolo[4,5-b]pyridine 4-oxide (B.1, Step a) (1.33 g, 4.15 mmol) in dry THF (65 mL) cooled down to -45 °C under an inert atmosphere was added isopropylmagnesium chloride 2.0M solution in THF (3.11 mL, 6.23 mmol) and the reaction mixture was stirred at -45 °C for 2 h. Iodine (1.58 g, 6.23 mmol) was added to the reaction mixture then the reaction was let slowly warm up to r.t. and stirred at r.t. for 60 min. Reaction was quenched by addition of a few drops of sat. aq. NH₄Cl solution, volatiles were removed in vacuo and the obtained crude residue was partitioned between ethyl acetate and a mixture of 15 mL aq. NaHCO₃ 1M/10 mL aq. sodium thiosulfate solution (0.1 mol/L). After extraction the organic phase was collected and the aqueous phase was back-extracted twice with ethyl acetate. The combined organic phases were dried over sodium sulfate and evaporated down to dryness. The crude material was purified by flash chromatography with an eluent mixture of methanol and dichloromethane (1% - 5% gradient) providing the title compound (1.23 g, 66.4%) as a dark red solid. MS (ESI): m/z = 447.2 [M+H]⁺

### Step b) tert-Butyl 4-(5-iodooxazolo[4,5-b]pyridin-2-yl)piperazine-1-carboxylate

To a suspension of 2-(4-(tert-butoxycarbonyl)piperazin-1-yl)-5-iodooxazolo[4,5-b]pyridine 4-oxide (1.22 g, 2.73 mmol) in CH₃CN (20 mL) was added 4,4,4',4',5,5,5',5'-octamethyl-2,2'-bi(1,3,2-dioxaborolane) (833 mg, 3.28 mmol) and the reaction mixture was stirred at 95 °C for 16 h. Volatiles were removed in vacuo and the crude residue was directly purified by flash chromatography with an eluent mixture of ethyl acetate and heptane (5% - 40% gradient) providing the title compound (962 mg, 81.8%) as a yellow solid. MS (ESI): m/z = 431.2 [M+H]⁺

### Example B.4

### 5-Chloro-2-(3-methylpiperazin-1-yl)oxazolo[4,5-b]pyridine

To a mixture of tert-butyl 4-(5-chlorooxazolo[4,5-b]pyridin-2-yl)-2-methyl-piperazine-1-carboxylate (310 mg, 0.880 mmol) in DCM (5 mL) was added trifluoroacetic acid (5.0 mL, 64.9 mmol) at 20 °C. Then the mixture was stirred at 20 °C for 2 h. The mixture was concentrated in vacuo to dryness. To the residue was added 10 mL saturated aq. NaHCO₃ solution and was extracted with ethyl acetate. Combined organic layers were dried over anhydrous sodium sulfate, filter and concentrated in vacuo to provide the title compound (200 mg, 0.790 mmol, 90.08%) as a yellow solid. MS (ESI): m/z = 253.0 [M+H]⁺

### Step a) tert-Butyl 4-(5-chlorooxazolo[4,5-b]pyridin-2-yl)-2-methyl-piperazine-1-carboxylate

To a mixture of 5-chloro-2-(methylthio)oxazolo[4,5-b]pyridine (0.5 g, 2.49 mmol) and N-Boc-2-methylpiperazine (0.75 g, 3.74 mmol) in DMSO (10 mL) was added N,N-diisopropylethylamine (0.65 mL, 3.74 mmol) at 20 °C. Then the mixture was stirred at 120 °C for 20 h. The mixture was added to 50 mL water and extracted with ethyl acetate. Combined organic layers were washed with brine, dried over anhydrous sodium sulfate, filtered and concentrated in vacuo. The crude product was purified by flash chromatography with an eluent mixture of EtOAc and petroleum ether (50%) providing the title compound (310 mg, 0.88 mmol, 35.3%) as a light yellow solid. MS (ESI): m/z = 353.1 [M+H]⁺

### Example B.5

### 5-Chloro-2-(2-methylpiperazin-1-yl)oxazolo[4,5-b]pyridine

In analogy to the procedure described for example B.4, the title compound was prepared from 5-chloro-2-methylsulfanyl-oxazolo[4,5-b]pyridine (0.5 g, 2.5 mmol) and 4-N-Boc-2-methylpiperazine (0.749 g, 3.7 mmol) as a yellow oil (70 mg, 0.28 mmol, 75.2 %). MS (ESI): m/z = 253.0 [M+H]⁺

### Example B.6

### (4-Nitrophenyl) 4-(5-chloro-[1,3]oxazolo[4,5-b]pyridin-2-yl)piperazine-1-carboxylate

To a solution of 5-chloro-2-(piperazin-1-yl)oxazolo[4,5-b]pyridine (Example B.2) (219 mg, 918 µmol) and TEA (279 mg, 384 µL, 2.75 mmol) in DCM (3 mL), cooled to 0 °C with an ice bath, was added 4-nitrophenyl carbonochloridate (203 mg, 1.01 mmol) in three portions and the reaction mixture was stirred at rt for 2 h. The crude material was purified by flash chromatography with an eluent mixture of EtOAc and heptane (0% to 100% gradient) providing the title compound (60 mg, 16.2%) as an off-white powder. MS (ESI): m/z = 404.2 [M+H]⁺

### Example B.7

### 4-Nitrophenyl 4-(5-methyloxazolo[4,5-b]pyridin-2-yl)piperazine-1-carboxylate

To a solution of 5-methyl-2-(piperazin-1-yl)oxazolo[4,5-b]pyridine (CAS: 1035840-99-3, 600 mg, 2.75 mmol) in dry CH₂Cl₂ (10 mL) cooled down to 0°C was added DIPEA (426 mg, 576 µl, 3.3 mmol) and 4-nitrophenyl carbonochloridate (582 mg, 2.89 mmol). The reaction mixture was then stirred at r.t. for 2 hours. The reaction mixture was diluted with dichloromethane and transferred into a separating funnel for extraction with aq. NaHCO₃ 1M solution. The organic phase was collected, dried over sodium sulfate and evaporated to get the title compound (1.02 g, 96.8%). MS (ESI): m/z = 384.3 [M+H]⁺

### Example B.8

### 4-(5-Methyloxazolo[4,5-b]pyridin-2-yl)piperazine-1-carbonyl chloride

To a solution of triphosgene (224 mg, 0.760 mmol) and sodium hydrogencarbonate (385 mg, 4.58 mmol) in DCM (10 mL) was added 5-methyl-2-piperazin-1-yl-oxazolo[4,5-b]pyridine (500 mg, 2.29 mmol) at -10 °C and the mixture was stirred at 20 °C for 16 h. The mixture was filtered and the filtrate was concentrated to provide the title crude compound (620 mg, 2.21 mmol, 96.4% yield) as a yellow solid which was used directly without further characterization.

### Example B.9

### 5-Chloro-2-(3,8-diazabicyclo [3.2.1] octan-8-yl)oxazolo[4,5-b]pyridine

A mixture of 9H-fluoren-9-ylmethyl 8-(5-chlorooxazolo[4,5-b]pyridin-2-yl)-3,8-diazabicyclo [3.2.1]octane-3-carboxylate (80.0 mg, 0.160 mmol) and piperidine (69.95 mg, 0.820 mmol) in DCM (8 mL) was stirred at 20 °C for 12 h. The mixture was concentrated under vacuum. The residue was purified by Prep-HPLC (0.5% v/v conc. NH₄OH) and concentrated under vacuum to give the desired product (35 mg, 0.130 mmol, 80.48% yield) as light yellow solid. MS (ESI): m/z = 265.0 [M+H]⁺.

### Step a) 6-Chloro-2-nitro-pyridin-3-ol

To a solution of 2-chloro-5-hydroxypyridine (25.0 g, 192.99 mmol, CAS RN 41288-96-4) in conc. H₂SO₄ (125 mL) was added KNO₃ (29.2 g, 289 mmol) portion-wise under ice-cooling (0 - 40 °C) over 1.5 h. The the mixture was stirred at 25 °C for 12 h. Two batches of the reaction mixture were poured into ice-water mixture (800 mL) and stirred for 30 min. The precipitate was collected by filtration, washed with water (100 mL three times) and dried under vacuum to give the desired product (25.3 g, 145 mmol, 75.1% yield) as yellow solid.

### Step b) 2-Amino-6-chloro-pyridin-3-ol

To a solution of 6-chloro-2-nitro-pyridin-3-ol (25.3 g, 145 mmol) in THF (200 mL) was added a solution of NH₄Cl (46.1 g, 870 mmol) in water (220 mL), and then zinc dust (47.1 g, 725 mmol) was added portion-wise at 0 °C for 0.5 h. Then the mixture was stirred at 20 °C for 12 h. The mixture was filtered. The filter cake was washed with EtOAc (300 mL three times). The filtered was extracted twice with EA. The combined organic was washed with brine, dried over Na₂SO₄ and filtered. The filtrate was concentrated under vacuum and purified by column chromatography (PE:EtOAc=10:1 to 3:1) to give the desired product (10.8 g, 74.7 mmol, 51.5% yield) as light yellow solid.

### Step c) 5-Chloro-3H-oxazolo[4,5-b]pyridine-2-thione

To a solution of 2-amino-6-chloro-pyridin-3-ol (2.0 g, 13.8 mmol) in DMF (10 mL) was added 1,1'-thiocarbonyldiimidazole (2.84 g, 15.9 mmol) at 0 - 10°C. The mixture was stirred at 20 °C for 16 h. The mixture was poured into water and adjusted to pH=5-6 with 1 N aq. HCl. The mixture was extracted twice with EtOAc (100 mL). The combined organic layers were washed with brine, dried over Na₂SO₄ and filtered. The filtrate was concentrated under vacuum to give the desired product (2.1 g, 11.3 mmol, 81.3% yield) as light yellow solid. MS (ESI): m/z = 187.0 [M+H]⁺.

### Step d) 2,5-Dichlorooxazolo[4,5-b]pyridine

A mixture of 5-chloro-3H-oxazolo[4,5-b]pyridine-2-thione (1.0 g, 5.36 mmol) and DMF (0.500 mL) in oxalyl chloride (10.0 mL, 5.36 mmol) was stirred at 20 °C for 3 h. The mixture was poured into water and extracted three times with EtOAc. The combined organic layers were washed with brine, dried over Na₂SO₄ and filtered. The filtrate was concentrated under vacuum to give the desired product (720 mg, 3.81 mmol, 71.1% yield) as off-white solid.

### Step e) O8-tert-Butyl O3-(9H-fluoren-9-ylmethyl) 3,8-diazabicyclo[3.2.1]octane-3,8-dicarboxylate

A mixture of tert-butyl 3,8-diazabicyclo[3.2.1]octane-8-carboxylate (200 mg, 0.940 mmol), FMOC-OSU (350 mg, 1.04 mmol) and sodium carbonate (150 mg, 1.41 mmol) in ACN (4 mL) and water (2 mL) was stirred at 25 °C for 2 h. The mixture was poured into water (2 mL) and extracted with EtOAc (5 mL twice). The combined organic layers were washed with brine, dried over Na₂SO₄ and filtered. The filtrate was concentrated under vacuum to give the crude desired product (350 mg, 0.810 mmol, 85.5% yield) as a colorless gum.

### Step f) 9H-Fluoren-9-ylmethyl 3,8-diazabicyclo[3.2.1]octane-3-carboxylate

A mixture of O8-tert-butyl O3-(9H-fluoren-9-ylmethyl) 3,8-diazabicyclo[3.2.1]octane-3,8-dicarboxylate (350 mg, 0.810 mmol), TFA (1.0 mL) and in DCM (10 mL) was stirred at 100 °C for 16 h. The mixture was concentrated under vacuum to give 450 mg of the crude product as light brown gum. 300 mg of the crude was diluted with EtOAc and the adjusted to pH=8-9 with saturated aq. NaHCO₃ solution. The aqueous layer was extracted three times with EtOAc. The combined organic layer was washed with brine, dried over Na₂SO₄ and filtered. The filtrate was concentrated under vacuum to give the crude product (200 mg, 0.600 mmol, 89.4% yield) as light yellow solid. MS (ESI): m/z = 335.2 [M+H]⁺.

### Step g) 9H-Fluoren-9ylmethyl 8-(5-chlorooxazolo [4,5-b]pyridin-2-yl)-3,8-diazabicyclo[3.2.1]octane-3-carboxylate

A mixture of 2,5-dichlorooxazolo[4,5-b]pyridine (136 mg, 0.720 mmol), and 9H-fluoren-9-ylmethyl 3,8-diazabicyclo[3.2.1]octane-3-carboxylate (200 mg, 0.600 mmol) in DMF (5 mL) was stirred at 20 °C for 12 h. Then TEA (0.17 mL, 1.2 mmol) was added and the mixture was stirred at 20 °C for 1 h. The mixture was poured into water and extracted three times with EtOAc. The combined organic layer was washed with brine, dried over Na₂SO₄ and filtered. The filtrate was concentrated and purified by column chromatography (PE:EtOAc=10:1 to 3:1) to give the desired product (85 mg, 0.170 mmol, 29.2% yield) as light yellow gum. MS (ESI): m/z = 487.2 [M+H]⁺.

### Example B.10

### 5-Chloro-2-(3,8-diazabicyclo[3.2.1]octan-3-yl)oxazolo[4,5-b]pyridine (trifluoroacetic acid salt)

A mixture of tert-butyl 3-(5-chlorooxazolo[4,5-b]pyridin-2-yl)-3,8-diazabicyclo[3.2.1]octane-8-carboxylate (250 mg, 0.690 mmol) and TFA (1.0 mL) in DCM (10 mL) was stirred at 25 °C for 12 h.. The mixture was concentrated under vacuum to give the desired product (260 mg, 0.690 mmol, 100.18% yield) as light brown oil as crude. MS (ESI): m/z = 265.1 [M+H]⁺.

### Step a) tert-Butyl 3-(5-chlorooxazolo[4,5-b]pyridin-2-yl)-3,8-diazabicyclo[3.2.1]octane-8-carboxylate

A mixture of 5-chloro-2-methylsulfanyl-oxazolo[4,5-b]pyridine (250 mg, 1.25 mmol) (CAS 1783370-92-2, Aurora Building Blocks), tert-butyl 3,8-diazabicyclo[3.2.1]octane-8-carboxylate (317 mg, 1.5 mmol) and DIPEA (241 mg, 1.87 mmol) in DMF (5 mL) was stirred at 100 °C for 16 h. The mixture was poured into water and extracted three times with EtOAc. The combined organic layer was washed with brine, dried over Na₂SO₄ and filtered. The filtrate was concentrated under vacuum and purified by Prep-HPLC (0.225% v/v FA) to give the desired product (255 mg, 0.700 mmol, 56.1% yield) as light yellow solid. MS (ESI): m/z =365.1 [M+H]⁺.

### Example B.11

### [1-(5-Chlorooxazolo[4,5-b]pyridin-2-yl)piperazin-2-yl] methanol (2,2,2-trifluoroacetic acid salt)

A mixture of tert-butyl 4-(5-chlorooxazolo[4,5-b]pyridin-2-yl)-3-(hydroxymethyl)piperazine-1-carboxylate (320 mg, 0.870 mmol) and TFA (2.0 mL) in DCM (20 mL) was stirred at 20 °C for 12 h. The mixture was concentrated under vacuum to give the crude product (390 mg, 1.02 mmol, 117 % yield) as light brown gum. MS (ESI): m/z = 269.1 [M+H]⁺.

### Step a) tert-Butyl 4-(5-chlorooxazolo[4,5-b]pyridin-2-yl)-3-(hydroxymethyl)piperazine-1-carboxylate

A mixture of 5-chloro-2-methylsulfanyl-oxazolo[4,5-b]pyridine (1.02 g, 5.09 mmol), 4-N-BOC-2-hydroxymethylpiperazine (1.0 g, 4.62 mmol, CAS RN 301673-16-5) and Na₂CO₃ (1.47 g, 13.87 mmol) in DMF (20 mL) was stirred at 100 °C for 36 h. The mixture was poured into water and extracted with a mixture of DCM:MeOH (1:10, 50 mL for three times). The combined organic layer was washed with brine, dried over Na₂SO₄ and filtered. The filtrate was concentrated under vacuum and purified by Prep-HPLC (0.225% v/v FA) and lyophilized to give (330 mg, 0.890 mmol, 20.5% yield) as light yellow solid. MS (ESI): m/z = 369.1 [M+H]⁺.

### Example B.12

### (4-(5-Chlorooxazolo[4,5-b] pyridin-2-yl)piperazin-2-yl)methanol(trifluoroacetic acid salt)

To a solution of tert-butyl 4-(5-chlorooxazolo[4,5-b]pyridin-2-yl)-2-(hydroxymethyl)piperazine-1-carboxylate (250 mg, 0.68 mmol) in DCM (6.25 mL) was added TFA (1.0 mL). The mixture was stirred at 25 °C for 12 hrs. The reaction mixture was concentrated in vacuum to get crude product (600 mg) as light yellow oil. MS (ESI): m/z = 269.0 [M+H-TFA]⁺.

### Step a) tert-Butyl 4-(5-chlorooxazolo[4,5-b]pyridin-2-yl)-2-(hydroxymethyl)piperazine-1-carboxylate

To a solution of tert-butyl 2-(hydroxymethyl)piperazine-1-carboxylate (1.08 g, 4.98 mmol, CAS RN 205434-75-9) and 5-chloro-2-methylsulfanyl-oxazolo[4,5-b]pyridine (1.00 g, 4.98 mmol) in DMF (20 mL) was added Na₂CO₃ (2.64 g, 24.9 mmol), the reaction was stirred at 100 °C for 12 h. The reaction mixture was diluted with water (100 mL) and extracted with EtOAc for three times, the combined organic layer was washed with water for three times and brine, dried over sodium sulfate and concentrated in vacuum to get yellow residue, which was purified with reverse phase chromatograph (0.05% v/v FA) to get desired product (1.40 g, 3.80 mmol, 76.2% yield) as light yellow solid. MS (ESI): m/z = 369.1 [M+H]⁺.

### Example B.13

### 5-Fluoro-2-piperazin-1-yl-oxazolo[4,5-b]pyridine ( 2,2,2-trifluoroacetic acid salt)

A mixture of tert-butyl 4-(5-fluorooxazolo[4,5-b]pyridin-2-yl)piperazine-1-carboxylate (540 mg, 1.68 mmol) in DCM (54 mL) was added TFA (0.05 mL). The mixture was stirred at 20 °C for 12 h. The mixture was concentrated under vacuum to give the crude desired product (570 mg, crude) as light brown gum. MS (ESI): m/z = 223.1 [M+H]⁺.

### Step a) 6-Fluoro-2-nitro-pyridin-3-ol

To a solution of 2-fluoro-5-hydroxypyridine (2.0 g, 17.7 mmol) in conc.H₂SO₄ (125 mL, 17.7 mmol) was added KNO₃ (2.68 g, 26.5 mmol) portion-wise under ice-cooling (0-40 °C) over 1.5 h. The mixture was stirred at 25 °C for 12 h. The mixture was poured into ice-water and stirred for 30 min. The mixture was extracted with EtOAc (100 mL three times). The combined organic layer was washed with brine (50 mL three times), dried over Na₂SO₄ and filtered. The filtrate was concentrated under vacuum to give the desired product (2.2 g, 13.9 mmol, 78.7% yield) as yellow solid.

### Step b) 2-Amino-6-fluoro-pyridin-3-ol

To a solution of 6-fluoro-2-nitro-pyridin-3-ol (2.2 g, 13.9 mmol) in THF (20 mL) was added a solution of NH₄Cl (4.43 g, 83.5 mmol) in water (20 mL), and then zinc dust (4.52 g, 69.6 mmol) was added portion-wise at 0 °C for 0.5 h. The mixture was stirred at 20 °C for 12 h. The mixture was filtered. The filter cake was washed with EtOAc (300 mL three times). The filtered was extracted twice with EtOAc. The combined organic layer was washed with brine, fried over Na₂SO₄ and filtered. The filtrate was concentrated under vacuum and purified by column chromatography (PE: EA=10:1~3:1) to give the desired product (0.950 g, 7.42 mmol, 53.29% yield) as light yellow solid.

### Step c) 5-Fluoro-3H-oxazolo[4,5-b]pyridine-2-thione

To a solution of 2-amino-6-fluoro-pyridin-3-ol (500 mg, 3.9 mmol) in DMF (10 mL) was added 1,1'-thiocarbonyldiimidazole (800 mg, 4.49 mmol) at 0-10°C. The mixture was then stirred at 20 °C for 16 h. The mixture was poured into water and adjusted to pH = 5-6 with 1 N HCl. The mixture was filtered and extracted twice with EA (100 mL). The combined organic layer was washed with brine, dried over Na₂SO₄ and filtered. The filtrate was concentrated under vacuum to give the desired product (420 mg, 2.47 mmol, 63.2% yield) as light brown solid. MS (ESI): m/z = 171.1 [M+H]⁺.

### Step d) 2-Chloro-5-fluoro-oxazolo[4,5-b]pyridine

A mixture of 5-fluoro-3H-oxazolo[4,5-b]pyridine-2-thione (400 mg, 2.35 mmol) and DMF (10 mL) in oxalyl chloride (5.0 mL, 2.35 mmol) was stirred at 20 °C for 3 h. The mixture was concentrated and the residue was diluted with water (30 mL) and extracted with EtOAc (30 mL three times). The combined organic was washed with brine, dried over Na₂SO₄ and filtered. The filtrate was concentrated under vacuum to give the desired product (375 mg, 2.17 mmol, 92.46% yield) as light brown solid.

### Step e) tert-Butyl 4-(5-fluorooxazolo[4,5-b]pyridin-2-yl)piperazine-1-carboxylate

A mixture of 2-chloro-5-fluoro-oxazolo[4,5-b]pyridine (370 mg, 2.14 mmol), TEA (0.9 mL, 6.43 mmol) and 1-BOC-piperazine (479 mg, 2.57 mmol, CAS RN 143238-38-4) in DMF (10 mL) was stirred at 20 °C for 12 h. The mixture was poured into water and extracted three times with EtOAc. The combined organic was washed with brine, dried over Na₂SO₄ and filtered. The filtrated was concentrated under vacuum to give the desired product (550 mg, 1.71 mmol, 79.57% yield) as light yellow solid. MS (ESI): m/z = 323.2 [M+H]⁺.

### Example C.1

### 1-[(3-Chloro-5-iodophenyl)carbonyl]-4-{5-methyl-[1,3]oxazolo[4,5-b]pyridin-2-yl} piperazine

To a solution of 1-(5-methyl-[1,3]oxazolo[4,5-b]pyridin-2-yl)piperazine, trifluoroacetic acid (550 mg, 1.66 mmol) in DMF (5 mL) was added triethylamine (0.92 mL, 6.62 mmol) and stirred for 10 minutes at r.t., followed by the addition of 3-chloro-5-iodobenzoic acid (468 mg, 1.66 mmol) and HATU (944 mg, 2.48 mmol). The mixture was then stirred for 17 h at the same temperature. The reaction mixture was diluted with EtOAc (50 mL), and water (50 mL). The solid part was filtered and washed with EtOAc and dried in vacuo providing the title compound (710 mg, 89%) as an off white solid. MS (ESI): m/z = 482.6 [M+H]⁺

Examples C.6 - C.8, C. 11 - C.26, C.28 - C.31, C.33 - C.34, C.36, C.38, C.40, C.41, C.43, C.44, C.46, C.47, C.50 - C.51, C.53 and C.55 of the following table were prepared in analogy to C. 1 using HATU or T₃P as coupling reagent.

| **Ex.** | **Systematic Name** | **Starting material 1** | **Starting material 2** | **Coupling reagent** | **MS (ESI): m/z** |
|---|---|---|---|---|---|
| C.6 | (4-iodophenyl)(4-(oxazolo[4,5-b]pyridin-2-yl)piperazin-1-yl)methanone | 2-piperazin-1-yloxazolo[4,5-b]pyridine | 4-iodobenzoic acid | HATU | 435.1 [M+H]⁺ |
| C.7 | (4-iodophenyl)(4-(5-methyloxazolo[4,5- b]pyridin-2-yl)piperazin-1-yl)methanone | 5-methyl-2-(piperazin-1-yl)oxazolo[4,5 -b]pyridine | 4-iodobenzoic acid | HATU | 449.1 [M+H]⁺ |
| C.8 | 1-({3-bromo-4-[5-(2,2-dimethylpropyl)-1,2,4-oxadiazol-3-yl]phenyl}carbonyl )-4-{5-methyl-[1,3]oxazolo[4,5-b]pyridin-2-yl}piperazine | 5-methyl-2-(piperazin-1-yl)oxazolo[4,5 -b]pyridine | 3-bromo-4-[5-(2,2-dimethylprop yl)-1,2,4-oxadiazol-3-yl]benzoic acid (A.14) | HATU | 539.0 [M+H]⁺ |
| C.11 | 1-{[4-bromo-3-(1H-imidazol-2-yl)phenyl]carbonyl }-4-{5-methyl-[1,3]oxazolo[4,5-b]pyridin-2-yl}piperazine | 5-methyl-2-(piperazin-1-yl)oxazolo[4,5 -b]pyridine | 4-bromo-3-(1H-imidazol-2-yl)benzoic acid (A.15) | HATU | 467.4 [M+H]⁺ |
| C.12 | (4-(5-chlorooxazolo[4,5-b]pyridin-2-yl)piperazin-1-yl)(4-iodophenyl)methan one | 5-chloro-2-(piperazin-1-yl)oxazolo[4,5 -b]pyridine hydrochloride (B.2) | 4-iodobenzoic acid | HATU | 469.1 [M+H]⁺ |
| C.13 | (6-fluoropyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 6-fluoronicotini c acid | HATU | 342.2 [M+H]⁺ |
| C.14 | (6-fluoro-5-methylpyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 6-fluoro-5-methylnicotin ic acid | HATU | 356.3 [M+H]⁺ |
| C.15 | (2-fluoro-4-iodophenyl)(4-(5-methyloxazolo[4,5- b]pyridin-2-yl)piperazin-1-yl)methanone | 5-methyl-2-(piperazin-1-yl)oxazolo[4,5 -b]pyridine | 2-fluoro-4-iodobenzoic acid | HATU | 467.1 [M+H]⁺ |
| C.16 | (3-fluoro-4-iodophenyl)(4-(5-methyloxazolo[4,5- b]pyridin-2-yl)piperazin-1-yl)methanone | 5-methyl-2-(piperazin-1-yl)oxazolo[4,5 -b]pyridine | 3-fluoro-4-iodobenzoic acid | HATU | 467.1 [M+H]⁺ |
| C.17 | (4-chloro-5,6-difluoropyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 4-chloro-5,6-difluoronicoti nic acid | HATU | 394.2 [M+H]⁺ |
| C.18 | (6-chloropyridazin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 6-chloropyridaz ine-3-carboxylic acid | HATU | 359.2 [M+H]⁺ |
| C.19 | (2-chloropyrimidin-5-yl)(4-(5-methyloxazolo[4,5- b]pyridin-2-yl)piperazin-1-yl)methanone | 5-methyl-2-(piperazin-1-yl)oxazolo[4,5 -b]pyridine | 2-chloropyrimi dine-5-carboxylic acid | HATU | 359.2 [M+H]⁺ |
| C.20 | (6-fluoro-4-methylpyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 6-fluoro-4-methylnicotin ic acid | HATU | 356.2 [M+H]⁺ |
| C.21 | (5,6-difluoropyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 5,6-difluoronicoti nic acid | HATU | 360.2 [M+H]⁺ |
| C.22 | (4-iodo-2-methylphenyl)(4-(5-methyloxazolo[4,5- b]pyridin-2-yl)piperazin-1-yl)methanone | 5-methyl-2-(piperazin-1-yl)oxazolo[4,5 -b]pyridine | 4-iodo-2-methylbenzoi c acid | HATU | 463.1 [M+H]⁺ |
| C.23 | (2,6-difluoro-4-iodophenyl)(4-(5-methyloxazolo[4,5- b]pyridin-2-yl)piperazin-1-yl)methanone | 5-methyl-2-(piperazin-1-yl)oxazolo[4,5 -b]pyridine | 2,6-difluoro-4-iodobenzoic acid | HATU | 463.1 [M+H]⁺ |
| C.24 | (2-chloro-4-iodophenyl)(4-(5-methyloxazolo[4,5- b]pyridin-2-yl)piperazin-1-yl)methanone | 5-methyl-2-(piperazin-1-yl)oxazolo[4,5 -b]pyridine | 2-chloro-4-iodobenzoic acid | HATU | 483.1 [M+H]⁺ |
| C.25 | (5-chloro-6-fluoropyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 5-chloro-6-fluoronicotini c acid | HATU | 376.1 [M+H]⁺ |
| C.26 | (4-(5-chlorooxazolo[4,5- b]pyridin-2-yl)piperazin-1-yl)(5,6-difluoropyridin-3-yl)methanone | 5-chloro-2-(piperazin-1-yl)oxazolo[4,5 -b]pyridine hydrochloride (B.2) | 5,6-difluoronicoti nic acid | HATU | 380.1 [M+H]⁺ |
| C.28 | (5-chloro-6-fluoropyridin-3-yl)(4-(5-chlorooxazolo[4,5- b]pyridin-2-yl)piperazin-1-yl)methanone | 5-chloro-2-(piperazin-1-yl)oxazolo[4,5 -b]pyridine hydrochloride (B.2) | 5-chloro-6-fluoronicotini c acid | HATU | 396.1 [M+H]⁺ |
| C.29 | (4-(5-chlorooxazolo[4,5-b]pyridin-2-yl)piperazin-1-yl)(6-fluoro-5-methylpyridin-3-yl)methanone | 5-chloro-2-(piperazin-1-yl)oxazolo[4,5 -b]pyridine hydrochloride (B.2) | 6-fluoro-5-methylnicotin ic acid | HATU | 376.1 [M+H]⁺ |
| C.30 | (6-chloro-5-(trifluoromethyl)py ridin-3-yl)(4-(5-chlorooxazolo[4,5- b]pyridin-2-yl)piperazin-1-yl)methanone | 5-chloro-2-(piperazin-1-yl)oxazolo[4,5 -b]pyridine hydrochloride (B.2) | 6-chloro-5-(trifluoromet hyl)nicotinic acid | HATU | 446.1 [M+H]⁺ |
| C.31 | 5-(4-(5-chlorooxazolo[4,5-b]pyridin-2-yl)piperazine-1-carbonyl)-2-ethynylbenzonitrile | 5-chloro-2-(piperazin-1-yl)oxazolo[4,5 -b]pyridine hydrochloride (B.2) | 3-cyano-4-ethynylbenzo ic acid (A.57) | HATU | 392.2 [M+H]⁺ |
| C.33 | (5-chloro-6-methyl-pyrazin-2-yl)-[4-(5-methyloxazolo[4,5- b]pyridin-2-yl)piperazin-1-yl]methanone | 5-methyl-2-(piperazin-1-yl)oxazolo[4,5 -b]pyridine | 5-chloro-6-methylpyrazine-2-carboxylic acid | T3P | 373.0 [M+H]⁺ |
| C.34 | 2-ethynyl-5-(4-(5-methyloxazolo[4,5 - b]pyridin-2-yl)piperazine-1-carbonyl)benzonitri le | 5-methyl-2-(piperazin-1-yl)oxazolo[4,5 -b]pyridine | 3-cyano-4-ethynylbenzo ic acid (A.57) | HATU | 372.2 [M+H]⁺ |
| C.36 | (4-(5-chlorooxazolo[4,5 - b]pyridin-2-yl)piperazin-1-yl)(4-ethynyl-3-methoxyphenyl)me thanone | 5-chloro-2-(piperazin-1-yl)oxazolo[4,5 -b]pyridine hydrochloride (B.2) | 4-Ethynyl-3-methoxybenz oic acid | HATU | 397.2 [M+H]⁺ |
| C.38 | (4-ethynyl-3-methoxyphenyl)(4-(5-methyloxazolo[4,5- b]pyridin-2-yl)piperazin-1-yl)methanone | 5-methyl-2-(piperazin-1-yl)oxazolo[4,5 -b]pyridine | 4-Ethynyl-3-methoxybenz oic acid | HATU | 377.2 [M+H]⁺ |
| C.40 | (6-chloro-5-methoxy-3-pyridyl)-[4-(5-methyloxazolo[4,5 - b]pyridin-2-yl)piperazin-1-yl]methanone | 5-methyl-2-(piperazin-1-yl)oxazolo[4,5 -b]pyridine | 6-chloro-5-methoxy-pyridine-3-carboxylic acid | T3P | 388.2 [M+H]⁺ |
| C.41 | (4-(5-chlorooxazolo[4,5-b]pyridin-2-yl)piperazin-1-yl)(6-ethynyl-5-fluoropyridin-3-yl)methanone | 5-chloro-2-(piperazin-1-yl)oxazolo[4,5 -b]pyridine hydrochloride (B.2) | 6-ethynyl-5-fluoronicotini c acid (A.61) | HATU | 386.2 [M+H]⁺ |
| C.43 | (4-(5-chlorooxazolo[4,5-b]pyridin-2-yl)piperazin-1-yl)(4-ethynyl-3-methylphenyl)meth anone | 5-chloro-2-(piperazin-1-yl)oxazolo[4,5 -b]pyridine hydrochloride (B.2) | 4-ethynyl-3-methylbenzoi c acid | HATU | 381.2 [M+H]⁺ |
| C.44 | (6-ethynyl-5-fluoropyridin-3-yl)(4-(5-methyloxazolo[4,5- b]pyridin-2-yl)piperazin-1-yl)methanone | 5-methyl-2-(piperazin-1-yl)oxazolo[4,5 -b]pyridine | 6-ethynyl-5-fluoronicotini c acid (A.61) | HATU | 366.2 [M+H]⁺ |
| C.46 | (6-bromo-5-methyl-3-pyridyl)-[4-(5-chlorooxazolo[4,5- b]pyridin-2-yl)piperazin-1-yl] methanone | 5-chloro-2-(piperazin-1-yl)oxazolo[4,5 -b]pyridine hydrochloride (B.2) | 6-Bromo-5-methyl-3-pyridinecarbo xylic acid | HATU | 438.0 [M+H]⁺ |
| C.47 | (6-bromo-5-methyl-3-pyridyl)-[4-(5-methyloxazolo[4,5 - b]pyridin-2-yl)piperazin-1-yl]methanone | 5-methyl-2-(piperazin-1-yl)oxazolo[4,5 -b]pyridine CAS 1035840-99-3 | 6-Bromo-5-methyl-3-pyridinecarbo xylic acid | HATU | 418.1 [M+H]⁺ |
| C.50 | [4-(5-chlorooxazolo[4,5-b]pyridin-2-yl)piperazin-1-yl]-(4-ethynylphenyl)met hanone | 5-chloro-2-(piperazin-1-yl)oxazolo[4,5 -b]pyridine hydrochloride (B.2) | 4-ethynylbenzo ic acid | T₃P | 367.1 [M+H]⁺ |
| C.51 | (4-(5-chlorooxazolo [4,5- b]pyridin-2-yl)piperazin-1-yl)(4-ethynyl-3-(trifluoromethyl)ph enyl)methanone | 5-chloro-2-(piperazin-1-yl)oxazolo[4,5 -b]pyridine hydrochloride (B.2) | 4-ethynyl-3-(trifluoromet hyl)benzoic acid (A.71) | HATU | 435.3 [M+H]⁺ |
| C.53 | (4-(5-chlorooxazolo[4,5-b]pyridin-2-yl)piperazin-1-yl)(6-ethynyl-5-methylpyridin-3-yl)methanone | 5-chloro-2-(piperazin-1-yl)oxazolo[4,5 -b]pyridine hydrochloride (B.2) | 6-ethynyl-5-methylnicotin ic acid | HATU | 382.2 [M+H]⁺ |
| C.55 | (2-chloro-6-hydroxy-3-pyridyl)-[4-(5-chlorooxazolo[4,5 - b]pyridin-2-yl)piperazin-1-yl]methanone | 5-chloro-2-(piperazin-1-yl)oxazolo[4,5 -b]pyridine hydrochloride (B.2) | 2-chloro-6-hydroxypyridine-3-carboxylic acid | T₃P | 394.0 [M+H]⁺ |
| C.56 | (2-chloro-6-hydroxy-3-pyridyl)-[4-(5-methyloxazolo[4,5- b]pyridin-2-yl)piperazin-1-yl]methanone | 5-methyl-2-(piperazin-1-yl)oxazolo[4,5 -b]pyridine CAS 1035840-99-3 | 2-chloro-6-hydroxypyridine-3-carboxylic acid | EDCI/HO Bt | 374.3 [M+H]⁺ |

### Example C.2

### 1-{[3-Bromo-5-(3,3,3-trifluoropropoxy)phenyl[carbonyl}-4-{5-methyl-[1,3] oxazolo [4,5-b] pyridin- 2-yl} piperazine

THF (2 mL) was added to 3-bromo-5-[(4-{5-methyl-[1,3]oxazolo[4,5-b]pyridin-2-yl}piperazin-1-yl)carbonyl]phenol (25 mg,0.06 mmol) and cooled to 0 °C. Sodium hydride (60% oil suspension) (4 mg, 0.06 mmol) was added and stirred for 10 minutes under an argon atmosphere. 3,3,3-Trifluoropropyl methanesulfonate (23.0 mg, 0.12 mmol) and potassium iodide (2.0 mg, 0.01 mmol) were added, and reaction mixture was heated at 80 °C for 18 h. The reaction was quenched by saturated aq. NH₄Cl solution, THF was removed in vacuo and the mixture was extracted with EtOAc. The combined organic layers were dried, filtered and concentrated in vacuo to get a crude product which was purified by flash column chromatography with an eluent mixture of methanol and dichloromethane (0% - 10% gradient) providing the title compound (7 mg, 23%) as an off white solid. MS (ESI): m/z = 416.9 [M+H]⁺

### Step a) 3-Bromo-5-[(4-{5-methyl-[1,3]oxazolo[4, 5-b]pyridin-2-yl}piperazin-1-yl)carbonyl]phenol

To a solution of 1-{5-methyl-[1,3]oxazolo[4,5-b]pyridin-2-yl}piperazine, trifluoroacetic acid salt (500 mg, 1.50 mmol) in DMF (30 mL) was added triethylamine (0.5 mL, 3.76 mmol) The reaction mixture was stirred at r.t. for 20 min. and then the 3-bromo-5-hydroxybenzoic acid (262 mg, 1.20 mmol) and HATU (859 mg, 2.25 mmol) were added. The mixture was then stirred at r.t. for 12 h, diluted with EtOAc (150 mL) and washed with H₂O and brine. The organic part was dried, filtered and concentrated in vacuo to dryness. The crude product was purified by column chromatography with an eluent mixture of methanol and DCM (0% - 10%) providing the title compound (310 mg, 49%) as a yellowish sticky solid. MS (ESI): m/z = 416.6 [M+H]⁺

### Example C.3

### 1-[(3-Iodo-4-{[1-(trifluoromethyl)cyclopropyl]methoxy}phenyl)carbonyl]-4-{5-methyl-[1,3] oxazolo[4,5-b]pyridin-2-yl}piperazine

THF (3 mL) was added to 2-iodo-4-[(4-{5-methyl-[1,3]oxazolo[4,5-b]pyridin-2-yl}piperazin-1-yl)carbonyl]phenol (70 mg, 0.17 mmol) and cooled to 0 °C. Sodium hydride (60% oil suspension) was added and the reaction mixture was stirred for 10 minutes under argon atmosphere. 1-(trifluoromethyl) cyclopropyl]methyl methanesulfonate (92 mg, 0.42 mmol) and KI (6.0 mg, 0.03 mmol) were added to the reaction mixture and allowed to stir for 16 h at 80 °C. The reaction mixture was quenched with saturated aq. NH₄Cl solution at 0 °C. THF was removed under vacuum, the residue was diluted with water (25 mL) and extracted with EtOAc. The combined organic part was washed with brine, dried, filtered and concentrated in vacuo to dryness. The crude product was purified by flash chromatography with an eluent mixture of methanol and DCM (0% - 10% gradient) providing the title compound (50 mg, 51%) as an off white solid. MS (ESI): m/z = 587.5 [M+H]⁺

### Step a) 2-Iodo-4-[(4-{5-methyl-[1,3]oxazolo[4,5-b]pyridin-2-yl}piperazin-1-yl)carbonyl]phenol

To a solution of 1-{5-methyl-[1,3]oxazolo[4,5-b]pyridin-2-yl}piperazine trifluoroacetic acid (CAS: 1035840-99-3; free base) (550 mg, 2.52 mmol) in DMF (30mL) was added triethylamine (0.9 mL, 6.30 mmol). The reaction mixture was stirred at r.t. for 20 min and then 4-hydroxy-3-iodobenzoic acid (533 mg, 2.01 mmol) and HATU (1.5 g, 3.78 mmol) were added to it. The mixture was then stirred at 25 °C for 12 h, diluted with EtOAc and washed with H₂O. The organic layer was dried, filtered and concentrated in vacuo to dryness. The crude product was purified by column chromatography with an eluent mixture of methanol and DCM (0% - 10%) providing the title compound (220 mg, 19%) as an off white solid. MS (ESI): m/z = 464.8 [M+H]⁺

### Example C.4

### 1-{[3-Iodo-4-(3,3,3-trifluoropropoxy)phenyl]carbonyl}-4-{5-methyl-[1,3]oxazolo[4,5-b]pyridin-2-yl}piperazine

In analogy to the procedure described for example C.3, the title compound was prepared from 2-iodo-4-[(4-{5-methyl-[1,3]oxazolo[4,5-b]pyridin-2-yl}piperazin-1-yl)carbonyl]phenol (C.3, Step a) (80 mg 0.17 mmol) and 3,3,3-trifluoropropyl methane sulfonate (83 mg, 0.43 mmol) as an off-white solid (50 mg, 52%). MS (ESI): m/z = 561.2 [M+H]⁺

### Example C.5

### 1-[(3-Bromo-5-{[1-(trifluoromethyl)cyclopropyl]methoxy}phenyl)carbonyl]-4-{5-methyl-[1,3]oxazolo [4,5-b] pyridin- 2-yl} piperazine

In analogy to the procedure described for example C.2 the title compound was prepared from 3-bromo-5-[(4-{5-methyl-[1,3]oxazolo[4,5-b]pyridin-2-yl}piperazin-1-yl)carbonyl]phenol (C.2, Step a) (80 mg 0.19 mmol) and1-(Trifluoromethyl)cyclopropyl]methylmethanesulfonate (105 mg, 0.48 mmol)as an off-white solid (70 mg, 68%). MS (ESI): m/z = 541.1 [M+H]⁺

### Example C.9

### 1-({3-Iodo-4-[(1-methylcyclopropyl)methoxy]phenyl}carbonyl)-4-{5-methyl-[1,3] oxazolo [4,5-b] pyridin- 2-yl} piperazine

A solution of (1-methylcyclopropyl)methanol (56.0 mg, 0.64 mmol) and DIAD (0.09 mL, 0.43 mmol) was taken in toluene (1.0 mL) and then slowly added to a mixture of 2-iodo-4-[(4- { 5 -methyl- [1,3] oxazolo[4,5-b]pyridin-2-yl}piperazin-1-yl)carbonyl]phenol (C.3, Step a) (100 mg, 0.22 mmol) and triphenylphosphine (85.0 mg, 0.32 mmol) in toluene (2.0 mL) at 100 °C over 4 h via a syringe pump. It was kept at 100 °C until the disappearance of the starting material was observed. The reaction mixture was cooled to r.t. and volatiles were removed in vacuo. The crude product was purified by column chromatography with an eluent mixture of methanol and DCM (0% - 10%) providing the title compound (80 mg, 70%) as an off white solid. MS (ESI): m/z = 533.2 [M+H]⁺

### Example C.10

### 1-({3-Bromo-5-[(1-methylcyclopropyl)methoxy]phenyl}carbonyl)-4-{5-methyl-[1,3]oxazolo[4,5-b]pyridin-2-yl}piperazine

A solution of (1-methylcyclopropyl)methanol (124 mg, 1.44 mmol) and DIAD (0.19 mL, 0.96 mmol) was taken in toluene (1.0 mL) and then slowly added to a mixture of 3-bromo-5 - [(4- { 5 -methyl- [1,3] oxazolo [4,5-b]pyridin-2-yl} piperazin-1 -yl)carbonyl]phenol (C.2, Step a) (200 mg, 0.48 mmol and triphenylphosphine (189 mg, 7.12 mmol) in toluene (3.0 mL) at 100 °C over 4 h via a syringe pump. It was kept at 100 °C until the disappearance of the starting material was observed. The reaction mixture was cooled to r.t. and volatiles were removed in vacuuo. The crude product was purified by column chromatography with en eluent mixture of methanol and DCM (0% - 10%) providing the title compound (210 mg, 90%) as an off white sticky solid. MS (ESI): m/z = 484.9 [M+H]⁺

### Example C.27

### (4-(5-Chlorooxazolo[4,5-b]pyridin-2-yl)piperazin-1-yl)(3-methyl-4-(1H-1,2,3-triazol-4-yl)phenyl)methanone

To a mixture of (4-(5-chlorooxazolo[4,5-b]pyridin-2-yl)piperazin-1-yl)(4-ethynyl-3-methylphenyl)methanone (C.43) (50 mg, 131 µmol), copper (I) iodide (1.25 mg, 6.56 µmol) in DMF (236 µL) and MeOH (26.2 µL) in a vial for microwave chemistry was added trimethylsilyl azide (24.1 mg, 27.8 µL, 197 µmol). The vial was closed and the mixture was heated to 100 °C for 6 h. The mixture was diluted with EtOAc and washed with sat. aq. NaHCO₃ sol. The aqueous phase was back extracted with EtOAc. The combined organics were washed with H₂O and brine, dried (MgSO₄), filtered and concentrated to leave the crude product as a light yellow liquid. The crude product was purified by silica column flash chromatography with an eluent mixture of AcOEt and heptane (0% - 100%) providing the title compound (27.6 mg, 49.6%) as a white solid. MS (ESI): m/z = 424.2 [M+H]⁺

Examples C.32, C.35, C.37, C.39, C.42, C.45,C.52 and C.54 were prepared in analogy to C.27

| **Ex.** | **Systematic Name** | **Starting material** | **MS (ESI): m/z** |
|---|---|---|---|
| C.32 | 5-(4-(5-chlorooxazolo[4,5-b]pyridin-2-yl)piperazine-1-carbonyl)-2-(1H-1,2,3-triazol-4-yl)benzonitrile | 5-(4-(5-chlorooxazolo [4,5 - b]pyridin-2-yl)piperazine-1-carbonyl)-2-ethynylbenzonitrile (C.31) | 435.3 [M+H]⁺ |
| C.35 | 5-(4-(5-methyloxazolo[4,5-b]pyridin-2-yl)piperazine-1-carbonyl)-2-(1H-1,2,3-triazol-4-yl)benzonitrile | 2-ethynyl-5-(4-(5-methyloxazolo [4,5-b]pyridin-2-yl)piperazine-1-carbonyl)benzonitrile (C.34) | 415.3 [M+H]⁺ |
| C.37 | (4-(5-chlorooxazolo[4,5-b]pyridin-2-yl)piperazin-1-yl)(3-methoxy-4-(1H-1,2,3-triazol-4-yl)phenyl)methanone | (4-(5-chlorooxazolo[4,5-b]pyridin-2-yl)piperazin-1-yl)(4-ethynyl-3-methoxyphenyl)methano ne (C.36) | 440.3 [M+H]⁺ |
| C.39 | (3-methoxy-4-(1H-1,2,3-triazol-4-yl)phenyl)(4-(5-methyloxazolo[4,5-b]pyridin-2-yl)piperazin-1-yl)methanone | (4-ethynyl-3-methoxyphenyl)(4-(5-methyloxazolo [4,5-b]pyridin-2-yl)piperazin-1-yl)methanone (C.38) | 420.3 [M+H]⁺ |
| C.42 | (4-(5-chlorooxazolo[4,5-b]pyridin-2-yl)piperazin-1-yl)(5-fluoro-6-(1H-1,2,3-triazol-4-yl)pyridin-3-yl)methanone | (4-(5-chlorooxazolo[4,5-b]pyridin-2-yl)piperazin-1-yl)(6-ethynyl-5-fluoropyridin-3-yl)methanone (C.41) | 429.2 [M+H]⁺ |
| C.45 | (5-fluoro-6-(1H-1,2,3-triazol-4-yl)pyridin-3 -yl)(4-(5 - methyloxazolo [4,5 -b]pyridin-2-yl)piperazin-1-yl)methanone | (6-ethynyl-5-fluoropyridin-3 -yl)(4-(5 - methyloxazolo [4,5-b]pyridin-2-yl)piperazin-1-yl)methanone (C.44) | 409.3 [M+H]⁺ |
| C.52 | (4-(1H-1,2,3-triazol-4-yl)-3-(trifluoromethyl)phenyl)(4-(5-chlorooxazolo [4,5 -b]pyridin-2-yl)piperazin-1-yl)methanone | (4-(5-chlorooxazolo[4,5-b]pyridin-2-yl)piperazin-1-yl)(4-ethynyl-3-(trifluoromethyl)phenyl) methanone (C.51) | 478.3 [M+H]⁺ |
| C.54 | (4-(5-chlorooxazolo[4,5-b]pyridin-2-yl)piperazin-1-yl)(5-methyl-6-(1H-1,2,3-triazol-4-yl)pyridin-3-yl)methanone | (4-(5-chlorooxazolo[4,5-b]pyridin-2-yl)piperazin-1-yl) (6-ethynyl-5 - methylpyridin-3-yl)methanone (C.53) | 425.3 [M+H]⁺ |

### Example C.48

### [4-(5-Chlorooxazolo [4,5-b] pyridin-2-yl)piperazin-1-yl]-[5-methyl-6-(1H-pyrazol-4-yl)-3-pyridyl] methanone

To a solution of (1H-pyrazol-4-yl)boronic acid pinacol ester (200 mg, 1.03 mmol) and (6-bromo-5-methyl-3-pyridyl)-[4-(5-chlorooxazolo[4,5-b]pyridin-2-yl)piperazin-1-yl]methanone (C.46) (420 mg, 962 µmol) in toluene (0.20 mL), water (0.20 mL) and EtOH (1.60 mL) was added Na₂CO₃ aq. stirred at 80 °C for 12 h. Water (5.00 mL) was added to the reaction mixture and extracted with The combined organic layer was washed with brine, dried over Na₂SO₄, filtered and concentrated to dryness. The crude product was purified by silica column chromatography providing the title compound (250 mg, 51.5%) as a yellow solid. MS (ESI): m/z = 424.2 [M+H]⁺

### Example C.49

### [4-(5-Methyloxazolo[4,5-b]pyridin-2-yl)piperazin-1-yl]-[5-methyl-6-(1H-pyrazol-4-yl)-3-pyridyl] methanone

In analogy to the procedure described for example C.48 the title compound was prepared from (6-bromo-5-methyl-3-pyridyl)-[4-(5-methyloxazolo[4,5-b]pyridin-2-yl)piperazin-1-yl]methanone (C.47) (400 mg 0.96 mmol) as a yellow solid (200 mg, 41.0%). MS (ESI): m/z = 404.3 [M+H]⁺

### Example 312

A compound of formula (I) can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 313

A compound of formula (I) can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. A compound of formula (I) 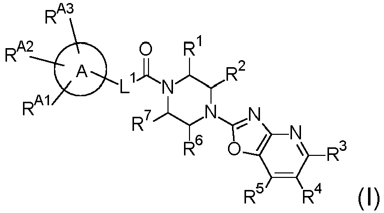 or a pharmaceutically acceptable salt thereof, wherein:
(i) R¹, R², R⁶, and R⁷ are each independently hydrogen, C₁-C₆-alkyl or hydroxy-C₁-C₆-alkyl; or
(ii) R¹ and R⁶ together form a bridge of formula -(CH₂)ₘ-, wherein m is 1, 2 or 3; and R² and R⁷ are independently hydrogen, C₁-C₆-alkyl or hydroxy-C₁-C₆-alkyl; or
(iii) R¹ and R⁷ together form a bridge of formula -(CH₂)ₘ-, wherein m is 1, 2 or 3; and R² and R⁶ are independently hydrogen, C₁-C₆-alkyl or hydroxy-C₁-C₆-alkyl; or
(iv) R² and R⁶ together form a bridge of formula -(CH₂)ₘ-, wherein m is 1, 2 or 3; and R¹ and R⁷ are independently hydrogen, C₁-C₆-alkyl or hydroxy-C₁-C₆-alkyl; or
(v) R² and R⁷ together form a bridge of formula -(CH₂)ₘ-, wherein m is 1, 2 or 3; and R¹ and R⁶ are independently hydrogen, C₁-C₆-alkyl or hydroxy-C₁-C₆-alkyl;
R³, R⁴, and R⁵ are each independently hydrogen, halogen, cyano, C₁-C₆-alkyl, halo-C₁-C₆-alkyl, hydroxy-C₁-C₆-alkyl, C₃₋₁₂-cycloalkyl, C₁-C₆-alkoxy or halo-C₁-C₆-alkoxy;
R^{A1} is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 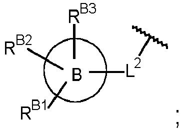
R^{A2} is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 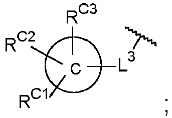
R^{A3} is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy or halo-C₁-C₆-alkyl;
R^{B1} is hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy, halo-C₁-C₆-alkyl or a group 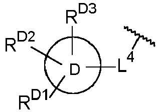
R^{B2}, R^{B3}, R^{C1}, R^{C2}, R^{C3}, R^{D1}, R^{D2}, and R^{D3} are each independently hydrogen, halogen, oxo, acetyl, cyano, cyano-C₁-C₆-alkyl, hydroxy, hydroxy-C₁-C₆-alkyl, C₁-C₆-alkyl, C₁-C₆-alkanoyl, C₂-C₆-alkenyl, C₂-C₆-alkynyl, C₁-C₆-alkoxy, C₁-C₆-alkoxy-C₁-C₆-alkyl, halo-C₁-C₆-alkoxy or halo-C₁-C₆-alkyl;
L¹ is a covalent bond, C₂-C₆-alkynyl, carbonyl, -C(O)NH-, -NHC(O)-, -CH₂O-, -OCH₂- or SO₂;
L², L³, and L⁴ are each independently a covalent bond, C₂-C₆-alkynyl, -CH₂-, -O-, carbonyl, -C(O)NH-, -NHC(O)-, -CH₂O-, -OCH₂- or SO₂; and
A, B, C, and D are each independently C₆₋₁₄-aryl, C₁₋₁₃-heteroaryl, C₃₋₁₂-cycloalkyl or C₂₋₉-heterocyclyl.

2. The compound of formula (I) according to claim 1, or a pharmaceutically acceptable salt thereof, wherein:
(i) R¹ and R² are independently hydrogen, C₁-C₆-alkyl or hydroxy-C₁-C₆-alkyl; and R⁶ and R⁷ are hydrogen; or
(ii) R¹ and R⁷ together form a bridge of formula -(CH₂)₂-; and R² and R⁶ are both hydrogen; or
(iii) R² and R⁶ together form a bridge of formula -(CH₂)₂-; and R¹ and R⁷ are both hydrogen.

3. The compound of formula (I) according to claim 1, or a pharmaceutically acceptable salt thereof, wherein:
R¹, R⁶, and R⁷ are all hydrogen; and
R² is hydrogen or C₁-C₆-alkyl.

4. The compound of formula (I) according to claim 1, or a pharmaceutically acceptable salt thereof, wherein:
R¹, R⁶, and R⁷ are all hydrogen; and
R² is hydrogen or methyl.

5. The compound of formula (I) according to any one of claims 1 to 4, or a pharmaceutically acceptable salt thereof, wherein:
R³ is hydrogen, halogen, cyano, C₁-C₆-alkyl, halo-C₁-C₆-alkyl, C₃₋₁₂-cycloalkyl or C₁-C₆-alkoxy;
R⁴ is hydrogen; and
R⁵ is hydrogen or halogen.

6. The compound of formula (I) according to any one of claims 1 to 4, or a pharmaceutically acceptable salt thereof, wherein:
R³ is C₁-C₆-alkyl or halogen; and
R⁴ and R⁵ are both hydrogen.

7. The compound of formula (I) according to any one of claims 1 to 4, or a pharmaceutically acceptable salt thereof, wherein:
R³ is methyl or chloro; and
R⁴ and R⁵ are both hydrogen.

8. The compound of formula (I) according to any one of claims 1 to 7, or a pharmaceutically acceptable salt thereof, wherein:
L¹ is a covalent bond or C₂-C₆-alkynyl;
A is C₆₋₁₄-aryl, C₁₋₁₃-heteroaryl or C₂₋₉-heterocyclyl;
R^{A1} is hydrogen, halogen, C₁-C₆-alkyl, C₂-C₆-alkenyl, C₂-C₆-alkynyl, C₁-C₆-alkoxy, halo-C₁-C₆-alkoxy, halo-C₁-C₆-alkyl or a group 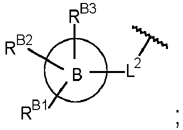
R^{A2} is hydrogen, halogen, cyano, hydroxy, C₁-C₆-alkyl, C₁-C₆-alkoxy, halo-C₁-C₆-alkoxy, halo-C₁-C₆-alkyl or a group 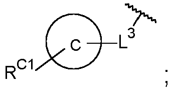
R^{A3} is hydrogen or halogen;
L² is a covalent bond, -C(O)NH-, -NHC(O)-, -CH₂O-, -OCH₂- or SO₂;
B is C₆₋₁₄-aryl, C₁₋₁₃-heteroaryl, C₃₋₁₂-cycloalkyl or C₂₋₉-heterocyclyl;
R^{B1} is hydrogen, halogen, hydroxy, acetyl, cyano, cyano-C₁-C₆-alkyl, C₁-C₆-alkyl, halo-C₁-C₆-alkyl, hydroxy-C₁-C₆-alkyl, C₂-C₆-alkenyl, C₁-C₆-alkoxy, halo-C₁-C₆-alkoxy, C₁-C₆-alkoxy-C₁-C₆-alkyl, C₁-C₆-alkanoyl, oxo or a group 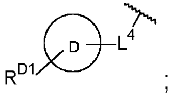
R^{B2} is hydrogen, halogen, oxo or C₁-C₆-alkyl;
R^{B3} is hydrogen or halogen;
L³ is a covalent bond or -CH₂O-;
C is C₆₋₁₄-aryl, C₁₋₁₃-heteroaryl, C₃₋₁₂-cycloalkyl or C₂₋₉-heterocyclyl;
R^{C1} is hydrogen, halogen, C₁-C₆-alkyl or halo-C₁-C₆-alkyl;
L⁴ is a covalent bond, -CH₂- or -O-;
D is C₆₋₁₄-aryl, C₃₋₁₂-cycloalkyl or C₂₋₉-heterocyclyl; and
R^{D1} is C₁-C₆-alkyl, halo-C₁-C₆-alkyl or halogen.

9. The compound of formula (I) according to any one of claims 1 to 8, or a pharmaceutically acceptable salt thereof, wherein:
L¹ is a covalent bond;
A is C₆₋₁₄-aryl or C₁₋₁₃-heteroaryl;
R^{A1} is a group 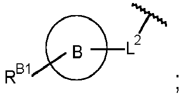
R^{A2} is hydrogen, halogen or C₁-C₆-alkyl;
R^{A3} is hydrogen or halogen;
L² is a covalent bond or -CH₂O-;
B is C₁₋₁₃-heteroaryl or C₃₋₁₂-cycloalkyl; and
R^{B1} is C₁-C₆-alkyl or halo-C₁-C₆-alkyl.

10. The compound of formula (I) according to any one of claims 1 to 9, or a pharmaceutically acceptable salt thereof, wherein:
L¹ is a covalent bond;
A is phenyl or pyridyl;
R^{A1} is a group 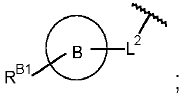
R^{A2} is hydrogen, fluoro, chloro or methyl;
R^{A3} is hydrogen or fluoro;
L² is a covalent bond or -CH₂O-;
B is oxadiazolyl, pyrazolyl, triazolyl, tetrazolyl or cyclopropyl; and
R^{B1} is *tert*-butyl, 2,2-dimethylpropyl, trifluoromethyl or 2-fluoro-1,1-dimethyl-ethyl.

11. The compound of formula (I) according to claim 1, or a pharmaceutically acceptable salt thereof, wherein the compound of formula (I) is:
[4-(5-methyloxazolo[4,5-b]pyridin-2-yl)piperazin-1-yl]-(2-phenyl-1,3-benzoxazol-6-yl)methanone;
(4-oxazolo[4,5-b]pyridin-2-ylpiperazin-1-yl)-(2-phenyl-1,3-benzoxazol-6-yl)methanone;
[4-(5-methyloxazolo[4,5-b]pyridin-2-yl)piperazin-1-yl]-(2-pyrrolidin-1-yl-1,3-benzoxazol-6-yl)methanone;
[4-(5-tert-butyl-1,2,4-oxadiazol-3-yl)phenyl]-[4-(5-methyloxazolo[4,5-b]pyridin-2-yl)piperazin-1-yl]methanone;
[4-[5-(2,2-dimethylpropyl)-1,2,4-oxadiazol-3-yl]phenyl]-[4-(5-methyloxazolo[4,5-b]pyridin-2-yl)piperazin-1-yl]methanone;
[2-(2-azaspiro [3.4] octan-2-yl)-1,3 -benzoxazol-6-yl]- [4-(5 -methyloxazolo [4,5-b]pyridin-2-yl)piperazin-1-yl]methanone;
[4-[5-(2-hydroxy-1,1-dimethyl-ethyl)-1,2,4-oxadiazol-3-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(7-chlorooxazolo[4,5-b]pyridin-2-yl)piperazin-1-yl]methanone;
[4-(7-chlorooxazolo[4,5-b]pyridin-2-yl)piperazin-1-yl]-[4-[5-(2,2-dimethylpropyl)-1,2,4-oxadiazol-3-yl]phenyl]methanone;
[4-(5-chlorooxazolo[4,5-b]pyridin-2-yl)piperazin-1-yl]-[4-[5-(2,2-dimethylpropyl)-1,2,4-oxadiazol-3-yl]phenyl]methanone;
[4-[5-(2-fluoro-1,1-dimethyl-ethyl)-1,2,4-oxadiazol-3-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(5-chlorooxazolo [4, 5-b]pyridin-2-yl)piperazin-1-yl]methanone;
[4-[5-(2,2-dimethylpropyl)-1,2,4-oxadiazol-3-yl]phenyl]-(4-oxazolo[4,5-b]pyridin-2-ylpiperazin-1-yl)methanone;
[4-(5-tert-butyl-1,2,4-oxadiazol-3-yl)phenyl]-(4-oxazolo[4,5-b]pyridin-2-ylpiperazin-1-yl)methanone;
2-[4-[4-[5-(2,2-dimethylpropyl)-1,2,4-oxadiazol-3-yl]benzoyl]piperazin-1-yl]oxazolo[4,5-b]pyridine-5-carbonitrile;
[4-[5-(2,2-dimethylpropyl)-1,2,4-oxadiazol-3-yl]phenyl]-[4-[5-(trifluoromethyl)oxazolo [4, 5-b]pyridin-2-yl]piperazin-1-yl] methanone;
[2-(1-ethylpropyl)-1,3-benzoxazol-6-yl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2,2-dimethylpropyl)-1,3,4-oxadiazol-2-yl]phenyl]methanone;
(2-cyclohexyl-1,3-benzoxazol-6-yl)-[4-(5-methyloxazolo[4,5-b]pyridin-2-yl)piperazin-1-yl]methanone;
[4-(5-methyloxazolo[4,5-b]pyridin-2-yl)piperazin-1-yl]-[2-(3,3,3-trifluoropropyl)-1,3-benzoxazol-6-yl]methanone;
[4-(5-chlorooxazolo[4,5-b]pyridin-2-yl)piperazin-1-yl]-[4-[1-(2,2-dimethylpropyl)pyrazol-4-yl]phenyl]methanone;
[3-chloro-5-[4-(hydroxymethyl)phenyl]phenyl]-[4-(5-methyloxazolo[4,5-b]pyridin-2-yl)piperazin-1-yl]methanone;
[3-chloro-5-(1,1-dioxo-1,4-thiazinan-4-yl)phenyl]-[4-(5-methyloxazolo[4,5-b]pyridin-2-yl)piperazin-1-yl]methanone;
1 - [4- [3 -chloro-5 - [4 - (5 -methyloxazolo [4,5 -b]pyridin-2-yl)piperazine-1 - carbonyl]phenyl]piperazin-1-yl]ethanone;
[3-chloro-5-(4,4-difluoro-1-piperidyl)phenyl]-[4-(5-methyloxazolo [4, 5-b]pyridin-2-yl)piperazin-1-yl]methanone;
[3-chloro-5-[4-(hydroxymethyl)-1-piperidyl]phenyl]-[4-(5-methyloxazolo [4, 5-b]pyridin-2-yl)piperazin-1-yl]methanone;
[3-chloro-5-(6-hydroxy-2-azaspiro[3.3]heptan-2-yl)phenyl]-[4-(5-methyloxazolo[4,5-b]pyridin-2-yl)piperazin-1-yl]methanone;
[2-(3-chlorophenyl)sulfonyl-2,6-diazaspiro[3.3]heptan-6-yl]-[4-(5-methyloxazolo[4,5-b]pyridin-2-yl)piperazin-1-yl]methanone;
[3-[4-(hydroxymethyl)phenyl]-5-(3,3,3-trifluoropropoxy)phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2-fluoro-1,1-dimethylethyl)-1,2,4-oxadiazol-3-yl]phenyl]methanone;
[2-[(E)-but-1-enyl]-1,3-benzoxazol-6-yl]-[4-(5-methyloxazolo[4,5-b]pyridin-2-yl)piperazin-1-yl]methanone;
(2-cyclopropyl-1,3-benzoxazol-6-yl)-[4-(5-methyloxazolo [4, 5-b]pyridin-2-yl)piperazin-1-yl]methanone;
1-[3-[4-[4-(5-chlorooxazolo [4, 5-b]pyridin-2-yl)piperazine-1-carbonyl]phenyl]-1, 2,4-oxadiazol-5-yl]propan-2-one;
[4-(5-chlorooxazolo[4,5-b]pyridin-2-yl)piperazin-1-yl]-[4-[5-(2,2-difluoropropyl)-1,2,4-oxadiazol-3-yl]phenyl]methanone;
[4-(5-chlorooxazolo[4,5-b]pyridin-2-yl)piperazin-1-yl]-[4-[5-(1-fluoro-1-methylethyl)-1,2,4-oxadiazol-3-yl]phenyl]methanone;
[4-(5-chlorooxazolo[4,5-b]pyridin-2-yl)piperazin-1-yl]-[4-[3-(2,2-dimethylpropyl)-1,2,4-oxadiazol-5-yl]phenyl]methanone;
[3-[4-(hydroxymethyl)phenyl]-4-[[1-(trifluoromethyl)cyclopropyl]methoxy]phenyl]-[4-(5-methyloxazolo[4,5-b]pyridin-2-yl)piperazin-1-yl]methanone;
[3-[4-(hydroxymethyl)phenyl]-4-(3,3,3-trifluoropropoxy)phenyl]-[4-(5-methyloxazolo[4,5-b]pyridin-2-yl)piperazin-1-yl]methanone;
[3-[4-(hydroxymethyl)phenyl]-5-[[1-(trifluoromethyl)cyclopropyl]methoxy]phenyl]-[4-(5-methyloxazolo[4,5-b]pyridin-2-yl)piperazin-1-yl]methanone;
(2-butyl-1,3-benzoxazol-6-yl)-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(1-fluorocyclopropyl)-1,2,4-oxadiazol-3-yl]phenyl]methanone;
[4-(5-chlorooxazolo[4,5-b]pyridin-2-yl)piperazin-1-yl]-[4-[1-(2,2-dimethylpropyl)-1,2,4-triazol-3-yl]phenyl]methanone;
[4-[1-(2,2-dimethylpropyl)-1,2,4-triazol-3-yl]phenyl]-[4-(5-methyloxazolo[4,5-b]pyridin-2-yl)piperazin-1-yl]methanone;
[4-(5-chlorooxazolo [4,5-b]pyridin-2-yl)piperazin-1-yl]-[4-[1-(2,2-dimethylpropyl)imidazol-4-yl]phenyl]methanone;
[4-(5-tert-butyl-1,3,4-oxadiazol-2-yl)phenyl]-[4-(5-chlorooxazolo[4,5-b]pyridin-2-yl)piperazin-1-yl]methanone;
[4-(5 -oxa-2-azaspiro [3.4] octan-2-yl)phenyl] -(4-oxazolo [4,5 -b]pyridin-2-ylpiperazin-1-yl)methanone;
(4-oxazolo [4, 5-b]pyridin-2-ylpiperazin-1-yl)-[4-[3-(2, 2,2-trifluoro-1-methyl-ethoxy)azetidin-1-yl]phenyl]methanone;
[4-(5-tert-butyl-1,3,4-oxadiazol-2-yl)phenyl]-[4-(5-methyloxazolo[4,5-b]pyridin-2-yl)piperazin-1-yl]methanone;
[4-(5-methyloxazolo [4, 5-b]pyridin-2-yl)piperazin-1-yl]-[4-(5-oxa-2-azaspiro [3.4] octan-2-yl)phenyl] methanone;
[4-(5-methyloxazolo [4, 5-b]pyridin-2-yl)piperazin-1-yl]-[4-[3-(2, 2,2-trifluoro-1-methyl-ethoxy)azetidin-1-yl]phenyl]methanone;
[4-(5-chlorooxazolo[4,5-b]pyridin-2-yl)piperazin-1-yl]-[4-[5-(2-fluoro-2-methylpropyl)-1,2,4-oxadiazol-3-yl]phenyl]methanone;
[4-(5-chlorooxazolo[4,5-b]pyridin-2-yl)piperazin-1-yl]-[4-[5-(2-methylallyl)-1,2,4-oxadiazol-3-yl]phenyl]methanone;
[4-(5-chlorooxazolo[4,5-b]pyridin-2-yl)piperazin-1-yl]-[4-[5-(2-methylprop-1-enyl)-1,2,4-oxadiazol-3-yl]phenyl]methanone;
[4-(5-chlorooxazolo[4,5-b]pyridin-2-yl)piperazin-1-yl]-[4-[5-(2-hydroxy-2-methylpropyl)-1,2,4-oxadiazol-3-yl]phenyl]methanone;
(4-oxazolo[4,5-b]pyridin-2-ylpiperazin-1-yl)-[4-(3-phenoxyazetidin-1-yl)phenyl] methanone;
[4-[5-(2-fluoro-1,1-dimethyl-ethyl)-1,3,4-oxadiazol-2-yl]phenyl]-[4-(5-methyloxazolo[4,5-b]pyridin-2-yl)piperazin-1-yl]methanone;
[4-(5-tert-butyl-1,2,4-oxadiazol-3-yl)phenyl]-[4-(5-methoxyoxazolo[4,5-b]pyridin-2-yl)piperazin-1-yl]methanone;
[4-(5-chlorooxazolo[4,5-b]pyridin-2-yl)piperazin-1-yl]-[4-[5-(2-fluoro-1,1-dimethylethyl)- 1,3,4-oxadiazol-2-yl]phenyl]methanone;
[4-[5-(1-fluorocyclopropyl)-1,2,4-oxadiazol-3-yl]phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[4-(3-phenoxyazetidin-1-yl)phenyl] methanone;
[4-[5-(2,2-dimethylpropyl)-1,2,4-oxadiazol-3-yl]-3-[4-(hydroxymethyl)phenyl]phenyl]-[4-(5-methyloxazolo[4,5-b]pyridin-2-yl)piperazin-1-yl]methanone;
[3-[4-(hydroxymethyl)phenyl]-4-[(1-methylcyclopropyl)methoxy]phenyl]-[4-(5-methyloxazolo[4,5-b]pyridin-2-yl)piperazin-1-yl]methanone;
[3-[4-(hydroxymethyl)phenyl]-5-[(1-methylcyclopropyl)methoxy]phenyl]-[4-(5-methyloxazolo[4,5-b]pyridin-2-yl)piperazin-1-yl]methanone;
[4-[(3-chlorophenyl)methoxy]-3-(1H-imidazol-2-yl)phenyl]-[4-(5-methyloxazolo[4,5-b]pyridin-2-yl)piperazin-1-yl]methanone;
[4-(2-azaspiro[3.4]octan-2-yl)-3-(1H-imidazol-2-yl)phenyl]-[4-(5-methyloxazolo[4,5-b]pyridin-2-yl)piperazin-1-yl]methanone;
[4-[5-[1-(fluoromethyl)cyclopropyl]-1,2,4-oxadiazol-3-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-l-yl]-[4-[5-[1-(fluoromethyl)cyclopropyl]-1,2,4-oxadiazol-3-yl]phenyl]methanone;
[4-(3-tert-butyl-1,2,4-oxadiazol-5-yl)phenyl]-[4-(5-methyloxazolo [4,5-b]pyridin-2-yl)piperazin-1-yl]methanone;
[4-(3-tert-butyl-1,2,4-oxadiazol-5-yl)phenyl]-[4-(5-chlorooxazolo[4,5-b]pyridin-2-yl)piperazin-1-yl]methanone;
[4-(5-tert-butyl-1,2,4-oxadiazol-3-yl)phenyl]-[4-(5-cyclopropyloxazolo[4,5-b]pyridin-2-yl)piperazin-1-yl]methanone;
[4-(5-chlorooxazolo[4,5-b]pyridin-2-yl)piperazin-1-yl]-[4-[5-[(1-methylcyclopropyl)methyl]-1,2,4-oxadiazol-3-yl]phenyl]methanone;
[4-(5-methyloxazolo[4,5-b]pyridin-2-yl)piperazin-1-yl]-[4-[3-[(1S)-2,2,2-trifluoro-1-methyl-ethoxy]azetidin-1-yl]phenyl]methanone;
[4-(5-methyloxazolo[4,5-b]pyridin-2-yl)piperazin-1-yl]-[4-[3-[(1R)-2,2,2-trifluoro-1-methyl-ethoxy]azetidin-1-yl]phenyl]methanone;
[4-(5-chlorooxazolo[4,5-b]pyridin-2-yl)piperazin-1-yl]-[4-(2,2-difluoro-5-azaspiro[2.4]heptan-5-yl)phenyl]methanone;
[4-(5-chlorooxazolo[4,5-b]pyridin-2-yl)piperazin-1-yl]-[4-(7,7-difluoro-2-azaspiro[3.3]heptan-2-yl)phenyl]methanone;
[4-(3-tert-butoxyazetidin-1-yl)phenyl]-[4-(5-chlorooxazolo [4, 5-b]pyridin-2-yl)piperazin-1-yl]methanone;
[4-(5-chlorooxazolo[4,5-b]pyridin-2-yl)piperazin-1-yl]-[4-[3-(1,1-dimethylpropoxy)azetidin-1-yl]phenyl]methanone;
[4-(1-tert-butyl-1,2,4-triazol-3-yl)phenyl]-[4-(5-chlorooxazolo[4,5-b]pyridin-2-yl)piperazin-1-yl]methanone;
[4-(1-tert-butyl-1,2,4-triazol-3-yl)phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]phenyl]methanone;
[4-(5-chlorooxazolo[4,5-b]pyridin-2-yl)piperazin-1-yl]-[4-[2-(2,2-dimethylpropyl)triazol-4-yl]phenyl]methanone;
[4-(5-tert-butyl-l,2,4-oxadiazol-3-yl)phenyl]-[4-(5-chlorooxazolo[4,5-b]pyridin-2-yl)-2-methyl-piperazin-1-yl]methanone;
[4-(5-tert-butyl-1,2,4-oxadiazol-3-yl)phenyl]-[4-(5-chlorooxazolo [4, 5-b]pyridin-2-yl)-3-methyl-piperazin-1-yl]methanone;
[4-(1-tert-butylpyrazol-4-yl)phenyl]-[4-(5-methyloxazolo[4,5-b]pyridin-2-yl)piperazin-1-yl]methanone;
[4-(1-tert-butylpyrazol-4-yl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[1-[(1-methylcyclopropyl)methyl]-1,2,4-triazol-3-yl]phenyl]methanone;
[4-(5-chlorooxazolo[4,5-b]pyridin-2-yl)piperazin-1-yl]-[4-[2-[(1-methylcyclopropyl)methyl]-1,2,4-triazol-3-yl]phenyl]methanone;
[4-[5-(1-methylcyclopropyl)-1,2,4-oxadiazol-3-yl]phenyl]-[4-(5-methyloxazolo[4,5-b]pyridin-2-yl)piperazin-1-yl]methanone;
[4-(4-tert-butylpyrazol-1-yl)phenyl]-[4-(5-chlorooxazolo[4,5-b]pyridin-2-yl)piperazin-1-yl]methanone;
[4-(4-tert-butylpyrazol-1-yl)phenyl]-[4-(5-methyloxazolo [4, 5-b]pyridin-2-yl)piperazin-1-yl]methanone;
[4-[1-(2,2-dimethylpropyl)pyrazol-4-yl]phenyl]-[4-(5-methyloxazolo[4,5-b]pyridin-2-yl)piperazin-1-yl] methanone;
[4-(7,7-difluoro-2-azaspiro[3.3]heptan-2-yl)phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[6-[3-(2,2,2-trifluoro-1-methyl-ethoxy)azetidin-1-yl]-3-pyridyl]methanone;
[4-(4-tert-butylimidazol-1-yl)phenyl]-[4-(5-methyloxazolo [4, 5-b]pyridin-2-yl)piperazin-1-yl]methanone;
[4-(5-tert-butyl-1,2,4-oxadiazol-3-yl)-3-methyl-phenyl]-[4-(5-methyloxazolo[4,5-b]pyridin-2-yl)piperazin-1-yl]methanone;
[4-(5-tert-butyl-1,2,4-oxadiazol-3-yl)-3-methyl-phenyl]-[4-(5-chlorooxazolo[4,5-b]pyridin-2-yl)piperazin-1-yl]methanone;
[4-(5-tert-butyl-1,2,4-oxadiazol-3-yl)-3-fluoro-phenyl]-[4-(5-methyloxazolo[4,5-b]pyridin-2-yl)piperazin-1-yl]methanone;
[4-(5-tert-butyl-1,2,4-oxadiazol-3-yl)-3-fluoro-phenyl]-[4-(5-chlorooxazolo[4,5-b]pyridin-2-yl)piperazin-1-yl]methanone;
[3-chloro-4-[(1-methylcyclopropyl)methoxy]phenyl]-[4-(5-chlorooxazolo[4,5-b]pyridin-2-yl)piperazin-1-yl]methanone;
[6-[3-(1,1-dimethylpropoxy)azetidin-1-yl]-3-pyridyl]-[4-(5-methyloxazolo[4,5-b]pyridin-2-yl)piperazin-1-yl]methanone;
[4-(5-methyloxazolo [4, 5-b]pyridin-2-yl)piperazin-1-yl]-[5-methyl-6-[3-(2,2,2-trifluoro-1-methyl-ethoxy)azetidin-1-yl]-3-pyridyl]methanone;
[6-(5-tert-butyl-1,2,4-oxadiazol-3-yl)-3-pyridyl]-[4-(5-chlorooxazolo[4,5-b]pyridin-2-yl)piperazin-1-yl] methanone;
[6-(5-tert-butyl-1,2,4-oxadiazol-3-yl)-3-pyridyl]-[4-(5-methyloxazolo[4,5-b]pyridin-2-yl)piperazin-1-yl]methanone;
[4-(4-tert-butylimidazol-1-yl)phenyl]-[4-(5-chlorooxazolo[4,5-b]pyridin-2-yl)piperazin-1-yl]methanone;
[2-fluoro-4-[3-(2,2,2-trifluoro-1-methyl-ethoxy)azetidin-1-yl]phenyl]-[4-(5-methyloxazolo [4, 5-b]pyridin-2-yl)piperazin-1-yl] methanone;
[4-(5-chlorooxazolo[4,5-b]pyridin-2-yl)piperazin-1-yl]-[4-(2,2-dimethylpropoxy)-3-methyl-phenyl] methanone;
[4-(2,2-dimethylpropoxy)-3-methyl-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[3-(2,2,2-trifluoro-1-methyl-ethoxy)azetidin-1-yl]phenyl]methanone;
[4-(5-chlorooxazolo[4,5-b]pyridin-2-yl)piperazin-1-yl]-[4-(3-ethoxy-3-methyl-azetidin-1-yl)phenyl]methanone;
[3-chloro-4-[(1-methylcyclopropyl)methoxy]phenyl]-[4-(5-methyloxazolo[4,5-b]pyridin-2-yl)piperazin-1-yl]methanone;
[3-fluoro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[4-(3-methyloxetan-3-yl)phenyl] methanone;
[4-(1-fluorocyclopropyl)phenyl]-[4-(5-methyloxazolo[4,5-b]pyridin-2-yl)piperazin-1-yl]methanone;
[4-chloro-5-fluoro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[6-[3-(2,2,2-trifluoro-1-methyl-ethoxy)azetidin-1-yl]pyridazin-3-yl]methanone;
[5-methyl-6-[(1-methylcyclopropyl)methoxy]-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[2-[3-(2,2,2-trifluoro-1-methyl-ethoxy)azetidin-1-yl]pyrimidin-5-yl]methanone;
[4-(5-methyloxazolo[4,5-b]pyridin-2-yl)piperazin-1-yl]-[4-methyl-6-[3-(2,2,2-trifluoro-1-methyl-ethoxy)azetidin-1-yl]-3-pyridyl]methanone;
[6-(7,7-difluoro-2-azaspiro[3.3]heptan-2-yl)-4-methyl-3-pyridyl]-[4-(5-methyloxazolo[4,5-b]pyridin-2-yl)piperazin-1-yl]methanone;
[2-(2,2-difluoro-5-azaspiro[2.4]heptan-5-yl)pyrimidin-5-yl]-[4-(5-methyloxazolo[4,5-b]pyridin-2-yl)piperazin-1-yl]methanone;
[6-(2,2-difluoro-5-azaspiro[2.4]heptan-5-yl)-4-methyl-3-pyridyl]-[4-(5-methyloxazolo[4,5-b]pyridin-2-yl)piperazin-1-yl]methanone;
[5-fluoro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[6-(2,2-difluoro-5-azaspiro[2.4]heptan-5-yl)-5-methyl-3-pyridyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[2-methyl-4-[3-(2,2,2-trifluoro-1-methyl-ethoxy)azetidin-1-yl]phenyl]methanone;
[2,6-difluoro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[2-chloro-4-[3-(2,2,2-trifluoro-1-methyl-ethoxy)azetidin-1-yl]phenyl]-[4-(5-methyloxazolo[4,5-b]pyridin-2-yl)piperazin-1-yl]methanone;
[4-(5-methyloxazolo[4,5-b]pyridin-2-yl)piperazin-1-yl]-[5-methyl-6-[[1-(trifluoromethyl)cyclopropyl]methoxy]-3-pyridyl]methanone;
[4-(5-chlorooxazolo[4,5-b]pyridin-2-yl)piperazin-1-yl]-[3-fluoro-4-[(1-methylcyclopropyl)methoxy]phenyl]methanone;
[4-(3-tert-butyl-1,2,4-triazol-1-yl)phenyl]-[4-(5-methyloxazolo [4, 5-b]pyridin-2-yl)piperazin-1-yl]methanone;
[3-fluoro-4-[(1-methylcyclopropyl)methoxy]phenyl]-[4-(5-methyloxazolo[4,5-b]pyridin-2-yl)piperazin-1-yl]methanone;
[4-(3-tert-butyl-1,2,4-triazol-1-yl)phenyl]-[4-(5-chlorooxazolo [4, 5-b]pyridin-2-yl)piperazin-1-yl]methanone;
[5-chloro-6-[3-(2,2,2-trifluoro-1-methyl-ethoxy)azetidin-1-yl]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[5-(2,2-dimethylpropyl)-4-methyl-1,2,4-triazol-3-yl]phenyl]methanone;
[5-chloro-6-(2,2-difluoro-5-azaspiro[2.4]heptan-5-yl)-3-pyridyl]-[4-(5-methyloxazolo[4,5-b]pyridin-2-yl)piperazin-1-yl]methanone;
[4-[5-(2,2-dimethylpropyl)-4-methyl-1,2,4-triazol-3-yl]phenyl]-[4-(5-methyloxazolo[4,5-b]pyridin-2-yl)piperazin-1-yl]methanone;
[6-(2,2-difluoro-5-azaspiro[2.4]heptan-5-yl)-5-fluoro-3-pyridyl]-[4-(5-methyloxazolo[4,5-b]pyridin-2-yl)piperazin-1-yl]methanone;
[4-[1-(2,2-dimethylpropyl)triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-fluoro-6-[3-(2,2,2-trifluoro-1-methyl-ethoxy)azetidin-1-yl]-3-pyridyl]methanone;
[4-(5-chlorooxazolo[4,5-b]pyridin-2-yl)piperazin-1-yl]-[6-(2,2-difluoro-5-azaspiro[2.4]heptan-5-yl)-5-fluoro-3-pyridyl]methanone;
[4-(5-chlorooxazolo[4,5-b]pyridin-2-yl)piperazin-1-yl]-[5-fluoro-6-[[1-(trifluoromethyl)cyclopropyl]methoxy]-3-pyridyl]methanone;
[4-(5-chlorooxazolo[4,5-b]pyridin-2-yl)piperazin-1-yl]-[5-fluoro-6-[(1-methylcyclopropyl)methoxy]-3-pyridyl]methanone;
[4-(5-tert-butyl-4-methyl-1,2,4-triazol-3-yl)phenyl]-[4-(5-chlorooxazolo[4,5-b]pyridin-2-yl)piperazin-1-yl]methanone;
[4-(5-tert-butyl-4-methyl-1,2,4-triazol-3-yl)phenyl]-[4-(5-methyloxazolo[4,5-b]pyridin-2-yl)piperazin-1-yl]methanone;
[5-chloro-6-[(1-methylcyclopropyl)methoxy]-3-pyridyl]-[4-(5-methyloxazolo[4,5-b]pyridin-2-yl)piperazin-1-yl]methanone;
[5-chloro-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-tert-butyl-1,2,4-oxadiazol-3-yl)-3-chloro-phenyl]-[4-(5-chlorooxazolo[4,5-b]pyridin-2-yl)piperazin-1-yl]methanone;
[4-(5-tert-butyl-1,2,4-oxadiazol-3-yl)-3-chloro-phenyl]-[4-(5-methyloxazolo[4,5-b]pyridin-2-yl)piperazin-1-yl]methanone;
[4-(5-tert-butyl-1,2,4-oxadiazol-3-yl)-3,5-difluoro-phenyl]-[4-(5-chlorooxazolo[4,5-b]pyridin-2-yl)piperazin-1-yl]methanone;
[4-(5-tert-butyl-1,2,4-oxadiazol-3-yl)-3-(trifluoromethyl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3-methyl-phenyl]methanone;
[4-(5-chlorooxazolo[4,5-b]pyridin-2-yl)piperazin-1-yl]-[4-[2-(2,2-dimethylpropyl)triazol-4-yl]-3-methyl-phenyl]methanone;
[5-fluoro-6-[(1-methylcyclopropyl)methoxy]-3-pyridyl]-[4-(5-methyloxazolo[4,5-b]pyridin-2-yl)piperazin-1-yl]methanone;
[5-chloro-6-[(1-methylcyclopropyl)methoxy]-3-pyridyl]-[4-(5-chlorooxazolo[4,5-b]pyridin-2-yl)piperazin-1-yl]methanone;
[5-fluoro-6-[[1-(trifluoromethyl)cyclopropyl]methoxy]-3-pyridyl]-[4-(5-methyloxazolo[4,5-b]pyridin-2-yl)piperazin-1-yl]methanone;
[5,6-bis[[1-(trifluoromethyl)cyclopropyl]methoxy]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-chloro-6-[[1-(trifluoromethyl)cyclopropyl]methoxy]-3-pyridyl]methanone;
[4-(5-chlorooxazolo[4,5-b]pyridin-2-yl)piperazin-1-yl]-[5-methyl-6-[[1-(trifluoromethyl)cyclopropyl]methoxy]-3-pyridyl]methanone;
[4-(5-chlorooxazolo[4,5-b]pyridin-2-yl)piperazin-1-yl]-[5-(trifluoromethyl)-6-[[1-(trifluoromethyl)cyclopropyl]methoxy]-3-pyridyl]methanone;
[4-(5-tert-butyl-1,3,4-oxadiazol-2-yl)-3-methyl-phenyl]-[4-(5-methyloxazolo [4, 5-b]pyridin-2-yl)piperazin-1-yl]methanone;
[4-(5-tert-butyl-1,3,4-oxadiazol-2-yl)-3-methyl-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)triazol-4-yl]-3-fluoro-phenyl]methanone;
[4-[2-(2,2-dimethylpropyl)triazol-4-yl]-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3-fluoro-phenyl]methanone;
[4-(1-tert-butyltriazol-4-yl)phenyl]-[4-(5-methyloxazolo[4,5-b]pyridin-2-yl)piperazin-1-yl]methanone;
[4-(1-tert-butyltriazol-4-yl)phenyl]-[4-(5-chlorooxazolo[4,5-b]pyridin-2-yl)piperazin-1-yl]methanone;
[4-(1-tert-butyl-1,2,4-triazol-3-yl)-3-chloro-phenyl]-[4-(5-methyloxazolo[4,5-b]pyridin-2-yl)piperazin-1-yl]methanone;
[4-(1-tert-butyl-1,2,4-triazol-3-yl)-3-chloro-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)triazol-4-yl] -3,5 -difluoro-phenyl] methanone;
[4-[2-(2,2-dimethylpropyl)triazol-4-yl]-3,5-difluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)triazol-4-yl]-3,5-difluoro-phenyl]methanone;
[4-(5-chlorooxazolo[4,5-b]pyridin-2-yl)piperazin-1-yl]-[6-(cyclopropylmethoxy)-5-methyl-3-pyridyl]methanone;
[4-(5-chlorooxazolo[4,5-b]pyridin-2-yl)piperazin-1-yl]-[5-methyl-6-[(1-methylcyclopropyl)methoxy]-3-pyridyl]methanone;
[4-(5-chlorooxazolo[4,5-b]pyridin-2-yl)piperazin-1-yl]-[5-methyl-6-[(3-methyloxetan-3-yl)methoxy]-3-pyridyl]methanone;
[4-(5-chlorooxazolo[4,5-b]pyridin-2-yl)piperazin-1-yl]-[6-[(2,2-difluoro-1-methylcyclopropyl)methoxy]-5-methyl-3-pyridyl]methanone;
[4-(5-chlorooxazolo[4,5-b]pyridin-2-yl)piperazin-1-yl]-[6-[(3-ethyloxetan-3-yl)methoxy]-5-methyl-3-pyridyl]methanone;
[4-(5-chlorooxazolo[4,5-b]pyridin-2-yl)piperazin-1-yl]-[6-[[1-(methoxymethyl)cyclopropyl]methoxy]-5-methyl-3-pyridyl]methanone;
[4-(5-chlorooxazolo[4,5-b]pyridin-2-yl)piperazin-1-yl]-[6-[(3-fluorooxetan-3-yl)methoxy]-5-methyl-3-pyridyl]methanone;
2-[1-[[5-[4-(5-chlorooxazolo[4,5-b]pyridin-2-yl)piperazine-1-carbonyl]-3-methyl-2-pyridyl] oxymethyl] cyclopropyl] acetonitrile;
1-[[5-[4-(5-chlorooxazolo[4,5-b]pyridin-2-yl)piperazine-1-carbonyl]-3-methyl-2-pyridyl]oxymethyl]cyclopropanecarbonitrile;
3-[[5-[4-(5-chlorooxazolo[4,5-b]pyridin-2-yl)piperazine-1-carbonyl]-3-methyl-2-pyridyl]oxymethyl]oxetane-3-carbonitrile;
[4-[1-(1-bicyclo[1.1.1]pentanyl)triazol-4-yl]phenyl]-[4-(5-methyloxazolo[4,5-b]pyridin-2-yl)piperazin-1-yl]methanone;
[4-[1-(1-bicyclo[1.1.1]pentanyl)triazol-4-yl]phenyl]-[4-(5-chlorooxazolo[4,5-b]pyridin-2-yl)piperazin-1-yl]methanone;
[4-(1-tert-butyl-1,2,4-triazol-3-yl)-3-methyl-phenyl]-[4-(5-methyloxazolo[4,5-b]pyridin-2-yl)piperazin-1-yl]methanone;
[4-(1-tert-butyl-1,2,4-triazol-3-yl)-3-methyl-phenyl]-[4-(5-chlorooxazolo[4,5-b]pyridin-2-yl)piperazin-1-yl]methanone;
3-[[5-[4-(5-chlorooxazolo[4,5-b]pyridin-2-yl)piperazine-1-carbonyl]-3-methyl-2-pyridyl]oxymethyl]-5-fluoro-benzonitrile;
[4-(5-chlorooxazolo[4,5-b]pyridin-2-yl)piperazin-1-yl]-[4-[1-(2,2,2-trifluoroethyl)triazol-4-yl]phenyl]methanone;
[4-(5-methyloxazolo[4,5-b]pyridin-2-yl)piperazin-1-yl]-[4-[1-(2,2,2-trifluoroethyl)triazol-4-yl]phenyl]methanone;
[4-(5-methyloxazolo[4,5-b]pyridin-2-yl)piperazin-1-yl]-[4-[1-[(3-methyloxetan-3-yl)methyl]triazol-4-yl]phenyl]methanone;
[4-(5-chlorooxazolo[4,5-b]pyridin-2-yl)piperazin-1-yl]-[4-[1-[(3-methyloxetan-3-yl)methyl]triazol-4-yl]phenyl]methanone;
1-[4-(5-chlorooxazolo[4,5-b]pyridin-2-yl)piperazin-1-yl]-3-phenyl-prop-2-yn-1-one;
1-[4-(5-chlorooxazolo[4,5-b]pyridin-2-yl)piperazin-1-yl]-3-(4-chlorophenyl)prop-2-yn-1-one;
[4-(5-chlorooxazolo[4,5-b]pyridin-2-yl)piperazin-1-yl]-[3-[2-(2,6-dichlorophenyl)ethynyl] azetidin-1-yl] methanone;
[3-[2-(2-chloro-4-fluoro-phenyl)ethynyl] azetidin-1-yl]-[4-(5-chlorooxazolo [4, 5-b]pyridin-2-yl)piperazin-1-yl]methanone;
[4-(5-chlorooxazolo[4,5-b]pyridin-2-yl)piperazin-1-yl]-(4-ethynyl-3-methoxyphenyl)methanone;
[4-[5-(1-bicyclo[1.1.1]pentanyl)-1,2,4-oxadiazol-3-yl]phenyl]-[4-(5-chlorooxazolo[4,5-b]pyridin-2-yl)piperazin-1-yl]methanone;
[4-(1-tert-butyl-1,2,4-triazol-3-yl)-3-fluoro-phenyl]-[4-(5-methyloxazolo[4,5-b]pyridin-2-yl)piperazin-1-yl]methanone;
[4-(1-tert-butyl-1,2,4-triazol-3-yl)-3-fluoro-phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[4-[1-[1-(trifluoromethyl)cyclopropyl]triazol-4-yl]phenyl]methanone;
[4-(5-methyloxazolo[4,5-b]pyridin-2-yl)piperazin-1-yl]-[4-[1-(3-methyloxetan-3-yl)triazol-4-yl]phenyl]methanone;
[4-(5-chlorooxazolo[4,5-b]pyridin-2-yl)piperazin-1-yl]-[4-[1-(3-methyloxetan-3-yl)triazol-4-yl]phenyl]methanone;
[4-(1-tert-butyl-1,2,4-triazol-3-yl)-3-(trifluoromethyl)phenyl]-[4-(5-methyloxazolo[4,5-b]pyridin-2-yl)piperazin-1-yl]methanone;
[4-(1-tert-butyl-1,2,4-triazol-3-yl)-3-(trifluoromethyl)phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[4-[1-(oxetan-3-ylmethyl)triazol-4-yl]phenyl]methanone;
[4-(5-chlorooxazolo[4,5-b]pyridin-2-yl)piperazin-1-yl]-[4-[1-(oxetan-3-ylmethyl)triazol-4-yl]phenyl]methanone;
[4-(5-tert-butyl-1,3,4-oxadiazol-2-yl)-3-chloro-phenyl]-[4-(5-chlorooxazolo[4,5-b]pyridin-2-yl)piperazin-1-yl]methanone;
[4-(5-tert-butyl-1,3,4-oxadiazol-2-yl)-3-fluoro-5-hydroxy-phenyl]-[4-(5-chlorooxazolo[4,5-b]pyridin-2-yl)piperazin-1-yl]methanone;
[4-(5-tert-butyl-1,3,4-oxadiazol-2-yl)-3-fluoro-5-hydroxy-phenyl]-[4-(5-methyloxazolo[4,5-b]pyridin-2-yl)piperazin-1-yl]methanone;
[4-(5-tert-butyl-2-methyl-1,2,4-triazol-3-yl)phenyl]-[4-(5-chlorooxazolo[4,5-b]pyridin-2-yl)piperazin-1-yl]methanone;
5-[4-(5-chlorooxazolo[4,5-b]pyridin-2-yl)piperazine-1-carbonyl]-2-[1-(2,2-dimethylpropyl)triazol-4-yl]benzonitrile;
5-[4-(5-chlorooxazolo[4,5-b]pyridin-2-yl)piperazine-1-carbonyl]-2-[2-(2,2-dimethylpropyl)triazol-4-yl]benzonitrile;
[6-(1-tert-butylpyrazol-4-yl)-5-methyl-3-pyridyl]-[4-(5-chlorooxazolo[4,5-b]pyridin-2-yl)piperazin-1-yl]methanone;
[6-(4-tert-butylpyrazol-1-yl)-5-methyl-3-pyridyl]-[4-(5-chlorooxazolo [4, 5-b]pyridin-2-yl)piperazin-1 -yl]methanone;
[6-(1-tert-butylpyrazol-4-yl)-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-methyl-6-(tetrahydrofuran-2-ylmethoxy)-3-pyridyl]methanone;
[4-(5-tert-butyl-1,3,4-oxadiazol-2-yl)-3-(trifluoromethyl)phenyl]-[4-(5-methyloxazolo[4,5-b]pyridin-2-yl)piperazin-1-yl]methanone;
[4-(5-tert-butyl-1,3,4-oxadiazol-2-yl)-3-(trifluoromethyl)phenyl]-[4-(5-chlorooxazolo[4,5-b]pyridin-2-yl)piperazin-1-yl]methanone;
[4-(5-methyloxazolo[4,5-b]pyridin-2-yl)piperazin-1-yl]-[6-methyl-5-[[1-(trifluoromethyl)cyclopropyl]methoxy]pyrazin-2-yl]methanone;
[4-(5-chlorooxazolo[4,5-b]pyridin-2-yl)piperazin-1-yl]-[5-methyl-6-(oxetan-2-ylmethoxy)-3-pyridyl]methanone;
2-[1-(2,2-dimethylpropyl)triazol-4-yl]-5-[4-(5-methyloxazolo[4,5-b]pyridin-2-yl)piperazine-1-carbonyl]benzonitrile;
2-[2-(2,2-dimethylpropyl)triazol-4-yl]-5-[4-(5-methyloxazolo[4,5-b]pyridin-2-yl)piperazine-1-carbonyl]benzonitrile;
[4-(5-chlorooxazolo[4,5-b]pyridin-2-yl)piperazin-1-yl]-[4-[1-(2,2-dimethylpropyl)triazol-4-yl]-3-methoxy-phenyl]methanone;
[4-(5-chlorooxazolo[4,5-b]pyridin-2-yl)piperazin-1-yl]-[4-[2-(2,2-dimethylpropyl)triazol-4-yl]-3-methoxy-phenyl]methanone;
[4-[1-(2,2-dimethylpropyl)triazol-4-yl]-3-methoxy-phenyl]-[4-(5-methyloxazolo[4,5-b]pyridin-2-yl)piperazin-1-yl]methanone;
[4-[2-(2,2-dimethylpropyl)triazol-4-yl]-3-methoxy-phenyl]-[4-(5-methyloxazolo[4,5-b]pyridin-2-yl)piperazin-1-yl]methanone;
[4-(1-tert-butyl-1,2,4-triazol-3-yl)-3-methoxy-phenyl]-[4-(5-methyloxazolo[4,5-b]pyridin-2-yl)piperazin-1-yl]methanone;
[4-(1-tert-butyl-1,2,4-triazol-3-yl)-3-methoxy-phenyl]-[4-(5-chlorooxazolo[4,5-b]pyridin-2-yl)piperazin-1-yl]methanone;
[5-methoxy-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-[(1-fluorocyclopropyl)methyl]triazol-4-yl]phenyl]methanone;
[4-(5-chlorooxazolo[4,5-b]pyridin-2-yl)piperazin-1-yl]-[5-methyl-6-(tetrahydropyran-2-ylmethoxy)-3-pyridyl]methanone;
[4-(5-chlorooxazolo[4,5-b]pyridin-2-yl)piperazin-1-yl]-[6-[(1-methoxycyclopropyl)methoxy]-5-methyl-3-pyridyl]methanone;
[4-[1-[(1-fluorocyclopropyl)methyl]triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[6-[1-(2,2-dimethylpropyl)triazol-4-yl]-5-fluoro-3-pyridyl]methanone;
[4-(5-chlorooxazolo[4,5-b]pyridin-2-yl)piperazin-1-yl]-[6-[3-(2,2-dimethylpropyl)triazol-4-yl]-5-fluoro-3-pyridyl]methanone;
[4-(5-chlorooxazolo[4,5-b]pyridin-2-yl)piperazin-1-yl]-[6-[2-(2,2-dimethylpropyl)triazol-4-yl]-5-fluoro-3-pyridyl]methanone;
[3-[(2-chloro-4-fluoro-phenoxy)methyl] azetidin-1-yl]-[4-(5-chlorooxazolo [4, 5-b]pyridin-2-yl)piperazin-1-yl]methanone;
[3-[(2-chloro-4-fluoro-phenoxy)methyl]azetidin-1-yl]-[4-(5-methyloxazolo[4,5-b]pyridin-2-yl)piperazin-1-yl]methanone;
2-chloro-N-[1-[4-(5-chlorooxazolo[4,5-b]pyridin-2-yl)piperazine-1-carbonyl]azetidin-3-yl]-4-fluoro-benzamide;
2-chloro-4-fluoro-N-[1-[4-(5-methyloxazolo[4,5-b]pyridin-2-yl)piperazine-1-carbonyl]azetidin-3-yl]benzamide;
[4-(5-chlorooxazolo[4,5-b]pyridin-2-yl)piperazin-1-yl]-[6-[(2S)-2,3-difluoropropoxy]-5-methyl-3-pyridyl]methanone;
N-(2-chloro-4-fluoro-phenyl)-1-[4-(5-chlorooxazolo[4,5-b]pyridin-2-yl)piperazine-1-carbonyl]azetidine-3 -carboxamide;
N-(2-chloro-4-fluoro-phenyl)-1-[4-(5-methyloxazolo[4,5-b]pyridin-2-yl)piperazine-1-carbonyl]azetidine-3 -carboxamide;
[4-(5-chlorooxazolo[4,5-b]pyridin-2-yl)piperazin-1-yl]-[5-methyl-6-(3,3,3-trifluoropropoxy)-3 -pyridyl] methanone;
[4-(1-tert-butyltriazol-4-yl)-3-methyl-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[6-[(2R)-2,3-difluoropropoxy]-5-methyl-3-pyridyl]methanone;
[4-(5-tert-butyl-2-methyl-1,2,4-triazol-3-yl)phenyl]-[4-(5-methyloxazolo[4,5-b]pyridin-2-yl)piperazin-1-yl]methanone;
[6-[1-(2,2-dimethylpropyl)triazol-4-yl]-5-fluoro-3-pyridyl]-[4-(5-methyloxazolo[4,5-b]pyridin-2-yl)piperazin-1-yl]methanone;
[6-[2-(2,2-dimethylpropyl)triazol-4-yl]-5-fluoro-3-pyridyl]-[4-(5-methyloxazolo[4,5-b]pyridin-2-yl)piperazin-1-yl]methanone;
[6-[3-(2,2-dimethylpropyl)triazol-4-yl]-5-fluoro-3-pyridyl]-[4-(5-methyloxazolo[4,5-b]pyridin-2-yl)piperazin-1-yl]methanone;
[4-(5-tert-butyl-1,3,4-oxadiazol-2-yl)phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-(1H-triazol-5-yl)phenyl] methanone;
[4-(5-tert-butyl-1,3,4-oxadiazol-2-yl)-3-chloro-phenyl]-[4-(5-methyloxazolo[4,5-b]pyridin-2-yl)piperazin-1-yl]methanone;
[6-(3-tert-butyl-1,2,4-triazol-1-yl)-5-methyl-3-pyridyl]-[4-(5-methyloxazolo[4,5-b]pyridin-2-yl)piperazin-1-yl]methanone;
[6-(3-tert-butyl-1,2,4-triazol-1-yl)-5-methyl-3-pyridyl]-[4-(5-chlorooxazolo[4,5-b]pyridin-2-yl)piperazin-1-yl]methanone;
[6-(4-tert-butylpyrazol-1-yl)-5-methyl-3-pyridyl]-[4-(5-methyloxazolo[4,5-b]pyridin-2-yl)piperazin-1-yl]methanone;
3-[4-[4-[4-(5-chlorooxazolo[4,5-b]pyridin-2-yl)piperazine-1-carbonyl]phenyl]triazol-1-yl]-2, 2-dimethyl-propanenitrile;
[4-(5-chlorooxazolo[4,5-b]pyridin-2-yl)piperazin-1-yl]-[4-[1-(2-fluoro-2-methylpropyl)triazol-4-yl]phenyl]methanone;
[4-[1-(2-fluoro-2-methyl-propyl)triazol-4-yl]phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[1-(2-fluoro-1,1-dimethylethyl)triazol-4-yl]phenyl]methanone;
[4-[1-(2-fluoro-1,1-dimethyl-ethyl)triazol-4-yl]phenyl]-[4-(5-methyloxazolo[4,5-b]pyridin-2-yl)piperazin-1-yl]methanone;
3-[4-[4-[4-(5-chlorooxazolo[4,5-b]pyridin-2-yl)piperazine-1-carbonyl]phenyl]triazol-2-yl]-2,2-dimethyl-propanenitrile;
[4-(5-chlorooxazolo[4,5-b]pyridin-2-yl)piperazin-1-yl]-[6-methyl-5-[[1-(trifluoromethyl)cyclopropyl]methoxy]pyrazin-2-yl]methanone;
[4-(5-chlorooxazolo[4,5-b]pyridin-2-yl)piperazin-1-yl]-[6-[1-(2,2-dimethylpropyl)pyrazol-4-yl]-5-methyl-3-pyridyl]methanone;
[6-[1-(2,2-dimethylpropyl)pyrazol-4-yl]-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[5-methoxy-6-[[1-(trifluoromethyl)cyclopropyl]methoxy]-3-pyridyl]methanone;
[4-(5-chlorooxazolo[4,5-b]pyridin-2-yl)piperazin-1-yl]-[4-[1-(2-methoxy-2-methylpropyl)triazol-4-yl]phenyl]methanone;
[4-(5-chlorooxazolo[4,5-b]pyridin-2-yl)piperazin-1-yl]-[4-(5-isobutyl-1,3,4-oxadiazol-2-yl)phenyl]methanone;
[4-(5-methyloxazolo[4,5-b]pyridin-2-yl)piperazin-1-yl]-[6-methyl-5-[[1-(trifluoromethyl)cyclopropyl]methoxy]-2-pyridyl]methanone;
[4-(5-chlorooxazolo[4,5-b]pyridin-2-yl)piperazin-1-yl]-[6-methyl-5-[[1-(trifluoromethyl)cyclopropyl]methoxy]-2-pyridyl]methanone;
[4-(5-chlorooxazolo[4,5-b]pyridin-2-yl)piperazin-1-yl]-[4-[1-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[4-[2-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[4-[3-(2,2-dimethylpropyl)triazol-4-yl]-3-(trifluoromethyl)phenyl]methanone;
[4-(5-chlorooxazolo[4,5-b]pyridin-2-yl)piperazin-1-yl]-[6-[1-(2,2-dimethylpropyl)triazol-4-yl]-5-methyl-3-pyridyl]methanone;
[4-(5-chlorooxazolo[4,5-b]pyridin-2-yl)piperazin-1-yl]-[6-[2-(2,2-dimethylpropyl)triazol-4-yl]-5-methyl-3-pyridyl]methanone;
[(3R)-4-(5-chlorooxazolo[4,5-b]pyridin-2-yl)-3-methyl-piperazin-1-yl]-[4-[1-(2,2-dimethylpropyl)triazol-4-yl]phenyl]methanone;
[(3S)-4-(5-chlorooxazolo[4,5-b]pyridin-2-yl)-3-methyl-piperazin-1-yl]-[4-[1-(2,2-dimethylpropyl)triazol-4-yl]phenyl]methanone;
[4-(5-chlorooxazolo[4,5-b]pyridin-2-yl)piperazin-1-yl]-[4-(5-isobutyl-1,3,4-oxadiazol-2-yl)-3-methyl-phenyl]methanone;
[4-(5-chlorooxazolo[4,5-b]pyridin-2-yl)piperazin-1-yl]-[2-chloro-6-[[1-(trifluoromethyl)cyclopropyl]methoxy]-3-pyridyl]methanone;
[4-[2-(2,2-dimethylpropyl)tetrazol-5-yl]phenyl]-[4-(5-methyloxazolo[4,5-b]pyridin-2-yl)piperazin-1-yl]methanone;
[6-(5-tert-butyl-1,3,4-oxadiazol-2-yl)-5-fluoro-3-pyridyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[6-[1-(2,2-dimethylpropyl)pyrazol-4-yl]-5-(trifluoromethyl)-3-pyridyl]methanone;
[4-(5-chlorooxazolo[4,5-b]pyridin-2-yl)piperazin-1-yl]-[3-fluoro-4-[5-(2-fluoro-1,1-dimethyl-ethyl)-1,3,4-oxadiazol-2-yl]phenyl]methanone;
[4-(5-tert-butyl-1,3,4-oxadiazol-2-yl)-3-fluoro-phenyl]-[4-(5-chlorooxazolo[4,5-b]pyridin-2-yl)piperazin-1-yl]methanone;
[6-[1-(2,2-dimethylpropyl)pyrazol-4-yl]-5-(trifluoromethyl)-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]phenyl]methanone;
[4-(5-tert-butyl-1,2,4-oxadiazol-3-yl)phenyl]-[8-(5-chlorooxazolo[4,5-b]pyridin-2-yl)-3,8-diazabicyclo[3.2.1]octan-3-yl]methanone;
[4-(5-chlorooxazolo[4,5-b]pyridin-2-yl)-3-(hydroxymethyl)piperazin-1-yl]-[4-[1-(2,2-dimethylpropyl)triazol-4-yl]phenyl]methanone;
[4-[1-(2,2-dimethylpropyl)triazol-4-yl]phenyl]-[4-(5-fluorooxazolo[4,5-b]pyridin-2-yl)piperazin-1-yl]methanone;
[4-(3-tert-butyl-1,2,4-triazol-1-yl)-3-methyl-phenyl]-[4-(5-chlorooxazolo[4,5-b]pyridin-2-yl)piperazin-1-yl]methanone;
[4-(3-tert-butyl-1,2,4-triazol-1-yl)-3-methyl-phenyl]-[4-(5-methyloxazolo [4, 5-b]pyridin-2-yl)piperazin-1-yl]methanone;
[(2S)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone;
[(2R)-4-(5-chlorooxazolo[4,5-b]pyridin-2-yl)-2-(hydroxymethyl)piperazin-1-yl]-[4-[1-(2,2-dimethylpropyl)triazol-4-yl]phenyl]methanone;
[4-(3-tert-butyl-1,2,4-triazol-1-yl)-3-chloro-phenyl]-[4-(5-methyloxazolo[4,5-b]pyridin-2-yl)piperazin-1-yl]methanone;
[4-(3-tert-butyl-1,2,4-triazol-1-yl)-3-chloro-phenyl]-[4-(5-chlorooxazolo[4,5-b]pyridin-2-yl)piperazin-1-yl]methanone;
[4-(5-chlorooxazolo[4,5-b]pyridin-2-yl)piperazin-1-yl]-[4-[1-(2,2-dimethylpropyl)-1,2,4-triazol-3-yl]-3-fluoro-phenyl]methanone;
[4-[1-(2,2-dimethylpropyl)-1,2,4-triazol-3-yl]-3-fluoro-phenyl]-[4-(5-methyloxazolo[4,5-b]pyridin-2-yl)piperazin-1-yl]methanone;
[4-(5-chlorooxazolo[4,5-b]pyridin-2-yl)-2-methyl-piperazin-1-yl]-[4-[1-(2,2-dimethylpropyl)triazol-4-yl]phenyl]methanone;
[2-chloro-6-[[1-(trifluoromethyl)cyclopropyl]methoxy]-3-pyridyl]-[4-(5-methyloxazolo[4,5-b]pyridin-2-yl)piperazin-1-yl]methanone;
[4-(5-tert-butyl-1,2,4-oxadiazol-3-yl)phenyl]-[3-(5-chlorooxazolo[4,5-b]pyridin-2-yl)-3,8-diazabicyclo[3.2.1]octan-8-yl]methanone;
[4-(5-chlorooxazolo[4,5-b]pyridin-2-yl)piperazin-1-yl]-[3-fluoro-4-[5-(2-fluoro-2-methyl-propyl)-1,3,4-oxadiazol-2-yl]phenyl]methanone;
[4-[2-(2,2-dimethylpropyl)tetrazol-5-yl]-3,5-difluoro-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5 -yl] -3,5 -difluoro-phenyl] methanone;
[4-[2-(2,2-dimethylpropyl)tetrazol-5-yl]-3-methyl-phenyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]-3-methyl-phenyl]methanone;
[4-[2-(2,2-dimethylpropyl)tetrazol-5-yl]-3-fluoro-phenyl-[4-(5-methyloxazolo[4,5-b]pyridin-2-yl)piperazin-1-yl]methanone;
[4-(5-chlorooxazolo[4,5-b]pyridin-2-yl)piperazin-1-yl]-[4-[2-(2,2-dimethylpropyl)tetrazol-5-yl]-3-fluoro-phenyl]methanone;
[4-(5-chlorooxazolo[4,5-b]pyridin-2-yl)piperazin-1-yl]-[6-[2-(2,2-dimethylpropyl)tetrazol-5-yl]-5-methoxy-3-pyridyl]methanone;
[6-[2-(2,2-dimethylpropyl)tetrazol-5-yl]-5-methoxy-3-pyridyl]-[4-(5-methyloxazolo[4,5-b]pyridin-2-yl)piperazin-1-yl]methanone;
[6-[2-(2,2-dimethylpropyl)tetrazol-5-yl]-5-methyl-3-pyridyl]-[4-(5-methyloxazolo[4,5-b]pyridin-2-yl)piperazin-1-yl]methanone;
[4-(5-chlorooxazolo[4,5-b]pyridin-2-yl)piperazin-1-yl]-[6-[2-(2,2-dimethylpropyl)tetrazol-5-yl]-5-methyl-3-pyridyl]methanone;
[6-[2-(2,2-dimethylpropyl)tetrazol-5-yl]-5-fluoro-3-pyridyl]-[4-(5-methyloxazolo[4,5-b]pyridin-2-yl)piperazin-1-yl]methanone; or
[4-(5-chlorooxazolo[4,5-b]pyridin-2-yl)piperazin-1-yl]-[6-[2-(2,2-dimethylpropyl)tetrazol-5-yl]-5-fluoro-3-pyridyl]methanone.

12. A process of manufacturing the compounds of formula (I) according to any one of claims 1 to 10, comprising:
reacting an acid of formula **1,** wherein L¹, A, and R^{A1} to R^{A3} are as defined in any one of claims 1 to 10, 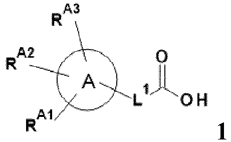
with an amine of formula **2,** wherein R¹ to R⁷ are as defined in any one of claims 1 to 10 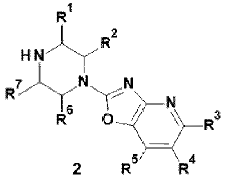
in the presence of a base and a coupling reagent,
to form said compound of formula (I).

13. A compound of formula (I) according to any one of claims 1 to 11 for use as therapeutically active substance.

14. A pharmaceutical composition comprising a compound of formula (I) according to any one of claims 1 to 11 and a therapeutically inert carrier.

15. A compound of formula (I) according to any one of claims 1 to 11 for use in the treatment or prophylaxis of neuroinflammation, neurodegenerative diseases, pain, cancer and/or mental disorders in a mammal, in particular for use in the treatment or prophylaxis of multiple sclerosis, Alzheimer's disease, Parkinson's disease, amyotrophic lateral sclerosis, traumatic brain injury, neurotoxicity, stroke, epilepsy, anxiety, migraine, depression, hepatocellular carcinoma, colon carcinogenesis, ovarian cancer, neuropathic pain, chemotherapy induced neuropathy, acute pain, chronic pain and/or spasticity associated with pain in a mammal.

## Patentansprüche

1. Verbindung der Formel (I) 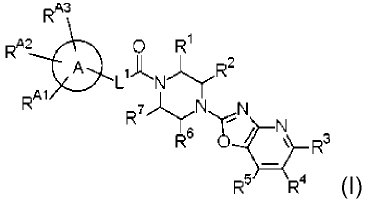 oder ein pharmazeutisch unbedenkliches Salz davon, wobei:
(i) R¹, R², R⁶ und R⁷ jeweils unabhängig voneinander Wasserstoff, C₁-C₆-Alkyl oder Hydroxy-C₁-C₆-alkyl sind; oder
(ii) R¹ und R⁶ zusammen eine Brücke der Formel -(CH₂)ₘ- bilden, wobei m 1, 2 oder 3 ist; und R² und R⁷ unabhängig voneinander Wasserstoff, C₁-C₆-Alkyl oder Hydroxy-C₁-C₆-alkyl sind; oder
(iii) R¹ und R⁷ zusammen eine Brücke der Formel -(CH₂)ₘ- bilden, wobei m 1, 2 oder 3 ist; und R² und R⁶ unabhängig voneinander Wasserstoff, C₁-C₆-Alkyl oder Hydroxy-C₁-C₆-alkyl sind; oder
(iv) R² und R⁶ zusammen eine Brücke der Formel -(CH₂)ₘ- bilden, wobei m 1, 2 oder 3 ist; und R¹ und R⁷ unabhängig voneinander Wasserstoff, C₁-C₆-Alkyl oder Hydroxy-C₁-C₆-alkyl sind; oder
(v) R² und R⁷ zusammen eine Brücke der Formel -(CH₂)ₘ- bilden, wobei m 1, 2 oder 3 ist; und R¹ und R⁶ unabhängig voneinander Wasserstoff, C₁-C₆-Alkyl oder Hydroxy-C₁-C₆-alkyl sind;
R³, R⁴ und R⁵ jeweils unabhängig voneinander Wasserstoff, Halogen, Cyano, C₁-C₆-Alkyl, Halogen-C₁-C₆-alkyl, Hydroxy-C₁-C₆-alkyl, C₃₋₁₂-Cycloalkyl, C₁-C₆-Alkoxy oder Halogen-C₁-C₆-alkoxy sind;
R^{A1} Wasserstoff, Halogen, Oxo, Acetyl, Cyano, Cyano-C₁-C₆-alkyl, Hydroxy, Hydroxy-C₁-C₆-alkyl, C₁-C₆-Alkyl, C₁-C₆-Alkanoyl, C₂-C₆-Alkenyl, C₂-C₆-Alkinyl, C₁-C₆-Alkoxy, C₁-C₆-Alkoxy-C₁-C₆-alkyl, Halogen-C₁-C₆-alkoxy, Halogen-C₁-C₆-alkyl oder eine Gruppe 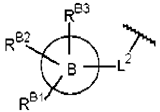 ist;
R^{A2} Wasserstoff, Halogen, Oxo, Acetyl, Cyano, Cyano-C₁-C₆-alkyl, Hydroxy, Hydroxy-C₁-C₆-alkyl, C₁-C₆-Alkyl, C₁-C₆-Alkanoyl, C₂-C₆-Alkenyl, C₂-C₆-Alkinyl, C₁-C₆-Alkoxy, C₁-C₆-Alkoxy-C₁-C₆-alkyl, Halogen-C₁-C₆-alkoxy, Halogen-C₁-C₆-alkyl oder eine Gruppe 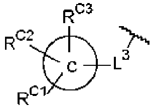 ist;
R^{A3} Wasserstoff, Halogen, Oxo, Acetyl, Cyano, Cyano-C₁-C₆-alkyl, Hydroxy, Hydroxy-C₁-C₆-alkyl, C₁-C₆-Alkyl, C₁-C₆-Alkanoyl, C₂-C₆-Alkenyl, C₂-C₆-Alkinyl, C₁-C₆-Alkoxy, C₁-C₆-Alkoxy-C₁-C₆-alkyl, Halogen-C₁-C₆-alkoxy oder Halogen-C₁-C₆-alkyl ist;
R^{B1} Wasserstoff, Halogen, Oxo, Acetyl, Cyano, Cyano-C₁-C₆-alkyl, Hydroxy, Hydroxy-C₁-C₆-alkyl, C₁-C₆-Alkyl, C₁-C₆-Alkanoyl, C₂-C₆-Alkenyl, C₂-C₆-Alkinyl, C₁-C₆-Alkoxy, C₁-C₆-Alkoxy-C₁-C₆-alkyl, Halogen-C₁-C₆-alkoxy, Halogen-C₁-C₆-alkyl oder eine Gruppe 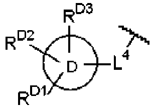 ist;
R^{B2}, R^{B3}, R^{C1}, R^{C2}, R^{C3}, R^{D1}, R^{D2} und R^{D3} jeweils unabhängig voneinander Wasserstoff, Halogen, Oxo, Acetyl, Cyano, Cyano-C₁-C₆-alkyl, Hydroxy, Hydroxy-C₁-C₆-alkyl, C₁-C₆-Alkyl, C₁-C₆-Alkanoyl, C₂-C₆-Alkenyl, C₂-C₆-Alkinyl, C₁-C₆-Alkoxy, C₁-C₆-Alkoxy-C₁-C₆-alkyl, Halogen-C₁-C₆-alkoxy oder Halogen-C₁-C₆-alkyl sind;
L¹ eine kovalente Bindung, C₂-C₆-Alkinyl, Carbonyl, -C(O)NH-, -NHC(O)-, -CH₂O-, -OCH₂- oder SO₂ ist;
L², L³ und L⁴ jeweils unabhängig voneinander eine kovalente Bindung, C₂-C₆-Alkinyl, -CH₂-, -O-, Carbonyl, -C(O)NH-, -NHC(O)-, -CH₂O-, -OCH₂- oder SO₂ sind; und
A, B, C und D jeweils unabhängig voneinander C₆₋₁₄-Aryl, C₁₋₁₃-Heteroaryl, C₃₋₁₂-Cycloalkyl oder C₂₋₉-Heterocyclyl sind.

2. Verbindung der Formel (I) nach Anspruch 1 oder ein pharmazeutisch unbedenkliches Salz davon, wobei:
(i) R¹ und R² unabhängig voneinander Wasserstoff, C₁-C₆-Alkyl oder Hydroxy-C₁-C₆-alkyl sind; und R⁶ und R⁷ Wasserstoff sind; oder
(ii) R¹ und R⁷ zusammen eine Brücke der Formel -(CH₂)₂- bilden; und R² und R⁶ beide Wasserstoff sind; oder
(iii) R² und R⁶ zusammen eine Brücke der Formel -(CH₂)₂- bilden; und R¹ und R⁷ beide Wasserstoff sind.

3. Verbindung der Formel (I) nach Anspruch 1 oder ein pharmazeutisch unbedenkliches Salz davon, wobei:
R¹, R⁶ und R⁷ alle Wasserstoff sind; und
R² Wasserstoff oder C₁-C₆-Alkyl ist.

4. Verbindung der Formel (I) nach Anspruch 1 oder ein pharmazeutisch unbedenkliches Salz davon, wobei:
R¹, R⁶ und R⁷ alle Wasserstoff sind; und
R² Wasserstoff oder Methyl ist.

5. Verbindung der Formel (I) nach einem der Ansprüche 1 bis 4 oder ein pharmazeutisch unbedenkliches Salz davon, wobei:
R³ Wasserstoff, Halogen, Cyano, C₁-C₆-Alkyl, Halogen-C₁-C₆-alkyl, C₃₋₁₂-Cycloalkyl oder C₁-C₆-Alkoxy ist;
R⁴ Wasserstoff ist; und
R⁵ Wasserstoff oder Halogen ist.

6. Verbindung der Formel (I) nach einem der Ansprüche 1 bis 4 oder ein pharmazeutisch unbedenkliches Salz davon, wobei:
R³ C₁-C₆-Alkyl oder Halogen ist; und
R⁴ und R⁵ beide Wasserstoff sind.

7. Verbindung der Formel (I) nach einem der Ansprüche 1 bis 4 oder ein pharmazeutisch unbedenkliches Salz davon, wobei:
R³ Methyl oder Chlor ist; und
R⁴ und R⁵ beide Wasserstoff sind.

8. Verbindung der Formel (I) nach einem der Ansprüche 1 bis 7 oder ein pharmazeutisch unbedenkliches Salz davon, wobei:
L¹ eine kovalente Bindung oder C₂-C₆-Alkinyl ist;
A C₆₋₁₄-Aryl, C₁₋₁₃-Heteroaryl oder C₂₋₉-Heterocyclyl ist;
R^{A1} Wasserstoff, Halogen, C₁-C₆-Alkyl, C₂-C₆-Alkenyl, C₂-C₆-Alkinyl, C₁-C₆-Alkoxy, Halogen-C₁-C₆-alkoxy, Halogen-C₁-C₆-alkyl oder eine Gruppe 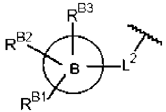 ist;
R^{A2} Wasserstoff, Halogen, Cyano, Hydroxy, C₁-C₆-Alkyl, C₁-C₆-Alkoxy, Halogen-C₁-C₆-alkoxy, Halogen-C₁-C₆-alkyl oder eine Gruppe 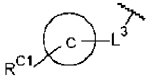 ist;
R^{A3} Wasserstoff oder Halogen ist;
L² eine kovalente Bindung, -C(O)NH-, -NHC(O)-, -CH₂O-, -OCH₂- oder SO₂ ist;
B C₆₋₁₄-Aryl, C₁₋₁₃-Heteroaryl, C₃₋₁₂-Cycloalkyl oder C₂₋₉-Heterocyclyl ist;
R^{B1} Wasserstoff, Halogen, Hydroxy, Acetyl, Cyano, Cyano-C₁-C₆-alkyl, C₁-C₆-Alkyl, Halogen-C₁-C₆-alkyl, Hydroxy-C₁-C₆-alkyl, C₂-C₆-Alkenyl, C₁-C₆-Alkoxy, Halogen-C₁-C₆-alkoxy, C₁-C₆-Alkoxy-C₁-C₆-alkyl, C₁-C₆-Alkanoyl, Oxo oder eine Gruppe 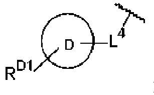 ist;
R^{B2} Wasserstoff, Halogen, Oxo oder C₁-C₆-Alkyl ist;
R^{B3} Wasserstoff oder Halogen ist;
L³ eine kovalente Bindung oder -CH₂O- ist;
C C₆₋₁₄-Aryl, C₁₋₁₃-Heteroaryl, C₃₋₁₂-Cycloalkyl oder C₂₋₉-Heterocyclyl ist;
R^{C1} Wasserstoff, Halogen, C₁-C₆-Alkyl oder Halogen-C₁-C₆-alkyl ist;
L⁴ eine kovalente Bindung, -CH₂- oder -O- ist;
D C₆₋₁₄-Aryl, C₃₋₁₂-Cycloalkyl oder C₂₋₉-Heterocyclyl ist; und
R^{D1} C₁-C₆-Alkyl, Halogen-C₁-C₆-alkyl oder Halogen ist.

9. Verbindung der Formel (I) nach einem der Ansprüche 1 bis 8 oder ein pharmazeutisch unbedenkliches Salz davon, wobei:
L¹ eine kovalente Bindung ist;
A C₆₋₁₄-Aryl oder C₁₋₁₃-Heteroaryl ist;
R^{A1} eine Gruppe 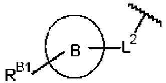 ist;
R^{A2} Wasserstoff, Halogen oder C₁-C₆-Alkyl ist;
R^{A3} Wasserstoff oder Halogen ist;
L² eine kovalente Bindung oder -CH₂O- ist;
B C₁₋₁₃-Heteroaryl oder C₃₋₁₂-Cycloalkyl ist; und
R^{B1} C₁-C₆-Alkyl oder Halogen-C₁-C₆-alkyl ist.

10. Verbindung der Formel (I) nach einem der Ansprüche 1 bis 9 oder ein pharmazeutisch unbedenkliches Salz davon, wobei:
L¹ eine kovalente Bindung ist;
A Phenyl oder Pyridyl ist;
R^{A1} eine Gruppe 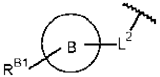 ist;
R^{A2} Wasserstoff, Fluor, Chlor oder Methyl ist;
R^{A3} Wasserstoff oder Fluor ist;
L² eine kovalente Bindung oder -CH₂O- ist;
B Oxadiazolyl, Pyrazolyl, Triazolyl, Tetrazolyl oder Cyclopropyl ist; und
R^{B1} *tert*-Butyl, 2,2-Dimethylpropyl, Trifluormethyl oder 2-Fluor-1,1-dimethylethyl ist.

11. Verbindung der Formel (I) nach Anspruch 1 oder ein pharmazeutisch unbedenkliches Salz davon, wobei die Verbindung der Formel (I) folgende ist:
[4-(5-Methyloxazolo[4,5-b]pyridin-2-yl)piperazin-1-yl]-(2-phenyl-1,3-benzoxazol-6-yl)methanon;
(4-Oxazolo[4,5-b]pyridin-2-ylpiperazin-1-yl)-(2-phenyl-1,3-benzoxazol-6-yl)methanon;
[4-(5-Methyloxazolo[4,5-b]pyridin-2-yl)piperazin-1-yl]-(2-pyrrolidin-1-yl-1,3-benzoxazol-6-yl)methanon;
[4-(5-tert-Butyl-1,2,4-oxadiazol-3-yl)phenyl]-[4-(5-methyloxazolo[4,5-b]pyridin-2-yl)piperazin-1-yl] methanon;
[4-[5-(2,2-Dimethylpropyl)-1,2,4-oxadiazol-3-yl]phenyl]-[4-(5-methyloxazolo[4,5-b]pyridin-2-yl)piperazin-1-yl]methanon;
[2-(2-Azaspiro[3.4]octan-2-yl)-1,3-benzoxazol-6-yl]-[4-(5-methyloxazolo[4,5-b]pyridin-2-yl)piperazin-1-yl]methanon;
[4-[5-(2-Hydroxy-1,1-dimethylethyl)-1,2,4-oxadiazol-3-yl]phenyl]-[4-(5-methyloxazolo[4,5-b]pyridin-2-yl)piperazin-1-yl]methanon;
[4-(5-tert-Butyl-1,2,4-oxadiazol-3-yl)phenyl]-[4-(7-chloroxazolo[4,5-b]pyridin-2-yl)piperazin-1-yl] methanon;
[4-(7-Chloroxazolo[4,5-b]pyridin-2-yl)piperazin-1-yl]-[4-[5-(2,2-dimethylpropyl)-1,2,4-oxadiazol-3-yl]phenyl]methanon;
[4-(5-Chloroxazolo[4,5-b]pyridin-2-yl)piperazin-1-yl]-[4-[5-(2,2-dimethylpropyl)-1,2,4-oxadiazol-3-yl]phenyl]methanon;
[4-[5-(2-Fluor-1,1-dimethylethyl)-1,2,4-oxadiazol-3-yl]phenyl]-[4-(5-methyloxazolo[4,5-b]pyridin-2-yl)piperazin-1-yl]methanon;
[4-(5-tert-Butyl-1,2,4-oxadiazol-3-yl)phenyl]-[4-(5-chloroxazolo[4,5-b]pyridin-2-yl)piperazin-1-yl] methanon;
[4-[5-(2,2-Dimethylpropyl)-1,2,4-oxadiazol-3-yl]phenyl]-(4-oxazolo[4,5-b]pyridin-2-ylpiperazin-1-yl)methanon;
[4-(5-tert-Butyl-1,2,4-oxadiazol-3-yl)phenyl]-(4-oxazolo[4,5-b]pyridin-2-yl)piperazin-1-yl)methanon;
2-[4-[4-[5-(2,2-Dimethylpropyl)-1,2,4-oxadiazol-3-yl]benzoyl]piperazin-1-yl]oxazolo[4,5-b]pyridin-5-carbonitril;
[4-[5-(2,2-Dimethylpropyl)-1,2,4-oxadiazol-3-yl]phenyl]-[4-[5-(trifluormethyl)oxazolo[4,5-b]pyridin-2-yl]piperazin-1-yl]methanon;
[2-(1-Ethylpropyl)-1,3-benzoxazol-6-yl]-[4-(5-methyloxazolo[4,5-b]pyridin-2-yl)piperazin-1-yl] methanon;
[4-(5-Chloroxazolo[4,5-b]pyridin-2-yl)piperazin-1-yl]-[4-[5-(2,2-dimethylpropyl)-1,3,4-oxadiazol-2-yl]phenyl]methanon;
(2-Cyclohexyl-1,3-benzoxazol-6-yl)-[4-(5-methyloxazolo[4,5-b]pyridin-2-yl)piperazin-1-yl]methanon;
[4-(5-Methyloxazolo[4,5-b]pyridin-2-yl)piperazin-1-yl]-[2-(3,3,3-trifluorpropyl)-1,3-benzoxazol-6-yl]methanon;
[4-(5-Chloroxazolo[4,5-b]pyridin-2-yl)piperazin-1-yl]-[4-[1-(2,2-dimethylpropyl)pyrazol-4-yl]phenyl]methanon;
[3-Chlor-5-[4-(hydroxymethyl)phenyl]phenyl]-[4-(5-methyloxazolo[4,5-b]pyridin-2-yl)piperazin-1-yl] methanon;
[3-Chlor-5-(1,1-dioxo-1,4-thiazinan-4-yl)phenyl]-[4-(5-methyloxazolo[4,5-b]pyridin-2-yl)piperazin-1-yl] methanon;
1-[4-[3-Chlor-5-[4-(5-methyloxazolo[4,5-b]pyridin-2-yl)piperazin-1-carbonyl]phenyl]piperazin-1-yl]ethanon;
[3-Chlor-5-(4,4-difluor-1-piperidyl)phenyl]-[4-(5-methyloxazolo[4,5-b]pyridin-2-yl)piperazin-1-yl] methanon;
[3-Chlor-5-[4-(hydroxymethyl)-l-piperidyl]phenyl]-[4-(5-methyloxazolo[4,5-b]pyridin-2-yl)piperazin-1 -yl]methanon;
[3-Chlor-5-(6-hydroxy-2-azaspiro[3.3]heptan-2-yl)phenyl]-[4-(5-methyloxazolo[4,5-b]pyridin-2-yl)piperazin-1-yl]methanon;
[2-(3-Chlorphenyl)sulfonyl-2,6-diazaspiro[3.3]heptan-6-yl]-[4-(5-methyloxazolo[4,5-b]pyridin-2-yl)piperazin-1-yl]methanon;
[3-[4-(Hydroxymethyl)phenyl]-5-(3,3,3-trifluorpropoxy)phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[5-(2-fluor-1,1-dimethylethyl)-1,2,4-oxadiazol-3-yl]phenyl]methanon;
[2-[(E)-But-1-enyl]-1,3-benzoxazol-6-yl]-[4-(5-methyloxazolo[4,5-b]pyridin-2-yl)piperazin-1-yl] methanon;
(2-Cyclopropyl-1,3-benzoxazol-6-yl)-[4-(5-methyloxazolo[4,5-b]pyridin-2-yl)piperazin-1-yl] methanon;
1-[3-[4-[4-(5-Chloroxazolo[4,5-b]pyridin-2-yl)piperazin-1-carbonyl]phenyl]-1,2,4-oxadiazol-5-yl]propan-2-on;
[4-(5-Chloroxazolo[4,5-b]pyridin-2-yl)piperazin-1-yl]-[4-[5-(2,2-difluorpropyl)-1,2,4-oxadiazol-3-yl]phenyl]methanon;
[4-(5-Chloroxazolo[4,5-b]pyridin-2-yl)piperazin-1-yl]-[4-[5-(1-fluor-1-methylethyl)-1,2,4-oxadiazol-3-yl]phenyl]methanon;
[4-(5-Chloroxazolo[4,5-b]pyridin-2-yl)piperazin-1-yl]-[4-[3-(2,2-dimethylpropyl)-1,2,4-oxadiazol-5-yl]phenyl]methanon;
[3-[4-(Hydroxymethyl)phenyl]-4-[[1-(trifluormethyl)cyclopropyl]methoxy]phenyl]-[4-(5-methyloxazolo[4,5-b]pyridin-2-yl)piperazin-1-yl]methanon;
[3-[4-(Hydroxymethyl)phenyl]-4-(3,3,3-trifluorpropoxy)phenyl]-[4-(5-methyloxazolo[4,5-b]pyridin-2-yl)piperazin-1-yl]methanon;
[3-[4-(Hydroxymethyl)phenyl]-5-[[1-(trifluormethyl)cyclopropyl]methoxy]phenyl]-[4-(5-methyloxazolo[4,5-b]pyridin-2-yl)piperazin-1-yl]methanon;
(2-Butyl-1,3-benzoxazol-6-yl)-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[5-(1-fluorcyclopropyl)-1,2,4-oxadiazol-3-yl]phenyl]methanon;
[4-(5-Chloroxazolo[4,5-b]pyridin-2-yl)piperazin-1-yl]-[4-[1-(2,2-dimethylpropyl)-1,2,4-triazol-3-yl]phenyl]methanon;
[4-[1-(2,2-Dimethylpropyl)-1,2,4-triazol-3-yl]phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[1-(2,2-dimethylpropyl)imidazol-4-yl]phenyl]methanon;
[4-(5-tert-Butyl-1,3,4-oxadiazol-2-yl)phenyl]-[4-(5-chloroxazolo[4,5-b]pyridin-2-yl)piperazin-1-yl] methanon;
[4-(5-Oxa-2-azaspiro[3.4]octan-2-yl)phenyl]-(4-oxazolo[4,5-b]pyridin-2-ylpiperazin-1-yl)methanon;
(4-Oxazolo[4,5-b]pyridin-2-ylpiperazin-1-yl)-[4-[3-(2,2,2-trifluor-1-methylethoxy)azetidin-1-yl]phenyl]methanon;
[4-(5-tert-Butyl-1,3,4-oxadiazol-2-yl)phenyl]-[4-(5-methyloxazolo[4,5-b]pyridin-2-yl)piperazin-1-yl] methanon;
[4-(5-Methyloxazolo[4,5-b]pyridin-2-yl)piperazin-1-yl]-[4-(5-oxa-2-azaspiro[3.4]octan-2-yl)phenyl] methanon;
[4-(5-Methyloxazolo[4,5-b]pyridin-2-yl)piperazin-1-yl]-[4-[3-(2,2,2-trifluor-1-methylethoxy)azetidin-1-yl]phenyl]methanon;
[4-(5-Chloroxazolo[4,5-b]pyridin-2-yl)piperazin-1-yl]-[4-[5-(2-fluor-2-methylpropyl)-1,2,4-oxadiazol-3-yl]phenyl]methanon;
[4-(5-Chloroxazolo[4,5-b]pyridin-2-yl)piperazin-1-yl]-[4-[5-(2-methylallyl)-1,2,4-oxadiazol-3-yl]phenyl]methanon;
[4-(5-Chloroxazolo[4,5-b]pyridin-2-yl)piperazin-1-yl]-[4-[5-(2-methylprop-1-enyl)-1,2,4-oxadiazol-3-yl]phenyl]methanon;
[4-(5-Chloroxazolo[4,5-b]pyridin-2-yl)piperazin-1-yl]-[4-[5-(2-hydroxy-2-methylpropyl)-1,2,4-oxadiazol-3-yl]phenyl]methanon;
(4-Oxazolo[4,5-b]pyridin-2-ylpiperazin-1-yl)-[4-(3-phenoxyazetidin-1-yl)phenyl] methanon;
[4-[5-(2-Fluor-1,1-dimethylethyl)-1,3,4-oxadiazol-2-yl]phenyl]-[4-(5-methyloxazolo[4,5-b]pyridin-2-yl)piperazin-1-yl]methanon;
[4-(5-tert-Butyl-1,2,4-oxadiazol-3-yl)phenyl]-[4-(5-methoxyoxazolo[4,5-b]pyridin-2-yl)piperazin-1-yl] methanon;
[4-(5-Chloroxazolo[4,5-b]pyridin-2-yl)piperazin-1-yl]-[4-[5-(2-fluor-1,1-dimethylethyl)-1,3,4-oxadiazol-2-yl]phenyl]methanon;
[4-[5-(1-Fluorcyclopropyl)-1,2,4-oxadiazol-3-yl]phenyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[4-(3-phenoxyazetidin-1-yl)phenyl] methanon;
[4-[5-(2,2-Dimethylpropyl)-1,2,4-oxadiazol-3-yl]-3-[4-(hydroxymethyl)phenyl]phenyl]-[4-(5-methyloxazolo[4,5-b]pyridin-2-yl)piperazin-1-yl]methanon;
[3-[4-(Hydroxymethyl)phenyl]-4-[(1-methylcyclopropyl)methoxy]phenyl]-[4-(5-methyloxazolo[4,5-b]pyridin-2-yl)piperazin-1-yl]methanon;
[3-[4-(Hydroxymethyl)phenyl]-5-[(1-methylcyclopropyl)methoxy]phenyl]-[4-(5-methyloxazolo[4,5-b]pyridin-2-yl)piperazin-1-yl]methanon;
[4-[(3-Chlorphenyl)methoxy]-3-(1H-imidazol-2-yl)phenyl]-[4-(5-methyloxazolo[4,5-b]pyridin-2-yl)piperazin-1-yl]methanon;
[4-(2-Azaspiro[3.4]octan-2-yl)-3-(1H-imidazol-2-yl)phenyl]-[4-(5-methyloxazolo[4,5-b]pyridin-2-yl)piperazin-1-yl]methanon;
[4-[5-[1-(Fluormethyl)cyclopropyl]-1,2,4-oxadiazol-3-yl]phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[5-[1-(fluormethyl)cyclopropyl]-1,2,4-oxadiazol-3-yl]phenyl]methanon;
[4-(3-tert-Butyl-1,2,4-oxadiazol-5-yl)phenyl]-[4-(5-methyloxazolo[4,5-b]pyridin-2-yl)piperazin-1-yl] methanon;
[4-(3-tert-Butyl-1,2,4-oxadiazol-5-yl)phenyl]-[4-(5-chloroxazolo[4,5-b]pyridin-2-yl)piperazin-1-yl] methanon;
[4-(5-tert-Butyl-1,2,4-oxadiazol-3-yl)phenyl]-[4-(5-cyclopropyloxazolo[4,5-b]pyridin-2-yl)piperazin-1-yl]methanon;
[4-(5-Chloroxazolo[4,5-b]pyridin-2-yl)piperazin-1-yl]-[4-[5-[(1-methylcyclopropyl)methyl]-1,2,4-oxadiazol-3-yl]phenyl]methanon;
[4-(5-Methyloxazolo[4,5-b]pyridin-2-yl)piperazin-1-yl]-[4-[3-[(1S)-2,2,2-trifluor-1-methylethoxy]azetidin-1-yl]phenyl]methanon;
[4-(5-Methyloxazolo[4,5-b]pyridin-2-yl)piperazin-1-yl]-[4-[3-[(1R)-2,2,2-trifluor-1-methylethoxy]azetidin-1-yl]phenyl]methanon;
[4-(5-Chloroxazolo[4,5-b]pyridin-2-yl)piperazin-1 -yl] -[4-(2,2-difluor-5-azaspiro[2.4]heptan-5-yl)phenyl]methanon;
[4-(5-Chloroxazolo[4,5-b]pyridin-2-yl)piperazin-1-yl]-[4-(7,7-difluor-2-azaspiro[3.3]heptan-2-yl)phenyl]methanon;
[4-(3-tert-Butoxyazetidin-1-yl)phenyl]-[4-(5-chloroxazolo[4,5-b]pyridin-2-yl)piperazin-1-yl]methanon;
[4-(5-Chloroxazolo[4,5-b]pyridin-2-yl)piperazin-1-yl]-[4-[3-(1,1-dimethylpropoxy)azetidin-1-yl]phenyl]methanon;
[4-(1-tert-Butyl-1,2,4-triazol-3-yl)phenyl]-[4-(5-chloroxazolo[4,5-b]pyridin-2-yl)piperazin-1-yl] methanon;
[4-(1-tert-Butyl-1,2,4-triazol-3-yl)phenyl]-[4-(5-methyloxazolo[4,5-b]pyridin-2-yl)piperazin-1-yl] methanon;
[4-(5-Chloroxazolo[4,5-b]pyridin-2-yl)piperazin-1-yl]-[4-[1-(2,2-dimethylpropyl)triazol-4-yl]phenyl]methanon;
[4-(5-Chloroxazolo[4,5-b]pyridin-2-yl)piperazin-1-yl]-[4-[2-(2,2-dimethylpropyl)triazol-4-yl]phenyl]methanon;
[4-(5-tert-Butyl-1,2,4-oxadiazol-3-yl)phenyl]-[4-(5-chloroxazolo[4,5-b]pyridin-2-yl)-2-methylpiperazin-1 -yl] methanon;
[4-(5-tert-Butyl-1,2,4-oxadiazol-3-yl)phenyl]-[4-(5-chloroxazolo[4,5-b]pyridin-2-yl)-3-methylpiperazin-1 -yl] methanon;
[4-(1-tert-Butylpyrazol-4-yl)phenyl]-[4-(5-methyloxazolo[4,5-b]pyridin-2-yl)piperazin-1-yl]methanon;
[4-(1-tert-Butylpyrazol-4-yl)phenyl]-[4-(5-chloroxazolo[4,5-b]pyridin-2-yl)piperazin-1-yl]methanon;
[4-(5-Chloroxazolo[4,5-b]pyridin-2-yl)piperazin-1-yl]-[4-[1-[(1-methylcyclopropyl)methyl]-1,2,4-triazol-3-yl]phenyl]methanon;
[4-(5-Chloroxazolo[4,5-b]pyridin-2-yl)piperazin-1-yl]-[4-[2-[(1-methylcyclopropyl)methyl]-1,2,4-triazol-3-yl]phenyl]methanon;
[4-[5-(1-Methylcyclopropyl)-1,2,4-oxadiazol-3-yl]phenyl]-[4-(5-methyloxazolo[4,5-b]pyridin-2-yl)piperazin-1-yl]methanon;
[4-(4-tert-Butylpyrazol-1-yl)phenyl]-[4-(5-chloroxazolo[4,5-b]pyridin-2-yl)piperazin-1-yl] methanon;
[4-(4-tert-Butylpyrazol-1-yl)phenyl]-[4-(5-methyloxazolo[4,5-b]pyridin-2-yl)piperazin-1-yl]methanon;
[4-[1-(2,2-Dimethylpropyl)pyrazol-4-yl]phenyl]-[4-(5-methyloxazolo[4,5-b]pyridin-2-yl)piperazin-1-yl] methanon;
[4-(7,7-Difluor-2-azaspiro[3.3]heptan-2-yl)phenyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[6-[3-(2,2,2-trifluor-1-methylethoxy)azetidin-1-yl]-3-pyridyl]methanon;
[4-(4-tert-Butylimidazol-1-yl)phenyl]-[4-(5-methyloxazolo[4,5-b]pyridin-2-yl)piperazin-1-yl] methanon;
[4-(5-tert-Butyl-1,2,4-oxadiazol-3-yl)-3-methylphenyl]-[4-(5-methyloxazolo[4,5-b]pyridin-2-yl)piperazin-1-yl]methanon;
[4-(5-tert-Butyl-1,2,4-oxadiazol-3-yl)-3-methylphenyl]-[4-(5-chloroxazolo[4,5-b]pyridin-2-yl)piperazin-1-yl]methanon;
[4-(5-tert-Butyl-1,2,4-oxadiazol-3-yl)-3-fluorphenyl]-[4-(5-methyloxazolo[4,5-b]pyridin-2-yl)piperazin-1-yl]methanon;
[4-(5-tert-Butyl-1,2,4-oxadiazol-3-yl)-3-fluorphenyl]-[4-(5-chloroxazolo[4,5-b]pyridin-2-yl)piperazin-1-yl]methanon;
[3-Chlor-4-[(1-methylcyclopropyl)methoxy]phenyl]-[4-(5-chloroxazolo[4,5-b]pyridin-2-yl)piperazin-1-yl]methanon;
[6-[3-(1,1-Dimethylpropoxy)azetidin-1-yl]-3-pyridyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[5-methyl-6-[3-(2,2,2-trifluor-1 -methylethoxy)azetidin-1 -yl] -3 -pyridyl]methanon;
[6-(5-tert-Butyl-1,2,4-oxadiazol-3-yl)-3-pyridyl]-[4-(5-chloroxazolo[4,5-b]pyridin-2-yl)piperazin-1-yl] methanon;
[6-(5-tert-Butyl-1,2,4-oxadiazol-3-yl)-3-pyridyl]-[4-(5-methyloxazolo[4,5-b]pyridin-2-yl)piperazin-1-yl] methanon;
[4-(4-tert-Butylimidazol-1-yl)phenyl]-[4-(5-chloroxazolo[4,5-b]pyridin-2-yl)piperazin-1-yl]methanon;
[2-Fluor-4-[3-(2,2,2-trifluor-1-methylethoxy)azetidin-1-yl]phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-(2,2-dimethylpropoxy)-3-methylphenyl]methanon;
[4-(2,2-Dimethylpropoxy)-3-methylphenyl]-[4-(5-methyloxazolo[4,5-b]pyridin-2-yl)piperazin-1-yl] methanon;
[4-(5-Chloroxazolo[4,5-b]pyridin-2-yl)piperazin-1-yl]-[4-[3-(2,2,2-trifluor-1-methylethoxy)azetidin-1-yl]phenyl]methanon;
[4-(5-Chloroxazolo[4,5-b]pyridin-2-yl)piperazin-1-yl]-[4-(3-ethoxy-3-methylazetidin-1-yl)phenyl]methanon;
[3-Chlor-4-[(1-methylcyclopropyl)methoxy]phenyl]-[4-(5-methyloxazolo[4,5-b]pyridin-2-yl)piperazin-1-yl]methanon;
[3-Fluor-4-[3-(2,2,2-trifluor-1-methylethoxy)azetidin-1-yl]phenyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[4-(3-methyloxetan-3-yl)phenyl]methanon;
[4-(1-Fluorcyclopropyl)phenyl]-[4-(5-methyloxazolo[4,5-b]pyridin-2-yl)piperazin-1-yl]methanon;
[4-Chlor-5-fluor-6-[3-(2,2,2-trifluor-1-methylethoxy)azetidin-1-yl]-3-pyridyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[6-[3-(2,2,2-trifluor-1-methylethoxy)azetidin-1-yl]pyridazin-3-yl]methanon;
[5-Methyl-6-[(1-methylcyclopropyl)methoxy]-3-pyridyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[2-[3-(2,2,2-trifluor-1-methylethoxy)azetidin-1-yl]pyrimidin-5-yl]methanon;
[4-(5-Methyloxazolo[4,5-b]pyridin-2-yl)piperazin-1-yl]-[4-methyl-6-[3-(2,2,2-trifluor-1-methylethoxy)azetidin-1-yl]-3-pyridyl]methanon;
[6-(7,7-Difluor-2-azaspiro[3.3]heptan-2-yl)-4-methyl-3-pyridyl]-[4-(5-methyloxazolo[4,5-b]pyridin-2-yl)piperazin-1-yl]methanon;
[2-(2,2-Difluor-5-azaspiro[2.4]heptan-5-yl)pyrimidin-5-yl]-[4-(5-methyloxazolo[4,5-b]pyridin-2-yl)piperazin-1-yl]methanon;
[6-(2,2-Difluor-5-azaspiro[2.4]heptan-5-yl)-4-methyl-3-pyridyl]-[4-(5-methyloxazolo[4,5-b]pyridin-2-yl)piperazin-1-yl]methanon;
[5-Fluor-6-[3-(2,2,2-trifluor-1-methylethoxy)azetidin-1-yl]-3-pyridyl]-[4-(5-methyloxazolo[4,5-b]pyridin-2-yl)piperazin-1-yl]methanon;
[6-(2,2-Difluor-5-azaspiro[2.4]heptan-5-yl)-5-methyl-3-pyridyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[2-methyl-4-[3-(2,2,2-trifluor-1-methylethoxy)azetidin-1-yl]phenyl]methanon;
[2,6-Difluor-4-[3-(2,2,2-trifluor-1-methylethoxy)azetidin-1-yl]phenyl]-[4-(5-methyloxazolo[4,5-b]pyridin-2-yl)piperazin-1-yl]methanon;
[2-Chlor-4-[3-(2,2,2-trifluor-1-methylethoxy)azetidin-1-yl]phenyl]-[4-(5-methyloxazolo[4,5-b]pyridin-2-yl)piperazin-1-yl]methanon;
[4-(5-Methyloxazolo[4,5-b]pyridin-2-yl)piperazin-1-yl]-[5-methyl-6-[[1-(trifluormethyl)cyclopropyl]methoxy]-3-pyridyl]methanon;
[4-(5-Chloroxazolo[4,5-b]pyridin-2-yl)piperazin-1-yl]-[3-fluor-4-[(1-methylcyclopropyl)methoxy]phenyl]methanon;
[4-(3-tert-Butyl-1,2,4-triazol-1-yl)phenyl]-[4-(5-methyloxazolo[4,5-b]pyridin-2-yl)piperazin-1-yl] methanon;
[3-Fluor-4-[(1-methylcyclopropyl)methoxy]phenyl]-[4-(5-methyloxazolo[4,5-b]pyridin-2-yl)piperazin-1-yl]methanon;
[4-(3-tert-Butyl-1,2,4-triazol-1-yl)phenyl]-[4-(5-chloroxazolo[4,5-b]pyridin-2-yl)piperazin-1-yl] methanon;
[5-Chlor-6-[3-(2,2,2-trifluor-1-methylethoxy)azetidin-1-yl]-3-pyridyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[5-(2,2-dimethylpropyl)-4-methyl-1,2,4-triazol-3-yl]phenyl]methanon;
[5-Chlor-6-(2,2-difluor-5-azaspiro[2.4]heptan-5-yl)-3-pyridyl]-[4-(5-methyloxazolo[4,5-b]pyridin-2-yl)piperazin-1-yl]methanon;
[4-[5-(2,2-Dimethylpropyl)-4-methyl-1,2,4-triazol-3-yl]phenyl]-[4-(5-methyloxazolo[4,5-b]pyridin-2-yl)piperazin-1-yl]methanon;
[6-(2,2-Difluor-5-azaspiro[2.4]heptan-5-yl)-5-fluor-3-pyridyl]-[4-(5-methyloxazolo[4,5-b]pyridin-2-yl)piperazin-1-yl]methanon;
[4-[1-(2,2-Dimethylpropyl)triazol-4-yl]phenyl]-[4-(5-methyloxazolo[4,5-b]pyridin-2-yl)piperazin-1-yl] methanon;
[4-(5-Chloroxazolo[4,5-b]pyridin-2-yl)piperazin-1-yl]-[5-fluor-6-[3-(2,2,2-trifluor-1-methylethoxy)azetidin-1-yl]-3-pyridyl]methanon;
[4-(5-Chloroxazolo[4,5-b]pyridin-2-yl)piperazin-1 -yl] -[6-(2,2-difluor-5-azaspiro[2.4]heptan-5-yl)-5-fluor-3-pyridyl]methanon;
[4-(5-Chloroxazolo[4,5-b]pyridin-2-yl)piperazin-1-yl]-[5-fluor-6-[[1-(trifluormethyl)cyclopropyl]methoxy]-3-pyridyl]methanon;
[4-(5-Chloroxazolo[4,5-b]pyridin-2-yl)piperazin-1-yl]-[5-fluor-6-[(1-methylcyclopropyl)methoxy]-3-pyridyl]methanon;
[4-(5-tert-Butyl-4-methyl-1,2,4-triazol-3-yl)phenyl]-[4-(5-chloroxazolo[4,5-b]pyridin-2-yl)piperazin-1-yl] methanon;
[4-(5-tert-Butyl-4-methyl-1,2,4-triazol-3-yl)phenyl]-[4-(5-methyloxazolo[4,5-b]pyridin-2-yl)piperazin-1-yl]methanon;
[5-Chlor-6-[(1-methylcyclopropyl)methoxy]-3-pyridyl]-[4-(5-methyloxazolo[4,5-b]pyridin-2-yl)piperazin-1-yl]methanon;
[5-Chlor-6-[[1-(trifluormethyl)cyclopropyl]methoxy]-3-pyridyl]-[4-(5-methyloxazolo[4,5-b]pyridin-2-yl)piperazin-1-yl]methanon;
[4-(5-tert-Butyl-1,2,4-oxadiazol-3-yl)-3-chlorphenyl]-[4-(5-chloroxazolo[4,5-b]pyridin-2-yl)piperazin-1-yl]methanon;
[4-(5-tert-Butyl-1,2,4-oxadiazol-3-yl)-3-chlorphenyl]-[4-(5-methyloxazolo[4,5-b]pyridin-2-yl)piperazin-1-yl]methanon;
[4-(5-tert-Butyl-1,2,4-oxadiazol-3-yl)-3,5-difluorphenyl]-[4-(5-chloroxazolo[4,5-b]pyridin-2-yl)piperazin-1-yl]methanon;
[4-(5-tert-Butyl-1,2,4-oxadiazol-3-yl)-3-(trifluormethyl)phenyl]-[4-(5-chloroxazolo[4,5-b]pyridin-2-yl)piperazin-1-yl]methanon;
[4-(5-Chloroxazolo[4,5-b]pyridin-2-yl)piperazin-1-yl]-[4-[1-(2,2-dimethylpropyl)triazol-4-yl] -3 -methylphenyl] methanon;
[4-(5-Chloroxazolo[4,5-b]pyridin-2-yl)piperazin-1-yl]-[4-[2-(2,2-dimethylpropyl)triazol-4-yl] -3 -methylphenyl] methanon;
[5-Fluor-6-[(1-methylcyclopropyl)methoxy]-3-pyridyl]-[4-(5-methyloxazolo[4,5-b]pyridin-2-yl)piperazin-1-yl]methanon;
[5-Chlor-6-[(1-methylcyclopropyl)methoxy]-3-pyridyl]-[4-(5-chloroxazolo[4,5-b]pyridin-2-yl)piperazin-1-yl]methanon;
[5-Fluor-6-[[1-(trifluormethyl)cyclopropyl]methoxy]-3-pyridyl]-[4-(5-methyloxazolo[4,5-b]pyridin-2-yl)piperazin-1-yl]methanon;
[5,6-Bis[[1-(trifluormethyl)cyclopropyl]methoxy]-3-pyridyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[5-chlor-6-[[1-(trifluormethyl)cyclopropyl]methoxy]-3-pyridyl]methanon;
[4-(5-Chloroxazolo[4,5-b]pyridin-2-yl)piperazin-1-yl]-[5-methyl-6-[[1-(trifluormethyl)cyclopropyl]methoxy]-3-pyridyl]methanon;
[4-(5-Chloroxazolo[4,5-b]pyridin-2-yl)piperazin-1-yl]-[5-(trifluormethyl)-6-[[1-(trifluormethyl)cyclopropyl]methoxy]-3-pyridyl]methanon;
[4-(5-tert-Butyl-1,3,4-oxadiazol-2-yl)-3-methylphenyl]-[4-(5-methyloxazolo[4,5-b]pyridin-2-yl)piperazin-1-yl]methanon;
[4-(5-tert-Butyl-1,3,4-oxadiazol-2-yl)-3-methylphenyl]-[4-(5-chloroxazolo[4,5-b]pyridin-2-yl)piperazin-1-yl]methanon;
[4-(5-Chloroxazolo[4,5-b]pyridin-2-yl)piperazin-1-yl]-[4-[2-(2,2-dimethylpropyl)triazol-4-yl]-3-fluorphenyl]methanon;
[4-[2-(2,2-Dimethylpropyl)triazol-4-yl]-3-fluorphenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[1-(2,2-dimethylpropyl)triazol-4-yl]-3-fluorphenyl]methanon;
[4-(1-tert-Butyltriazol-4-yl)phenyl]-[4-(5-methyloxazolo[4,5-b]pyridin-2-yl)piperazin-1-yl]methanon;
[4-(1-tert-Butyltriazol-4-yl)phenyl]-[4-(5-chloroxazolo[4,5-b]pyridin-2-yl)piperazin-1-yl] methanon;
[4-(1-tert-Butyl-1,2,4-triazol-3-yl)-3-chlorphenyl]-[4-(5-methyloxazolo[4,5-b]pyridin-2-yl)piperazin-1-yl] methanon;
[4-(1-tert-Butyl-1,2,4-triazol-3-yl)-3-chlorphenyl]-[4-(5-chloroxazolo[4,5-b]pyridin-2-yl)piperazin-1-yl] methanon;
[4-(5-Chloroxazolo[4,5-b]pyridin-2-yl)piperazin-1-yl]-[4-[2-(2,2-dimethylpropyl)triazol-4-yl]-3,5-difluorphenyl]methanon;
[4-[2-(2,2-Dimethylpropyl)triazol-4-yl]-3,5-difluorphenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[1-(2,2-dimethylpropyl)triazol-4-yl]-3,5-difluorphenyl]methanon;
[4-(5-Chloroxazolo[4,5-b]pyridin-2-yl)piperazin-1-yl]-[6-(cyclopropylmethoxy)-5-methyl-3-pyridyl]methanon;
[4-(5-Chloroxazolo[4,5-b]pyridin-2-yl)piperazin-1-yl]-[5-methyl-6-[(1-methylcyclopropyl)methoxy]-3-pyridyl]methanon;
[4-(5-Chloroxazolo[4,5-b]pyridin-2-yl)piperazin-1-yl]-[5-methyl-6-[(3-methyloxetan-3-yl)methoxy]-3-pyridyl]methanon;
[4-(5-Chloroxazolo[4,5-b]pyridin-2-yl)piperazin-1-yl]-[6-[(2,2-difluor-1-methylcyclopropyl)methoxy]-5-methyl-3-pyridyl]methanon;
[4-(5-Chloroxazolo[4,5-b]pyridin-2-yl)piperazin-1-yl]-[6-[(3-ethyloxetan-3-yl)methoxy]-5-methyl-3-pyridyl]methanon;
[4-(5-Chloroxazolo[4,5-b]pyridin-2-yl)piperazin-1-yl]-[6-[[1-(methoxymethyl)cyclopropyl]methoxy]-5-methyl-3-pyridyl]methanon;
[4-(5-Chloroxazolo[4,5-b]pyridin-2-yl)piperazin-1-yl]-[6-[(3-fluoroxetan-3-yl)methoxy]-5-methyl-3-pyridyl]methanon;
2-[1-[[5-[4-(5-Chloroxazolo[4,5-b]pyridin-2-yl)piperazin-1-carbonyl]-3-methyl-2-pyridyl]oxymethyl]cyclopropyl]acetonitril;
1-[[5-[4-(5-Chloroxazolo[4,5-b]pyridin-2-yl)piperazin-1-carbonyl]-3-methyl-2-pyridyl] oxymethyl] cyclopropancarbonitril;
3-[[5-[4-(5-Chloroxazolo[4,5-b]pyridin-2-yl)piperazin-1-carbonyl]-3-methyl-2-pyridyl] oxymethyl] oxetan-3-carbonitril;
[4-[1-(1-Bicyclo[1.1.1]pentanyl)triazol-4-yl]phenyl]-[4-(5-methyloxazolo[4,5-b]pyridin-2-yl)piperazin-1-yl]methanon;
[4-[1-(1-Bicyclo[1.1.1]pentanyl)triazol-4-yl]phenyl]-[4-(5-chloroxazolo[4,5-b]pyridin-2-yl)piperazin-1 -yl]methanon;
[4-(1-tert-Butyl-1,2,4-triazol-3-yl)-3-methylphenyl]-[4-(5-methyloxazolo[4,5-b]pyridin-2-yl)piperazin-1 -yl]methanon;
[4-(1-tert-Butyl-1,2,4-triazol-3-yl)-3-methylphenyl]-[4-(5-chloroxazolo[4,5-b]pyridin-2-yl)piperazin-1-yl] methanon;
3-[[5-[4-(5-Chloroxazolo[4,5-b]pyridin-2-yl)piperazin-1-carbonyl]-3-methyl-2-pyridyl]oxymethyl]-5-fluorbenzonitril;
[4-(5-Chloroxazolo[4,5-b]pyridin-2-yl)piperazin-1-yl]-[4-[1-(2,2,2-trifluorethyl)triazol-4-yl]phenyl]methanon;
[4-(5-Methyloxazolo[4,5-b]pyridin-2-yl)piperazin-1-yl]-[4-[1-(2,2,2-trifluorethyl)triazol-4-yl]phenyl]methanon;
[4-(5-Methyloxazolo[4,5-b]pyridin-2-yl)piperazin-1-yl]-[4-[1-[(3-methyloxetan-3-yl)methyl]triazol-4-yl]phenyl]methanon;
[4-(5-Chloroxazolo[4,5-b]pyridin-2-yl)piperazin-1-yl]-[4-[1-[(3-methyloxetan-3-yl)methyl]triazol-4-yl]phenyl]methanon;
1-[4-(5-Chloroxazolo[4,5-b]pyridin-2-yl)piperazin-1-yl]-3-phenylprop-2-in-1-on;
1-[4-(5-Chloroxazolo[4,5-b]pyridin-2-yl)piperazin-1-yl]-3-(4-chlorphenyl)prop-2-in-1-on;
[4-(5-Chloroxazolo[4,5-b]pyridin-2-yl)piperazin-1-yl]-[3-[2-(2,6-dichlorphenyl)ethinyl]azetidin-1-yl]methanon;
[3-[2-(2-Chlor-4-fluorphenyl)ethinyl]azetidin-1-yl]-[4-(5-chloroxazolo[4,5-b]pyridin-2-yl)piperazin-1-yl] methanon;
[4-(5-Chloroxazolo[4,5-b]pyridin-2-yl)piperazin-1-yl]-(4-ethinyl-3-methoxyphenyl)methanon;
[4-[5-(1-Bicyclo[1.1.1]pentanyl)-1,2,4-oxadiazol-3-yl]phenyl]-[4-(5-chloroxazolo[4,5-b]pyridin-2-yl)piperazin-1-yl]methanon;
[4-(1-tert-Butyl-1,2,4-triazol-3-yl)-3-fluorphenyl]-[4-(5-methyloxazolo[4,5-b]pyridin-2-yl)piperazin-1-yl] methanon;
[4-(1-tert-Butyl-1,2,4-triazol-3-yl)-3-fluorphenyl]-[4-(5-chloroxazolo[4,5-b]pyridin-2-yl)piperazin-1-yl] methanon;
[4-(5-Methyloxazolo[4,5-b]pyridin-2-yl)piperazin-1-yl]-[4-[1-[1-(trifluormethyl)cyclopropyl]triazol-4-yl]phenyl]methanon;
[4-(5-Methyloxazolo[4,5-b]pyridin-2-yl)piperazin-1-yl]-[4-[1-(3-methyloxetan-3-yl)triazol-4-yl]phenyl]methanon;
[4-(5-Chloroxazolo[4,5-b]pyridin-2-yl)piperazin-1-yl]-[4-[1-(3-methyloxetan-3-yl)triazol-4-yl]phenyl]methanon;
[4-(1-tert-Butyl-1,2,4-triazol-3-yl)-3-(trifluormethyl)phenyl]-[4-(5-methyloxazolo[4,5-b]pyridin-2-yl)piperazin-1-yl]methanon;
[4-(1-tert-Butyl-1,2,4-triazol-3-yl)-3-(trifluormethyl)phenyl]-[4-(5-chloroxazolo[4,5-b]pyridin-2-yl)piperazin-1-yl]methanon;
[4-(5-Methyloxazolo[4,5-b]pyridin-2-yl)piperazin-1-yl]-[4-[1-(oxetan-3-ylmethyl)triazol-4-yl]phenyl]methanon;
[4-(5-Chloroxazolo[4,5-b]pyridin-2-yl)piperazin-1-yl]-[4-[1-(oxetan-3-ylmethyl)triazol-4-yl]phenyl]methanon;
[4-(5-tert-Butyl-1,3,4-oxadiazol-2-yl)-3-chlorphenyl]-[4-(5-chloroxazolo[4,5-b]pyridin-2-yl)piperazin-1-yl]methanon;
[4-(5-tert-Butyl-1,3,4-oxadiazol-2-yl)-3-fluor-5-hydroxyphenyl]-[4-(5-chloroxazolo[4,5-b]pyridin-2-yl)piperazin-1-yl]methanon;
[4-(5-tert-Butyl-1,3,4-oxadiazol-2-yl)-3-fluor-5-hydroxyphenyl]-[4-(5-methyloxazolo[4,5-b]pyridin-2-yl)piperazin-1-yl]methanon;
[4-(5-tert-Butyl-2-methyl-1,2,4-triazol-3-yl)phenyl]-[4-(5-chloroxazolo[4,5-b]pyridin-2-yl)piperazin-1-yl] methanon;
5-[4-(5-Chloroxazolo[4,5-b]pyridin-2-yl)piperazin-1-carbonyl]-2-[1-(2,2-dimethylpropyl)triazol-4-yl]benzonitril;
5-[4-(5-Chloroxazolo[4,5-b]pyridin-2-yl)piperazin-1-carbonyl]-2-[2-(2,2-dimethylpropyl)triazol-4-yl]benzonitril;
[6-(1-tert-Butylpyrazol-4-yl)-5-methyl-3-pyridyl]-[4-(5-chloroxazolo[4,5-b]pyridin-2-yl)piperazin-1-yl] methanon;
[6-(4-tert-Butylpyrazol-1-yl)-5-methyl-3-pyridyl]-[4-(5-chloroxazolo[4,5-b]pyridin-2-yl)piperazin-1-yl] methanon;
[6-(1-tert-Butylpyrazol-4-yl)-5-methyl-3-pyridyl]-[4-(5-methyloxazolo[4,5-b]pyridin-2-yl)piperazin-1-yl] methanon;
[4-(5-Chloroxazolo[4,5-b]pyridin-2-yl)piperazin-1-yl]-[5-methyl-6-(tetrahydrofuran-2-ylmethoxy)-3-pyridyl] methanon;
[4-(5-tert-Butyl-1,3,4-oxadiazol-2-yl)-3-(trifluormethyl)phenyl]-[4-(5-methyloxazolo[4,5-b]pyridin-2-yl)piperazin-1-yl]methanon;
[4-(5-tert-Butyl-1,3,4-oxadiazol-2-yl)-3-(trifluormethyl)phenyl]-[4-(5-chloroxazolo[4,5-b]pyridin-2-yl)piperazin-1-yl]methanon;
[4-(5-Methyloxazolo[4,5-b]pyridin-2-yl)piperazin-1-yl]-[6-methyl-5-[[1-(trifluormethyl)cyclopropyl]methoxy]pyrazin-2-yl]methanon;
[4-(5-Chloroxazolo[4,5-b]pyridin-2-yl)piperazin-1-yl]-[5-methyl-6-(oxetan-2-ylmethoxy)-3-pyridyl]methanon;
2-[1-(2,2-Dimethylpropyl)triazol-4-yl]-5-[4-(5-methyloxazolo[4,5-b]pyridin-2-yl)piperazin-1-carbonyl] benzonitril;
2-[2-(2,2-Dimethylpropyl)triazol-4-yl]-5-[4-(5-methyloxazolo[4,5-b]pyridin-2-yl)piperazin-1-carbonyl] benzonitril;
[4-(5-Chloroxazolo[4,5-b]pyridin-2-yl)piperazin-1-yl]-[4-[1-(2,2-dimethylpropyl)triazol-4-yl] -3 -methoxyphenyl] methanon;
[4-(5-Chloroxazolo[4,5-b]pyridin-2-yl)piperazin-1-yl]-[4-[2-(2,2-dimethylpropyl)triazol-4-yl] -3 -methoxyphenyl] methanon;
[4-[1-(2,2-Dimethylpropyl)triazol-4-yl]-3-methoxyphenyl]-[4-(5-methyloxazolo[4,5-b]pyridin-2-yl)piperazin-1-yl]methanon;
[4-[2-(2,2-Dimethylpropyl)triazol-4-yl]-3-methoxyphenyl]-[4-(5-methyloxazolo[4,5-b]pyridin-2-yl)piperazin-1-yl]methanon;
[4-(1-tert-Butyl-1,2,4-triazol-3-yl)-3-methoxyphenyl]-[4-(5-methyloxazolo[4,5-b]pyridin-2-yl)piperazin-1-yl]methanon;
[4-(1-tert-Butyl-1,2,4-triazol-3-yl)-3-methoxyphenyl]-[4-(5-chloroxazolo[4,5-b]pyridin-2-yl)piperazin-1-yl]methanon;
[5-Methoxy-6-[[1-(trifluormethyl)cyclopropyl]methoxy]-3-pyridyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[1-[(1-fluorcyclopropyl)methyl]triazol-4-yl]phenyl]methanon;
[4-(5-Chloroxazolo[4,5-b]pyridin-2-yl)piperazin-1-yl]-[5-methyl-6-(tetrahydropyran-2-ylmethoxy)-3-pyridyl] methanon;
[4-(5-Chloroxazolo[4,5-b]pyridin-2-yl)piperazin-1-yl]-[6-[(1-methoxycyclopropyl)methoxy]-5-methyl-3-pyridyl]methanon;
[4-[1-[(1-Fluorcyclopropyl)methyl]triazol-4-yl]phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[6-[1-(2,2-dimethylpropyl)triazol-4-yl] -5 -fluor-3-pyridyl] methanon;
[4-(5-Chloroxazolo[4,5-b]pyridin-2-yl)piperazin-1-yl]-[6-[3-(2,2-dimethylpropyl)triazol-4-yl] -5 -fluor-3-pyridyl] methanon;
[4-(5-Chloroxazolo[4,5-b]pyridin-2-yl)piperazin-1-yl]-[6-[2-(2,2-dimethylpropyl)triazol-4-yl]-5-fluor-3-pyridyl]methanon;
[3-[(2-Chlor-4-fluorphenoxy)methyl]azetidin-1-yl]-[4-(5-chloroxazolo[4,5-b]pyridin-2-yl)piperazin-1-yl] methanon;
[3-[(2-Chlor-4-fluorphenoxy)methyl]azetidin-1-yl]-[4-(5-methyloxazolo[4,5-b]pyridin-2-yl)piperazin-1-yl]methanon;
2-Chlor-N-[1-[4-(5-chloroxazolo[4,5-b]pyridin-2-yl)piperazin-1-carbonyl]azetidin-3-yl]-4-fluorbenzamid;
2-Chlor-4-fluor-N-[1-[4-(5-methyloxazolo[4,5-b]pyridin-2-yl)piperazin-1-carbonyl]azetidin-3-yl]benzamid;
[4-(5-Chloroxazolo[4,5-b]pyridin-2-yl)piperazin-1-yl]-[6-[(2S)-2,3-difluorpropoxy]-5-methyl-3-pyridyl]methanon;
N-(2-Chlor-4-fluorphenyl)-1-[4-(5-chloroxazolo[4,5-b]pyridin-2-yl)piperazin-1-carbonyl]azetidin-3-carboxamid;
N-(2-Chlor-4-fluorphenyl)-1-[4-(5-methyloxazolo[4,5-b]pyridin-2-yl)piperazin-1-carbonyl]azetidin-3-carboxamid;
[4-(5-Chloroxazolo[4,5-b]pyridin-2-yl)piperazin-1-yl]-[5-methyl-6-(3,3,3-trifluorpropoxy)-3-pyridyl]methanon;
[4-(1-tert-Butyltriazol-4-yl)-3-methylphenyl]-[4-(5-chloroxazolo[4,5-b]pyridin-2-yl)piperazin-1-yl] methanon;
[4-(5-Chloroxazolo[4,5-b]pyridin-2-yl)piperazin-1-yl]-[6-[(2R)-2,3-difluorpropoxy]-5-methyl-3-pyridyl]methanon;
[4-(5-tert-Butyl-2-methyl-1,2,4-triazol-3-yl)phenyl]-[4-(5-methyloxazolo[4,5-b]pyridin-2-yl)piperazin-1 -yl]methanon;
[6-[1-(2,2-Dimethylpropyl)triazol-4-yl]-5-fluor-3-pyridyl]-[4-(5-methyloxazolo[4,5-b]pyridin-2-yl)piperazin-1-yl]methanon;
[6-[2-(2,2-Dimethylpropyl)triazol-4-yl]-5-fluor-3-pyridyl]-[4-(5-methyloxazolo[4,5-b]pyridin-2-yl)piperazin-1-yl]methanon;
[6-[3-(2,2-Dimethylpropyl)triazol-4-yl]-5-fluor-3-pyridyl]-[4-(5-methyloxazolo[4,5-b]pyridin-2-yl)piperazin-1-yl]methanon;
[4-(5-tert-Butyl-1,3,4-oxadiazol-2-yl)phenyl]-[4-(5-chloroxazolo[4,5-b]pyridin-2-yl)piperazin-1-yl] methanon;
[4-(5-Chloroxazolo[4,5-b]pyridin-2-yl)piperazin-1-yl]-[4-(1H-triazol-5-yl)phenyl] methanon;
[4-(5-tert-Butyl-1,3,4-oxadiazol-2-yl)-3-chlorphenyl]-[4-(5-methyloxazolo[4,5-b]pyridin-2-yl)piperazin-1-yl]methanon;
[6-(3-tert-Butyl-1,2,4-triazol-1-yl)-5-methyl-3-pyridyl]-[4-(5-methyloxazolo[4,5-b]pyridin-2-yl)piperazin-1-yl]methanon;
[6-(3-tert-Butyl-1,2,4-triazol-1-yl)-5-methyl-3-pyridyl]-[4-(5-chloroxazolo[4,5-b]pyridin-2-yl)piperazin-1-yl]methanon;
[6-(4-tert-Butylpyrazol-1-yl)-5-methyl-3-pyridyl]-[4-(5-methyloxazolo[4,5-b]pyridin-2-yl)piperazin-1-yl] methanon;
3-[4-[4-[4-(5-Chloroxazolo[4,5-b]pyridin-2-yl)piperazin-1-carbonyl]phenyl]triazol-1-yl]-2,2-dimethylpropannitril;
[4-(5-Chloroxazolo[4,5-b]pyridin-2-yl)piperazin-1-yl]-[4-[1-(2-fluor-2-methylpropyl)triazol-4-yl]phenyl]methanon;
[4-[1-(2-Fluor-2-methylpropyl)triazol-4-yl]phenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[1-(2-fluor-1,1-dimethylethyl)triazol-4-yl]phenyl]methanon;
[4-[1-(2-Fluor-1,1-dimethylethyl)triazol-4-yl]phenyl]-[4-(5-methyloxazolo[4,5-b]pyridin-2-yl)piperazin-1-yl]methanon;
3-[4-[4-[4-(5-Chloroxazolo[4,5-b]pyridin-2-yl)piperazin-1-carbonyl]phenyl]triazol-2-yl]-2,2-dimethylpropannitril;
[4-(5-Chloroxazolo[4,5-b]pyridin-2-yl)piperazin-1-yl]-[6-methyl-5-[[1-(trifluormethyl)cyclopropyl]methoxy]pyrazin-2-yl]methanon;
[4-(5-Chloroxazolo[4,5-b]pyridin-2-yl)piperazin-1-yl]-[6-[1-(2,2-dimethylpropyl)pyrazol-4-yl]-5-methyl-3-pyridyl]methanon;
[6-[1-(2,2-Dimethylpropyl)pyrazol-4-yll-5-methyl-3-pyridyll-[4-(5-methyloxazolo[4,5-b]pyridin-2-yl)piperazin-1-yl]methanon;
[4-(5-Chloroxazolo[4,5-b]pyridin-2-yl)piperazin-1-yl]-[5-methoxy-6-[[1-(trifluormethyl)cyclopropyl]methoxy]-3-pyridyl]methanon;
[4-(5-Chloroxazolo[4,5-b]pyridin-2-yl)piperazin-1-yl]-[4-[1-(2-methoxy-2-methylpropyl)triazol-4-yl]phenyl]methanon;
[4-(5-Chloroxazolo[4,5-b]pyridin-2-yl)piperazin-1-yl]-[4-(5-isobutyl-1,3,4-oxadiazol-2-yl)phenyl] methanon;
[4-(5-Methyloxazolo[4,5-b]pyridin-2-yl)piperazin-1-yl]-[6-methyl-5-[[1-(trifluormethyl)cyclopropyl]methoxy]-2-pyridyl]methanon;
[4-(5-Chloroxazolo[4,5-b]pyridin-2-yl)piperazin-1-yl]-[6-methyl-5-[[1-(trifluormethyl)cyclopropyl]methoxy]-2-pyridyl]methanon;
[4-(5-Chloroxazolo[4,5-b]pyridin-2-yl)piperazin-1-yl]-[4-[1-(2,2-dimethylpropyl)triazol-4-yl] -3 -(trifluormethyl)phenyl] methanon;
[4-(5-Chloroxazolo[4,5-b]pyridin-2-yl)piperazin-1-yl]-[4-[2-(2,2-dimethylpropyl)triazol-4-yl] -3 -(trifluormethyl)phenyl] methanon;
[4-(5-Chloroxazolo[4,5-b]pyridin-2-yl)piperazin-1-yl]-[4-[3-(2,2-dimethylpropyl)triazol-4-yl] -3 -(trifluormethyl)phenyl] methanon;
[4-(5-Chloroxazolo[4,5-b]pyridin-2-yl)piperazin-1-yl]-[6-[1-(2,2-dimethylpropyl)triazol-4-yl] -5 -methyl-3 -pyridyl] methanon;
[4-(5-Chloroxazolo[4,5-b]pyridin-2-yl)piperazin-1-yl]-[6-[2-(2,2-dimethylpropyl)triazol-4-yl] -5 -methyl-3 -pyridyl] methanon;
[(3R)-4-(5-Chloroxazolo[4,5-b]pyridin-2-yl)-3-methylpiperazin-1-yl]-[4-[1-(2,2-dimethylpropyl)triazol-4-yl]phenyl]methanon;
[(3S)-4-(5-Chloroxazolo[4,5-b]pyridin-2-yl)-3-methylpiperazin-1-yl]-[4-[1-(2,2-dimethylpropyl)triazol-4-yl]phenyl]methanon;
[4-(5-Chloroxazolo[4,5-b]pyridin-2-yl)piperazin-1-yl]-[4-(5-isobutyl-1,3,4-oxadiazol-2-yl)-3-methylphenyl] methanon;
[4-(5-Chloroxazolo[4,5-b]pyridin-2-yl)piperazin-1-yl]-[2-chlor-6-[[1-(trifluormethyl)cyclopropyl]methoxy]-3-pyridyl]methanon;
[4-[2-(2,2-Dimethylpropyl)tetrazol-5-yl]phenyl]-[4-(5-methyloxazolo[4,5-b]pyridin-2-yl)piperazin-1-yl] methanon;
[6-(5-tert-Butyl-1,3,4-oxadiazol-2-yl)-5-fluor-3-pyridyl]-[4-(5-chloroxazolo[4,5-b]pyridin-2-yl)piperazin-1-yl]methanon;
[4-(5-Chloroxazolo[4,5-b]pyridin-2-yl)piperazin-1-yl]-[6-[1-(2,2-dimethylpropyl)pyrazol-4-yl] -5-(trifluormethyl)-3-pyridyl] methanon;
[4-(5-Chloroxazolo[4,5-b]pyridin-2-yl)piperazin-1-yl]-[3-fluor-4-[5-(2-fluor-1,1-dimethylethyl)-1,3,4-oxadiazol-2-yl]phenyl]methanon;
[4-(5-tert-Butyl-1,3,4-oxadiazol-2-yl)-3-fluorphenyl]-[4-(5-chloroxazolo[4,5-b]pyridin-2-yl)piperazin-1-yl]methanon;
[6-[1-(2,2-Dimethylpropyl)pyrazol-4-yl]-5-(trifluormethyl)-3-pyridyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[2-(2,2-dimethylpropyl)tetrazol-5-yl]phenyl]methanon;
[4-(5-tert-Butyl-1,2,4-oxadiazol-3-yl)phenyl]-[8-(5-chloroxazolo[4,5-b]pyridin-2-yl)-3,8-diazabicyclo[3.2.1]octan-3-yl]methanon;
[4-(5-Chloroxazolo[4,5-b]pyridin-2-yl)-3-(hydroxymethyl)piperazin-l-yl]-[4-[1-(2,2-dimethylpropyl)triazol-4-yl]phenyl]methanon;
[4-[1-(2,2-Dimethylpropyl)triazol-4-yl]phenyl]-[4-(5-fluoroxazolo[4,5-b]pyridin-2-yl)piperazin-1-yl] methanon;
[4-(3-tert-Butyl-1,2,4-triazol-1-yl)-3-methylphenyl]-[4-(5-chloroxazolo[4,5-b]pyridin-2-yl)piperazin-1-yl] methanon;
[4-(3-tert-Butyl-1,2,4-triazol-1-yl)-3-methylphenyl]-[4-(5-methyloxazolo[4,5-b]pyridin-2-yl)piperazin-1-yl]methanon;
[(2S)-4-(5-Chloroxazolo[4,5-b]pyridin-2-yl)-2-(hydroxymethyl)piperazin-l-yl]-[4-[1-(2,2-dimethylpropyl)triazol-4-yl]phenyl] methanon;
[(2R)-4-(5-Chloroxazolo[4,5-b]pyridin-2-yl)-2-(hydroxymethyl)piperazin-l-yl]-[4-[1-(2,2-dimethylpropyl)triazol-4-yl]phenyl]methanon;
[4-(3-tert-Butyl-1,2,4-triazol-1-yl)-3-chlorphenyl]-[4-(5-methyloxazolo[4,5-b]pyridin-2-yl)piperazin-1-yl] methanon;
[4-(3-tert-Butyl-1,2,4-triazol-1-yl)-3-chlorphenyl]-[4-(5-chloroxazolo[4,5-b]pyridin-2-yl)piperazin-1-yl] methanon;
[4-(5-Chloroxazolo[4,5-b]pyridin-2-yl)piperazin-1-yl]-[4-[1-(2,2-dimethylpropyl)-1,2,4-triazol-3-yl]-3-fluorphenyl]methanon;
[4-[1-(2,2-Dimethylpropyl)-1,2,4-triazol-3-yl]-3-fluorphenyl]-[4-(5-methyloxazolo[4,5-b]pyridin-2-yl)piperazin-1-yl]methanon;
[4-(5-Chloroxazolo[4,5-b]pyridin-2-yl)-2-methylpiperazin-1-yl]-[4-[1-(2,2-dimethylpropyl)triazol-4-yl]phenyl]methanon;
[2-Chlor-6-[[1-(trifluormethyl)cyclopropyl]methoxy]-3-pyridy1]-[4-(5-methyloxazolo[4,5-b]pyridin-2-yl)piperazin-1-yl]methanon;
[4-(5-tert-Butyl-1,2,4-oxadiazol-3-yl)phenyl]-[3-(5-chloroxazolo[4,5-b]pyridin-2-yl)-3,8-diazabicyclo[3.2.1]octan-8-yl]methanon;
[4-(5-Chloroxazolo[4,5-b]pyridin-2-yl)piperazin-1-yl]-[3-fluor-4-[5-(2-fluor-2-methylpropyl)-1,3,4-oxadiazol-2-yl]phenyl]methanon;
[4-[2-(2,2-Dimethylpropyl)tetrazol-5-yl]-3,5-difluorphenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[2-(2,2-dimethylpropyl)tetrazol-5-yl]-3,5-difluorphenyl]methanon;
[4-[2-(2,2-Dimethylpropyl)tetrazol-5-yl]-3-methylphenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[2-(2,2-dimethylpropyl)tetrazol-5-yl]-3-methylphenyl]methanon;
[4-[2-(2,2-Dimethylpropyl)tetrazol-5-yl]-3-fluorphenyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[4-[2-(2,2-dimethylpropyl)tetrazol-5-yl]-3-fluorphenyl]methanon;
[4-(5-Chloroxazolo[4,5-b]pyridin-2-yl)piperazin-1-yl]-[6-[2-(2,2-dimethylpropyl)tetrazol-5-yl]-5-methoxy-3-pyridyl]methanon;
[6-[2-(2,2-Dimethylpropyl)tetrazol-5-yl]-5-methoxy-3-pyridyl]-[4-(5-methyloxazolo[4,5-b]pyridin-2-yl)piperazin-1-yl]methanon;
[6-[2-(2,2-Dimethylpropyl)tetrazol-5-yl]-5-methyl-3-pyridyl]-[4-(5-methyloxazolo[4,5-b]pyridin-2-yl)piperazin-1-yl]methanon;
[4-(5-Chloroxazolo[4,5-b]pyridin-2-yl)piperazin-1-yl]-[6-[2-(2,2-dimethylpropyl)tetrazol-5-yl]-5-methyl-3-pyridyl]methanon;
[6-[2-(2,2-Dimethylpropyl)tetrazol-5-yl]-5-fluor-3-pyridyl]-[4-(5-methyloxazolo[4,5-b]pyridin-2-yl)piperazin-1-yl]methanon; oder
[4-(5-Chloroxazolo[4,5-b]pyridin-2-yl)piperazin-1-yl]-[6-[2-(2,2-dimethylpropyl)tetrazol-5-yl]-5-fluor-3-pyridyl]methanon.

12. Verfahren zur Herstellung der Verbindungen der Formel (I) nach einem der Ansprüche 1 bis 10, umfassend:
das Reagieren einer Säure der Formel 1, wobei L¹, A und R^{A1} bis R^{A3} wie in einem der Ansprüche 1 bis 10 definiert sind, 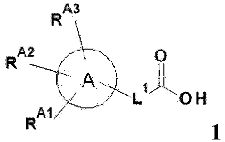
mit einem Amin der Formel 2, wobei R¹ bis R⁷ wie in einem der Ansprüche 1 bis 10 definiert sind, 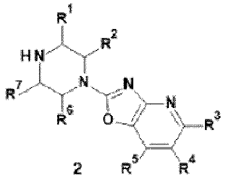
in der Gegenwart einer Base und eines Kopplungsreagens,
unter Bildung der Verbindung der Formel (I).

13. Verbindung der Formel (I) nach einem der Ansprüche 1 bis 11 zur Verwendung als therapeutisch wirksame Substanz.

14. Pharmazeutische Zusammensetzung, umfassend eine Verbindung der Formel (I) nach einem der Ansprüche 1 bis 11 und einen therapeutisch inerten Träger.

15. Verbindung der Formel (I) nach einem der Ansprüche 1 bis 11 zur Verwendung bei der Behandlung oder Prophylaxe von Neuroinflammation, neurodegenerativen Krankheiten, Schmerz, Krebs und/oder mentalen Störungen bei einem Säuger, insbesondere zur Verwendung bei der Behandlung oder Prophylaxe von multipler Sklerose, Alzheimer-Krankheit, Parkinson-Krankheit, amyotropher Lateralsklerose, traumatischer Hirnverletzung, Neurotoxizität, Schlaganfall, Epilepsie, Angst, Migräne, Depression, Leberzellenkarzinom, Kolonkarzinogenese, Eierstockkrebs, neuropathischem Schmerz, durch Chemotherapie induzierter Neuropathie, akutem Schmerz, chronischem Schmerz und/oder Spastizität in Verbindung mit Schmerz bei einem Säuger.

## Revendications

1. Composé de formule (I) 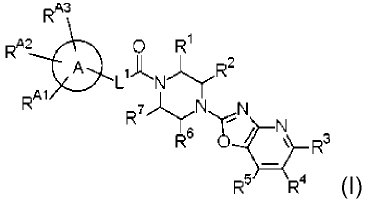 ou un sel pharmaceutiquement acceptable de celui-ci, dans lequel :
(i) R¹, R², R⁶ et R⁷ représentent chacun indépendamment un hydrogène, un alkyle en C₁-C₆ ou un hydroxyalkyle en C₁-C₆ ; ou
(ii) R¹ et R⁶ forment conjointement un pont de formule -(CH₂)ₘ-, dans lequel m représente 1, 2 ou 3 ; et R² et R⁷ représentent indépendamment un hydrogène, un alkyle en C₁-C₆ ou un hydroxyalkyle en C₁-C₆ ; ou
(iii) R¹ et R⁷ forment conjointement un pont de formule -(CH₂)ₘ-, dans lequel m représente 1, 2 ou 3 ; et R² et R⁶ représentent indépendamment un hydrogène, un alkyle en C₁-C₆ ou un hydroxyalkyle en C₁-C₆ ; ou
(iv) R² et R⁶ forment conjointement un pont de formule -(CH₂)ₘ-, dans lequel m représente 1, 2 ou 3 ; et R¹ et R⁷ représentent indépendamment un hydrogène, un alkyle en C₁-C₆ ou un hydroxyalkyle en C₁-C₆ ; ou
(v) R² et R⁷ forment conjointement un pont de formule -(CH₂)ₘ-, dans lequel m représente 1, 2 ou 3 ; et R¹ et R⁶ représentent indépendamment un hydrogène, un alkyle en C₁-C₆ ou un hydroxyalkyle en C₁-C₆ ;
R³, R⁴ et R⁵ représentent chacun indépendamment un hydrogène, un halogène, un cyano, un alkyle en C₁-C₆, un halogénoalkyle en C₁-C₆, un hydroxyalkyle en C₁-C₆, un cycloalkyle en C₃₋₁₂, un alcoxy en C₁-C₆ ou un halogénoalcoxy en C₁-C₆ ;
R^{A1} représente un hydrogène, un halogène, un oxo, un acétyle, un cyano, un cyanoalkyle en C₁-C₆, un hydroxy, un hydroxyalkyle en C₁-C₆, un alkyle en C₁-C₆, un alcanoyle en C₁-C₆, un alcényle en C₂-C₆, un alcynyle en C₂-C₆, un alcoxy en C₁-C₆, un alcoxy en C₁-C₆-alkyle en C₁-C₆, un halogénoalcoxy en C₁-C₆, un halogénoalkyle en C₁-C₆ ou un groupe 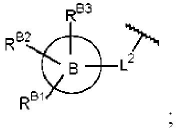
R^{A2} représente un hydrogène, un halogène, un oxo, un acétyle, un cyano, un cyanoalkyle en C₁-C₆, un hydroxy, un hydroxyalkyle en C₁-C₆, un alkyle en C₁-C₆, un alcanoyle en C₁-C₆, un alcényle en C₂-C₆, un alcynyle en C₂-C₆, un alcoxy en C₁-C₆, un alcoxy en C₁-C₆-alkyle en C₁-C₆, un halogénoalcoxy en C₁-C₆, un halogénoalkyle en C₁-C₆ ou un groupe 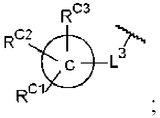
R^{A3} représente un hydrogène, un halogène, un oxo, un acétyle, un cyano, un cyanoalkyle en C₁-C₆, un hydroxy, un hydroxyalkyle en C₁-C₆, un alkyle en C₁-C₆, un alcanoyle en C₁-C₆, un alcényle en C₂-C₆, un alcynyle en C₂-C₆, un alcoxy en C₁-C₆, un alcoxy en C₁-C₆-alkyle en C₁-C₆, un halogénoalcoxy en C₁-C₆ ou un halogénoalkyle en C₁-C₆ ;
R^{B1} représente un hydrogène, un halogène, un oxo, un acétyle, un cyano, un cyanoalkyle en C₁-C₆, un hydroxy, un hydroxyalkyle en C₁-C₆, un alkyle en C₁-C₆, un alcanoyle en C₁-C₆, un alcényle en C₂-C₆, un alcynyle en C₂-C₆, un alcoxy en C₁-C₆, un alcoxy en C₁-C₆-alkyle en C₁-C₆, un halogénoalcoxy en C₁-C₆, un halogénoalkyle en C₁-C₆ ou un groupe 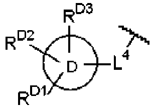
R^{B2}, R^{B3}, R^{C1}, R^{C2}, R^{C3}, R^{D1}, R^{D2} et R^{D3} représentent chacun indépendamment un hydrogène, un halogène, un oxo, un acétyle, un cyano, un cyanoalkyle en C₁-C₆, un hydroxy, un hydroxyalkyle en C₁-C₆, un alkyle en C₁-C₆, un alcanoyle en C₁-C₆, un alcényle en C₂-C₆, un alcynyle en C₂-C₆, un alcoxy en C₁-C₆, un alcoxy en C₁-C₆-alkyle en C₁-C₆, un halogénoalcoxy en C₁-C₆ ou un halogénoalkyle en C₁-C₆ ;
L¹ représente une liaison covalente, un alcynyle en C₂-C₆, un carbonyle, -C(O)NH-, -NHC(O)-, -CH₂O-, -OCH₂- ou SO₂ ;
L², L³ et L⁴ représentent chacun indépendamment une liaison covalente, un alcynyle en C₂-C₆, -CH₂-, -O-, un carbonyle, -C(O)NH-, -NHC(O)-, -CH₂O-, -OCH₂-ou SO₂ ; et
A, B, C et D représentent chacun indépendamment un aryle en C₆₋₁₄, un hétéroaryle en C₁₋₁₃, un cycloalkyle en C₃₋₁₂ ou un hétérocyclyle en C₂₋₉.

2. Composé de formule (I) selon la revendication 1, ou un sel pharmaceutiquement acceptable de celui-ci, dans lequel :
(i) R¹ et R² représentent indépendamment un hydrogène, un alkyle en C₁-C₆ ou un hydroxyalkyle en C₁-C₆ ; et R⁶ et R⁷ représentent un hydrogène ; ou
(ii) R¹ et R⁷ forment conjointement un pont de formule -(CH₂)₂-; et R² et R⁶ représentent tous deux un hydrogène ; ou
(iii) R² et R⁶ forment conjointement un pont de formule -(CH₂)₂-; et R¹ et R⁷ représentent tous deux un hydrogène.

3. Composé de formule (I) selon la revendication 1, ou un sel pharmaceutiquement acceptable de celui-ci, dans lequel :
R¹, R⁶ et R⁷ représentent tous un hydrogène ; et
R² représente un hydrogène ou un alkyle en C₁-C₆.

4. Composé de formule (I) selon la revendication 1, ou un sel pharmaceutiquement acceptable de celui-ci, dans lequel :
R¹, R⁶ et R⁷ représentent tous un hydrogène ; et
R² représente un hydrogène ou un méthyle.

5. Composé de formule (1) selon l'une quelconque des revendications 1 à 4, ou un sel pharmaceutiquement acceptable de celui-ci, dans lequel :
R³ représente un hydrogène, un halogène, un cyano, un alkyle en C₁-C₆, un halogénoalkyle en C₁-C₆, un cycloalkyle en C₃₋₁₂ ou un alcoxy en C₁-C₆ ;
R⁴ représente un hydrogène ; et
R⁵ représente un hydrogène ou un halogène.

6. Composé de formule (1) selon l'une quelconque des revendications 1 à 4, ou un sel pharmaceutiquement acceptable de celui-ci, dans lequel :
R³ représente un alkyle en C₁-C₆ ou un halogène ; et
R⁴ et R⁵ représentent tous deux un hydrogène.

7. Composé de formule (1) selon l'une quelconque des revendications 1 à 4, ou un sel pharmaceutiquement acceptable de celui-ci, dans lequel :
R³ représente un méthyle ou un chlore ; et
R⁴ et R⁵ représentent tous deux un hydrogène.

8. Composé de formule (1) selon l'une quelconque des revendications 1 à 7, ou un sel pharmaceutiquement acceptable de celui-ci, dans lequel :
L¹ représente une liaison covalente ou un alcynyle en C₂-C₆ ;
A représente un aryle en C₆₋₁₄, un hétéroaryle en C₁₋₁₃ ou un hétérocyclyle en C₂₋₉ ;
R^{A1} représente un hydrogène, un halogène, un alkyle en C₁-C₆, un alcényle en C₂-C₆, un alcynyle en C₂-C₆, un alcoxy en C₁-C₆, un halogénoalcoxy en C₁-C₆, un halogénoalkyle en C₁-C₆ ou un groupe 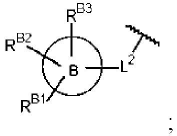
R^{A2} représente un hydrogène, un halogène, un cyano, un hydroxy, un alkyle en C₁-C₆, un alcoxy en C₁-C₆, un halogénoalcoxy en C₁-C₆, un halogénoalkyle en C₁-C₆ ou un groupe 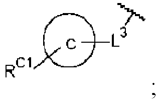
R^{A3} représente un hydrogène ou un halogène ;
L² représente une liaison covalente, -C(O)NH-, -NHC(O)-, -CH₂O-, -OCH₂- ou SO₂ ;
B représente un aryle en C₆₋₁₄, un hétéroaryle en C₁₋₁₃, un cycloalkyle en C₃₋₁₂ ou un hétérocyclyle en C₂₋₉ ;
R^{B1} représente un hydrogène, un halogène, un hydroxy, un acétyle, un cyano, un cyanoalkyle en C₁-C₆, un alkyle en C₁-C₆, un halogénoalkyle en C₁-C₆, un hydroxyalkyle en C₁-C₆, un alcényle en C₂-C₆, un alcoxy en C₁-C₆, un halogénoalcoxy en C₁-C₆, un alcoxy en C₁-C₆-alkyle en C₁-C₆, un alcanoyle en C₁-C₆, un oxo ou un groupe 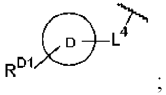
R^{B2} représente un hydrogène, un halogène, un oxo ou un alkyle en C₁-C₆ ;
R^{B3} représente un hydrogène ou un halogène ;
L³ représente une liaison covalente ou -CH₂O- ;
C représente un aryle en C₆₋₁₄, un hétéroaryle en C₁₋₁₃, un cycloalkyle en C₃₋₁₂ ou un hétérocyclyle en C₂₋₉ ;
R^{C1} représente un hydrogène, un halogène, un alkyle en C₁-C₆ ou un halogénoalkyle en C₁-C₆ ;
L⁴ représente une liaison covalente, -CH₂- ou -O- ;
D représente un aryle en C₆₋₁₄, un cycloalkyle en C₃₋₁₂ ou un hétérocyclyle en C₂₋₉ ; et
R^{D1} représente un alkyle en C₁-C₆, un halogénoalkyle en C₁-C₆ ou un halogène.

9. Composé de formule (1) selon l'une quelconque des revendications 1 à 8, ou un sel pharmaceutiquement acceptable de celui-ci, dans lequel :
L¹ représente une liaison covalente ;
A représente un aryle en C₆₋₁₄ ou un hétéroaryle en C₁₋₁₃ ;
R^{A1} représente un groupe 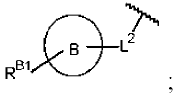
R^{A2} représente un hydrogène, un halogène ou un alkyle en C₁-C₆ ;
R^{A3} représente un hydrogène ou un halogène ;
L² représente une liaison covalente ou -CH₂O- ;
B représente un hétéroaryle en C₁₋₁₃ ou un cycloalkyle en C₃₋₁₂ ; et
R^{B1} représente un alkyle en C₁-C₆ ou un halogénoalkyle en C₁-C₆.

10. Composé de formule (1) selon l'une quelconque des revendications 1 à 9, ou un sel pharmaceutiquement acceptable de celui-ci, dans lequel :
L¹ représente une liaison covalente ;
A représente un phényle ou un pyridyle ;
R^{A1} représente un groupe 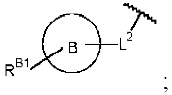
R^{A2} représente un hydrogène, un fluor, un chlore ou un méthyle ;
R^{A3} représente un hydrogène ou un fluor ;
L² représente une liaison covalente ou -CH₂O- ;
B représente un oxadiazolyle, un pyrazolyle, un triazolyle, un tétrazolyle ou un cyclopropyle ; et
R^{B1} représente un tert-butyle, un 2,2-diméthylpropyle, un trifluorométhyle ou un 2-fluoro-1,1-diméthyl-éthyle.

11. Composé de formule (I) selon la revendication 1, ou un sel pharmaceutiquement acceptable de celui-ci, dans lequel le composé de formule (I) est :
la [4-(5-méthyloxazolo[4,5-b]pyridin-2-yl)pipérazin-1-yl]-(2-phényl-1,3-benzoxazol-6-yl)méthanone ;
la (4-oxazolo[4,5-b]pyridin-2-ylpipérazin-1-yl)-(2-phényl-1,3-benzoxazol-6-yl)méthanone ;
la [4-(5-méthyloxazolo[4,5-b]pyridin-2-yl)pipérazin-1-yl]-(2-pyrrolidin-1-yl-1,3-benzoxazol-6-yl)méthanone ;
la [4-(5-tert-butyl-1,2,4-oxadiazol-3-yl)phényl]-[4-(5-méthyloxazolo[4,5-b]pyridin-2-yl)pipérazin-1-yl]méthanone ;
la [4-[5-(2,2-diméthylpropyl)-1,2,4-oxadiazol-3-yl]phényl]-[4-(5-méthyloxazolo[4,5-b]pyridin-2-yl)pipérazin-1-yl]méthanone ;
la [2-(2-azaspiro[3.4]octan-2-yl)-1,3-benzoxazol-6-yl]-[4-(5-méthyloxazolo[4,5-b]pyridin-2-yl)pipérazin-1-yl]méthanone ;
la [4-[5-(2-hydroxy-1,1-diméthyl-éthyl)-1,2,4-oxadiazol-3-yl]phényl]-[4-(5-méthyloxazolo[4,5-b]pyridin-2-yl)pipérazin-1-yl]méthanone ;
la [4-(5-tert-butyl-1,2,4-oxadiazol-3-yl)phényl]-[4-(7-chlorooxazolo[4,5-b]pyridin-2-yl)pipérazin-1-yl]méthanone ;
la [4-(7-chlorooxazolo[4,5-b]pyridin-2-yl)pipérazin-1-yl]-[4-[5-(2,2-diméthylpropyl)-1,2,4-oxadiazol-3-yl]phényl]méthanone ;
la [4-(5-chlorooxazolo[4,5-b]pyridin-2-yl)pipérazin-1-yl]-[4-[5-(2,2-diméthylpropyl)-1,2,4-oxadiazol-3-yl]phényl]méthanone ;
la [4-[5-(2-fluoro-1,1-diméthyl-éthyl)-1,2,4-oxadiazol-3-yl]phényl]-[4-(5-méthyloxazolo[4,5-b]pyridin-2-yl)pipérazin-1-yl]méthanone ;
la [4-(5-tert-butyl-1,2,4-oxadiazol-3-yl)phényl]-[4-(5-chlorooxazolo[4,5-b]pyridin-2-yl)pipérazin-1-yl]méthanone ;
la [4-[5-(2,2-diméthylpropyl)-1,2,4-oxadiazol-3-yl]phényl]-(4-oxazolo[4,5-b]pyridin-2-ylpipérazin-1-yl)méthanone ;
la [4-(5-tert-butyl-1,2,4-oxadiazol-3-yl)phényl]-(4-oxazolo[4,5-b]pyridin-2-yl)pipérazin-1-yl)méthanone ;
le 2-[4-[4-[5-(2,2-diméthylpropyl)-1,2,4-oxadiazol-3-yl]benzoyl]pipérazin-1-yl]oxazolo[4,5-b]pyridine-5-carbonitrile ;
la [4-[5-(2,2-diméthylpropyl)-1,2,4-oxadiazol-3-yl]phényl]-[4-[5-(trifluorométhyl)oxazolo[4,5-b]pyridin-2-yl]pipérazin-1-yl]méthanone ;
la [2-(1-éthylpropyl)-1,3-benzoxazol-6-yl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-(2,2-diméthylpropyl)-1,3,4-oxadiazol-2-yl]phényl]méthanone ;
la (2-cyclohexyl-1,3-benzoxazol-6-yl)-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[2-(3,3,3-trifluoropropyl)-1,3-benzoxazol-6-yl]méthanone ;
la [4-(5-chlorooxazolo[4,5-b]pyridin-2-yl)pipérazin-1-yl]-[4-[1-(2,2-diméthylpropyl)pyrazol-4-yl]phényl]méthanone ;
la [3-chloro-5-[4-(hydroxyméthyl)phényl]phényl]-[4-(5-méthyloxazolo[4,5-b]pyridin-2-yl)pipérazin-1-yl]méthanone ;
la [3-chloro-5-(1,1-dioxo-1,4-thiazinan-4-yl)phényl]-[4-(5-méthyloxazolo[4,5-b]pyridin-2-yl)pipérazin-1-yl]méthanone ;
la 1-[4-[3-chloro-5-[4-(5-méthyloxazolo[4,5-b]pyridin-2-yl)pipérazine-1-carbonyl]phényl]pipérazin-1-yl]éthanone ;
la [3-chloro-5-(4,4-difluoro-1-pipéridyl)phényl]-[4-(5-méthyloxazolo[4,5-b]pyridin-2-yl)pipérazin-1-yl]méthanone ;
la [3-chloro-5-[4-(hydroxyméthyl)-1-pipéridyl]phényl]-[4-(5-méthyloxazolo[4,5-b]pyridin-2-yl)pipérazin-1-yl]méthanone ;
la [3-chloro-5-(6-hydroxy-2-azaspiro[3.3]heptan-2-yl)phényl]-[4-(5-méthyloxazolo[4,5-b]pyridin-2-yl)pipérazin-1-yl]méthanone ;
la [2-(3-chlorophényl)sulfonyl-2,6-diazaspiro[3.3]heptan-6-yl]-[4-(5-méthyloxazolo[4,5-b]pyridin-2-yl)pipérazin-1-yl]méthanone ;
la [3-[4-(hydroxyméthyl)phényl]-5-(3,3,3-trifluoropropoxy)phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-(2-fluoro-1,1-diméthyl-éthyl)-1,2,4-oxadiazol-3-yl]phényl]méthanone ;
la [2-[(E)-but-1-ényl]-1,3-benzoxazol-6-yl]-[4-(5-méthyloxazolo[4,5-b]pyridin-2-yl)pipérazin-1-yl]méthanone ;
la (2-cyclopropyl-1,3-benzoxazol-6-yl)-[4-(5-méthyloxazolo[4,5-b]pyridin-2-yl)pipérazin-1-yl]méthanone ;
la 1-[3-[4-[4-(5-chlorooxazolo[4,5-b]pyridin-2-yl)pipérazine-1-carbonyl]phényl]-1,2,4-oxadiazol-5-yl]propan-2-one ;
la [4-(5-chlorooxazolo[4,5-b]pyridin-2-yl)pipérazin-1-yl]-[4-[5-(2,2-difluoropropyl)-1,2,4-oxadiazol-3-yl]phényl]méthanone ;
la [4-(5-chlorooxazolo[4,5-b]pyridin-2-yl)pipérazin-1-yl]-[4-[5-(1-fluoro-1-méthyl-éthyl)-1,2,4-oxadiazol-3-yl]phényl]méthanone ;
la [4-(5-chlorooxazolo[4,5-b]pyridin-2-yl)pipérazin-1-yl]-[4-[3-(2,2-diméthylpropyl)-1,2,4-oxadiazol-5-yl]phényl]méthanone ;
la [3-[4-(hydroxyméthyl)phényl]-4-[[1-(trifluorométhyl)cyclopropyl]méthoxy]phényl]-[4-(5-méthyloxazolo[4,5-b]pyridin-2-yl)pipérazin-1-yl]méthanone ;
la [3-[4-(hydroxyméthyl)phényl]-4-(3,3,3-trifluoropropoxy)phényl]-[4-(5-méthyloxazolo[4,5-b]pyridin-2-yl)pipérazin-1-yl]méthanone ;
la [3-[4-(hydroxyméthyl)phényl]-5-[[1-(trifluorométhyl)cyclopropyl]méthoxy]phényl]-[4-(5-méthyloxazolo[4,5-b]pyridin-2-yl)pipérazin-1-yl]méthanone ;
la (2-butyl-1,3-benzoxazol-6-yl)-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-(1-fluorocyclopropyl)-1,2,4-oxadiazol-3-yl]phényl]méthanone ;
la [4-(5-chlorooxazolo[4,5-b]pyridin-2-yl)pipérazin-1-yl]-[4-[1-(2,2-diméthylpropyl)-1,2,4-triazol-3-yl]phényl]méthanone ;
la [4-[1-(2,2-diméthylpropyl)-1,2,4-triazol-3-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)imidazol-4-yl]phényl]méthanone ;
la [4-(5-tert-butyl-1,3,4-oxadiazol-2-yl)phényl]-[4-(5-chlorooxazolo[4,5-b]pyridin-2-yl)pipérazin-1-yl]méthanone ;
la [4-(5-oxa-2-azaspiro[3.4]octan-2-yl)phényl]-(4-oxazolo[4,5-b]pyridin-2-ylpipérazin-1-yl)méthanone ;
la (4-oxazolo[4,5-b]pyridin-2-ylpipérazin-1-yl)-[4-[3-(2,2,2-trifluoro-1-méthyl-éthoxy)azétidin-1-yl]phényl]méthanone ;
la [4-(5-tert-butyl-1,3,4-oxadiazol-2-yl)phényl]-[4-(5-méthyloxazolo[4,5-b]pyridin-2-yl)pipérazin-1-yl]méthanone ;
la [4-(5-méthyloxazolo [4,5 -b]pyridin-2-yl)pipérazin-1-yl] -[4-(5 -oxa-2-azaspiro[3.4]octan-2-yl)phényl]méthanone ;
la [4-(5-méthyloxazolo[4,5-b]pyridin-2-yl)pipérazin-1-yl]-[4-[3-(2,2,2-trifluoro-1-méthyl-éthoxy)azétidin-1-yl]phényl]méthanone ;
la [4-(5-chlorooxazolo[4,5-b]pyridin-2-yl)pipérazin-1-yl]-[4-[5-(2-fluoro-2-méthyl-propyl)-1,2,4-oxadiazol-3-yl]phényl]méthanone ;
la [4-(5-chlorooxazolo[4,5-b]pyridin-2-yl)pipérazin-1-yl]-[4-[5-(2-méthylallyl)-1,2,4-oxadiazol-3-yl]phényl]méthanone ;
la [4-(5-chlorooxazolo[4,5-b]pyridin-2-yl)pipérazin-1-yl]-[4-[5-(2-méthylprop-1-ényl)-1,2,4-oxadiazol-3-yl]phényl]méthanone ;
la [4-(5-chlorooxazolo[4,5-b]pyridin-2-yl)pipérazin-1-yl]-[4-[5-(2-hydroxy-2-méthyl-propyl)-1,2,4-oxadiazol-3-yl]phényl]méthanone ;
la (4-oxazolo[4,5-b]pyridin-2-ylpipérazin-1-yl)-[4-(3-phénoxyazétidin-1-yl)phényl]méthanone ;
la [4-[5-(2-fluoro-1,1-diméthyl-éthyl)-1,3,4-oxadiazol-2-yl]phényl]-[4-(5-méthyloxazolo[4,5-b]pyridin-2-yl)pipérazin-1-yl]méthanone ;
la [4-(5-tert-butyl-1,2,4-oxadiazol-3-yl)phényl]-[4-(5-méthoxyoxazolo[4,5-b]pyridin-2-yl)pipérazin-1-yl]méthanone ;
la [4-(5-chlorooxazolo[4,5-b]pyridin-2-yl)pipérazin-1-yl]-[4-[5-(2-fluoro-1,1-diméthyl-éthyl)-1,3,4-oxadiazol-2-yl]phényl]méthanone ;
la [4-[5-(1-fluorocyclopropyl)-1,2,4-oxadiazol-3-yl]phényl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[4-(3-phénoxyazétidin-1-yl)phényl]méthanone ;
la [4-[5-(2,2-diméthylpropyl)-1,2,4-oxadiazol-3-yl]-3-[4-(hydroxyméthyl)phényl]phényl]-[4-(5-méthyloxazolo[4,5-b]pyridin-2-yl)pipérazin-1-yl]méthanone ;
la [3-[4-(hydroxyméthyl)phényl]-4-[(1-méthylcyclopropyl)méthoxy]phényl]-[4-(5-méthyloxazolo[4,5-b]pyridin-2-yl)pipérazin-1-yl]méthanone ;
la [3-[4-(hydroxyméthyl)phényl]-5-[(1-méthylcyclopropyl)méthoxy]phényl]-[4-(5-méthyloxazolo[4,5-b]pyridin-2-yl)pipérazin-1-yl]méthanone ;
la [4-[(3-chlorophényl)méthoxy]-3-(1H-imidazol-2-yl)phényl]-[4-(5-méthyloxazolo[4,5-b]pyridin-2-yl)pipérazin-1-yl]méthanone ;
la [4-(2-azaspiro[3.4]octan-2-yl)-3-(1H-imidazol-2-yl)phényl]-[4-(5-méthyloxazolo[4,5-b]pyridin-2-yl)pipérazin-1-yl]méthanone ;
la [4-[5-[1-(fluorométhyl)cyclopropyl]-1,2,4-oxadiazol-3-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-[1-(fluorométhyl)cyclopropyl]-1,2,4-oxadiazol-3-yl]phényl]méthanone ;
la [4-(3-tert-butyl-1,2,4-oxadiazol-5-yl)phényl]-[4-(5-méthyloxazolo[4,5-b]pyridin-2-yl)pipérazin-1-yl]méthanone ;
la [4-(3-tert-butyl-1,2,4-oxadiazol-5-yl)phényl]-[4-(5-chlorooxazolo[4,5-b]pyridin-2-yl)pipérazin-1-yl]méthanone ;
la [4-(5-tert-butyl-1,2,4-oxadiazol-3-yl)phényl]-[4-(5-cyclopropyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-[(1-méthylcyclopropyl)méthyl]-1,2,4-oxadiazol-3-yl]phényl]méthanone ;
la [4-(5-méthyloxazolo[4,5-b]pyridin-2-yl)pipérazin-1-yl]-[4-[3-[(1S)-2,2,2-trifluoro-1-méthyl-éthoxy]azétidin-1-yl]phényl]méthanone ;
la [4-(5-méthyloxazolo[4,5-b]pyridin-2-yl)pipérazin-1-yl]-[4-[3-[(1R)-2,2,2-trifluoro-1-méthyl-éthoxy]azétidin-1-yl]phényl]méthanone ;
la [4-(5-chlorooxazolo[4,5-b]pyridin-2-yl)pipérazin-1-yl]-[4-(2,2-difluoro-5-azaspiro[2.4]heptan-5-yl)phényl]méthanone ;
la [4-(5-chlorooxazolo[4,5-b]pyridin-2-yl)pipérazin-1-yl]-[4-(7,7-difluoro-2-azaspiro[3.3]heptan-2-yl)phényl]méthanone ;
la [4-(3-tert-butoxyazétidin-1-yl)phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[3-(1,1-diméthylpropoxy)azétidin-1-yl]phényl]méthanone ;
la [4-(1-tert-butyl-1,2,4-triazol-3-yl)phényl]-[4-(5-chlorooxazolo[4,5-b]pyridin-2-yl)pipérazin-1-yl]méthanone ;
la [4-(1-tert-butyl-1,2,4-triazol-3-yl)phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)triazol-4-yl]phényl]méthanone ;
la [4-(5-chlorooxazolo[4,5-b]pyridin-2-yl)pipérazin-1-yl]-[4-[2-(2,2-diméthylpropyl)triazol-4-yl]phényl]méthanone ;
la [4-(5-tert-butyl-1,2,4-oxadiazol-3-yl)phényl]-[4-(5-chlorooxazolo[4,5-b]pyridin-2-yl)-2-méthyl-pipérazin-1-yl]méthanone ;
la [4-(5-tert-butyl-1,2,4-oxadiazol-3-yl)phényl]-[4-(5-chlorooxazolo[4,5-b]pyridin-2-yl)-3-méthyl-pipérazin-1-yl]méthanone ;
la [4-(1-tert-butylpyrazol-4-yl)phényl]-[4-(5-méthyloxazolo[4,5-b]pyridin-2-yl)pipérazin-1-yl]méthanone ;
la [4-(1-tert-butylpyrazol-4-yl)phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[1-[(1-méthylcyclopropyl)méthyl]-1,2,4-triazol-3-yl]phényl]méthanone ;
la [4-(5-chlorooxazolo[4,5-b]pyridin-2-yl)pipérazin-1-yl]-[4-[2-[(1-méthylcyclopropyl)méthyl]-1,2,4-triazol-3-yl]phényl]méthanone ;
la [4-[5-(1-méthylcyclopropyl)-1,2,4-oxadiazol-3-yl]phényl]-[4-(5-méthyloxazolo[4,5-b]pyridin-2-yl)pipérazin-1-yl]méthanone ;
la [4-(4-tert-butylpyrazol-1-yl)phényl]-[4-(5-chlorooxazolo[4,5-b]pyridin-2-yl)pipérazin-1-yl]méthanone ;
la [4-(4-tert-butylpyrazol-1-yl)phényl]-[4-(5-méthyloxazolo[4,5-b]pyridin-2-yl)pipérazin-1-yl]méthanone ;
la [4-[1-(2,2-diméthylpropyl)pyrazol-4-yl]phényl]-[4-(5-méthyloxazolo[4,5-b]pyridin-2-yl)pipérazin-1-yl]méthanone ;
la [4-(7,7-difluoro-2-azaspiro[3.3]heptan-2-yl)phényl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[6-[3-(2,2,2-trifluoro-1-méthyl-éthoxy)azétidin-1-yl]-3-pyridyl]méthanone ;
la [4-(4-tert-butylimidazol-1-yl)phényl]-[4-(5-méthyloxazolo[4,5-b]pyridin-2-yl)pipérazin-1-yl]méthanone ;
la [4-(5-tert-butyl-1,2,4-oxadiazol-3-yl)-3-méthyl-phényl]-[4-(5-méthyloxazolo[4,5-b]pyridin-2-yl)pipérazin-1-yl]méthanone ;
la [4-(5-tert-butyl-1,2,4-oxadiazol-3-yl)-3-méthyl-phényl]-[4-(5-chlorooxazolo[4,5-b] pyridin-2-yl)pipérazin-1 -yl] méthanone ;
la [4-(5-tert-butyl-1,2,4-oxadiazol-3-yl)-3-fluoro-phényl]-[4-(5-méthyloxazolo[4,5-b] pyridin-2-yl)pipérazin-1 -yl] méthanone ;
la [4-(5-tert-butyl-1,2,4-oxadiazol-3-yl)-3-fluoro-phényl]-[4-(5-chlorooxazolo[4,5-b] pyridin-2-yl)pipérazin-1 -yl] méthanone ;
la [3-chloro-4-[(1-méthylcyclopropyl)méthoxy]phényl]-[4-(5-chlorooxazolo[4,5-b] pyridin-2-yl)pipérazin-1 -yl] méthanone ;
la [6-[3-(1,1-diméthylpropoxy)azétidin-1-yl]-3-pyridyl]-[4-(5-méthyloxazolo[4,5-b] pyridin-2-yl)pipérazin-1 -yl] méthanone ;
la [4-(5-méthyloxazolo[4,5-b]pyridin-2-yl)pipérazin-1-yl]-[5-méthyl-6-[3-(2,2,2-trifluoro-1-méthyl-éthoxy)azétidin-1-yl]-3-pyridyl]méthanone ;
la [6-(5-tert-butyl-1,2,4-oxadiazol-3-yl)-3-pyridyl]-[4-(5-chlorooxazolo[4,5-b]pyridin-2-yl)pipérazin-1-yl]méthanone ;
la [6-(5-tert-butyl-1,2,4-oxadiazol-3-yl)-3-pyridyl]-[4-(5-méthyloxazolo[4,5-b]pyridin-2-yl)pipérazin-1-yl]méthanone ;
la [4-(4-tert-butylimidazol-1-yl)phényl]-[4-(5-chlorooxazolo[4,5-b]pyridin-2-yl)pipérazin-1-yl]méthanone ;
la [2-fluoro-4-[3-(2,2,2-trifluoro-1-méthyl-éthoxy)azétidin-1-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-(2,2-diméthylpropoxy)-3-méthyl-phényl]méthanone ;
la [4-(2,2-diméthylpropoxy)-3-méthyl-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[3-(2,2,2-trifluoro-1-méthyl-éthoxy)azétidin-1-yl]phényl]méthanone ;
la [4-(5-chlorooxazolo[4,5-b]pyridin-2-yl)pipérazin-l-yl]-[4-(3-éthoxy-3-méthyl-azétidin-1-yl)phényl]méthanone ;
la [3-chloro-4-[(1-méthylcyclopropyl)méthoxy]phényl]-[4-(5-méthyloxazolo[4,5-b]pyridin-2-yl)pipérazin-1-yl]méthanone ;
la [3-fluoro-4-[3-(2,2,2-trifluoro-1-méthyl-éthoxy)azétidin-1-yl]phényl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[4-(3-méthyloxétan-3-yl)phényl]méthanone ;
la [4-(1-fluorocyclopropyl)phényl]-[4-(5-méthyloxazolo[4,5-b]pyridin-2-yl)pipérazin-1-yl]méthanone ;
la [4-chloro-5-fluoro-6-[3-(2,2,2-trifluoro-1-méthyl-éthoxy)azétidin-l-yl]-3-pyridyl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[6-[3-(2,2,2-trifluoro-1-méthyl-éthoxy)azétidin-1-yl]pyridazin-3-yl]méthanone ;
la [5-méthyl-6-[(1-méthylcyclopropyl)méthoxy]-3-pyridyl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[2-[3-(2,2,2-trifluoro-1-méthyl-éthoxy)azétidin-1-yl]pyrimidin-5-yl]méthanone ;
la [4-(5-méthyloxazolo[4,5-b]pyridin-2-yl)pipérazin-1-yl]-[4-méthyl-6-[3-(2,2,2-trifluoro-1-méthyl-éthoxy)azétidin-1-yl]-3-pyridyl]méthanone ;
la [6-(7,7-difluoro-2-azaspiro[3.3]heptan-2-yl)-4-méthyl-3-pyridyl]-[4-(5-méthyloxazolo[4,5-b]pyridin-2-yl)pipérazin-1-yl]méthanone ;
la [2-(2,2-difluoro-5-azaspiro[2.4]heptan-5-yl)pyrimidin-5-yl]-[4-(5-méthyloxazolo[4,5-b]pyridin-2-yl)pipérazin-1-yl]méthanone ;
la [6-(2,2-difluoro-5-azaspiro[2.4]heptan-5-yl)-4-méthyl-3-pyridyl]-[4-(5-méthyloxazolo[4,5-b]pyridin-2-yl)pipérazin-1-yl]méthanone ;
la [5-fluoro-6-[3-(2,2,2-trifluoro-l-méthyl-éthoxy)azétidin-1-yl]-3-pyridyl]-[4-(5-méthyloxazolo[4,5-b]pyridin-2-yl)pipérazin-1-yl]méthanone ;
la [6-(2,2-difluoro-5-azaspiro[2.4]heptan-5-yl)-5-méthyl-3-pyridyl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[2-méthyl-4-[3-(2,2,2-trifluoro-1-méthyl-éthoxy)azétidin-1-yl]phényl]méthanone ;
la [2,6-difluoro-4-[3-(2,2,2-trifluoro-1-méthyl-éthoxy)azétidin-1-yl]phényl]-[4-(5-méthyloxazolo[4,5-b]pyridin-2-yl)pipérazin-1-yl]méthanone ;
la [2-chloro-4-[3-(2,2,2-trifluoro-1-méthyl-éthoxy)azétidin-1-yl]phényl]-[4-(5-méthyloxazolo[4,5-b]pyridin-2-yl)pipérazin-1-yl]méthanone ;
la [4-(5-méthyloxazolo[4,5-b]pyridin-2-yl)pipérazin-1-yl]-[5-méthyl-6-[[1-(trifluorométhyl)cyclopropyl]méthoxy]-3-pyridyl]méthanone ;
la [4-(5-chlorooxazolo[4,5-b]pyridin-2-yl)pipérazin-1-yl]-[3-fluoro-4-[(1-méthylcyclopropyl)méthoxy]phényl]méthanone ;
la [4-(3-tert-butyl-1,2,4-triazol-1-yl)phényl]-[4-(5-méthyloxazolo[4,5-b]pyridin-2-yl)pipérazin-1-yl]méthanone ;
la [3-fluoro-4-[(1-méthylcyclopropyl)méthoxy]phényl]-[4-(5-méthyloxazolo[4,5-b]pyridin-2-yl)pipérazin-1-yl]méthanone ;
la [4-(3-tert-butyl-1,2,4-triazol-1-yl)phényl]-[4-(5-chlorooxazolo[4,5-b]pyridin-2-yl)pipérazin-1-yl]méthanone ;
la [5-chloro-6-[3-(2,2,2-trifluoro-1-méthyl-éthoxy)azétidin-1-yl]-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[5-(2,2-diméthylpropyl)-4-méthyl-1,2,4-triazol-3-yl]phényl]méthanone ;
la [5-chloro-6-(2,2-difluoro-5-azaspiro[2.4]heptan-5-yl)-3-pyridyl]-[4-(5-méthyloxazolo[4,5-b]pyridin-2-yl)pipérazin-1-yl]méthanone ;
la [4-[5-(2,2-diméthylpropyl)-4-méthyl-1,2,4-triazol-3-yl]phényl]-[4-(5-méthyloxazolo[4,5-b]pyridin-2-yl)pipérazin-1-yl]méthanone ;
la [6-(2,2-difluoro-5-azaspiro[2.4]heptan-5-yl)-5-fluoro-3-pyridyl]-[4-(5-méthyloxazolo[4,5-b]pyridin-2-yl)pipérazin-1-yl]méthanone ;
la [4-[1-(2,2-diméthylpropyl)triazol-4-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[5-fluoro-6-[3-(2,2,2-trifluoro-1 -méthyl-éthoxy)azétidin-1 -yl] -3-pyridyl] méthanone ;
la [4-(5-chlorooxazolo[4,5-b]pyridin-2-yl)pipérazin-1-yl]-[6-(2,2-difluoro-5-azaspiro[2.4]heptan-5-yl)-5-fluoro-3-pyridyl]méthanone ;
la [4-(5 -chlorooxazolo [4,5-b]pyridin-2-yl)pipérazin-1-yl]-[5-fluoro-6-[[1-(trifluorométhyl)cyclopropyl]méthoxy]-3-pyridyl]méthanone ;
la [4-(5 -chlorooxazolo [4,5-b]pyridin-2-yl)pipérazin-1-yl]-[5-fluoro-6-[(1-méthylcyclopropyl)méthoxy]-3-pyridyl]méthanone ;
la [4-(5-tert-butyl-4-méthyl-1,2,4-triazol-3-yl)phényl]-[4-(5-chlorooxazolo[4,5-b]pyridin-2-yl)pipérazin-1-yl]méthanone ;
la [4-(5-tert-butyl-4-méthyl-1,2,4-triazol-3-yl)phényl]-[4-(5-méthyloxazolo[4,5-b]pyridin-2-yl)pipérazin-1-yl]méthanone ;
la [5-chloro-6-[(1-méthylcyclopropyl)méthoxy]-3-pyridyl]-[4-(5-méthyloxazolo[4,5-b]pyridin-2-yl)pipérazin-1-yl]méthanone ;
la [5-chloro-6-[[1-(trifluorométhyl)cyclopropyl]méthoxy]-3-pyridyl]-[4-(5-méthyloxazolo[4,5-b]pyridin-2-yl)pipérazin-1-yl]méthanone ;
la [4-(5-tert-butyl-1,2,4-oxadiazol-3-yl)-3-chloro-phényl]-[4-(5-chlorooxazolo[4,5-b]pyridin-2-yl)pipérazin-1-yl]méthanone ;
la [4-(5-tert-butyl-1,2,4-oxadiazol-3-yl)-3-chloro-phényl]-[4-(5-méthyloxazolo[4,5-b]pyridin-2-yl)pipérazin-1-yl]méthanone ;
la [4-(5-tert-butyl-1,2,4-oxadiazol-3-yl)-3,5-difluoro-phényl]-[4-(5-chlorooxazolo[4,5-b]pyridin-2-yl)pipérazin-1-yl]méthanone ;
la [4-(5-tert-butyl-1,2,4-oxadiazol-3-yl)-3-(trifluorométhyl)phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)triazol-4-yl]-3-méthyl-phényl]méthanone ;
la [4-(5-chlorooxazolo[4,5-b]pyridin-2-yl)pipérazin-1-yl]-[4-[2-(2,2-diméthylpropyl)triazol-4-yl]-3-méthyl-phényl]méthanone ;
la [5-fluoro-6-[(1-méthylcyclopropyl)méthoxy]-3-pyridyl]-[4-(5-méthyloxazolo[4,5-b]pyridin-2-yl)pipérazin-1-yl]méthanone ;
la [5-chloro-6-[(1-méthylcyclopropyl)méthoxy]-3-pyridyl]-[4-(5-chlorooxazolo[4,5-b]pyridin-2-yl)pipérazin-1-yl]méthanone ;
la [5-fluoro-6-[[1-(trifluorométhyl)cyclopropyl]méthoxy]-3-pyridyl]-[4-(5-méthyloxazolo[4,5-b]pyridin-2-yl)pipérazin-1-yl]méthanone ;
la [5,6-bis[[1-(trifluorométhyl)cyclopropyl]méthoxy]-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[5-chloro-6-[[1-(trifluorométhyl)cyclopropyl]méthoxy]-3-pyridyl]méthanone ;
la [4-(5-chlorooxazolo[4,5-b]pyridin-2-yl)pipérazin-1-yl]-[5-méthyl-6-[[1-(trifluorométhyl)cyclopropyl]méthoxy]-3-pyridyl]méthanone ;
la [4-(5-chlorooxazolo[4,5-b]pyridin-2-yl)pipérazin-1-yl]-[5-(trifluorométhyl)-6-[[1-(trifluorométhyl)cyclopropyl]méthoxy]-3-pyridyl]méthanone ;
la [4-(5-tert-butyl-1,3,4-oxadiazol-2-yl)-3-méthyl-phényl]-[4-(5-méthyloxazolo[4,5-b]pyridin-2-yl)pipérazin-1-yl]méthanone ;
la [4-(5-tert-butyl-1,3,4-oxadiazol-2-yl)-3-méthyl-phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)triazol-4-yl]-3-fluoro-phényl]méthanone ;
la [4-[2-(2,2-diméthylpropyl)triazol-4-yl]-3-fluoro-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)triazol-4-yl]-3-fluoro-phényl]méthanone ;
la [4-(1-tert-butyltriazol-4-yl)phényl] -[4-(5-méthyloxazolo[4,5-b]pyridin-2-yl)pipérazin-1-yl]méthanone ;
la [4-(1-tert-butyltriazol-4-yl)phényl]-[4-(5-chlorooxazolo[4,5-b]pyridin-2-yl)pipérazin-1-yl]méthanone ;
la [4-(1-tert-butyl-1,2,4-triazol-3-yl)-3-chloro-phényl]-[4-(5-méthyloxazolo[4,5-b]pyridin-2-yl)pipérazin-1-yl]méthanone ;
la [4-(1-tert-butyl-1,2,4-triazol-3-yl)-3-chloro-phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)triazol-4-yl]-3,5-difluoro-phényl]méthanone ;
la [4-[2-(2,2-diméthylpropyl)triazol-4-yl]-3,5-difluoro-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)triazol-4-yl]-3,5-difluoro-phényl]méthanone ;
la [4-(5-chlorooxazolo[4,5-b]pyridin-2-yl)pipérazin-1-yl]-[6-(cyclopropylméthoxy)-5-méthyl-3-pyridyl]méthanone ;
la [4-(5-chlorooxazolo[4,5-b]pyridin-2-yl)pipérazin-1-yl]-[5-méthyl-6-[(1-méthylcyclopropyl)méthoxy]-3-pyridyl]méthanone ;
la [4-(5-chlorooxazolo[4,5-b]pyridin-2-yl)pipérazin-1-yl]-[5-méthyl-6-[(3-méthyloxétan-3-yl)méthoxy]-3-pyridyl]méthanone ;
la [4-(5-chlorooxazolo[4,5-b]pyridin-2-yl)pipérazin-1-yl]-[6-[(2,2-difluoro-1-méthyl-cyclopropyl)méthoxy]-5-méthyl-3-pyridyl]méthanone ;
la [4-(5-chlorooxazolo[4,5-b]pyridin-2-yl)pipérazin-1-yl]-[6-[(3-éthyloxétan-3-yl)méthoxy]-5-méthyl-3-pyridyl]méthanone ;
la [4-(5-chlorooxazolo[4,5-b]pyridin-2-yl)pipérazin-1-yl]-[6-[[1-(méthoxyméthyl)cyclopropyl]méthoxy]-5-méthyl-3-pyridyl]méthanone ;
la [4-(5-chlorooxazolo[4,5-b]pyridin-2-yl)pipérazin-1-yl]-[6-[(3-fluorooxétan-3-yl)méthoxy]-5-méthyl-3-pyridyl]méthanone ;
le 2-[1-[[5-[4-(5-chlorooxazolo[4,5-b]pyridin-2-yl)pipérazine-1-carbonyl]-3-méthyl-2-pyridyl]oxyméthyl]cyclopropyl]acétonitrile ;
le 1-[[5-[4-(5-chlorooxazolo[4,5-b]pyridin-2-yl)pipérazine-1-carbonyl]-3-méthyl-2-pyridyl]oxyméthyl]cyclopropanecarbonitrile ;
le 3-[[5-[4-(5-chlorooxazolo[4,5-b]pyridin-2-yl)pipérazine-1-carbonyl]-3-méthyl-2-pyridyl] oxyméthyl] oxétane-3 -carbonitrile ;
la [4-[1-(1-bicyclo[1.1.1]pentanyl)triazol-4-yl]phényl]-[4-(5-méthyloxazolo[4,5-b]pyridin-2-yl)pipérazin-1-yl]méthanone ;
la [4-[1-(1-bicyclo[1.1.1]pentanyl)triazol-4-yl]phényl]-[4-(5-chlorooxazolo[4,5-b]pyridin-2-yl)pipérazin-1-yl]méthanone ;
la [4-(1-tert-butyl-1,2,4-triazol-3-yl)-3-méthyl-phényl]-[4-(5-méthyloxazolo[4,5-b]pyridin-2-yl)pipérazin-1-yl]méthanone ;
la [4-(1-tert-butyl-1,2,4-triazol-3-yl)-3-méthyl-phényl]-[4-(5-chlorooxazolo[4,5-b]pyridin-2-yl)pipérazin-1-yl]méthanone ;
le 3-[[5-[4-(5-chlorooxazolo[4,5-b]pyridin-2-yl)pipérazine-1-carbonyl]-3-méthyl-2-pyridyl]oxyméthyl]-5-fluoro-benzonitrile ;
la [4-(5 -chlorooxazolo [4,5-b]pyridin-2-yl)pipérazin-1-yl]-[4-[1-(2,2,2-trifluoroéthyl)triazol-4-yl]phényl]méthanone ;
la [4-(5-méthyloxazolo[4,5-b]pyridin-2-yl)pipérazin-1-yl]-[4-[1-(2,2,2-trifluoroéthyl)triazol-4-yl]phényl]méthanone ;
la [4-(5-méthyloxazolo[4,5-b]pyridin-2-yl)pipérazin-1-yl]-[4-[1-[(3-méthyloxétan-3-yl)méthyl]triazol-4-yl]phényl]méthanone ;
la [4-(5-chlorooxazolo[4,5-b]pyridin-2-yl)pipérazin-1-yl]-[4-[1-[(3-méthyloxétan-3-yl)méthyl]triazol-4-yl]phényl]méthanone ;
la 1-[4-(5-chlorooxazolo[4,5-b]pyridin-2-yl)pipérazin-1-yl]-3-phényl-prop-2-yn-1-one ;
la 1-[4-(5-chlorooxazolo[4,5-b]pyridin-2-yl)pipérazin-1-yl]-3-(4-chlorophényl)prop-2-yn-1-one ;
la [4-(5-chlorooxazolo[4,5-b]pyridin-2-yl)pipérazin-1-yl]-[3-[2-(2,6-dichlorophényl)éthynyl]azétidin-1-yl]méthanone ;
la [3-[2-(2-chloro-4-fluoro-phényl)éthynyl]azétidin-1-yl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-(4-éthynyl-3-méthoxy-phényl)méthanone ;
la [4-[5-(1-bicyclo[1.1.1]pentanyl)-1,2,4-oxadiazol-3-yl]phényl]-[4-(5-chlorooxazolo[4,5-b]pyridin-2-yl)pipérazin-1 -yl]méthanone ;
la [4-(1-tert-butyl-1,2,4-triazol-3-yl)-3-fluoro-phényl]-[4-(5-méthyloxazolo[4,5-b]pyridin-2-yl)pipérazin-1-yl]méthanone ;
la [4-(1-tert-butyl-1,2,4-triazol-3-yl)-3-fluoro-phényl]-[4-(5-chlorooxazolo[4,5-b]pyridin-2-yl)pipérazin-1-yl]méthanone ;
la [4-(5-méthyloxazolo[4,5-b]pyridin-2-yl)pipérazin-1-yl]-[4-[1-[1-(trifluorométhyl)cyclopropyl]triazol-4-yl]phényl]méthanone ;
la [4-(5-méthyloxazolo[4,5-b]pyridin-2-yl)pipérazin-1-yl]-[4-[1-(3-méthyloxétan-3-yl)triazol-4-yl]phényl]méthanone ;
la [4-(5-chlorooxazolo[4,5-b]pyridin-2-yl)pipérazin-1-yl]-[4-[1-(3-méthyloxétan-3-yl)triazol-4-yl]phényl]méthanone ;
la [4-(1-tert-butyl-1,2,4-triazol-3-yl)-3-(trifluorométhyl)phényl]-[4-(5-méthyloxazolo[4,5-b]pyridin-2-yl)pipérazin-1-yl]méthanone ;
la [4-(1-tert-butyl-1,2,4-triazol-3-yl)-3-(trifluorométhyl)phényl]-[4-(5-chlorooxazolo[4,5-b]pyridin-2-yl)pipérazin-1-yl]méthanone ;
la [4-(5-méthyloxazolo[4,5-b]pyridin-2-yl)pipérazin-1-yl]-[4-[1-(oxétan-3-ylméthyl)triazol-4-yl]phényl]méthanone ;
la [4-(5-chlorooxazolo[4,5-b]pyridin-2-yl)pipérazin-1-yl]-[4-[1-(oxétan-3-ylméthyl)triazol-4-yl]phényl]méthanone ;
la [4-(5-tert-butyl-1,3,4-oxadiazol-2-yl)-3 -chloro-phényl] -[4-(5-chlorooxazolo[4,5-b]pyridin-2-yl)pipérazin-1-yl]méthanone ;
la [4-(5-tert-butyl-1,3,4-oxadiazol-2-yl)-3-fluoro-5-hydroxy-phényl]-[4-(5-chlorooxazolo[4,5-b]pyridin-2-yl)pipérazin-1-yl]méthanone ;
la [4-(5-tert-butyl-1,3,4-oxadiazol-2-yl)-3-fluoro-5-hydroxy-phényl]-[4-(5-méthyloxazolo[4,5-b]pyridin-2-yl)pipérazin-1-yl]méthanone ;
la [4-(5-tert-butyl-2-méthyl-1,2,4-triazol-3-yl)phényl]-[4-(5-chlorooxazolo[4,5-b]pyridin-2-yl)pipérazin-1-yl]méthanone ;
le 5-[4-(5-chlorooxazolo[4,5-b]pyridin-2-yl)pipérazine-1-carbonyl]-2-[1-(2,2-diméthylpropyl)triazol-4-yl]benzonitrile ;
le 5-[4-(5-chlorooxazolo[4,5-b]pyridin-2-yl)pipérazine-1-carbonyl]-2-[2-(2,2-diméthylpropyl)triazol-4-yl]benzonitrile ;
la [6-(1-tert-butylpyrazol-4-yl)-5-méthyl-3-pyridyl]-[4-(5-chlorooxazolo[4,5-b]pyridin-2-yl)pipérazin-1-yl]méthanone ;
la [6-(4-tert-butylpyrazol-1-yl)-5-méthyl-3-pyridyl]-[4-(5-chlorooxazolo[4,5-b]pyridin-2-yl)pipérazin-1-yl]méthanone ;
la [6-(1-tert-butylpyrazol-4-yl)-5-méthyl-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[5-méthyl-6-(tétrahydrofuran-2-ylméthoxy)-3-pyridyl]méthanone ;
la [4-(5-tert-butyl-1,3,4-oxadiazol-2-yl)-3-(trifluorométhyl)phényl]-[4-(5-méthyloxazolo[4,5-b]pyridin-2-yl)pipérazin-1-yl]méthanone ;
la [4-(5-tert-butyl-1,3,4-oxadiazol-2-yl)-3-(trifluorométhyl)phényl]-[4-(5-chlorooxazolo[4,5-b]pyridin-2-yl)pipérazin-1-yl]méthanone ;
la [4-(5-méthyloxazolo[4,5-b]pyridin-2-yl)pipérazin-1-yl]-[6-méthyl-5-[[1-(trifluorométhyl)cyclopropyl]méthoxy]pyrazin-2-yl]méthanone ;
la [4-(5-chlorooxazolo[4,5-b]pyridin-2-yl)pipérazin-1-yl]-[5-méthyl-6-(oxétan-2-ylméthoxy)-3-pyridyl]méthanone ;
le 2-[1-(2,2-diméthylpropyl)triazol-4-yl]-5-[4-(5-méthyloxazolo[4,5-b]pyridin-2-yl)pipérazine-1-carbonyl]benzonitrile ;
le 2-[2-(2,2-diméthylpropyl)triazol-4-yl]-5-[4-(5-méthyloxazolo[4,5-b]pyridin-2-yl)pipérazine-1-carbonyl]benzonitrile ;
la [4-(5-chlorooxazolo[4,5-b]pyridin-2-yl)pipérazin-1-yl]-[4-[1-(2,2-diméthylpropyl)triazol-4-yl] -3 -méthoxy-phényl] méthanone ;
la [4-(5-chlorooxazolo[4,5-b]pyridin-2-yl)pipérazin-1-yl]-[4-[2-(2,2-diméthylpropyl)triazol-4-yl] -3 -méthoxy-phényl] méthanone ;
la [4-[1-(2,2-diméthylpropyl)triazol-4-yl]-3-méthoxy-phényl]-[4-(5-méthyloxazolo[4,5-b]pyridin-2-yl)pipérazin-1-yl]méthanone ;
la [4-[2-(2,2-diméthylpropyl)triazol-4-yl]-3-méthoxy-phényl]-[4-(5-méthyloxazolo[4,5-b]pyridin-2-yl)pipérazin-1-yl]méthanone ;
la [4-(1-tert-butyl-1,2,4-triazol-3-yl)-3-méthoxy-phényl]-[4-(5-méthyloxazolo[4,5-b]pyridin-2-yl)pipérazin-1-yl]méthanone ;
la [4-(1-tert-butyl-1,2,4-triazol-3-yl)-3-méthoxy-phényl]-[4-(5-chlorooxazolo[4,5-b]pyridin-2-yl)pipérazin-1-yl]méthanone ;
la [5-méthoxy-6-[[1-(trifluorométhyl)cyclopropyl]méthoxy]-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-[(1-fluorocyclopropyl)méthyl]triazol-4-yl]phényl]méthanone ;
la [4-(5-chlorooxazolo[4,5-b]pyridin-2-yl)pipérazin-1-yl]-[5-méthyl-6-(tétrahydropyran-2-ylméthoxy)-3-pyridyl]méthanone ;
la [4-(5-chlorooxazolo[4,5-b]pyridin-2-yl)pipérazin-1-yl]-[6-[(1-méthoxycyclopropyl)méthoxy]-5-méthyl-3-pyridyl]méthanone ;
la [4-[1-[(1-fluorocyclopropyl)méthyl]triazol-4-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[6-[1-(2,2-diméthylpropyl)triazol-4-yl]-5-fluoro-3-pyridyl] méthanone ;
la [4-(5-chlorooxazolo[4,5-b]pyridin-2-yl)pipérazin-1-yl]-[6-[3-(2,2-diméthylpropyl)triazol-4-yl]-5-fluoro-3-pyridyl] méthanone ;
la [4-(5-chlorooxazolo[4,5-b]pyridin-2-yl)pipérazin-1-yl]-[6-[2-(2,2-diméthylpropyl)triazol-4-yl]-5-fluoro-3-pyridyl]méthanone ;
la [3-[(2-chloro-4-fluoro-phénoxy)méthyl]azétidin-1-yl]-[4-(5-chlorooxazolo[4,5-b]pyridin-2-yl)pipérazin-1-yl]méthanone ;
la [3-[(2-chloro-4-fluoro-phénoxy)méthyl]azétidin-1-yl]-[4-(5-méthyloxazolo[4,5-b]pyridin-2-yl)pipérazin-1-yl]méthanone ;
le 2-chloro-N-[1-[4-(5-chlorooxazolo[4,5-b]pyridin-2-yl)pipérazine-1-carbonyl]azétidin-3-yl]-4-fluoro-benzamide ;
le 2-chloro-4-fluoro-N-[1-[4-(5-méthyloxazolo[4,5-b]pyridin-2-yl)pipérazine-1-carbonyl]azétidin-3-yl]benzamide ;
la [4-(5-chlorooxazolo[4,5-b]pyridin-2-yl)pipérazin-1-yl]-[6-[(2S)-2,3-difluoropropoxy]-5-méthyl-3-pyridyl]méthanone ;
le N-(2-chloro-4-fluoro-phényl)-1-[4-(5-chlorooxazolo[4,5-b]pyridin-2-yl)pipérazine-1-carbonyl]azétidine-3-carboxamide ;
le N-(2-chloro-4-fluoro-phényl)-1-[4-(5-méthyloxazolo[4,5-b]pyridin-2-yl)pipérazine-1-carbonyl]azétidine-3-carboxamide ;
la [4-(5-chlorooxazolo[4,5-b]pyridin-2-yl)pipérazin-1-yl]-[5-méthyl-6-(3,3,3-trifluoropropoxy)-3-pyridyl]méthanone ;
la [4-(1-tert-butyltriazol-4-yl)-3-méthyl-phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[6-[(2R)-2,3-difluoropropoxy]-5-méthyl-3-pyridyl]méthanone ;
la [4-(5-tert-butyl-2-méthyl-1,2,4-triazol-3-yl)phényl]-[4-(5-méthyloxazolo[4,5-b]pyridin-2-yl)pipérazin-1-yl]méthanone ;
la [6-[1-(2,2-diméthylpropyl)triazol-4-yl]-5-fluoro-3-pyridyl]-[4-(5-méthyloxazolo[4,5-b]pyridin-2-yl)pipérazin-1-yl]méthanone ;
la [6-[2-(2,2-diméthylpropyl)triazol-4-yl]-5-fluoro-3-pyridyl]-[4-(5-méthyloxazolo[4,5-b]pyridin-2-yl)pipérazin-1-yl]méthanone ;
la [6-[3-(2,2-diméthylpropyl)triazol-4-yl]-5-fluoro-3-pyridyl]-[4-(5-méthyloxazolo[4,5-b]pyridin-2-yl)pipérazin-1-yl]méthanone ;
la [4-(5-tert-butyl-1,3,4-oxadiazol-2-yl)phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-(1H-triazol-5-yl)phényl]méthanone ;
la [4-(5-tert-butyl-1,3,4-oxadiazol-2-yl)-3-chloro-phényl]-[4-(5-méthyloxazolo[4,5-b]pyridin-2-yl)pipérazin-1-yl]méthanone ;
la [6-(3-tert-butyl-1,2,4-triazol-1-yl)-5-méthyl-3-pyridyl]-[4-(5-méthyloxazolo[4,5-b]pyridin-2-yl)pipérazin-1-yl]méthanone ;
la [6-(3-tert-butyl-1,2,4-triazol-1-yl)-5-méthyl-3-pyridyl]-[4-(5-chlorooxazolo[4,5-b]pyridin-2-yl)pipérazin-1-yl]méthanone ;
la [6-(4-tert-butylpyrazol-1-yl)-5-méthyl-3-pyridyl]-[4-(5-méthyloxazolo[4,5-b]pyridin-2-yl)pipérazin-1-yl]méthanone ;
le 3-[4-[4-[4-(5-chlorooxazolo[4,5-b]pyridin-2-yl)pipérazine-1-carbonyl]phényl]triazol-1 -yl] -2,2-diméthyl-propanenitrile ;
la [4-(5-chlorooxazolo[4,5-b]pyridin-2-yl)pipérazin-1-yl]-[4-[1-(2-fluoro-2-méthyl-propyl)triazol-4-yl]phényl]méthanone ;
la [4-[1-(2-fluoro-2-méthyl-propyl)triazol-4-yl]phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2-fluoro-1,1-diméthyl-éthyl)triazol-4-yl]phényl]méthanone ;
la [4-[1-(2-fluoro-1,1-diméthyl-éthyl)triazol-4-yl]phényl]-[4-(5-méthyloxazolo[4,5-b]pyridin-2-yl)pipérazin-1-yl]méthanone ;
le 3-[4-[4-[4-(5-chlorooxazolo[4,5-b]pyridin-2-yl)pipérazine-1-carbonyl]phényl]triazol-2-yl] -2,2-diméthyl-propanenitrile ;
la [4-(5-chlorooxazolo[4,5-b]pyridin-2-yl)pipérazin-1-yl]-[6-méthyl-5-[[1-(trifluorométhyl)cyclopropyl]méthoxy]pyrazin-2-yl]méthanone ;
la [4-(5-chlorooxazolo[4,5-b]pyridin-2-yl)pipérazin-1-yl]-[6-[1-(2,2-diméthylpropyl)pyrazol-4-yl]-5-méthyl-3-pyridyl]méthanone ;
la [6-[1-(2,2-diméthylpropyl)pyrazol-4-yl]-5-méthyl-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[5-méthoxy-6-[[1-(trifluorométhyl)cyclopropyl]méthoxy]-3-pyridyl]méthanone ;
la [4-(5-chlorooxazolo[4,5-b]pyridin-2-yl)pipérazin-1-yl]-[4-[1-(2-méthoxy-2-méthyl-propyl)triazol-4-yl]phényl]méthanone ;
la [4-(5-chlorooxazolo[4,5-b]pyridin-2-yl)pipérazin-1-yl]-[4-(5-isobutyl-1,3,4-oxadiazol-2-yl)phényl]méthanone ;
la [4-(5-méthyloxazolo[4,5-b]pyridin-2-yl)pipérazin-1-yl]-[6-méthyl-5-[[1-(trifluorométhyl)cyclopropyl]méthoxy]-2-pyridyl]méthanone ;
la [4-(5-chlorooxazolo[4,5-b]pyridin-2-yl)pipérazin-1-yl]-[6-méthyl-5-[[1-(trifluorométhyl)cyclopropyl]méthoxy]-2-pyridyl]méthanone ;
la [4-(5-chlorooxazolo[4,5-b]pyridin-2-yl)pipérazin-1-yl]-[4-[1-(2,2-diméthylpropyl)triazol-4-yl] -3 -(trifluorométhyl)phényl] méthanone ;
la [4-(5-chlorooxazolo[4,5-b]pyridin-2-yl)pipérazin-1-yl]-[4-[2-(2,2-diméthylpropyl)triazol-4-yl] -3 -(trifluorométhyl)phényl] méthanone ;
la [4-(5-chlorooxazolo[4,5-b]pyridin-2-yl)pipérazin-1-yl]-[4-[3-(2,2-diméthylpropyl)triazol-4-yl] -3 -(trifluorométhyl)phényl] méthanone ;
la [4-(5-chlorooxazolo[4,5-b]pyridin-2-yl)pipérazin-1-yl]-[6-[1-(2,2-diméthylpropyl)triazol-4-yl]-5-méthyl-3-pyridyl]méthanone ;
la [4-(5-chlorooxazolo[4,5-b]pyridin-2-yl)pipérazin-1-yl]-[6-[2-(2,2-diméthylpropyl)triazol-4-yl]-5-méthyl-3-pyridyl]méthanone ;
la [(3R)-4-(5-chlorooxazolo[4,5-b]pyridin-2-yl)-3-méthyl-pipérazin-1-yl]-[4-[1-(2,2-diméthylpropyl)triazol-4-yl]phényl]méthanone ;
la [(3S)-4-(5-chlorooxazolo[4,5-b]pyridin-2-yl)-3-méthyl-pipérazin-1-yl]-[4-[1-(2,2-diméthylpropyl)triazol-4-yl]phényl]méthanone ;
la [4-(5-chlorooxazolo[4,5-b]pyridin-2-yl)pipérazin-1-yl]-[4-(5-isobutyl-1,3,4-oxadiazol-2-yl)-3-méthyl-phényl]méthanone ;
la [4-(5-chlorooxazolo[4,5-b]pyridin-2-yl)pipérazin-1-yl]-[2-chloro-6-[[1-(trifluorométhyl)cyclopropyl]méthoxy]-3-pyridyl]méthanone ;
la [4-[2-(2,2-diméthylpropyl)tétrazol-5-yl]phényl]-[4-(5-méthyloxazolo[4,5-b]pyridin-2-yl)pipérazin-1-yl]méthanone ;
la [6-(5-tert-butyl-1,3,4-oxadiazol-2-yl)-5-fluoro-3-pyridyl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[6-[1-(2,2-diméthylpropyl)pyrazol-4-yl]-5-(trifluorométhyl)-3-pyridyl]méthanone ;
la [4-(5-chlorooxazolo[4,5-b]pyridin-2-yl)pipérazin-1-yl]-[3-fluoro-4-[5-(2-fluoro-1,1-diméthyl-éthyl)-1,3,4-oxadiazol-2-yl]phényl]méthanone ;
la [4-(5-tert-butyl-1,3,4-oxadiazol-2-yl)-3-fluoro-phényl]-[4-(5-chlorooxazolo[4,5-b]pyridin-2-yl)pipérazin-1-yl]méthanone ;
la [6-[1-(2,2-diméthylpropyl)pyrazol-4-yl]-5-(trifluorométhyl)-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)tétrazol-5-yl]phényl]méthanone ;
la [4-(5-tert-butyl-1,2,4-oxadiazol-3-yl)phényl]-[8-(5-chlorooxazolo[4,5-b]pyridin-2-yl)-3,8-diazabicyclo[3.2.1]octan-3-yl]méthanone ;
la [4-(5-chlorooxazolo[4,5-b]pyridin-2-yl)-3-(hydroxyméthyl)pipérazin-1-yl]-[4-[1-(2,2-diméthylpropyl)triazol-4-yl]phényl]méthanone ;
la [4-[1-(2,2-diméthylpropyl)triazol-4-yl]phényl]-[4-(5-fluorooxazolo[4,5-b]pyridin-2-yl)pipérazin-1-yl]méthanone ;
la [4-(3-tert-butyl-1,2,4-triazol-1-yl)-3-méthyl-phényl]-[4-(5-chlorooxazolo[4,5-b]pyridin-2-yl)pipérazin-1-yl]méthanone ;
la [4-(3-tert-butyl-1,2,4-triazol-1-yl)-3-méthyl-phényl]-[4-(5-méthyloxazolo[4,5-b]pyridin-2-yl)pipérazin-1-yl]méthanone ;
la [(2S)-4-(5-chlorooxazolo[4,5-b]pyridin-2-yl)-2-(hydroxyméthyl)pipérazin-1-yl]-[4-[1-(2,2-diméthylpropyl)triazol-4-yl]phényl]méthanone ;
la [(2R)-4-(5-chlorooxazolo[4,5-b]pyridin-2-yl)-2-(hydroxyméthyl)pipérazin-1-yl]-[4-[1-(2,2-diméthylpropyl)triazol-4-yl]phényl]méthanone ;
la [4-(3-tert-butyl-1,2,4-triazol-1-yl)-3-chloro-phényl]-[4-(5-méthyloxazolo[4,5-b]pyridin-2-yl)pipérazin-1-yl]méthanone ;
la [4-(3-tert-butyl-1,2,4-triazol-1-yl)-3-chloro-phényl]-[4-(5-chlorooxazolo[4,5-b]pyridin-2-yl)pipérazin-1-yl]méthanone ;
la [4-(5-chlorooxazolo[4,5-b]pyridin-2-yl)pipérazin-1-yl]-[4-[1-(2,2-diméthylpropyl)-1,2,4-triazol-3-yl]-3-fluoro-phényl]méthanone ;
la [4-[1-(2,2-diméthylpropyl)-1,2,4-triazol-3-yl]-3-fluoro-phényl]-[4-(5-méthyloxazolo[4,5-b]pyridin-2-yl)pipérazin-1-yl]méthanone ;
la [4-(5-chlorooxazolo[4,5-b]pyridin-2-yl)-2-méthyl-pipérazin-1-yl]-[4-[1-(2,2-diméthylpropyl)triazol-4-yl]phényl]méthanone ;
la [2-chloro-6-[[1-(trifluorométhyl)cyclopropyl]méthoxy]-3-pyridyl]-[4-(5-méthyloxazolo[4,5-b]pyridin-2-yl)pipérazin-1-yl]méthanone ;
la [4-(5-tert-butyl-1,2,4-oxadiazol-3-yl)phényl]-[3-(5-chlorooxazolo[4,5-b]pyridin-2-yl)-3,8-diazabicyclo[3.2.1]octan-8-yl]méthanone ;
la [4-(5-chlorooxazolo[4,5-b]pyridin-2-yl)pipérazin-1-yl]-[3-fluoro-4-[5-(2-fluoro-2-méthyl-propyl)-1,3,4-oxadiazol-2-yl]phényl]méthanone ;
la [4-[2-(2,2-diméthylpropyl)tétrazol-5-yl]-3,5-difluoro-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)tétrazol-5-yl]-3,5-difluoro-phényl]méthanone ;
la [4-[2-(2,2-diméthylpropyl)tétrazol-5-yl]-3-méthyl-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)tétrazol-5-yl]-3-méthyl-phényl]méthanone ;
la [4-[2-(2,2-diméthylpropyl)tétrazol-5-yl]-3-fluoro-phényl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[4-[2-(2,2-diméthylpropyl)tétrazol-5-yl]-3-fluoro-phényl]méthanone ;
la [4-(5-chlorooxazolo[4,5-b]pyridin-2-yl)pipérazin-1-yl]-[6-[2-(2,2-diméthylpropyl)tétrazol-5-yl]-5-méthoxy-3-pyridyl]méthanone ;
la [6-[2-(2,2-diméthylpropyl)tétrazol-5-yl]-5-méthoxy-3-pyridyl]-[4-(5-méthyloxazolo[4,5-b]pyridin-2-yl)pipérazin-1-yl]méthanone ;
la [6-[2-(2,2-diméthylpropyl)tétrazol-5-yl]-5-méthyl-3-pyridyl]-[4-(5-méthyloxazolo[4,5-b]pyridin-2-yl)pipérazin-1-yl]méthanone ;
la [4-(5-chlorooxazolo[4,5-b]pyridin-2-yl)pipérazin-1-yl]-[6-[2-(2,2-diméthylpropyl)tétrazol-5-yl]-5-méthyl-3-pyridyl]méthanone ;
la [6-[2-(2,2-diméthylpropyl)tétrazol-5-yl]-5-fluoro-3-pyridyl]-[4-(5-méthyloxazolo[4,5-b]pyridin-2-yl)pipérazin-1-yl]méthanone ; ou
la [4-(5-chlorooxazolo[4,5-b]pyridin-2-yl)pipérazin-1-yl]-[6-[2-(2,2-diméthylpropyl)tétrazol-5-yl]-5-fluoro-3-pyridyl]méthanone.

12. Procédé de fabrication des composés de formule (1) selon l'une quelconque des revendications 1 à 10, comprenant :
la réaction d'un acide de formule 1, dans lequel L¹, A et R^{A1} à R^{A3} sont tels que définis dans l'une quelconque des revendications 1 à 10, 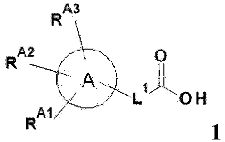
avec une amine de formule 2, dans laquelle R¹ à R⁷ sont tels que définis dans l'une quelconque des revendications 1 à 10 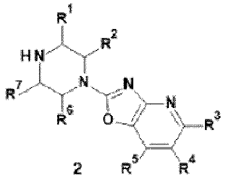
en présence d'une base et d'un réactif de couplage,
pour former ledit composé de formule (I).

13. Composé de formule (1) selon l'une quelconque des revendications 1 à 11 pour une utilisation comme substance thérapeutiquement active.

14. Composition pharmaceutique comprenant un composé de formule (1) selon l'une quelconque des revendications 1 à 11 et un véhicule thérapeutiquement inerte.

15. Composé de formule (1) selon l'une quelconque des revendications 1 à 11 pour une utilisation dans le traitement ou la prophylaxie d'une neuro-inflammation, de maladies neurodégénératives, d'une douleur, d'un cancer et/ou de troubles mentaux chez un mammifère, en particulier pour une utilisation dans le traitement ou la prophylaxie de la sclérose en plaques, de la maladie d'Alzheimer, de la maladie de Parkinson, de la sclérose latérale amyotrophique, de la lésion cérébrale traumatique, de la neurotoxicité, de l'accident vasculaire cérébral, de l'épilepsie, de l'anxiété, de la migraine, de la dépression, du carcinome hépatocellulaire, de la carcinogenèse du côlon, du cancer de l'ovaire, de la douleur neuropathique, de la neuropathie induite par la chimiothérapie, de la douleur aiguë, de la douleur chronique et/ou de la spasticité associée à la douleur chez un mammifère.
